# EUROPEAN PATENT APPLICATION

(11) **EP 2 889 291 A1**
(43) Date of publication of application: **01.07.2015**
(21) Application number: 13830309.4
(22) Date of filing: 23.08.2013
(51) Int. Cl.: C07D 239/80, A61K 31/517, A61K 31/519, A61K 31/5377, A61P 9/10, A61P 43/00, C07D 401/04, C07D 401/06, C07D 401/12, C07D 401/14, C07D 403/12, C07D 405/14, C07D 409/12, C07D 413/04, C07D 417/12, C07D 471/04, C07D 487/04, C07D 513/04, C07D 519/00

(54) **HETEROCYCLIC COMPOUND**

(30) Priority: 24.08.2012 JP 2012185725
(71) Applicant: Takeda Pharmaceutical Company Limited, Osaka-shi, Osaka 541-0045 (JP)
(72) Inventor: OGINO, Masaki, Fujisawa-shi Kanagawa 251-0012 (JP); IKEDA, Zenichi, Fujisawa-shi Kanagawa 251-0012 (JP); FUJIMOTO, Jun, Fujisawa-shi Kanagawa 251-0012 (JP); OHBA, Yusuke, Fujisawa-shi Kanagawa 251-0012 (JP); ISHII, Naoki, Fujisawa-shi Kanagawa 251-0012 (JP); FUJIMOTO, Takuya, Fujisawa-shi Kanagawa 251-0012 (JP); ODA, Tsuneo, Fujisawa-shi Kanagawa 251-0012 (JP); TAYA, Naohiro, Fujisawa-shi Kanagawa 251-0012 (JP); YAMASHITA, Toshiro, Fujisawa-shi Kanagawa 251-0012 (JP); MATSUNAGA, Nobuyuki, Fujisawa-shi Kanagawa 251-0012 (JP)
(74) Representative: König, Gregor Sebastian
(86) International application number: PCT/JP2013/072562
(87) International publication number: WO 2014/030743

(57) **Abstract**

Provided is a compound having a superior FLAP inhibitory action and useful as a prophylactic or therapeutic agent for arteriosclerosis and the like, and a salt thereof. The present invention relates to a compound represented by the formula 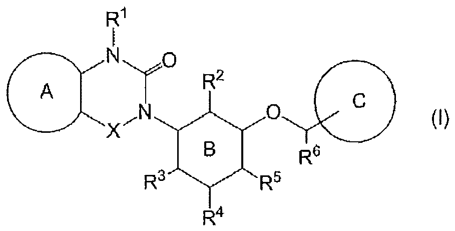 wherein each symbol is as defined in the present DESCRIPTION, or a salt thereof.

## Description

### Technical Field

The present invention relates to a heterocyclic compound having a 5-lipoxygenase activating protein (sometimes to be abbreviated as "FLAP" in the present specification) inhibitory action, and useful for the treatment of arteriosclerosis and the like, a pharmaceutical composition containing same and the like.

### (Background of the Invention)

Leukotriene biosynthesized from arachidonic acid is one kind of lipid mediator that mediates various actions such as neutrophil chemotaxis, vasopermeability promotion, vasoconstriction action, bronchoconstriction action and the like. As leukotriene biosynthesis inhibitors, two kinds are mainly known. One is a 5-lipoxygenase (5-LO) inhibitor, and the other is 5-lipoxygenase activating protein (FLAP) inhibitor. These inhibitors have been studied with the focus on the treatment of asthma. As one example, zileuton, which is a 5-LO inhibitor, has already been used for application to asthma treatments, and AM-803, which is a FLAP inhibitor, is under clinical trial with asthma patients. On the other hand, as for the treatment or prophylaxis of arteriosclerosis, phase 2 clinical trials of DG-031, which is a FLAP inhibitor, and VIA-2291, which is a 5-LO inhibitor, were conducted from the thought that suppression of leukotriene biosynthesis is useful based on the report of Helgadottir et al. (Nature Genetics. 2004: vol36; 233-239) and the like. However, progress was not made in the study thereafter for the reasons of low effect, hepatotoxicity and the like. There exists no pharmaceutical product among 5-LO inhibitors and FLAP inhibitors which is superior in the safety and effectiveness for circulatory diseases such as arteriosclerosis and the like, respiratory diseases such as asthma and the like, and allergic diseases such as topic dermatitis and the like. Therefore, the study of a FLAP inhibitor having a novel structure leads to the creation of a more superior pharmaceutical product for circulatory diseases such as peripheral artery disease (PAD) arteriosclerosis including myocardial infarction and the like, respiratory diseases such as asthma and the like, and allergic diseases such as atopic dermatitis and the like.

Examples of the compound having a structure similar to that of the compound described in the present specification include the following.
(1) A compound represented by the following formula: 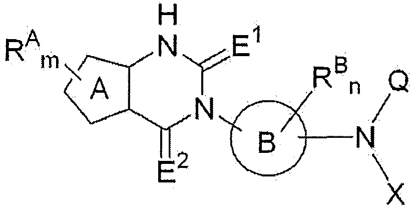 wherein
   ring A is 5-membered unsaturated hydrocarbon or 5-membered heteroaryl;
   R^{A} is halogen, cyano, nitro, optionally substituted lower alkyl and the like;
   ring B is aryl or heteroaryl;
   R^{B}: halogen, OW⁴ (W⁴ is H, optionally substituted lower alkyl and the like) and the like;
   E¹ is O, S or N-CN;
   E²: O or NH;
   Q: H, optionally substituted lower alkyl and the like;
   X is -L-Z, -CO-Y (L is optionally substituted lower alkylene, and Y is Z and the like) and the like;
   Z is optionally substituted and optionally fused aryl, optionally substituted and optionally fused heteroaryl and the like, which is a nonpeptidic GnRH antagonist and useful for the treatment of sex hormone-dependent diseases (prostatomegaly, uterine myoma etc.) and the like (patent document 1).
(2) A compound represented by the following formula: 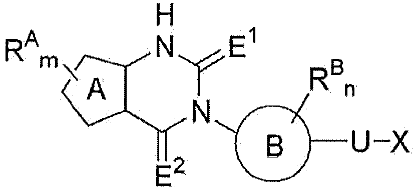 wherein
   ring A is 5-membered unsaturated hydrocarbon or 5-membered heteroaryl;
   R^{A} is halogen, cyano, nitro, optionally substituted lower alkyl and the like;
   ring B is aryl or heteroaryl;
   R^{B} is halogen, OW⁴, -COW⁴ (W⁴ is optionally substituted lower alkyl and the like) and the like;
   E¹ is O, S or N-CN;
   E² is O or NH;
   U is a single bond or optionally substituted lower alkylene;
   X is Y, -O-L-Y (L is optionally substituted lower alkylene, and
   Y is Z and the like) and the like;
   Z is optionally substituted and optionally fused aryl, optionally substituted and optionally fused heteroaryl and the like, which is a nonpeptidic GnRH antagonist and useful for the treatment of sex hormone-dependent diseases (prostatomegaly, uterine myoma etc.) and the like (patent document 2).
(3) A compound represented by the following formula: 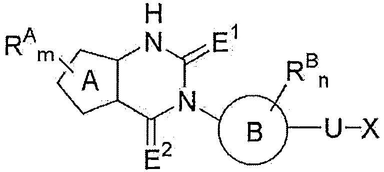 wherein
   ring A is 5-membered unsaturated hydrocarbon or 5-membered heteroaryl;
   R^{A} is halogen, cyano, nitro, or optionally substituted lower alkyl;
   ring B is aryl or heteroaryl;
   R^{B} is halogen, OW⁴, or -COW⁴ (W⁴ is optionally substituted lower alkyl and the like);
   E¹ is O, S or N-CN;
   E² is O or NH;
   U is a single bond or optionally substituted lower alkylene;
   X is Y, -O-L-Y (L is optionally substituted lower alkylene, and
   Y is Z and the like) and the like;
   Z is optionally substituted and optionally fused aryl, optionally substituted and optionally fused heteroaryl and the like, which is a nonpeptidic GnRH antagonist and useful for the treatment of sex hormone-dependent diseases (prostatomegaly, uterine myoma etc.) and the like (patent document 3).
(4) A compound represented by the following formula: 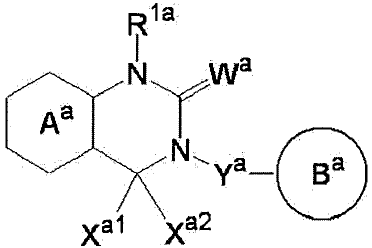 wherein
   R^{1a} is an optionally substituted hydrocarbon group;
   ring A^{a} is an optionally substituted aromatic 6-membered ring;
   ring B^{a} is an optionally substituted homocyclic ring or optionally substituted heterocycle;
   W^{a} is O or S;
   X^{a1} and X^{a2} are each independently H and the like, or X^{a1} and X^{a2} in combination show O and the like;
   Y^{a} is a bond or optionally substituted C₁₋₆ alkylene, which is a GnRH antagonist and useful for the treatment of sex hormone-dependent diseases (prostatomegaly, uterine myoma etc.) and the like (patent document 4).
(5) A compound represented by the following formula: 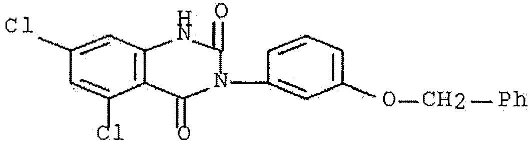 is registered in the Chemical Abstract (registry no.: 195253-31-7).

### [Document List]

### [patent documents]

patent document 1: WO 2008/133127
patent document 2: WO 2008/133128
patent document 3: WO 2007/046392
patent document 4: WO 2005/019188

### SUMMARY OF THE INVENTION

### Problems to be Solved by the Invention

An object of the present invention is to provide a compound having a superior FLAP inhibitory action, and useful as a prophylactic or therapeutic agent for arteriosclerosis and the like.

### Means of Solving the Problems

The present inventors have conducted intensive studies in an attempt to solve the above-mentioned problems and found that a compound represented by the following formula (I) has a superior FLAP inhibitory action, which resulted in the completion of the present invention.

Accordingly, the present invention provides the following.
[1] A compound represented by the formula (I): 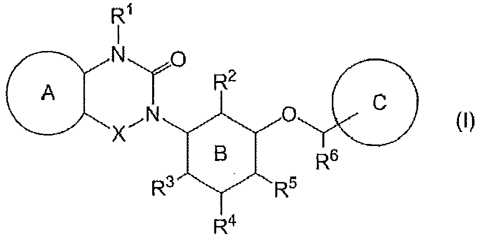 wherein
   X is C(=O) or CH₂;
   ring A is an optionally substituted 5-membered nitrogen-containing heterocycle, an optionally substituted 6-membered heterocycle, or an optionally substituted benzene;
   ring B is a 6-membered aromatic heterocycle or benzene;
   ring C is an optionally further substituted 5- or 6-membered heterocycle or a benzene further having substituent(s);
   R¹ is a hydrogen atom or an optionally substituted C₁₋₆ alkyl group;
   R² is a hydrogen atom or an optionally substituted C₁₋₆ alkyl group or absent;
   R³ is a hydrogen atom or a substituent or absent;
   R⁴ is a hydrogen atom or an optionally substituted C₁₋₆ alkyl group or absent;
   R⁵ is a hydrogen atom, a halogen atom or a C₁₋₆ alkyl group or absent;
   R⁶ is a hydrogen atom or a C₁₋₆ alkyl group,
   or R⁵ and R⁶ optionally form a dihydropyran structure together with a carbon atom bonded thereto,
   or a salt thereof (hereinafter to be also referred to as compound (I)).
[2] The compound of the above-mentioned [1], wherein the formula (I) is the following formula (IA): 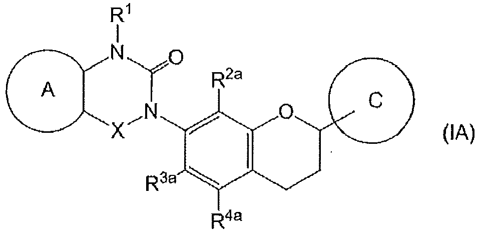 wherein
   X is C (=O) or CH₂;
   ring A is an optionally substituted 5-membered nitrogen-containing heterocycle, an optionally substituted 6-membered heterocycle, or an optionally substituted benzene;
   ring C is an optionally further substituted 5- or 6-membered heterocycle, or benzene further having substituent(s);
   R¹ is a hydrogen atom or an optionally substituted C₁₋₆ alkyl group;
   R^{2a} is a hydrogen atom or an optionally substituted C₁₋₆ alkyl group;
   R^{3a} is a hydrogen atom or a substituent; and
   R^{4a} is a hydrogen atom or an optionally substituted C₁₋₆ alkyl group,
   or a salt thereof (hereinafter to be also referred to as compound (IA)).
[3] 5-Chloro-3-((2S)-6-chloro-2-(3-fluoropyridin-2-yl)-3,4-dihydro-2H-chromen-7-yl)-8-methyl-3,4-dihydropyrido[3,4-d]pyrimidine-2(1H)-one or a salt thereof.
[4] (+)-5-Chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidine-2(1H)-one or a salt thereof.
[5] 3-((2R)-2-(3-Fluoropyridin-2-yl)-6-methyl-3,4-dihydro-2H-chromen-7-yl)-5-(trifluoromethyl)-3,4-dihydropyrido[4,3-d]pyrimidine-2(1H)-one or a salt thereof.
[6] A medicament comprising the compound of the above-mentioned [1] or a salt thereof.
[7] The medicament of the above-mentioned [6], which is a 5-lipoxygenase activating protein inhibitor.
[8] The medicament of the above-mentioned [6], which is a prophylactic or therapeutic agent for arteriosclerosis.
[9] The compound of the above-mentioned [1] or a salt thereof for use for the prophylaxis or treatment of arteriosclerosis.
[10] A method of inhibiting a 5-lipoxygenase activating protein in a mammal, comprising administering an effective amount of the compound of the above-mentioned [1] or a salt thereof to the mammal.
[11] A method for the prophylaxis or treatment of arteriosclerosis in a mammal, comprising administering an effective amount of the compound of the above-mentioned [1] or a salt thereof to the mammal.
[12] Use of the compound of the above-mentioned [1] or a salt thereof in the production of a prophylactic or therapeutic agent for arteriosclerosis.

### Effect of the Invention

Compound (I) has a superior FLAP inhibitory action, and is useful as a prophylactic or therapeutic agent for arteriosclerosis and the like.

### (Detailed Description of the Invention)

In the present specification, the "halogen atom" means a fluorine atom, a chlorine atom, a bromine atom or an iodine atom.

In the present specification, the "C₁₋₆ alkyl (group)" means, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.

In the present specification, the "C₁₋₁₀ alkyl (group)" means, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl or the like. Of these, a C₁₋₆ alkyl group is preferable.

In the present specification, the "C₂₋₆ alkenyl (group)" means, for example, vinyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl or the like.

In the present specification, the "C₂₋₁₀ alkenyl (group)" means, for example, vinyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl or the like. Of these, a C₂₋₆ alkenyl group is preferable.

In the present specification, the "C₂₋₆ alkynyl (group)" means, for example, ethynyl, 1-propynyl, 2-propynyl, 1-butynyl, 2-butynyl, 3-butynyl, 1-pentynyl, 2-pentynyl, 3-pentynyl, 4-pentynyl, 1,1-dimethylprop-2-yn-1-yl, 1-hexynyl, 2-hexynyl, 3-hexynyl, 4-hexynyl, 5-hexynyl or the like.

In the present specification, the "C₂₋₁₀ alkynyl (group)" means, for example, ethynyl, 1-propynyl, 2-propynyl, 1-butynyl, 2-butynyl, 3-butynyl, 1-pentynyl, 2-pentynyl, 3-pentynyl, 4-pentynyl, 1,1-dimethylprop-2-yn-1-yl, 1-hexynyl, 2-hexynyl, 3-hexynyl, 4-hexynyl, 5-hexynyl, 1-heptynyl, 1-octynyl or the like. Of these, a C₂₋₆ alkynyl group is preferable.

In the present specification, the "C₁₋₆ alkoxy (group)" means, for example, methoxy, ethoxy, propoxy, isopropoxy, butoxy, isobutoxy, sec-butoxy, tert-butoxy, pentyloxy, isopentyloxy, hexyloxy or the like.

In the present specification, the "C₂₋₆ alkenyloxy (group)" means, for example, vinyloxy, 1-propenyloxy, 2-propenyloxy, 2-methyl-1-propenyloxy, 1-butenyloxy, 2-butenyloxy, 3-butenyloxy, 3-methyl-2-butenyloxy, 1-pentenyloxy, 2-pentenyloxy, 3-pentenyloxy, 4-pentenyloxy, 4-methyl-3-pentenyloxy, 1-hexenyl, 3-hexenyloxy, 5-hexenyloxy or the like.

In the present specification, the "C₂₋₆ alkynyloxy (group)" means, for example, ethynyloxy, 1-propynyloxy, 2-propynyloxy, 1-butynyloxy, 2-butynyloxy, 3-butynyloxy, 1-pentynyloxy, 2-pentynyloxy, 3-pentynyloxy, 4-pentynyloxy, 1,1-dimethylprop-2-yn-1-yloxy, 1-hexynyloxy, 2-hexynyloxy, 3-hexynyloxy, 4-hexynyloxy, 5-hexynyloxy or the like.

In the present specification, the "C₁₋₆ alkylenedioxy (group)" means, for example, methylenedioxy, ethylenedioxy or the like.

In the present specification, the "C₁₋₆ alkoxy-carbonyl (group)" means, for example, methoxycarbonyl, ethoxycarbonyl, propoxycarbonyl, isopropoxycarbonyl, butoxycarbonyl, isobutoxycarbonyl, tert-butoxycarbonyl or the like.

In the present specification, the "C₁₋₆ alkyl-carbonyl (group)" means, for example, acetyl, propanoyl, butanoyl, 2-methylpropanoyl or the like.

In the present specification, the "mono C₁₋₆ alkylamino (group)" means, for example, methylamino, ethylamino, propylamino, isopropylamino, butylamino, isobutylamino, tert-butylamino or the like.

In the present specification, the "di C₁₋₆ alkylamino (group)" means, for example, dimethylamino, diethylamino, dipropylamino, diisopropylamino, dibutylamino, diisobutylamino, di-tert-butylamino or the like.

In the present specification, the "C₃₋₈ cycloalkyl (group)" means, for example, cyclopropyl, cyclobutyl, cyclopentyl, cyclohexyl, cycloheptyl, cyclooctyl or the like.

In the present specification, the "C₃₋₁₀ cycloalkyl (group)" means, for example, cyclopropyl, cyclobutyl, cyclopentyl, cyclohexyl, cycloheptyl, cyclooctyl, cyclononyl, cyclodecyl or the like. Of these, a C₃₋₈ cycloalkyl group is preferable.

In the present specification, the "C₃₋₈ cycloalkenyl (group)" means, for example, cyclopropenyl (e.g., 2-cyclopropen-1-yl), cyclobutenyl (e.g., 2-cyclobuten-1-yl), cyclopentenyl (e.g., 2-cyclopenten-1-yl, 3-cyclopenten-1-yl), cyclohexenyl (e.g., 2-cyclohexen-1-yl, 3-cyclohexen-1-yl) or the like.

In the present specification, the "C₃₋₁₀ cycloalkenyl (group)" means, for example, 2-cyclopenten-1-yl, 3-cyclopenten-1-yl, 2-cyclohexen-1-yl, 3-cyclohexen-1-yl or the like. Of these, a C₃₋₈ cycloalkenyl group is preferable.

In the present specification, the "C₄₋₁₀ cycloalkadienyl (group)" means, for example, 2,4-cyclopentadien-1-yl, 2,4-cyclohexadien-1-yl, 2,5-cyclohexadien-1-yl or the like.

The above-mentioned C₃₋₁₀ cycloalkyl group, C₃₋₁₀ cycloalkenyl group and C₄₋₁₀ cycloalkadienyl group each optionally form a fused ring group by fusing with a benzene ring. Examples of such fused ring group include indanyl, dihydronaphthyl, tetrahydronaphthyl, fluorenyl and the like.

The above-mentioned C₃₋₁₀ cycloalkyl group, C₃₋₁₀ cycloalkenyl group and C₄₋₁₀ cycloalkadienyl group may be C₇₋₁₀ bridged hydrocarbon groups. Examples of the C₇₋₁₀ bridged hydrocarbon group include bicyclo[2.2.1]heptyl (norbornyl), bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.

Furthermore, the above-mentioned C₃₋₁₀ cycloalkyl group, C₃₋₁₀ cycloalkenyl group and C₄₋₁₀ cycloalkadienyl group each optionally form a spiro ring group with C₃₋₁₀ cycloalkane, C₃₋₁₀ cycloalkene and C₄₋₁₀ cycloalkadiene, respectively. Examples of the C₃₋₁₀ cycloalkane, C₃₋₁₀ cycloalkene and C₄₋₁₀ cycloalkadiene include rings corresponding to the above-mentioned C₃₋₁₀ cycloalkyl group, C₃₋₁₀ cycloalkenyl group and C₄₋₁₀ cycloalkadienyl group. Examples of such spiro ring group include spiro[4.5]decan-8-yl and the like.

In the present specification, the "C₃₋₈ cycloalkyloxy (group)" means, for example, cyclopropyloxy, cyclobutyloxy, cyclopentyloxy, cyclohexyloxy, cycloheptyloxy, cyclooctyloxy or the like.

In the present specification, the "C₃₋₆ cycloalkyloxy (group)" means, for example, cyclopropyloxy, cyclobutyloxy, cyclopentyloxy, cyclohexyloxy or the like.

In the present specification, the "C₃₋₈ cycloalkenyloxy (group)" means, for example, cyclopropenyloxy (e.g., 2-cyclopropen-1-yloxy), cyclobutenyloxy (e.g., 2-cyclobuten-1-yloxy), cyclopentenyloxy (e.g., 2-cyclopenten-1-yloxy, 3-cyclopenten-1-yloxy), cyclohexenyloxy (e.g., 2-cyclohexen-1-yloxy, 3-cyclohexen-1-yloxy) or the like.

In the present specification, the "C₆₋₁₄ aryl (group)" means, for example, phenyl, 1-naphthyl, 2-naphthyl or the like.

In the present specification, the "C₆₋₁₄ aryloxy (group)" means, for example, phenoxy, 1-naphthyloxy, 2-naphthyloxy or the like.

In the present specification, the "C₇₋₁₄ aralkyl (group)" means, for example, benzyl, phenethyl or the like.

In the present specification, the "C₇₋₁₄ aralkyloxy (group)" means, for example, benzyloxy, phenethyloxy or the like.

In the present specification, the "C₈₋₁₃ arylalkenyl (group)" means, for example, styryl or the like.

In the present specification, the "heterocyclic group" means an aromatic heterocyclic group and a non-aromatic heterocyclic group.

In the present specification, the "aromatic heterocyclic group" means a monocyclic aromatic heterocyclic group and a fused aromatic heterocyclic group.

In the present specification, examples of the "monocyclic aromatic heterocyclic group" include a 5- to 7-membered (preferably 5- or 6-membered) monocyclic aromatic heterocyclic group containing, as a ring-constituting atom besides carbon atoms, 1 to 4 hetero atoms selected from an oxygen atom, a sulfur atom (optionally oxidized) and a nitrogen atom (optionally oxidized). Examples thereof include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like.

In the present specification, examples of the "fused aromatic heterocyclic group" include an 8- to 12-membered fused aromatic heterocyclic group, specifically, a group derived from a fused ring wherein a ring corresponding to the above-mentioned 5- to 7-membered monocyclic aromatic heterocyclic group is fused with a C₆₋₁₄ aromatic hydrocarbon; and a group derived from a fused ring wherein rings corresponding to the above-mentioned 5- to 7-membered monocyclic aromatic heterocyclic groups are fused. Examples thereof include quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridyl (e.g., 1H-imidazo[4,5-b]pyridin-2-yl, 1H-imidazo[4,5-c]pyridin-2-yl, 2H-imidazo[1,2-a]pyridin-3-yl), thienopyridyl (e.g., thieno[2,3-b]pyridin-3-yl), imidazopyrazinyl (e.g., 1H-imidazo[4,5-b]pyrazin-2-yl), pyrazolopyridyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like.

In the present specification, the "non-aromatic heterocyclic group" means a monocyclic non-aromatic heterocyclic group and a fused non-aromatic heterocyclic group.

In the present specification, examples of the "monocyclic non-aromatic heterocyclic group" include a 3- to 8-membered (preferably 5- or 6-membered) monocyclic non-aromatic heterocyclic group containing, as a ring-constituting.atom besides carbon atoms, 1 to 4 hetero atoms selected from an oxygen atom, a sulfur atom (optionally oxidized) and a nitrogen atom (optionally oxidized). Examples thereof include azetidinyl (e.g., 1-azetidinyl, 2-azetidinyl), pyrrolidinyl (e.g., 1-pyrrolidinyl, 2-pyrrolidinyl), piperidyl (e.g., piperidino, 2-piperidyl, 3-piperidyl, 4-piperidyl), morpholinyl (e.g., morpholino), thiomorpholinyl (e.g., thiomorpholino), piperazinyl (e.g., 1-piperazinyl, 2-piperazinyl, 3-piperazinyl), oxazolidinyl (e.g., oxazolidin-2-yl), thiazolidinyl (e.g., thiazolidin-2-yl), isothiazolidinyl (e.g., isothiazolidin-2-yl), dihydrothiopyranyl (e.g., dihydrothiopyran-3-yl, dihydrothiopyran-4-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), pyranyl (e.g., 2-pyranyl, 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), oxetanyl (e.g., oxetan-2-yl, oxetan-3-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl), azepanyl (e.g., 1-azepanyl, 2-azepanyl, 3-azepanyl, 4-azepanyl), dihydropyridyl (e.g., dihydropyridin-1-yl, dihydropyridin-2-yl, dihydropyridin-3-yl, dihydropyridin-4-yl), tetrahydropyridyl (e.g., 1,2,3,4-tetrahydropyridin-1-yl, 1,2,3,4-tetrahydropyridin-2-yl, 1,2,3,4-tetrahydropyridin-3-yl, 1,2,3,4-tetrahydropyridin-4-yl) and the like.

In the present specification, examples of the "fused non-aromatic heterocyclic group" include an 8- to 12-membered fused non-aromatic heterocyclic group, specifically, a group derived from a fused ring wherein a ring corresponding to the above-mentioned 3- to 8-membered monocyclic non-aromatic heterocyclic group is fused with a C₆₋₁₄ aromatic hydrocarbon; a group derived from a fused ring wherein rings corresponding to the above-mentioned 3- to 8-membered monocyclic non-aromatic heterocyclic groups are fused; a group derived from a fused ring wherein a ring corresponding to the above-mentioned 3- to 8-membered monocyclic non-aromatic heterocyclic group is fused with a ring corresponding to the above-mentioned 5- to 7-membered monocyclic aromatic heterocyclic group; and a group wherein the above-mentioned group is partially saturated. Examples thereof include dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), tetrahydrobenzofuranyl (e.g., 4,5,6,7-tetrahydro-1-benzofuran-3-yl), dihydrobenzodioxinyl (e.g., 2,3-dihydro-1,4-benzodioxin-2-yl), dihydrobenzodioxepinyl (e.g., 3,4-dihydro-2H-1,5-benzodioxepin-2-yl), chromenyl (e.g., 4H-chromen-2-yl, 2H-chromen-3-yl), dihydrochromenyl (e.g., 3,4-dihydro-2H-chromen-2-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like.

Furthermore, the above-mentioned heterocyclic groups each optionally form a spiro ring group with 5- to 7-membered monocyclic aromatic heterocycle, 3- to 8-membered monocyclic non-aromatic heterocycle, C₃₋₁₀ cycloalkane, C₃₋₁₀ cycloalkene or C₄₋₁₀ cycloalkadiene. Examples of the 5- to 7-membered monocyclic aromatic heterocycle and 3- to 8-membered monocyclic non-aromatic heterocycle include rings corresponding to 5- to 7-membered monocyclic aromatic heterocyclic group and 3- to 8-membered monocyclic non-aromatic heterocyclic group. Examples of the C₃₋₁₀ cycloalkane, C₃₋₁₀ cycloalkene and C₄₋₁₀ cycloalkadiene include rings corresponding to the above-mentioned C₃₋₁₀ cycloalkyl group, C₃₋₁₀ cycloalkenyl group and C₄₋₁₀ cycloalkadienyl group. Examples of such spiro ring group include 2-oxa-6-azaspiro[3.3]heptan-6-yl and the like.

In the present specification, examples of the "C₆₋₁₄ aromatic hydrocarbon" include benzene and naphthalene.

In the present specification, examples of the "aromatic heterocycle" include rings corresponding to the above-mentioned aromatic heterocyclic groups.

In the present specification, examples of the "non-aromatic heterocycle" include rings corresponding to the above-mentioned non-aromatic heterocyclic groups.

In the present specification, examples of the "5- or 6-membered heterocycle" include 5- or 6-membered aromatic heterocycles such as furan, thiophene, pyridine, pyrimidine, pyridazine, pyrazine, pyrrole, imidazole, pyrazole, thiazole, isothiazole, oxazole, isoxazole, oxadiazole, thiadiazole, triazole, tetrazole, triazine and the like; and 5- or 6-membered non-aromatic heterocycles such as pyrrolidine, piperidine, piperazine, morpholine, thiomorpholine, oxazolidine, thiazolidine, imidazolidine, pyrazolidine, oxazoline, thiazoline, imidazoline, pyrazoline, dioxole, dioxolane, dihydrooxadiazole, pyran, dihydropyran, tetrahydropyran, thiopyran, dihydrothiopyran, tetrahydrothiopyran, 1-oxidotetrahydrothiopyran, 1,1-dioxidotetrahydrothiopyran, tetrahydrofuran, oxetane, dihydropyridine, tetrahydropyridine, dihydropyrimidine, tetrahydropyrimidine, dihydrotriazole, tetrahydrotriazole and the like.

In the present specification, examples of the "6-membered heterocycle" include 6-membered aromatic heterocycles such as pyridine, pyrimidine, pyridazine, pyrazine, triazine and the like; 6-membered non-aromatic heterocycles such as piperidine, piperazine, morpholine, thiomorpholine, dioxolane, pyran, dihydropyran, tetrahydropyran, thiopyran, dihydrothiopyran, tetrahydrothiopyran, 1-oxidotetrahydrothiopyran, 1,1-dioxidotetrahydrothiopyran, dihydropyridine, tetrahydropyridine, dihydropyrimidine, tetrahydropyrimidine and the like.

In the present specification, examples of the "6-membered aromatic heterocycle" include pyridine, pyrimidine, pyridazine, pyrazine, triazine and the like.

In the present specification, examples of the "5-membered nitrogen-containing heterocycle" include 5-membered nitrogen-containing heterocycle containing, besides carbon atom, at least one nitrogen atom as a ring constituting atom, and optionally further containing 1 or 2 hetero atoms selected from an oxygen atom, a sulfur atom and a nitrogen atom. Examples thereof include 5-membered nitrogen-containing aromatic heterocycles such as pyrrole, imidazole, pyrazole, thiazole, isothiazole, oxazole, isoxazole, oxadiazole, thiadiazole, triazole, tetrazole and the like; 5-membered nitrogen-containing non-aromatic heterocycles such as pyrrolidine, oxazolidine, thiazolidine, imidazolidine, pyrazolidine, oxazoline, thiazoline, imidazoline, pyrazoline, dihydrooxadiazole, dihydrotriazole, tetrahydrotriazole and the like; and the like.

Each symbol of the formula (I) is explained below.

R¹ in the formula (I) is a hydrogen atom or an optionally substituted C₁₋₆ alkyl group.

The "C₁₋₆ alkyl group" of the "optionally substituted C₁₋₆ alkyl group" for R¹ optionally has 1 to 5 (preferably 1 to 3) substituents at substitutable position(s). Examples of the substituent include those selected from the following Substituent Group A. When plural substituents are present, the respective substituents may be the same or different.

Substituent Group A:
(1) a halogen atom;
(2) a cyano group;
(3) a nitro group;
(4) a hydroxy group;
(5) a C₃₋₁₀ cycloalkyl group optionally substituted by 1 to 3 substituents selected from
   (a) a halogen atom,
   (b) a cyano group,
   (c) a C₁₋₆ alkyl group optionally substituted by 1 to 3 halogen atoms, and
   (d) a C₁₋₆ alkoxy group optionally substituted by 1 to 3 halogen atoms;
(6) a C₆₋₁₄ aryl group optionally substituted by 1 to 3 substituents selected from
   (a) a halogen atom,
   (b) a cyano group,
   (c) a C₁₋₆ alkyl group optionally substituted by 1 to 3 halogen atoms,
   (d) a C₁₋₆ alkoxy group optionally substituted by 1 to 3 halogen atoms;
   (e) a carboxy group, and
   (f) a C₁₋₆ alkoxy-carbonyl group;
(7) a C₁₋₆ alkoxy group optionally substituted by 1 to 3 substituents selected from
   (a) a halogen atom,
   (b) a cyano group,
   (c) a C₃₋₁₀ cycloalkyl group optionally having 1 to 3 halogen atoms,
   (d) a C₃₋₈ cycloalkenyl group optionally having 1 to 3 halogen atoms,
   (e) a C₆₋₁₄ aryl group optionally having 1 to 3 halogen atoms, and
   (f) a 5- or 6-membered monocyclic aromatic heterocyclic group;
(8) a C₂₋₆ alkenyloxy group (e.g., vinyloxy, propenyloxy, butenyloxy, pentenyloxy, hexenyloxy) optionally having 1 to 3 halogen atoms;
(9) a C₂₋₆ alkynyloxy group (e.g., ethynyloxy, propynyloxy, butynyloxy, pentynyloxy, hexynyloxy) optionally having 1 to 3 halogen atoms;
(10) a C₃₋₈ cycloalkyloxy group (e.g., cyclopropyloxy, cyclobutyloxy, cyclopentyloxy, cyclohexyloxy) optionally having 1 to 3 halogen atoms;
(11) a C₃₋₈ cycloalkenyloxy group (e.g., cyclopropenyloxy, cyclobutenyloxy, cyclopentenyloxy, cyclohexenyloxy) optionally having 1 to 3 halogen atoms;
(12) a C₆₋₁₄ aryloxy group optionally having 1 to 3 halogen atoms;
(13) a C₇₋₁₄ aralkyloxy group optionally having 1 to 3 halogen atoms;
(14) a carbamoyl group optionally mono- or di-substituted by substituent(s) selected from
   (a) a C₁₋₆ alkyl group optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₈ cycloalkyl group,
   (c) a C₆₋₁₄ aryl group,
   (d) a C₁₋₆ alkoxy group,
   (e) an aromatic heterocyclic group,
   (f) a non-aromatic heterocyclic group, and
   (g) a C₁₋₆ alkylsulfonyl group;
(15) a sulfamoyl group optionally mono- or di-substituted by substituent(s) selected from
   (a) a C₁₋₆ alkyl group,
   (b) a C₃₋₈ cycloalkyl group,
   (c) a C₆₋₁₄ aryl group,
   (d) a C₁₋₆ alkoxy group,
   (e) a 5- or 6-membered monocyclic aromatic heterocyclic group,
   (f) an 8- to 12-membered fused aromatic heterocyclic group,
   (g) a 3- to 8-membered monocyclic non-aromatic heterocyclic group, and
   (h) an 8- to 12-membered fused non-aromatic heterocyclic
   group;
(16) formyl;
(17) a C₁₋₆ alkyl-carbonyl group;
(18) a C₂₋₆ alkenyl-carbonyl group (e.g., acryloyl, butenoyl, pentenoyl, hexenoyl, heptenoyl);
(19) a C₂₋₆ alkynyl-carbonyl group (e.g., propioloyl, propynylcarbonyl, butynylcarbonyl, pentynylcarbonyl, hexynylcarbonyl);
(20) a C₃₋₈ cycloalkyl-carbonyl group (e.g., cyclopropylcarbonyl, cyclobutylcarbonyl, cyclopentylcarbonyl, cyclohexylcarbonyl);
(21) a C₃₋₈ cycloalkenyl-carbonyl group (e.g., cyclopropenylcarbonyl, cyclobutenylcarbonyl, cyclopentenylcarbonyl, cyclohexenylcarbonyl);
(22) a C₆₋₁₄ aryl-carbonyl group (e.g., benzoyl, 1-naphthylcarbonyl, 2-naphthylcarbonyl);
(23) a C₃₋₈ cycloalkyl-C₁₋₆ alkyl-carbonyl group (e.g., cyclopropylacetyl, 3-cyclopropylpropionyl, cyclobutylacetyl, cyclopentylacetyl, cyclohexylacetyl, cyclohexylpropionyl);
(24) a C₃₋₈ cycloalkenyl-C₁₋₆ alkyl-carbonyl group (e.g., cyclopentenylacetyl, cyclohexenylacetyl, 3-cyclohexenylpropionyl, 3-cyclohexenylpropionyl);
(25) a C₇₋₁₄ aralkyl-carbonyl group (e.g., phenylacetyl, 3-phenylpropionyl);
(26) a 5- or 6-membered monocyclic aromatic heterocyclylcarbonyl group (e.g., furylcarbonyl, thienylcarbonyl, pyrrolylcarbonyl, oxazolylcarbonyl, isoxazolylcarbonyl, thiazolylcarbonyl, isothiazolylcarbonyl, imidazolylcarbonyl, pyridylcarbonyl, pyrazolylcarbonyl);
(27) an 8- to 12-membered fused aromatic heterocyclylcarbonyl group (e.g., benzofuranylcarbonyl, isobenzofuranylcarbonyl, benzothienylcarbonyl, isobenzothienylcarbonyl, indolylcarbonyl, isoindolylcarbonyl, indazolylcarbonyl, benzimidazolylcarbonyl, benzoxazolylcarbonyl);
(28) a 3- to 8-membered monocyclic non-aromatic heterocyclylcarbonyl group (e.g., oxiranylcarbonyl, azetidinylcarbonyl, oxetanylcarbonyl, thietanylcarbonyl, pyrrolidinylcarbonyl, tetrahydrofurylcarbonyl, thiolanylcarbonyl, piperidylcarbonyl);
(29) an 8- to 12-membered fused non-aromatic heterocyclylcarbonyl group (e.g., dihydrobenzofuranyl);
(30) an amino group optionally mono- or di-substituted by substituent(s) selected from
   (a) a C₁₋₆ alkyl group optionally having 1 to 3 halogen atoms,
   (b) a C₁₋₆ alkyl-carbonyl group optionally having 1 to 3 halogen atoms,
   (c) a C₃₋₈ cycloalkyl-carbonyl group,
   (d) a C₆₋₁₄ aryl-carbonyl group optionally having 1 to 3 halogen atoms,
   (e) a 5- or 6-membered monocyclic aromatic heterocyclylcarbonyl group,
   (f) an 8- to 12-membered fused aromatic heterocyclylcarbonyl group,
   (g) a 3- to 8-membered monocyclic non-aromatic heterocyclylcarbonyl group,
   (h) an 8- to 12-membered fused non-aromatic heterocyclylcarbonyl group;
   (i) a C₃₋₁₀ cycloalkyl-carbonyl group, and
   (j) a C₃₋₁₀ cycloalkylsulfonyl group;
(31) a sulfanyl group;
(32) a C₁₋₆ alkylsulfanyl group (e.g., methylsulfanyl, ethylsulfanyl);
(33) a C₂₋₆ alkenylsulfanyl group (e.g., vinylsulfanyl, propenylsulfanyl);
(34) a C₂₋₆ alkynylsulfanyl group (e.g., ethynylsulfanyl, propynylsulfanyl);
(35) a C₃₋₈ cycloalkylsulfanyl group (e.g., cyclopropylsulfanyl, cyclobutylsulfanyl);
(36) a C₃₋₈ cycloalkenylsulfanyl group (e.g., cyclopropenylsulfanyl, cyclobutenylsulfanyl);
(37) a C₆₋₁₄ arylsulfanyl group (e.g., phenylsulfanyl);
(38) a C₃₋₈ cycloalkyl-C₁₋₆ alkylsulfanyl group (e.g., cyclopropylmethylsulfanyl);
(39) a C₃₋₈ cycloalkenyl-C₁₋₆ alkylsulfanyl group (e.g., cyclopentenylmethylsulfanyl);
(40) a C₁₋₆ alkylsulfinyl group (e.g., methylsulfinyl, ethylsulfinyl);
(41) a C₂₋₆ alkenylsulfinyl group (e.g., vinylsulfinyl, propenylsulfinyl);
(42) a C₂₋₆ alkynylsulfinyl group (e.g., ethynylsulfinyl, propynylsulfinyl);
(43) a C₃₋₈ cycloalkylsulfinyl group (e.g., cyclopropylsulfinyl, cyclobutylsulfinyl);
(44) a C₃₋₈ cycloalkenylsulfinyl group (e.g., cyclopropenylsulfinyl, cyclobutenylsulfinyl);
(45) a C₆₋₁₄ arylsulfinyl group (e.g., phenylsulfinyl);
(46) a C₃₋₈ cycloalkyl-C₁₋₆ alkylsulfinyl group (e.g., cyclopropylmethylsulfinyl);
(47) a C₃₋₈ cycloalkenyl-C₁₋₆ alkylsulfinyl group (e.g., cyclopentenylmethylsulfinyl);
(48) a C₁₋₆ alkylsulfonyl group (e.g., methylsulfonyl, ethylsulfonyl);
(49) a C₂₋₆ alkenylsulfonyl group (e.g., vinylsulfonyl, propenylsulfonyl);
(50) a C₂₋₆ alkynylsulfonyl group (e.g., ethynylsulfonyl, propynylsulfonyl);
(51) a C₃₋₈ cycloalkylsulfonyl group (e.g., cyclopropylsulfonyl, cyclobutylsulfonyl);
(52) a C₃₋₈ cycloalkenylsulfonyl group (e.g., cyclopropenylsulfonyl, cyclobutenylsulfonyl);
(53) a C₆₋₁₄ arylsulfonyl group (e.g., phenylsulfonyl);
(54) a C₃₋₈ cycloalkyl-C₁₋₆ alkylsulfonyl group (e.g., cyclopropylmethylsulfonyl);
(55) a C₃₋₈ cycloalkenyl-C₁₋₆ alkylsulfonyl group (e.g., cyclopentenylmethylsulfonyl);
(56) a C₆₋₁₄ aryl-C₁₋₆ alkylsulfonyl group (e.g., benzylsulfonyl);
(57) a 5- or 6-membered monocyclic aromatic heterocyclylsulfonyl group (e.g., furylsulfonyl, thienylsulfonyl, pyridylsulfonyl);
(58) an 8- to 12-membered fused aromatic heterocyclylsulfonyl group (e.g., benzofuranylsulfonyl, isobenzofuranylsulfonyl);
(59) a 3- to 8-membered monocyclic non-aromatic heterocyclylsulfonyl group (e.g., oxiranylsulfonyl, azetidinylsulfonyl);
(60) an 8- to 12-membered fused non-aromatic heterocyclylsulfonyl group (e.g., dihydrobenzofuranylsulfonyl);
(61) an aromatic heterocyclic group (e.g., furyl, thienyl, pyrrolyl, oxazolyl, isoxazolyl, thiazolyl, isothiazolyl, imidazolyl, pyridyl, pyrazolyl, morpholinyl, triazolyl, oxodiazolyl, benzofuranyl, isobenzofuranyl, benzothienyl, isobenzothienyl, indolyl, isoindolyl, indazolyl, benzimidazolyl, benzoxazolyl) optionally substituted by 1 to 3 substituents selected from
   (a) a halogen atom,
   (b) a C₁₋₆ alkyl group optionally substituted by 1 to 3 halogen atoms or a hydroxy group,
   (c) a C₁₋₆ alkoxy group optionally substituted by 1 to 3 halogen atoms, and
   (d) a carbamoyl group;
(62) a non-aromatic heterocyclic group (e.g., oxiranyl, azetidinyl, oxetanyl, thietanyl, pyrrolidinyl, tetrahydrofuryl, thiolanyl, piperidyl, piperazinyl, dihydrooxadiazolyl, thiazolinyl, morpholinyl, dihydrothiadiazolyl, dihydrooxazolyl, tetrahydrooxazolyl, oxetanyl, 1,1-dioxidotetrahydroisothiazolyl, tetrahydroimidazolyl, dihydrobenzofuranyl) optionally substituted by 1 to 3 substituents selected from
   (a) a halogen atom,
   (b) a C₁₋₆ alkyl group optionally substituted by 1 to 3 halogen atoms,
   (c) a C₁₋₆ alkoxy group optionally substituted by 1 to 3 halogen atoms,
   (d) an oxo group,
   (e) a hydroxy group,
   (f) a thioxo group, and
   (g) an aromatic heterocyclyl-carbonyl group;
(63) a 5- or 6-membered monocyclic aromatic heterocyclyloxy group (e.g., furyloxy, thienyloxy, pyrrolyloxy, oxazolyloxy, isooxazolyloxy, thiazolyloxy, isothiazolyloxy, imidazolyloxy, pyridyloxy, pyrazolyloxy) optionally substituted by 1 to 3 C₁₋₆ alkyl groups;
(64) an 8- to 12-membered fused aromatic heterocyclyloxy group (e.g., benzofuranyloxy, isobenzofuranyloxy, benzothienyloxy, isobenzothienyloxy, indolyloxy, isoindolyloxy, indazolyloxy, benzimidazolyloxy, benzoxazolyloxy) optionally substituted by 1 to 3 C₁₋₆ alkyl groups;
(65) a 3- to 8-membered monocyclic non-aromatic heterocyclyloxy group (e.g., oxiranyloxy, azetidinyloxy, oxetanyloxy, thietanyloxy, pyrrolidinyloxy, tetrahydrofuryloxy, thiolanyloxy, piperidyloxy);
(66) an 8- to 12-membered fused non-aromatic heterocyclyloxy group (e.g., dihydrobenzofuranyloxy);
(67) a carboxy group;
(68) a C₁₋₆ alkoxy-carbonyl group;
(69) a C₂₋₆ alkenyloxy-carbonyl group (e.g., vinyloxycarbonyl, propenyloxycarbonyl, butenyloxycarbonyl, pentenyloxycarbonyl, hexenyloxycarbonyl);
(70) a C₂₋₆ alkynyloxy-carbonyl group (e.g., ethynyloxycarbonyl, propynyloxycarbonyl, butynyloxycarbonyl, pentynyloxycarbonyl, hexynyloxycarbonyl);
(71) a C₃₋₈ cycloalkyloxy-carbonyl group (e.g., cyclopropyloxycarbonyl, cyclobutyloxycarbonyl, cyclopentyloxycarbonyl, cyclohexyloxycarbonyl);
(72) a C₃₋₈ cycloalkenyloxy-carbonyl group (e.g., cyclopropenyloxycarbonyl, cyclobutenyloxycarbonyl, cyclopentenyloxycarbonyl, cyclohexenyloxycarbonyl);
(73) a C₆₋₁₄ aryloxy-carbonyl group (e.g., phenoxycarbonyl, 1-naphthyloxycarbonyl, 2-naphthyloxycarbonyl);
(74) a C₃₋₈ cycloalkyl-C₁₋₆ alkoxy-carbonyl group (e.g., cyclopropylmethyloxycarbonyl, cyclopropylethyloxycarbonyl, cyclobutylmethyloxycarbonyl, cyclopentylmethyloxycarbonyl, cyclohexylmethyloxycarbonyl, cyclohexylethyloxycarbonyl);
(75) a C₃₋₈ cycloalkenyl-C₁₋₆ alkoxy-carbonyl group (e.g., cyclopentenylmethyloxycarbonyl, cyclohexenylmethyloxycarbonyl, cyclohexenylethyloxycarbonyl, cyclohexenylpropyloxycarbonyl);
(76) a C₇₋₁₄ aralkyloxy-carbonyl group (e.g., benzyloxycarbonyl, phenethyloxycarbonyl);
(77) a mono-C₁₋₆ alkylthio-carbamoyl group (e.g., methylthiocarbamoyl, ethylthiocarbamoyl, propylthiocarbamoyl);
(78) a di-C₁₋₆ alkylthio-carbamoyl group (e.g., dimethylthiocarbamoyl, diethylthiocarbamoyl, dipropylthiocarbamoyl);
(79) a C₁₋₆ alkyl-carbonyloxy group (e.g., acetyloxy, propanoyloxy, butanoyloxy, 2-methylpropanoyloxy);
(80) an imino group optionally substituted by a hydroxy group;
(81) a C₁₋₆ alkylenedioxy group (e.g., methylenedioxy, ethylenedioxy);
(82) a non-aromatic heterocyclyl-carbonyl group (e.g., morpholinylcarbonyl, piperidylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom, and
   (c) a C₁₋₆ alkoxy group; and
(83) a C₆₋₁₄ arylsulfonyloxy group (e.g., phenylsulfonyloxy) optionally substituted by 1 to 3 C₁₋₆ alkyl groups.

R¹ is preferably
(1) a hydrogen atom, or
(2) a C₁₋₆ alkyl group (e.g., methyl, ethyl) optionally substituted by 1 to 3 substituents selected from
   (a) a carboxy group,
   (b) a C₃₋₁₀ cycloalkyl group (e.g., cyclopropyl),
   (c) a C₁₋₆ alkoxy group (e.g., methoxy),
   (d) a C₆₋₁₄ aryl group (e.g., phenyl) optionally substituted by 1 to 3 substituents selected from
      (i) a C₁₋₆ alkoxy group (e.g., methoxy),
      (ii) a carboxy group, and
      (iii) a C₁₋₆ alkoxy-carbonyl group (e.g., methoxycarbonyl), and
   (e) a carbamoyl group.
R¹ is more preferably
(1) a hydrogen atom, or
(2) a C₁₋₆ alkyl group (e.g., methyl, ethyl) optionally substituted by 1 to 3 C₁₋₆ alkoxy groups (e.g., methoxy).

R¹ is particularly preferably a hydrogen atom.

R² in the formula (I) is a hydrogen atom or an optionally substituted C₁₋₆ alkyl group, or absent.

The "C₁₋₆ alkyl group" of the "optionally substituted C₁₋₆ alkyl group" for R² optionally has 1 to 5 (preferably 1 to 3) substituents at substitutable position(s). Examples of the substituent include those selected from the following Substituent Group A. When plural substituents are present, the respective substituents may be the same or different.

R² is preferably a hydrogen atom or an optionally substituted C₁₋₆ alkyl group.

R² is more preferably a hydrogen atom or a C₁₋₆ alkyl group.

R² is particularly preferably a hydrogen atom.

R³ in the formula (I) is a hydrogen atom or a substituent, or absent.

Examples of the "substituent" for R³ include "halogen atom", "nitro group", "cyano group", "optionally substituted hydrocarbon group", "optionally substituted heterocyclic group", "optionally substituted hydroxy group", "optionally substituted amino group", "optionally substituted mercapto group", "acyl group" and the like.

Examples of the "hydrocarbon group" of the aforementioned "optionally substituted hydrocarbon group" include a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₄ aralkyl group, a C₈₋₁₃ arylalkenyl group and the like.

The C₁₋₁₀ alkyl group, C₂₋₁₀ alkenyl group and C₂₋₁₀ alkynyl group exemplified as the aforementioned "hydrocarbon group" optionally have 1 to 5 (preferably 1 to 3) substituents at substitutable position(s). Examples of such substituent include those selected from the above-mentioned Substituent Group A. When plural substituents are present, the respective substituents may be the same or different.

In addition, the C₃₋₁₀ cycloalkyl group, C₃₋₁₀ cycloalkenyl group, C₄₋₁₀ cycloalkadienyl group, C₆₋₁₄ aryl group, C₇₋₁₄ aralkyl group and C₈₋₁₃ arylalkenyl group, exemplified as the aforementioned "hydrocarbon group", optionally have 1 to 5 (preferably 1 to 3) substituents at substitutable position(s). Examples of such substituent include those selected from the following Substituent Group B. When plural substituents are present, the respective substituents may be the same or different.

Substituent Group B:
(1) the above-mentioned Substituent Group A;
(2) a C₁₋₆ alkyl group optionally substituted by 1 to 3 substituents selected from
   (a) a halogen atom,
   (b) a cyano group,
   (c) a hydroxy group,
   (d) a C₃₋₈ cycloalkyl group optionally substituted by 1 to 3 substituents selected from
      (i) a halogen atom,
      (ii) a cyano group, and
      (iii) a C₁₋₆ alkyl group optionally substituted by 1 to 3 halogen atoms;
   (e) a C₆₋₁₄ aryl group optionally substituted by 1 to 3 substituents selected from
      (i) a halogen atom,
      (ii) a cyano group, and
      (iii) a C₁₋₆ alkyl group optionally substituted by 1 to 3 halogen atoms,
   (f) a C₁₋₆ alkoxy group optionally substituted by 1 to 3 halogen atoms,
   (g) an amino group optionally mono- or di-substituted by C₁₋₆ alkyl,
   (h) a 5- or 6-membered monocyclic aromatic heterocyclic group,
   (i) a 8- to 12-membered fused aromatic heterocyclic group,
   (j) a 3- to 8-membered monocyclic non-aromatic heterocyclic group,
   (k) a 8- to 12-membered fused non-aromatic heterocyclic group,
   (l) a carboxy group, and
   (m) a C₁₋₆ alkoxy-carbonyl group optionally substituted by 1 to 3 halogen atoms;
(3) a C₂₋₆ alkenyl group optionally substituted by 1 to 3 substituents selected from
   (a) a halogen atom,
   (b) a hydroxy group,
   (c) a C₁₋₆ alkoxy group,
   (d) an amino group optionally mono- or di-substituted by C₁₋₆ alkyl,
   (e) a carboxy group, and
   (f) a C₁₋₆ alkoxy-carbonyl group;
(4) a C₇₋₁₄ aralkyl group optionally substituted by 1 to 3 substituents selected from
   (a) a halogen atom,
   (b) a hydroxy group,
   (c) a C₁₋₆ alkoxy group, and
   (d) a C₁₋₆ alkyl group optionally substituted by 1 to 3 halogen atoms; and
(5) an oxo group.

The "heterocyclic group" of the aforementioned "optionally substituted heterocyclic group" optionally has 1 to 5 (preferably 1 to 3) substituents at substitutable position(s). Examples of the substituent include those selected from the aforementioned Substituent Group B. When plural substituents are present, the respective substituents may be the same or different.

Examples of the aforementioned "optionally substituted hydroxy group" include a hydroxyl group optionally substituted by substituent (s) selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₄ aralkyl group, a C₈₋₁₃ arylalkenyl group, a C₁₋₆ alkyl-carbonyl group, a heterocyclic group and the like, each of which is optionally substituted.

Here, the C₁₋₁₀ alkyl group, C₂₋₁₀ alkenyl group and C₁₋₆ alkyl-carbonyl group optionally have 1 to 5 (preferably 1 to 3) substituents at substitutable position(s). Examples of the substituent include those selected from the following Substituent Group A. When plural substituents are present, the respective substituents may be the same or different.

In addition, the C₃₋₁₀ cycloalkyl group, C₃₋₁₀ cycloalkenyl group, C₆₋₁₄ aryl group, C₇₋₁₄ aralkyl group, C₈₋₁₃ arylalkenyl group and heterocyclic group optionally have 1 to 5 (preferably 1 to 3) substituents at substitutable position(s). Examples of the substituent include those selected from the following Substituent Group B. When plural substituents are present, the respective substituents may be the same or different.

Examples of the aforementioned "optionally substituted mercapto group" include a mercapto group optionally substituted by substituent (s) selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₄ aralkyl group, a C₈₋₁₃ arylalkenyl group, a C₁₋₆ alkyl-carbonyl group, a heterocyclic group and the like, each of which is optionally substituted.

Examples of the substituent include those exemplified as each substituent for the aforementioned "optionally substituted hydroxy group".

Examples of the aforementioned "optionally substituted amino group" include an amino group optionally mono- or di-substituted by substituent (s) selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₄ aralkyl group, a C₈₋₁₃ arylalkenyl group and a heterocyclic group; an acyl group and the like, each of which is optionally substituted.

The C₁₋₁₀ alkyl group and C₂₋₁₀ alkenyl group optionally have 1 to 5 (preferably 1 to 3) substituents at substitutable position(s). Examples of the substituent include those selected from the aforementioned Substituent Group A. When plural substituents are present, the respective substituents may be the same or different.

In addition, the C₃₋₁₀ cycloalkyl group, C₃₋₁₀ cycloalkenyl group, C₆₋₁₄ aryl group, C₇₋₁₄ aralkyl group, C₈₋₁₃ arylalkenyl group and heterocyclic group optionally have 1 to 5 (preferably 1 to 3) substituents at substitutable position(s). Examples of the substituent include those selected from the aforementioned Substituent Group B. When plural substituents are present, the respective substituents may be the same or different.

Examples of the "acyl group" exemplified as the substituent for the "optionally substituted amino group" include those similar to the "acyl group" to be exemplified as the "substituent" for R³ shown below.

Examples of the "acyl group" exemplified as the substituent for R³ include a group represented by the formula: -COR^{A}, -CO-OR^{A}, -SO₃R^{A}, -S(O)₂R^{A}, -SOR^{A}, -CO-NR^{A}'R^{B}', -CS-NR^{A}'R^{B}' or -S(O)₂NR^{A}'R^{B}' wherein R^{A} is a hydrogen atom, an optionally substituted hydrocarbon group, or an optionally substituted heterocyclic group, and R^{A}' and R^{B}' are the same or different and each is a hydrogen atom, an optionally substituted hydrocarbon group, or an optionally substituted heterocyclic group, or R^{A}' and R^{B}' in combination optionally form, together with the adjacent nitrogen atom, an optionally substituted nitrogen-containing heterocycle, and the like.

Examples of the "optionally substituted hydrocarbon group" and "optionally substituted heterocyclic group" for R^{A}, R^{A}' or R^{B}' include those similar to the "optionally substituted hydrocarbon group" and "optionally substituted heterocyclic group" each exemplified as the "substituent" for R³.

Examples of the "nitrogen-containing heterocycle" of the "optionally substituted nitrogen-containing heterocycle" formed by R^{A}' and R^{B}' together with the adjacent nitrogen atom include a 5- to 7-membered nitrogen-containing heterocycle containing, as a ring-constituting atom besides carbon atoms, at least one nitrogen atom and optionally further containing one or two hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine and the like.

The nitrogen-containing heterocycle optionally has 1 to 5 (preferably 1 to 3) substituents at substitutable position(s). Examples of the substituent include those selected from the aforementioned Substituent Group B. When plural substituents are present, the respective substituents may be the same or different.

Preferable examples of the "acyl group" include
(1) a formyl group;
(2) a carboxy group;
(3) a C₁₋₆ alkyl-carbonyl group (e.g., acetyl) optionally substituted by 1 to 3 halogen atoms;
(4) a C₁₋₆ alkoxy-carbonyl group (e.g., methoxycarbonyl, ethoxycarbonyl, propoxycarbonyl, tert-butoxycarbonyl) optionally substituted by 1 to 3 halogen atoms;
(5) a C₃₋₁₀ cycloalkyl-carbonyl group (e.g., cyclopropylcarbonyl, cyclopentylcarbonyl, cyclohexylcarbonyl);
(6) a C₆₋₁₄ aryl-carbonyl group (e.g., benzoyl, 1-naphthoyl, 2-naphthoyl) optionally substituted by 1 to 3 halogen atoms;
(7) a carbamoyl group optionally mono- or di-substituted by substituent(s) selected from
   (a) a C₁₋₆ alkyl group optionally substituted by 1 to 3 substituents selected from a halogen atom, a hydroxy group, a C₁₋₆ alkoxy group, a C₁₋₆ alkoxy-carbonyl group, an aromatic heterocyclic group and a carboxy group,
   (b) an amino group optionally mono- or di-substituted by a C₁₋₆ alkoxy-carbonyl group, and
   (c) a C₃₋₁₀ cycloalkyl group;
(8) a C₁₋₆ alkylsulfonyl group (e.g., methylsulfonyl, ethylsulfonyl, isopropylsulfonyl) optionally substituted by 1 to 3 halogen atoms;
(9) a C₆₋₁₄ arylsulfonyl group (e.g., benzenesulfonyl) ;
(10) a sulfamoyl group;
(11) a thiocarbamoyl group;
(12) an aromatic heterocyclylcarbonyl group (e.g., furylcarbonyl, thienylcarbonyl) optionally substituted by 1 to 3 substituents selected from a C₁₋₆ alkyl group optionally substituted by 1 to 3 halogen atoms;
(13) a non-aromatic heterocyclylcarbonyl group (e.g., tetrahydrofurylcarbonyl, pyrrolidinylcarbonyl) optionally substituted by 1 to 3 substituents selected from a C₁₋₆ alkyl group optionally substituted by 1 to 3 halogen atoms;
and the like.

R³ is preferably a hydrogen atom, a halogen atom, a cyano group, an optionally substituted hydrocarbon group (preferably, C₁₋₆ alkyl group, C₂₋₆ alkenyl group, C₃₋₁₀ cycloalkyl group), an acyl group (preferably, carboxy group, C₁₋₆ alkoxy-carbonyl group, optionally substituted carbamoyl group) or the like, or absent.

To be specific, R³ is preferably
(1) a hydrogen atom,
(2) a cyano group,
(3) a halogen atom (e.g., fluorine atom, chlorine atom, bromine atom),
(4) a C₁₋₆ alkyl group (e.g., methyl, ethyl, propyl, butyl, 3-methylbutyl, neopentyl) optionally substituted by 1 to 3 substituents selected from
   (a) a hydroxy group,
   (b) a halogen atom (e.g., fluorine atom),
   (c) a carboxy group,
   (d) a C₁₋₆ alkoxy-carbonyl group (e.g., ethoxycarbonyl, butoxycarbonyl),
   (e) a C₃₋₁₀ cycloalkyl group (e.g., cyclohexyl),
   (f) a C₆₋₁₄ aryl group (e.g., phenyl),
   (g) an aromatic heterocyclic group (e.g., pyridyl),
   (h) a non-aromatic heterocyclic group (e.g., pyrrolidinyl, morpholinyl, piperazinyl) optionally substituted by 1 to 3 C₁₋₆ alkyl groups (e.g., propyl),
   (i) an amino group optionally mono- or di-substituted by a C₃₋₁₀ cycloalkyl group (e.g., cyclopropyl), and
   (j) a carbamoyl group optionally mono- or di-substituted by a C₃₋₁₀ cycloalkyl group (e.g., cyclopropyl),
(5) a C₂₋₆ alkenyl group (e.g., vinyl) optionally substituted by 1 to 3 C₁₋₆ alkoxy-carbonyl groups (e.g., butoxycarbonyl),
(6) a C₃₋₁₀ cycloalkyl group (e.g., cyclopropyl),
(7) a C₁₋₆ alkoxy-carbonyl group (e.g., methoxycarbonyl), or
(8) a carbamoyl group optionally mono- or di-substituted by substituent(s) selected from
   (a) a C₁₋₆ alkyl group (e.g., methyl, isobutyl) optionally substituted by 1 to 3 substituents selected from
      (i) a hydroxy group, and
      (ii) an aromatic heterocyclic group (e.g., pyridyl), and
   (b) a C₃₋₁₀ cycloalkyl group (e.g., cyclopropyl),
or absent.

R³ is more preferably a hydrogen atom, a halogen atom, a cyano group, an optionally substituted hydrocarbon group (preferably, C₁₋₆ alkyl group, C₂₋₆ alkenyl group, C₃₋₁₀ cycloalkyl group), an acyl group (preferably, carboxy group, C₁₋₆ alkoxy-carbonyl group, optionally substituted carbamoyl group) or the like.

To be specific, R³ is more preferably
(1) a hydrogen atom,
(2) a cyano group,
(3) a halogen atom (e.g., fluorine atom, chlorine atom, bromine atom),
(4) a C₁₋₆ alkyl group (e.g., methyl, ethyl, propyl, butyl, 3-methylbutyl, neopentyl) optionally substituted by 1 to 3 substituents selected from
   (a) a hydroxy group,
   (b) a halogen atom (e.g., fluorine atom),
   (c) a carboxy group,
   (d) a C₁₋₆ alkoxy-carbonyl group (e.g., ethoxycarbonyl, butoxycarbonyl),
   (e) a C₃₋₁₀ cycloalkyl group (e.g., cyclohexyl),
   (f) a C₆₋₁₄ aryl group (e.g., phenyl),
   (g) an aromatic heterocyclic group (e.g., pyridyl),
   (h) a non-aromatic heterocyclic group (e.g., pyrrolidinyl, morpholinyl, piperazinyl) optionally substituted by 1 to 3 C₁₋₆ alkyl groups (e.g., propyl),
   (i) an amino group optionally mono- or di-substituted by a C₃₋₁₀ cycloalkyl group (e.g., cyclopropyl), and
   (j) a carbamoyl group optionally mono- or di-substituted by a C₃₋₁₀ cycloalkyl group (e.g., cyclopropyl),
(5) a C₂₋₆ alkenyl group (e.g., vinyl) optionally substituted by 1 to 3 C₁₋₆ alkoxy-carbonyl groups (e.g., butoxycarbonyl),
(6) a C₃₋₁₀ cycloalkyl group (e.g., cyclopropyl),
(7) a C₁₋₆ alkoxy-carbonyl group (e.g., methoxycarbonyl), or
(8) a carbamoyl group optionally mono- or di-substituted by substituent(s) selected from
   (a) a C₁₋₆ alkyl group (e.g., methyl, isobutyl) optionally substituted by 1 to 3 substituents selected from
      (i) a hydroxy group, and
      (ii) an aromatic heterocyclic group (e.g., pyridyl), and
   (b) a C₃₋₁₀ cycloalkyl group (e.g., cyclopropyl).

R³ is particularly preferably
(1) a hydrogen atom,
(2) a halogen atom (e.g., fluorine atom, chlorine atom, bromine atom), or
(3) a C₁₋₆ alkyl group (e.g., methyl) optionally substituted by 1 to 3 halogen atoms (e.g., fluorine atom).

R⁴ in the formula (I) is a hydrogen atom or an optionally substituted C₁₋₆ alkyl group, or absent.

The "C₁₋₆ alkyl group" of the "optionally substituted C₁₋₆ alkyl group" for R⁴ optionally has 1 to 5 (preferably 1 to 3) substituents at substitutable position(s). Examples of the substituent include those selected from the following Substituent Group A. When plural substituents are present, the respective substituents may be the same or different.

R⁴ is preferably a hydrogen atom or an optionally substituted C₁₋₆ alkyl group.

R⁴ is more preferably a hydrogen atom or a C₁₋₆ alkyl group.

R⁴ is particularly preferably a hydrogen atom.

R⁵ in the formula (I) is a hydrogen atom, a halogen atom or a C₁₋₆ alkyl group, or absent.

R⁶ in the formula (I) is a hydrogen atom or a C₁₋₆ alkyl group.

Alternatively, R⁵ and R⁶ optionally form a dihydropyran structure together with a carbon atom bonded thereto.

R⁵ is preferably
(1) a hydrogen atom,
(2) a halogen atom (e.g., fluorine atom, iodine atom), or
(3) a C₁₋₆ alkyl group.

R⁵ is more preferably
(1) a hydrogen atom, or
(2) a halogen atom (e.g., fluorine atom, iodine atom).

R⁶ is preferably
(1) a hydrogen atom, or
(2) a C₁₋₆ alkyl group (e.g., methyl).

R⁶ is more preferably a hydrogen atom.

Alternatively, R⁵ and R⁶ optionally form a dihydropyran structure together with a carbon atom bonded thereto.

Ring A is optionally substituted 5-membered nitrogen-containing heterocycle, optionally substituted 6-membered heterocycle, or optionally substituted benzene.

The "5-membered nitrogen-containing heterocycle" of the "optionally substituted 5-membered nitrogen-containing heterocycle", the "6-membered heterocycle" of the "optionally substituted 6-membered heterocycle" and the "benzene" of the "optionally substituted benzene" for ring A optionally have 1 to 5 (preferably 1 to 3) substituents at substitutable position(s). Examples of the substituent include those selected from the aforementioned Substituent Group B. When plural substituents are present, the respective substituents may be the same or different.

The "5-membered nitrogen-containing heterocycle" of the "optionally substituted 5-membered nitrogen-containing heterocycle" for ring A is preferably 5-membered nitrogen-containing aromatic heterocycle, more preferably pyrrole, thiazole or pyrazole.

The "6-membered heterocycle" of the "optionally substituted 6-membered heterocycle" is preferably 6-membered nitrogen-containing heterocycle, more preferably pyridine, pyrimidine or dihydropyridine, and particularly preferably pyridine.

Ring A is preferably a 5-membered nitrogen-containing aromatic heterocycle (preferably, pyrrole, thiazole, pyrazole), 6-membered nitrogen-containing heterocycle (preferably, pyridine, pyrimidine, dihydropyridine, more preferably pyridine) or benzene, each of which is optionally substituted by 1 to 4 substituents selected from
(1) a halogen atom (e.g., fluorine atom, chlorine atom, bromine atom),
(2) a hydroxy group,
(3) a carboxy group,
(4) a cyano group,
(5) a nitro group,
(6) a C₁₋₆ alkyl group (e.g., methyl, ethyl, isopropyl) optionally substituted by 1 to 3 substituents selected from
   (a) a hydroxy group,
   (b) a halogen atom (e.g., fluorine atom),
   (c) an amino group,
   (d) a carboxy group,
   (e) a C₁₋₆ alkoxy group (e.g., methoxy), and
   (f) a C₃₋₁₀ cycloalkyl group (e.g., cyclopropyl),
(7) a C₂₋₆ alkenyl group (e.g., vinyl) optionally substituted by 1 to 3 substituents selected from
   (a) a carboxy group, and
   (b) a C₁₋₆ alkoxy-carbonyl group (e.g., methoxycarbonyl),
(8) a C₃₋₁₀ cycloalkyl group (e.g., cyclopropyl),
(9) a C₁₋₆ alkoxy group (e.g., methoxy, ethoxy) optionally substituted by 1 to 3 substituents selected from
   (a) a C₃₋₁₀ cycloalkyl group (e.g., cyclopropyl),
   (b) a C₁₋₆ alkoxy group (e.g., methoxy), and
   (c) a halogen atom (e.g., fluorine atom),
(10) a C₁₋₆ alkyl-carbonyl group (e.g., acetyl),
(11) a C₁₋₆ alkoxy-carbonyl group (e.g., methoxycarbonyl, ethoxycarbonyl),
(12) an aromatic heterocyclic group (e.g., oxazolyl, pyrazolyl, triazolyl, oxadiazolyl, isoxazolyl, furyl) optionally substituted by 1 to 3 substituents selected from
   (a) a C₁₋₆ alkyl group (e.g., methyl) optionally substituted by 1 to 3 substituents selected from
      (i) a hydroxy group, and
      (ii) a halogen atom (e.g., fluorine atom), and
   (b) a carbamoyl group,
(13) a non-aromatic heterocyclic group (e.g., morpholinyl, dihydrooxadiazolyl, dihydrothiadiazolyl, dihydrooxazolyl, tetrahydrooxazolyl, oxetanyl, azetidinyl, pyrrolidinyl, 1,1-dioxidotetrahydroisothiazolyl, piperidyl, piperazinyl, dihydropyranyl, thiomorpholinyl, tetrahydroimidazolyl) optionally substituted by 1 to 3 substituents selected from
   (a) a hydroxy group,
   (b) an oxo group,
   (c) a thioxo group,
   (d) a C₁₋₆ alkyl group (e.g., methyl), and
   (e) an aromatic heterocyclyl-carbonyl group (e.g., imidazolylcarbonyl),
(14) an amino group optionally mono- or di-substituted by substituent(s) selected from
   (a) a C₁₋₆ alkyl group (e.g., methyl),
   (b) a C₃₋₁₀ cycloalkyl-carbonyl group (e.g., cyclopropylcarbonyl), and
   (c) a C₃₋₁₀ cycloalkylsulfonyl group (e.g., cyclopropylsulfonyl),
(15) a carbamoyl group optionally mono- or di-substituted by substituent(s) selected from
   (a) C₁₋₆ alkyl group (e.g., methyl, ethyl, propyl, isopropyl, isobutyl) optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₁₀ cycloalkyl group (e.g., cyclopropyl),
   (c) a C₁₋₆ alkylsulfonyl group (e.g., methylsulfonyl),
   (d) a C₆₋₁₄ aryl group (e.g., phenyl),
   (e) an aromatic heterocyclic group (e.g., pyridyl), and
   (f) a non-aromatic heterocyclic group (e.g., oxetanyl),
(16) an aromatic heterocyclyloxy group (e.g., pyrazolyloxy) optionally substituted by 1 to 3 C₁₋₆ alkyl groups (e.g., methyl),
(17) a non-aromatic heterocyclyl-carbonyl group (e.g., morpholinylcarbonyl, piperidylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom (e.g., fluorine atom), and
   (c) a C₁₋₆ alkoxy group (e.g., methoxy),
(18) a C₆₋₁₄ arylsulfonyloxy group (e.g., phenylsulfonyloxy) optionally substituted by 1 to 3 C₁₋₆ alkyl groups (e.g., methyl), and
(19) an oxo group (preferably, pyrrole, thiazole, pyrazole, pyridine, pyrimidine, dihydropyridine or benzene, each of which is optionally substituted by the above-mentioned substituents, more preferably, pyridine or benzene, each of which is optionally substituted by the above-mentioned substituents).

Ring A is more preferably a 6-membered nitrogen-containing heterocycle (preferably, pyridine, pyrimidine, more preferably pyridine) or benzene, each of which is optionally substituted by 1 to 4 substituents selected from
(1) a halogen atom (e.g., fluorine atom, chlorine atom, bromine atom),
(2) a carboxy group,
(3) a cyano group,
(4) C₁₋₆ alkyl group (e.g., methyl, ethyl, isopropyl) optionally substituted by 1 to 3 substituents selected from
   (a) a hydroxy group,
   (b) a halogen atom (e.g., fluorine atom), and
   (c) a carboxy group,
(5) a C₂₋₆ alkenyl group (e.g., vinyl) optionally substituted by 1 to 3 substituents selected from
   (a) a carboxy group, and
   (b) a C₁₋₆ alkoxy-carbonyl group (e.g., methoxycarbonyl),
(6) a C₃₋₁₀ cycloalkyl group (e.g., cyclopropyl),
(7) a C₁₋₆ alkoxy group (e.g., methoxy, ethoxy) optionally substituted by 1 to 3 substituents selected from
   (a) a C₁₋₆ alkoxy group (e.g., methoxy), and
   (b) a halogen atom (e.g., fluorine atom),
(8) a C₁₋₆ alkyl-carbonyl group (e.g., acetyl),
(9) a C₁₋₆ alkoxy-carbonyl group (e.g., methoxycarbonyl, ethoxycarbonyl),
(10) an aromatic heterocyclic group (e.g., oxazolyl, pyrazolyl, triazolyl, oxadiazolyl, isoxazolyl, furyl) optionally substituted by 1 to 3 substituents selected from
   (a) a C₁₋₆ alkyl group (e.g., methyl) optionally substituted by 1 to 3 substituents selected from
      (i) a hydroxy group, and
      (ii) a halogen atom (e.g., fluorine atom), and
   (b) a carbamoyl group,
(11) a non-aromatic heterocyclic group (e.g., morpholinyl, dihydrooxadiazolyl, dihydrothiadiazolyl, tetrahydrooxazolyl, oxetanyl, azetidinyl, pyrrolidinyl, 1,1-dioxidotetrahydroisothiazolyl, piperidyl, piperazinyl, dihydropyranyl, thiomorpholinyl, tetrahydroimidazolyl) optionally substituted by 1 to 3 substituents selected from
   (a) a hydroxy group,
   (b) an oxo group,
   (c) a thioxo group, and
   (d) an aromatic heterocyclyl-carbonyl group (e.g., imidazolylcarbonyl),
(12) an amino group optionally mono- or di-substituted by a C₁₋₆ alkyl group (e.g., methyl),
(13) a carbamoyl group optionally mono- or di-substituted by substituent(s) selected from
   (a) C₁₋₆ alkyl group (e.g., methyl, ethyl, isopropyl, isobutyl) optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₁₀ cycloalkyl group (e.g., cyclopropyl), and
   (c) a non-aromatic heterocyclic group (e.g., oxetanyl),
(14) an aromatic heterocyclyloxy group (e.g., pyrazolyloxy) optionally substituted by 1 to 3 C₁₋₆ alkyl groups (e.g., methyl),
(15) a non-aromatic heterocyclyl-carbonyl group (e.g., morpholinylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom (e.g., fluorine atom), and
   (c) a C₁₋₆ alkoxy group (e.g., methoxy), and
(16) an oxo group,
(preferably, pyridine, pyrimidine or benzene, each of which is optionally substituted by the above-mentioned substituents, more preferably, pyridine or benzene, each of which is optionally substituted by the above-mentioned substituents).

Ring B is 6-membered aromatic heterocycle or benzene.

Ring B is preferably pyridine or benzene.

Ring B is more preferably benzene.

Ring C is optionally further substituted 5- or 6-membered heterocycle, or benzene further having substituent(s).

The "5- or 6-membered heterocycle" of the "optionally substituted 5- or 6-membered heterocycle", and the "benzene" of the "optionally substituted benzene" for ring C optionally have 1 to 5 (preferably 1 to 3) substituents at substitutable position(s). Examples of the substituent include those selected from the above-mentioned Substituent Group B. When plural substituents are present, the respective substituents may be the same or different.

The "5- or 6-membered heterocycle" of the "optionally further substituted 5- or 6-membered heterocycle" represented by ring C is preferably 5- or 6-membered aromatic heterocycle, more preferably thiophene, thiazole, pyridine, pyrimidine, and particularly preferably pyridine.

Ring C is preferably
(1) a 5- or 6-membered heterocycle (preferably 5- or 6-membered aromatic heterocycle, e.g., thiophene, thiazole, pyridine, pyrimidine, preferably pyridine) optionally substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy); or
(2) benzene substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy).
Ring C is more preferably
(1) a 5- or 6-membered heterocycle (preferably 5- or 6-membered aromatic heterocycle, e.g., pyridine) optionally substituted by 1 to 4 substituents selected from
   (a) a halogen atom (e.g., fluorine atom), and
   (b) a C₁₋₆ alkoxy group (e.g., methoxy); or
(2) benzene substituted by 1 to 4 halogen atoms (e.g., fluorine atom).

X is C(=O) or CH₂.

X is preferably CH₂.

When the atom of ring B to which R², R³, R⁴ or R⁵ is bonded is -N=, R², R³, R⁴ and R⁵ are absent.

In the present invention, an embodiment wherein R⁵ and R⁶ form a dihydropyran structure together with a carbon atom bonded thereto is preferable. That is, a compound represented by the formula (IA); 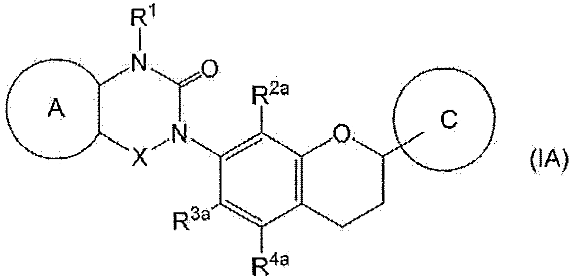 wherein
X is C(=O) or CH₂;
ring A is optionally substituted 5-membered nitrogen-containing heterocycle, optionally substituted 6-membered heterocycle, or optionally substituted benzene;
ring C is optionally further substituted 5- or 6-membered heterocycle, or benzene further having substituent(s); R¹ is a hydrogen atom or an optionally substituted C₁₋₆ alkyl group;
R^{2a} is a hydrogen atom or an optionally substituted C₁₋₆ alkyl group;
R^{3a} is a hydrogen atom or a substituent; and
R^{4a} is a hydrogen atom or an optionally substituted C₁₋₆ alkyl group,
or a salt thereof (compound (IA)).

The "C₁₋₆ alkyl group" of the "optionally substituted C₁₋₆ alkyl group" for R^{2a} optionally has 1 to 5 (preferably 1 to 3) substituents at substitutable position(s). Examples of the substituent include those selected from the above-mentioned Substituent Group A. When plural substituents are present, the respective substituents may be the same or different.

R^{2a} is preferably a hydrogen atom or a C₁₋₆ alkyl group.

R^{2a} is particularly preferably a hydrogen atom.

Examples of the "substituent" for R^{3a} include those similar to the "substituents" for R³.

R^{3a} is preferably a hydrogen atom, a halogen atom, a cyano group, an optionally substituted hydrocarbon group (preferably, C₁₋₆ alkyl group, C₂₋₆ alkenyl group, C₃₋₁₀ cycloalkyl group), an acyl group (preferably, carboxy group, C₁₋₆ alkoxy-carbonyl group, optionally substituted carbamoyl group) or the like.

R^{3a} is more preferably
(1) a hydrogen atom,
(2) a cyano group,
(3) a halogen atom (e.g., fluorine atom, chlorine atom, bromine atom),
(4) a C₁₋₆ alkyl group (e.g., methyl, ethyl, propyl, butyl, 3-methylbutyl, neopentyl) optionally substituted by 1 to 3 substituents selected from
   (a) a hydroxy group,
   (b) a halogen atom (e.g., fluorine atom),
   (c) a carboxy group,
   (d) a C₁₋₆ alkoxy-carbonyl group (e.g., ethoxycarbonyl, butoxycarbonyl),
   (e) a C₃₋₁₀ cycloalkyl group (e.g., cyclohexyl),
   (f) a C₆₋₁₄ aryl group (e.g., phenyl),
   (g) an aromatic heterocyclic group (e.g., pyridyl),
   (h) a non-aromatic heterocyclic group (e.g., pyrrolidinyl, morpholinyl, piperazinyl) optionally substituted by 1 to 3 C₁₋₆ alkyl groups (e.g., propyl),
   (i) an amino group optionally mono- or di-substituted by a C₃₋₁₀ cycloalkyl group (e.g., cyclopropyl), and
   (j) a carbamoyl group optionally mono- or di-substituted by a C₃₋₁₀ cycloalkyl group (e.g., cyclopropyl),
(5) a C₂₋₆ alkenyl group (e.g., vinyl) optionally substituted by 1 to 3 C₁₋₆ alkoxy-carbonyl groups (e.g., butoxycarbonyl),
(6) a C₃₋₁₀ cycloalkyl group (e.g., cyclopropyl),
(7) a C₁₋₆ alkoxy-carbonyl group (e.g., methoxycarbonyl), or
(8) a carbamoyl group optionally mono- or di-substituted by substituent(s) selected from
   (a) a C₁₋₆ alkyl group (e.g., methyl, isobutyl) optionally substituted by 1 to 3 substituents selected from
      (i) a hydroxy group, and
      (ii) an aromatic heterocyclic group (e.g., pyridyl), and
   (b) a C₃₋₁₀ cycloalkyl group (e.g., cyclopropyl).

R^{3a} is particularly preferably
(1) a hydrogen atom,
(2) a halogen atom (e.g., fluorine atom, chlorine atom, bromine atom), or
(3) a C₁₋₆ alkyl group (e.g., methyl) optionally substituted by 1 to 3 halogen atoms (e.g., fluorine atom).

The "C₁₋₆ alkyl group" of the "optionally substituted C₁₋₆ alkyl group" for R^{4a} optionally has 1 to 5 (preferably 1 to 3) substituents at substitutable position(s). Examples of the substituent include those selected from the above-mentioned Substituent Group A. When plural substituents are present, the respective substituents may be the same or different.

R^{4a} is preferably a hydrogen atom or a C₁₋₆ alkyl group.

R^{4a} is particularly preferably a hydrogen atom.

Specific preferable examples of compound (I) include as follows:

### [Compound A]

Compound (I) wherein
X is C(=O) or CH₂;
ring A is a 5-membered nitrogen-containing aromatic heterocycle (preferably, pyrrole, thiazole, pyrazole), 6-membered nitrogen-containing heterocycle (preferably, pyridine, pyrimidine, dihydropyridine, more preferably pyridine) or benzene, each of which is optionally substituted by 1 to 4 substituents selected from
(1) a halogen atom (e.g., fluorine atom, chlorine atom, bromine atom),
(2) a hydroxy group,
(3) a carboxy group,
(4) a cyano group,
(5) a nitro group,
(6) a C₁₋₆ alkyl group (e.g., methyl, ethyl, isopropyl) optionally substituted by 1 to 3 substituents selected from
   (a) a hydroxy group,
   (b) a halogen atom (e.g., fluorine atom),
   (c) an amino group,
   (d) a carboxy group,
   (e) a C₁₋₆ alkoxy group (e.g., methoxy), and
   (f) a C₃₋₁₀ cycloalkyl group (e.g., cyclopropyl),
(7) a C₂₋₆ alkenyl group (e.g., vinyl) optionally substituted by 1 to 3 substituents selected from
   (a) a carboxy group, and
   (b) a C₁₋₆ alkoxy-carbonyl group (e.g., methoxycarbonyl),
(8) a C₃₋₁₀ cycloalkyl group (e.g., cyclopropyl),
(9) a C₁₋₆ alkoxy group (e.g., methoxy, ethoxy) optionally substituted by 1 to 3 substituents selected from
   (a) a C₃₋₁₀ cycloalkyl group (e.g., cyclopropyl),
   (b) a C₁₋₆ alkoxy group (e.g., methoxy), and
   (c) a halogen atom (e.g., fluorine atom),
(10) a C₁₋₆ alkyl-carbonyl group (e.g., acetyl),
(11) a C₁₋₆ alkoxy-carbonyl group (e.g., methoxycarbonyl, ethoxycarbonyl),
(12) an aromatic heterocyclic group (e.g., oxazolyl, pyrazolyl, triazolyl, oxadiazolyl, isoxazolyl, furyl) optionally substituted by 1 to 3 substituents selected from
   (a) a C₁₋₆ alkyl group (e.g., methyl) optionally substituted by 1 to 3 substituents selected from
      (i) a hydroxy group, and
      (ii) a halogen atom (e.g., fluorine atom), and
   (b) a carbamoyl group,
(13) a non-aromatic heterocyclic group (e.g., morpholinyl, dihydrooxadiazolyl, dihydrothiadiazolyl, dihydrooxazolyl, tetrahydrooxazolyl, oxetanyl, azetidinyl, pyrrolidinyl, 1,1-dioxidotetrahydroisothiazolyl, piperidyl, piperazinyl, dihydropyranyl, thiomorpholinyl, tetrahydroimidazolyl) optionally substituted by 1 to 3 substituents selected from
   (a) a hydroxy group,
   (b) an oxo group,
   (c) a thioxo group,
   (d) a C₁₋₆ alkyl group (e.g., methyl), and
   (e) an aromatic heterocyclyl-carbonyl group (e.g., imidazolylcarbonyl),
(14) an amino group optionally mono- or di-substituted by substituent(s) selected from
   (a) a C₁₋₆ alkyl group (e.g., methyl),
   (b) a C₃₋₁₀ cycloalkyl-carbonyl group (e.g., cyclopropylcarbonyl), and
   (c) a C₃₋₁₀ cycloalkylsulfonyl group (e.g., cyclopropylsulfonyl),
(15) a carbamoyl group optionally mono- or di-substituted by substituent(s) selected from
   (a) a C₁₋₆ alkyl group (e.g., methyl, ethyl, propyl, isopropyl, isobutyl) optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₁₀ cycloalkyl group (e.g., cyclopropyl),
   (c) a C₁₋₆ alkylsulfonyl group (e.g., methylsulfonyl),
   (d) a C₆₋₁₄ aryl group (e.g., phenyl),
   (e) an aromatic heterocyclic group (e.g., pyridyl), and
   (f) a non-aromatic heterocyclic group (e.g., oxetanyl),
(16) an aromatic heterocyclyloxy group (e.g., pyrazolyloxy) optionally substituted by 1 to 3 C₁₋₆ alkyl groups (e.g., methyl),
(17) a non-aromatic heterocyclyl-carbonyl group (e.g., morpholinylcarbonyl, piperidylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom (e.g., fluorine atom), and
   (c) a C₁₋₆ alkoxy group (e.g., methoxy),
(18) a C₆₋₁₄ arylsulfonyloxy group (e.g., phenylsulfonyloxy) optionally substituted by 1 to 3 C₁₋₆ alkyl groups (e.g., methyl), and
(19) an oxo group,
   (preferably, pyrrole, thiazole, pyrazole, pyridine, pyrimidine, dihydropyridine or benzene, each of which is optionally substituted by the above-mentioned substituents, more preferably, pyridine or benzene, each of which is optionally substituted by the above-mentioned substituents);
ring B is pyridine or benzene;
ring C is
(1) a 5- or 6-membered heterocycle (preferably 5- or 6-membered aromatic heterocycle, e.g., thiophene, thiazole, pyridine, pyrimidine, preferably pyridine) optionally substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy); or
(2) benzene substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy);
R¹ is
(1) a hydrogen atom, or
(2) a C₁₋₆ alkyl group (e.g., methyl, ethyl) optionally substituted by 1 to 3 substituents selected from
   (a) a carboxy group,
   (b) a C₃₋₁₀ cycloalkyl group (e.g., cyclopropyl),
   (c) a C₁₋₆ alkoxy group (e.g., methoxy),
   (d) a C₆₋₁₄ aryl group (e.g., phenyl) optionally substituted by 1 to 3 substituents selected from
      (i) a C₁₋₆ alkoxy group (e.g., methoxy),
      (ii) a carboxy group, and
      (iii) a C₁₋₆ alkoxy-carbonyl group (e.g., methoxycarbonyl), and
   (e) a carbamoyl group;
R² is a hydrogen atom or a C₁₋₆ alkyl group;
R³ is
(1) a hydrogen atom,
(2) a cyano group,
(3) a halogen atom (e.g., fluorine atom, chlorine atom, bromine atom),
(4) a C₁₋₆ alkyl group (e.g., methyl, ethyl, propyl, butyl, 3-methylbutyl, neopentyl) optionally substituted by 1 to 3 substituents selected from
   (a) a hydroxy group,
   (b) a halogen atom (e.g., fluorine atom),
   (c) a carboxy group,
   (d) a C₁₋₆ alkoxy-carbonyl group (e.g., ethoxycarbonyl, butoxycarbonyl),
   (e) a C₃₋₁₀ cycloalkyl group (e.g., cyclohexyl),
   (f) a C₆₋₁₄ aryl group (e.g., phenyl),
   (g) an aromatic heterocyclic group (e.g., pyridyl),
   (h) a non-aromatic heterocyclic group (e.g., pyrrolidinyl, morpholinyl, piperazinyl) optionally substituted by 1 to 3 C₁₋₆ alkyl groups (e.g., propyl),
   (i) an amino group optionally mono- or di-substituted by a C₃₋₁₀ cycloalkyl group (e.g., cyclopropyl), and
   (j) a carbamoyl group optionally mono- or di-substituted by a C₃₋₁₀ cycloalkyl group (e.g., cyclopropyl),
(5) a C₂₋₆ alkenyl group (e.g., vinyl) optionally substituted by 1 to 3 C₁₋₆ alkoxy-carbonyl groups (e.g., butoxycarbonyl),
(6) a C₃₋₁₀ cycloalkyl group (e.g., cyclopropyl),
(7) a C₁₋₆ alkoxy-carbonyl group (e.g., methoxycarbonyl), or
(8) a carbamoyl group optionally mono- or di-substituted by substituent(s) selected from
   (a) a C₁₋₆ alkyl group (e.g., methyl, isobutyl) optionally substituted by 1 to 3 substituents selected from
      (i) a hydroxy group, and
      (ii) an aromatic heterocyclic group (e.g., pyridyl), and
   (b) a C₃₋₁₀ cycloalkyl group (e.g., cyclopropyl), or absent;
R⁴ is a hydrogen atom or a C₁₋₆ alkyl group;
R⁵ is
(1) a hydrogen atom,
(2) a halogen atom (e.g., fluorine atom, chlorine atom, iodine atom), or
(3) a C₁₋₆ alkyl group; and
R⁶ is
(1) a hydrogen atom, or
(2) a C₁₋₆ alkyl group (e.g., methyl); or,
R⁵ and R⁶ form a dihydropyran structure together with a carbon atom bonded thereto.

### [Compound B]

Compound (IA) wherein
X is C(=O) or CH₂;
ring A is a 5-membered nitrogen-containing aromatic heterocycle (preferably, pyrrole, thiazole, pyrazole), 6-membered nitrogen-containing heterocycle(preferably, pyridine, pyrimidine, dihydropyridine, more preferably pyridine) or benzene, each of which is optionally substituted by 1 to 4 substituents selected from
(1) a halogen atom (e.g., fluorine atom, chlorine atom, bromine atom),
(2) a hydroxy group,
(3) a carboxy group,
(4) a cyano group,
(5) a nitro group,
(6) a C₁₋₆ alkyl group (e.g., methyl, ethyl, isopropyl) optionally substituted by 1 to 3 substituents selected from
   (a) a hydroxy group,
   (b) a halogen atom (e.g., fluorine atom),
   (c) an amino group,
   (d) a carboxy group,
   (e) a C₁₋₆ alkoxy group (e.g., methoxy), and
   (f) a C₃₋₁₀ cycloalkyl group (e.g., cyclopropyl),
(7) a C₂₋₆ alkenyl group (e.g., vinyl) optionally substituted by 1 to 3 substituents selected from
   (a) a carboxy group, and
   (b) a C₁₋₆ alkoxy-carbonyl group (e.g., methoxycarbonyl),
(8) a C₃₋₁₀ cycloalkyl group (e.g., cyclopropyl),
(9) a C₁₋₆ alkoxy group (e.g., methoxy, ethoxy) optionally substituted by 1 to 3 substituents selected from
   (a) a C₃₋₁₀ cycloalkyl group (e.g., cyclopropyl),
   (b) a C₁₋₆ alkoxy group (e.g., methoxy), and
   (c) a halogen atom (e.g., fluorine atom),
(10) a C₁₋₆ alkyl-carbonyl group (e.g., acetyl),
(11) a C₁₋₆ alkoxy-carbonyl group (e.g., methoxycarbonyl, ethoxycarbonyl),
(12) an aromatic heterocyclic group (e.g., oxazolyl, pyrazolyl, triazolyl, oxadiazolyl, isoxazolyl, furyl) optionally substituted by 1 to 3 substituents selected from
   (a) a C₁₋₆ alkyl group (e.g., methyl) optionally substituted by 1 to 3 substituents selected from
      (i) a hydroxy group, and
      (ii) a halogen atom (e.g., fluorine atom), and
   (b) a carbamoyl group,
(13) a non-aromatic heterocyclic group (e.g., morpholinyl, dihydrooxadiazolyl, dihydrothiadiazolyl, dihydrooxazolyl, tetrahydrooxazolyl, oxetanyl, azetidinyl, pyrrolidinyl, 1,1-dioxidotetrahydroisothiazolyl, piperidyl, piperazinyl, dihydropyranyl, thiomorpholinyl, tetrahydroimidazolyl) optionally substituted by 1 to 3 substituents selected from
   (a) a hydroxy group,
   (b) an oxo group,
   (c) a thioxo group,
   (d) a C₁₋₆ alkyl group (e.g., methyl), and
   (e) an aromatic heterocyclyl-carbonyl group (e.g., imidazolylcarbonyl),
(14) an amino group optionally mono- or di-substituted by substituent(s) selected from
   (a) a C₁₋₆ alkyl group (e.g., methyl),
   (b) a C₃₋₁₀ cycloalkyl-carbonyl group (e.g., cyclopropylcarbonyl), and
   (c) a C₃₋₁₀ cycloalkylsulfonyl group (e.g., cyclopropylsulfonyl),
(15) a carbamoyl group optionally mono- or di-substituted by substituent(s) selected from
   (a) a C₁₋₆ alkyl group (e.g., methyl, ethyl, propyl, isopropyl, isobutyl) optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₁₀ cycloalkyl group (e.g., cyclopropyl),
   (c) a C₁₋₆ alkylsulfonyl group (e.g., methylsulfonyl),
   (d) a C₆₋₁₄ aryl group (e.g., phenyl),
   (e) an aromatic heterocyclic group (e.g., pyridyl), and
   (f) a non-aromatic heterocyclic group (e.g., oxetanyl),
(16) an aromatic heterocyclyloxy group (e.g., pyrazolyloxy) optionally substituted by 1 to 3 C₁₋₆ alkyl groups (e.g., methyl),
(17) a non-aromatic heterocyclyl-carbonyl group (e.g., morpholinylcarbonyl, piperidylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom (e.g., fluorine atom), and
   (c) a C₁₋₆ alkoxy group (e.g., methoxy),
(18) a C₆₋₁₄ arylsulfonyloxy group (e.g., phenylsulfonyloxy) optionally substituted by 1 to 3 C₁₋₆ alkyl groups (e.g., methyl), and
(19) an oxo group,
(preferably, pyrrole, thiazole, pyrazole, pyridine, pyrimidine, dihydropyridine or benzene, each of which is optionally substituted by the above-mentioned substituents, more preferably, pyridine or benzene, each of which is optionally substituted by the above-mentioned substituents);
ring C is
(1) a 5- or 6-membered heterocycle (preferably 5- or 6-membered aromatic heterocycle, e.g., thiophene, thiazole, pyridine, pyrimidine, preferably pyridine) optionally substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy); or
(2) benzene substituted by 1 to 4 substituents selected from
   (a) a halogen atom (e.g., fluorine atom, chlorine atom, bromine atom),
   (b) a C₁₋₆ alkyl group (e.g., methyl), and
   (c) a C₁₋₆ alkoxy group (e.g., methoxy);
R¹ is
(1) a hydrogen atom, or
(2) a C₁₋₆ alkyl group (e.g., methyl, ethyl) optionally substituted by 1 to 3 substituents selected from
   (a) a carboxy group,
   (b) a C₃₋₁₀ cycloalkyl group (e.g., cyclopropyl),
   (c) a C₁₋₆ alkoxy group (e.g., methoxy),
   (d) a C₆₋₁₄ aryl group (e.g., phenyl) optionally substituted by 1 to 3 substituents selected from
      (i) a C₁₋₆ alkoxy group (e.g., methoxy),
      (ii) a carboxy group, and
      (iii) a C₁₋₆ alkoxy-carbonyl group (e.g., methoxycarbonyl), and
   (e) a carbamoyl group;
R^{2a} is a hydrogen atom;
R^{3a} is
(1) a hydrogen atom,
(2) a cyano group,
(3) a halogen atom (e.g., fluorine atom, chlorine atom, bromine atom),
(4) a C₁₋₆ alkyl group (e.g., methyl, ethyl, propyl, butyl, 3-methylbutyl, neopentyl) optionally substituted by 1 to 3 substituents selected from
   (a) a hydroxy group,
   (b) a halogen atom (e.g., fluorine atom),
   (c) a carboxy group,
   (d) a C₁₋₆ alkoxy-carbonyl group (e.g., ethoxycarbonyl, butoxycarbonyl),
   (e) a C₃₋₁₀ cycloalkyl group (e.g., cyclohexyl),
   (f) a C₆₋₁₄ aryl group (e.g., phenyl),
   (g) an aromatic heterocyclic group (e.g., pyridyl),
   (h) a non-aromatic heterocyclic group (e.g., pyrrolidinyl, morpholinyl, piperazinyl) optionally substituted by 1 to 3 C₁₋₆ alkyl groups (e.g., propyl),
   (i) an amino group optionally mono- or di-substituted by a C₃₋₁₀ cycloalkyl group (e.g., cyclopropyl), and
   (j) a carbamoyl group optionally mono- or di-substituted by a C₃₋₁₀ cycloalkyl group (e.g., cyclopropyl),
(5) a C₂₋₆ alkenyl group (e.g., vinyl) optionally substituted by 1 to 3 C₁₋₆ alkoxy-carbonyl groups (e.g., butoxycarbonyl),
(6) a C₃₋₁₀ cycloalkyl group (e.g., cyclopropyl),
(7) a C₁₋₆ alkoxy-carbonyl group (e.g., methoxycarbonyl), or
(8) a carbamoyl group optionally mono- or di-substituted by substituent(s) selected from
   (a) a C₁₋₆ alkyl group (e.g., methyl, isobutyl) optionally substituted by 1 to 3 substituents selected from
      (i) a hydroxy group, and
      (ii) an aromatic heterocyclic group (e.g., pyridyl), and
   (b) a C₃₋₁₀ cycloalkyl group (e.g., cyclopropyl) ; and
R^{4a} is a hydrogen atom.

### [Compound C]

Compound (IA) wherein
X is C(=O) or CH₂;
ring A is a 6-membered nitrogen-containing heterocycle (preferably, pyridine, pyrimidine, more preferably pyridine) or benzene, each of which is optionally substituted by 1 to 4 substituents selected from
(1) a halogen atom (e.g., fluorine atom, chlorine atom, bromine atom),
(2) a carboxy group,
(3) a cyano group,
(4) a C₁₋₆ alkyl group (e.g., methyl, ethyl, isopropyl) optionally substituted by 1 to 3 substituents selected from
   (a) a hydroxy group,
   (b) a halogen atom (e.g., fluorine atom), and
   (c) a carboxy group,
(5) a C₂₋₆ alkenyl group (e.g., vinyl) optionally substituted by 1 to 3 substituents selected from
   (a) a carboxy group, and
   (b) a C₁₋₆ alkoxy-carbonyl group (e.g., methoxycarbonyl),
(6) a C₃₋₁₀ cycloalkyl group (e.g., cyclopropyl),
(7) a C₁₋₆ alkoxy group (e.g., methoxy, ethoxy) optionally substituted by 1 to 3 substituents selected from
   (a) a C₁₋₆ alkoxy group (e.g., methoxy), and
   (b) a halogen atom (e.g., fluorine atom),
(8) a C₁₋₆ alkyl-carbonyl group (e.g., acetyl),
(9) a C₁₋₆ alkoxy-carbonyl group (e.g., methoxycarbonyl, ethoxycarbonyl),
(10) an aromatic heterocyclic group (e.g., oxazolyl, pyrazolyl, triazolyl, oxadiazolyl, isoxazolyl, furyl) optionally substituted by 1 to 3 substituents selected from
   (a) a C₁₋₆ alkyl group (e.g., methyl) optionally substituted by 1 to 3 substituents selected from
      (i) a hydroxy group, and
      (ii) a halogen atom (e.g., fluorine atom), and
   (b) a carbamoyl group,
(11) a non-aromatic heterocyclic group (e.g., morpholinyl, dihydrooxadiazolyl, dihydrothiadiazolyl, tetrahydrooxazolyl, oxetanyl, azetidinyl, pyrrolidinyl, 1,1-dioxidotetrahydroisothiazolyl, piperidyl, piperazinyl, dihydropyranyl, thiomorpholinyl, tetrahydroimidazolyl) optionally substituted by 1 to 3 substituents selected from
   (a) a hydroxy group,
   (b) an oxo group,
   (c) a thioxo group, and
   (d) an aromatic heterocyclyl-carbonyl group (e.g., imidazolylcarbonyl),
(12) an amino group optionally mono- or di-substituted by a C₁₋₆ alkyl group (e.g., methyl),
(13) a carbamoyl group optionally mono- or di-substituted by substituent(s) selected from
   (a) a C₁₋₆ alkyl group (e.g., methyl, ethyl, isopropyl, isobutyl) optionally substituted by 1 to 3 substituents selected from
      (i) a hydroxy group,
      (ii) a carboxy group,
      (iii) a halogen atom (e.g., fluorine atom),
      (iv) a C₁₋₆ alkoxy group (e.g., methoxy), and
      (v) a C₁₋₆ alkylsulfinyl group (e.g., methylsulfinyl),
   (b) a C₃₋₁₀ cycloalkyl group (e.g., cyclopropyl), and
   (c) a non-aromatic heterocyclic group (e.g., oxetanyl),
(14) an aromatic heterocyclyloxy group (e.g., pyrazolyloxy) optionally substituted by 1 to 3 C₁₋₆ alkyl groups (e.g., methyl),
(15) a non-aromatic heterocyclyl-carbonyl group (e.g., morpholinylcarbonyl, azetidinylcarbonyl, 2-oxa-6-azaspiro[3.3]heptan-6-ylcarbonyl) optionally substituted by 1 to 3 substituents selected from
   (a) a hydroxy group,
   (b) a halogen atom (e.g., fluorine atom), and
   (c) a C₁₋₆ alkoxy group (e.g., methoxy), and
(16) an oxo group,
(preferably, pyridine, pyrimidine or benzene, each of which is optionally substituted by the above-mentioned substituents, more preferably, pyridine or benzene, each of which is optionally substituted by the above-mentioned substituents);
ring C is
(1) a 5- or 6-membered heterocycle (preferably 5- or 6-membered aromatic heterocycle, e.g., pyridine) optionally substituted by 1 to 4 substituents selected from
   (a) a halogen atom (e.g., fluorine atom), and
   (b) a C₁₋₆ alkoxy group (e.g., methoxy); or
(2) benzene substituted by 1 to 4 halogen atoms (e.g., fluorine atom);
R¹ is
(1) a hydrogen atom, or
(2) a C₁₋₆ alkyl group (e.g., methyl, ethyl) optionally substituted by 1 to 3 C₁₋₆ alkoxy groups (e.g., methoxy);
R^{2a} is a hydrogen atom;
R^{3a} is
(1) a hydrogen atom,
(2) a halogen atom (e.g., fluorine atom, chlorine atom, bromine atom), or
(3) a C₁₋₆ alkyl group (e.g., methyl) optionally substituted by 1 to 3 halogen atoms (e.g., fluorine atom); and
R^{4a} is a hydrogen atom.

### [Compound D]

Compounds described in Examples 1 - 373 or a salt thereof. Preferably, compounds described in Examples 1 - 169, 182 - 185, 326 - 329, 372 and 373, or a salt thereof.

### [Compound E]

5-chloro-3-((2S)-6-chloro-2-(3-fluoropyridin-2-yl)-3,4-dihydro-2H-chromen-7-yl)-8-methyl-3,4-dihydropyrido[3,4-d]pyrimidine-2(1H)-one or a salt thereof;
(+)-5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidine-2(1H)-one or a salt thereof; or
3-((2R)-2-(3-fluoropyridin-2-yl)-6-methyl-3,4-dihydro-2H-chromen-7-yl)-5-(trifluoromethyl)-3,4-dihydropyrido[4,3-d]pyrimidine-2(1H)-one or a salt thereof.

Examples of salts of compounds represented by the formula (I) include metal salt, ammonium salt, salt with organic base, salt with inorganic acid, salt with organic acid, salt with basic or acidic amino acid and the like. Preferable examples of the metal salt include alkali metal salts such as sodium salt, potassium salt and the like; alkaline earth metal salts such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salt with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like, and preferable examples of the salt with acidic amino acid include salts with aspartic acid, glutamic acid and the like.

Of these, a pharmaceutically acceptable salt is preferable. For example, when an acidic functional group is contained in the compound, inorganic salts such as alkali metal salt (e.g., sodium salt, potassium salt etc.), alkaline earth metal salt (e.g., calcium salt, magnesium salt etc.) and the like, ammonium salt and the like can be mentioned. In addition, when a basic functional group is contained in the compound, for example, salts with inorganic acids such as hydrochloric acid, hydrobromic acid, nitric acid, sulfuric acid, phosphoric acid and the like, or salts with organic acids such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

### [Production Method]

As examples of the production method of compound (I), representative production methods are described below, which are not to be construed as limitative. Compound (I) can also be produced by the methods shown in the following Production Methods 1 - 9 or a method analogous thereto or the methods described in the Examples and the like.

Unless particularly indicated, the reaction time of each reaction is generally 1 min - 200 hr.

Unless particularly indicated, the reaction temperature of each reaction is -100 - 300°C.

Examples of the base or deoxidizer include the following. inorganic bases: lithium hydroxide, sodium hydroxide, potassium hydroxide, calcium hydroxide, magnesium hydroxide, barium hydroxide and the like; basic salts: sodium carbonate, potassium carbonate, lithium carbonate, cesium carbonate, calcium carbonate, sodium hydrogen carbonate, potassium hydrogen carbonate, hydrogencarbonate lithium, calcium hydrogencarbonate, sodium phosphate, potassium phosphate, sodium acetate, potassium acetate, acetic acid cesium and the like; organic bases: triethylamine, diisopropylethylamine, tributylamine, cyclohexyldimethylamine, pyridine, picoline, lutidine, collidine, 4-dimethylaminopyridine, N,N-dimethylaniline, piperidine, N-methylpiperidine, N-methylpyrrolidine, N-methylmorpholine, 1,5-diazabicyclo[4.3.0]-5-nonene, 1,4-diazabicyclo[2.2.2]octane, 1,8-diazabicyclo[5.4.0]-7-undecene, tetramethylethylenediamine, imidazole and the like; metal alkoxides: sodium methoxide, sodium ethoxide, potassium tert-butoxide and the like; alkali metal hydrides: sodium hydride, potassium hydride and the like; metal amides: sodium amide, lithiumdiisopropylamide, lithiumhexamethyl disilazide and the like; organic lithium reagents: methyllithium, n-butyllithium, sec-butyllithium, tert-butyllithium and the like.

Unless particularly indicated, the equivalent of the reagents and reactants used in each reaction is 0.001 mol equivalents - 100 mol equivalents relative to the substrate of each reaction.

### Production Method 1

Of compounds (I) of the present invention, compound (Ia) wherein X is carbonyl (C=O) and R¹ is a hydrogen atom can be produced, for example, by the method of Production Method 1. 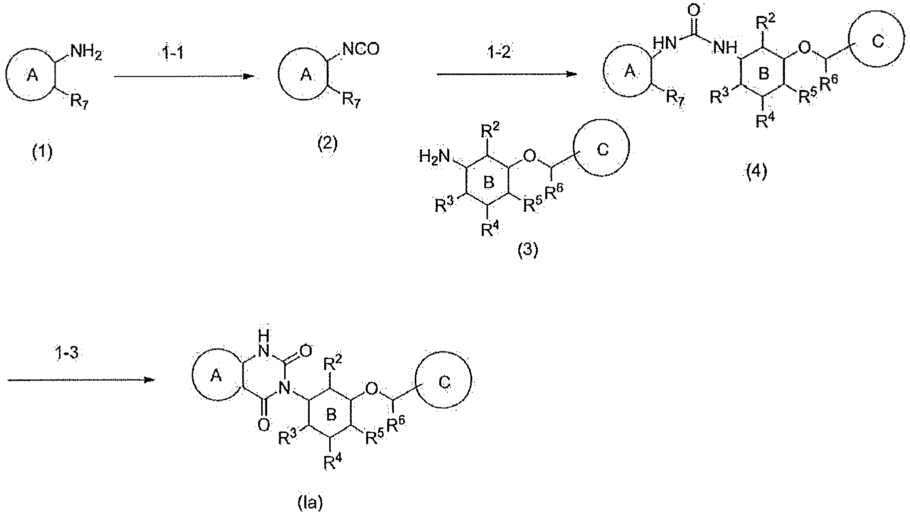 wherein R⁷ is a C₁₋₆ alkoxycarbonyl group, and other groups are as defined above.

### step 1-1

Amine compound (1) can be converted to isocyanate compound (2) by a treatment with a reagent such as triphosgene, diphenylphosphoryl azide and the like in an inert solvent (e.g., tetrahydrofuran, toluene, or a mixed solvent thereof) in the presence of a base at generally -78°C - 200°C for 1 hr - 1 day. The synthesized isocyanate compound (2) is sometimes used in one-pot, without isolation, for the next step 1-2.

Amine compound (1) used as a starting compound is, for example, a commercially available product, or can be synthesized according to the method described in a document or a method combining general synthesis methods and the like.

### step 1-2

Isocyanate compound (2) can be converted to urea compound (4) by a treatment with amine compound (3) in an inert solvent (e.g., tetrahydrofuran, toluene, or a mixed solvent thereof) in the presence or absence of a base at generally -20°C - 200°C for 1 hr - 1 day.

### step 1-3

Compound (Ia) can be produced by a treatment with urea compound (4) in an inert solvent (e.g., tetrahydrofuran, toluene, or a mixed solvent thereof) in the presence or absence of a base at generally -20°C - 200°C for 1 hr - 1 day.

### Production Method 2

Of compounds (I) of the present invention, compound (Ia) wherein X is carbonyl(C=O) and R¹ is a hydrogen atom can also be produced, for example, by the method of Production Method 2. 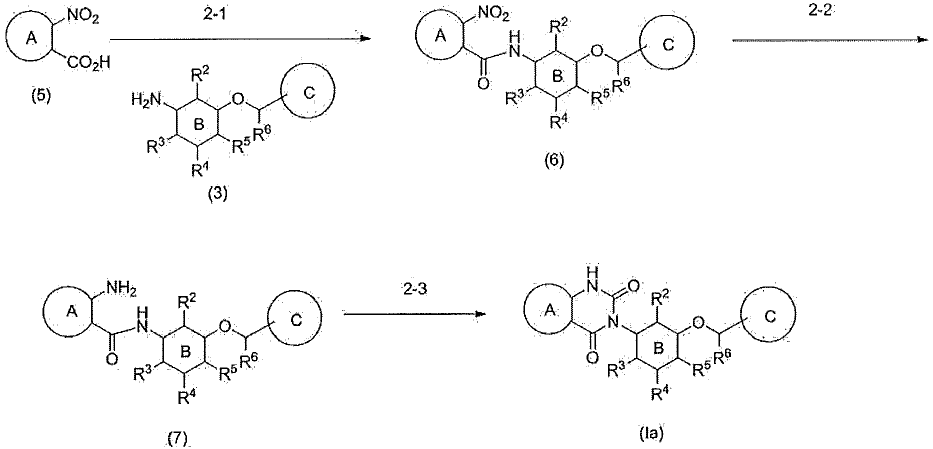 wherein the groups are as defined above.

### step 2-1

Carboxylic acid compound (5) and amine compound (3) are condensed by a general acid chloride method or condensing agent method to produce amide compound (6). In the acid chloride method, for example, acid chloride is obtained by a treatment of carboxylic acid compound (5) in an inert solvent (e.g., tetrahydrofuran, toluene, or a mixed solvent thereof) using a reagent such as thionyl chloride, oxalyl chloride and the like in the presence or absence of an additive (e.g., N,N-dimethylformamide and the like) at generally -20°C - 200°C for 1 hr - 1 day, and reacted with amine compound (3) in an inert solvent (e.g., tetrahydrofuran, pyridine, or a mixed solvent thereof) in the presence or absence of a base (e.g., triethylamine) at generally -20°C - 200°C for 1 hr - 1 day. In the condensing agent method, for example, carboxylic acid compound (5) and amine compound (3) are reacted in an inert solvent (e.g., N,N-dimethylformamide, acetonitrile, or a mixed solvent thereof) using a condensing agent (1-ethyl-3-(3-dimethylaminopropyl)carbodiimide hydrochloride and the like) in the presence or absence of an additive (1-hydroxybenzotriazole and the like) at generally -20°C - 200°C for 1 hr - 1 day.

Carboxylic acid compound (5) used as a starting compound is, for example, a commercially available product, or can be synthesized according to the method described in a document or a method combining general synthesis methods and the like.

### step 2-2

The nitro group of amide compound (6) is reduced by a general catalytic reduction method or a method using a metal and the like to produced amine compound (7). In the catalytic reduction method, for example, amide compound (6) is reacted under a hydrogen atmosphere in an inert solvent (e.g., methanol, ethanol, tetrahydrofuran, or a mixed solvent thereof) using a catalyst (palladium carbon powder and the like) at generally 0°C - 200°C for 1 hr - 1 day. In the method using a metal, for example, amide compound (6) is reacted in an inert solvent (e.g., ethanol, water, or a mixed solvent thereof) using a metal (reduced iron and the like) in the presence or absence of an additive (calcium chloride, ammonium chloride and the like) at generally -20°C - 200°C for 1 hr - 1 day.

### step 2-3

Amine compound (7) is treated in an inert solvent (e.g., tetrahydrofuran, N,N-dimethylformamide, or a mixed solvent thereof) using a reagent such as triphosgene, 1,1'-carbonyldiimidazole and the like in the presence or absence of a base (1,8-diazabicyclo[5.4.0]undec-7-ene, triethylamine and the like) at generally -20°C - 200°C for 1 hr - 1 day to produce compound (Ia).

### Production Method 3

Of compounds (I), compound (Ib) wherein X is methylene (CH₂) and R¹ is a hydrogen atom can be produced, for example, by the method of Production Method 3. 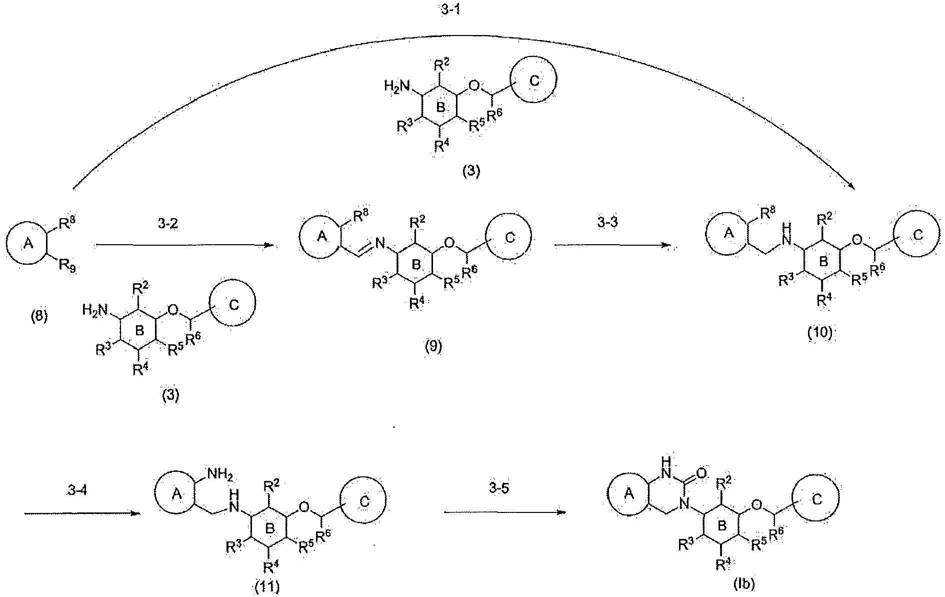 wherein R⁸ is an amino group, a protected amino group (e.g., protecting group is pivaloyl group, 4-methoxybenzyl group, t-butoxycarbonyl group and the like) or a nitro group, R⁹ is a formyl group or an acetal thereof (e.g., dimethylacetal and the like) or a methyl group optionally substituted by 1 or 3 halogen atoms, and other groups are as defined above.

### step 3-1

Compound (8) wherein R⁹ is a halogenomethyl group and amine compound (3) are treated in an inert solvent (e.g., N,N-dimethylacetamide) in the presence or absence of a basic additive (potassium carbonate and the like) at generally 0°C - 200°C for 1 hr - 1 day to produce amine compound (10).

### step 3-2

Compound (8) wherein R⁹ is a formyl group or acetal thereof (e.g., dimethylacetal and the like)) and amine compound (3) are treated in an inert solvent (e.g., toluene, tetrahydrofuran or a mixed solvent thereof) in the presence or absence of an acidic additive (e.g., acetic acid, p-toluenesulfonic acid monohydrate and the like) at generally - 20°C - 200°C for 1 hr - 1 day to produce imine compound (9). Synthesized imine compound (9) is sometimes used in one-pot, without isolation, for the next step 3-3.

### step 3-3

Imine compound (9) is reacted in an inert solvent (e.g., tetrahydrofuran, methanol, ethanol, toluene, or a mixed solvent thereof) using a metal hydride complex compound (sodium acetoxyborohydride, sodium borohydride, lithium aluminum hydride and the like) at generally -20°C - 200°C for 1 hr - 1 day to produce amine compound (10).

### step 3-4

When R⁸ of amine compound (10) is other than amino group, amine compound (11) can be produced by a general method. When R⁸ is a protected amino group, the compound is reacted in an inert solvent (e.g., acetic acid, water, or a mixed solvent thereof) with an acid (hydrochloric acid and the like) at generally 0°C - 200°C for 1 hr - 1 day. When R⁸ is a nitro group, the compound is reduced by a general catalytic reduction method or a method using a metal and the like. In the catalytic reduction method, for example, amine compound (10) reacted under a hydrogen atmosphere in an inert solvent (e.g., methanol, ethanol, tetrahydrofuran, or a mixed solvent thereof) using a catalyst (palladium carbon powder and the like) at generally 0°C - 200°C for 1 hr - 1 day. In the method using a metal, for example, amine compound (10) is reacted in an inert solvent (e.g., ethanol, water, or a mixed solvent thereof) using a metal (reduced iron and the like) in the presence or absence of an additive (calcium chloride and the like) at generally -20°C - 200°C for 1 hr - 1 day.

### step 3-5

Amine compound (11) is treated in an inert solvent (e.g., tetrahydrofuran, N,N-dimethylformamide, or a mixed solvent thereof) using a reagent such as triphosgene, 1,1'-carbonyldiimidazole and the like in the presence or absence of a base (1,8-diazabicyclo[5.4.0]undec-7-ene, triethylamine and the like) at generally -20°C - 200°C for 1 hr - 1 day to produce compound (Ib).

### Production Method 4

Amine compound (3) used as a starting compound in the above-mentioned Production Methods 1, 2 and 3 is, for example, a commercially available product, or can be obtained by reducing nitro compound (12), synthesized by the method described in a document or a method combining general synthesis methods and the like, by a general reduction method and the like. For example, it can be produced by the method of the following Production Method 4. 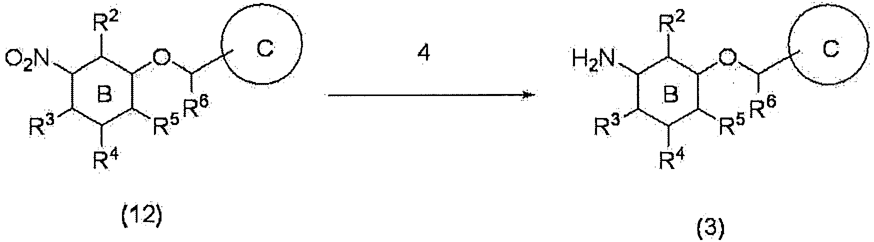 wherein the groups are as defined above.

### step 4

The nitro group of nitro compound (12) is reduced by a general catalytic reduction method or a method using a metal and the like to produced amine compound (3). In the catalytic reduction method, for example, nitro compound (12) is reacted under a hydrogen atmosphere in an inert solvent (e.g., methanol, ethanol, tetrahydrofuran, or a mixed solvent thereof) using a catalyst (palladium carbon powder and the like) at generally 0°C - 200°C for 1 hr - 1 day. In the method using a metal, for example, nitro compound (12) is reacted in an inert solvent (e.g., ethanol, water, or a mixed solvent thereof) using a metal (reduced iron and the like) in the presence or absence of an additive (calcium chloride and the like) at generally -20°C - 200°C for 1 hr - 1 day.

### Production Method 5

Of the amine compounds (3) used as a starting compound in the above-mentioned Production Methods 1, 2 and 3, amine compound (3a) wherein R⁵ and R⁶ form a tetrahydropyran ring can be produced by the method of the following Production Method 5. Compound (13) used as the starting compound is, for example, a commercially available product, or can be synthesized according to the method described in a document or a method combining general synthesis methods and the like. 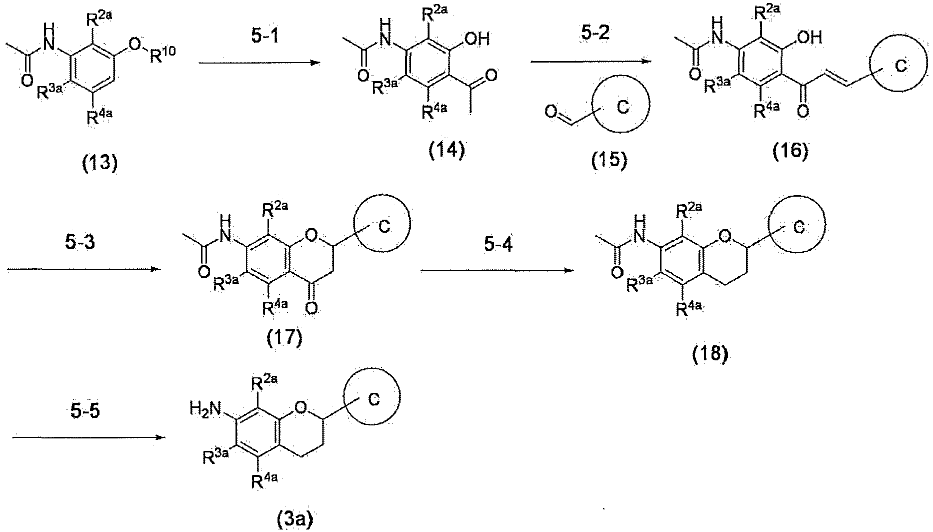 wherein R¹⁰ is an acetyl group or a C₁₋₆ alkyl group, and other groups are as defined above.

### step 5-1

When R¹⁰ of compound (13) is an acetyl group, acetophenone compound (14) can be produced using the Fries rearrangement reaction. For example, compound (13) is reacted in the presence of an additive (sodium chloride and the like) using Lewis acid (e.g., aluminum chloride and the like) at generally 100 - 200°C for 1 hr - 1 day. When R¹⁰ of compound (13) is a C₁₋₆ alkyl group, acetophenone compound (14) can be produced using the Friedel-Crafts reaction. For example, compound (13) is reacted with an acetylation reagent (e.g., acetyl chloride, acetic anhydride and the like) in an inert solvent (e.g., nitrobenzene and the like) using a Lewis acid (e.g., aluminum chloride and the like) at generally -20°C - 200°C for 1 hr - 1 day.

### step 5-2

Acetophenone compound (14) and formyl compound (15) are treated in an inert solvent (e.g., ethanol, water or a mixed solvent thereof) in the presence or absence of a base (e.g., sodium hydroxide and the like) at generally -20°C - 200°C for 1 hr - 1 day to produce compound (16).

Formyl compound (15) used as a starting compound is, for example, a commercially available product, or can be synthesized according to the method described in a document or a method combining general synthesis methods and the like.

### step 5-3

Compound (16) is reacted in an inert solvent (e.g., ethanol and the like) in the presence or absence of a base (e.g., potassium hydroxide, sodium acetate and the like) at generally -20°C - 200°C for 1 hr - 1 day to produce chromane compound (17).

### step 5-4

Chromane compound (17) is reacted in a solvent (e.g., trifluoroacetic acid etc.) using a reducing agent (e.g., triethylsilane and the like) at generally 0°C - 200°C for 1 hr - 3 days to produce chromane compound (18). In addition, amine compound (3a) obtained in the next step 5-5 is partly obtained in some cases.

### step 5-5

Chromane compound (18) is treated in the presence or absence of an iniert solvent (e.g., acetic acid and the like) using an acid (e.g., hydrochloric acid and the like) at generally 0°C - 200°C for 1 hr - 1 day to produce amine compound (3a).

### Production Method 6

Of the amine compounds (8) used as a starting compound in the above-mentioned Production Method 3, amine compound (8a') wherein ring A is an optionally substituted a pyridine ring can be produced by the method of the following Production Method 6. Compound (19) used as the starting compound is, for example, a commercially available product, or can be synthesized according to the method described in a document or a method combining general synthesis methods and the like. 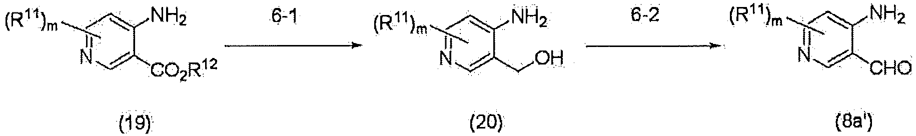 wherein R¹¹ is a substituent, m is 0 - 3, and R¹² is a C₁₋₆ alkyl group (e.g., methyl group, ethyl group, tert-butyl group and the like).

### step 6-1

Ester compound (19) is reacted in an inert solvent (e.g., tetrahydrofuran and the like) using a metal hydride complex compound (lithium aluminum hydride, sodium borohydride and the like) at generally -20°C - 200°C for 1 hr - 1 day to produce alcohol compound (20).

### step 6-2

Alcohol compound (20) is reacted in an inert solvent (e.g., toluene, tetrahydrofuran, ethanol and the like) using an oxidant (e.g., manganese dioxide and the like) at generally-20°C - 200°C for 1 hr - 1 day to produce aldehyde compound (8a').

### Production Method 7

Of the amine compounds (8) used as a starting compound in the above-mentioned Production Method 3, compound (8b) can be produced by the method of the following Production Method 7. Compound (8a) used as the starting compound is, for example, a commercially available product, or can be synthesized according to the method of the above-mentioned Production Method 6 and the like.

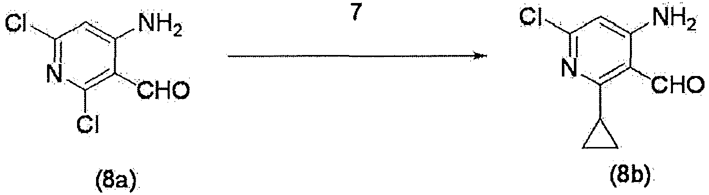

### step 7

Compound (8a) is reacted in an inert solvent (e.g., toluene, water, or a mixed solution thereof and the like) using a cyclopropylboron compound (e.g., cyclopropylboronic acid and the like) and a base (tripotassium phosphate and the like), a palladium compound (e.g., palladium acetate and the like) and a phosphine compound (e.g., tricyclohexylphosphine and the like) at generally 0°C - 200°C, or under microwave irradiation for 1 hr - 1 day to produce aldehyde compound (8b).

### Production Method 8

Of the amine compounds (8) used as a starting compound in the above-mentioned Production Method 3, compound (8d), (8e) or (8f) can be produced by, for example, the method of the following Production Method 8. 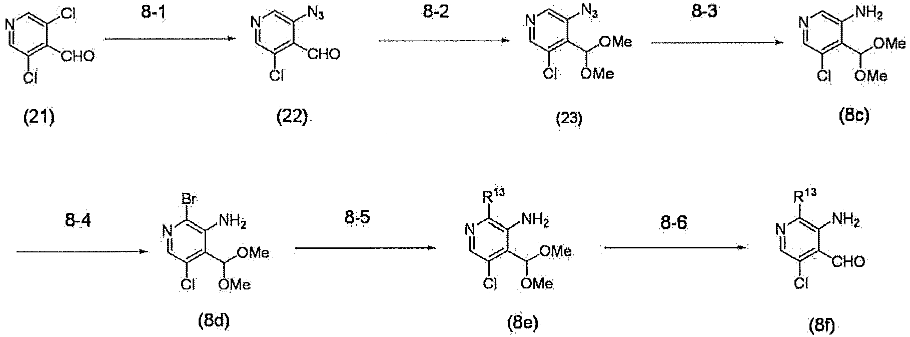 wherein R¹³ is an alkyl group, an aryl group or a heterocyclic group, each of which is optionally substituted.

### step 8-1

Compound (21) is reacted in an inert solvent (e.g., DMF and the like) using an azidation reagent (e.g., sodium azide and the like) at generally 0°C - 200°C for 1 hr - 1 day to produce compound (22).

Compound (21) used as a starting compound is, for example, a commercially available product, or can be synthesized according to the method described in a document or a method combining general synthesis methods and the like.

### step 8-2

Compound (22) is reacted in a methanol solvent and using an acid catalyst (e.g., p-toluenesulfonic acid monohydrate and the like) at generally 0°C - 200°C for 1 hr - 1 day to produce compound (23).

### step 8-3

Compound (23) is reacted in an inert solvent (e.g., ethanol and the like) using a reducing agent (e.g., sodium borohydride and the like) in the presence or absence of an additive (2,2'-bipyridine and the like) at generally 0°C - 200°C for 1 hr - 1 day to produce compound (8c).

### step 8-4

Compound (8c) is reacted in an inert solvent (e.g., acetic acid and the like) using a bromination reagent (e.g., bromine and the like) in the presence or absence of an additive (sodium acetate and the like) at generally 0°C - 200°C for 1 hr - 1 day to produce compound (8d).

### step 8-5

Compound (8d) is reacted in an inert solvent (e.g., toluene, water, or a mixed solution thereof and the like) using a boron compound (e.g., alkylboronic acid, arylboronic acid, heterocyclylboronic acid and the like) and a base (tripotassium phosphate and the like), a palladium compound (e.g., palladium acetate and the like) and a phosphine compound (e.g., tricyclohexylphosphine and the like) at generally 0°C - 200°C, or under microwave irradiation for 1 hr - 1 day to produce compound (8e).

### step 8-6

Compound (8e) is reacted in an inert solvent (e.g., THF and the like) using an acid (e.g., aqueous hydrochloric acid solution and the like) at generally 0°C - 200°C for 1 hr - 1 day to produce compound (8f).

### Production Method 9

Of compounds (I), a compound having an asymmetric carbon can be produced, for example, by separating into each optically active compound by HPLC using a chiral column. In addition, optically active compound (1c) can also be produced, for example, by the method of the following Production Method 9. Compound (3b) used as a starting compound can be produced by using compound (3a) synthesized by the above-mentioned Production Method 4, for example, by separating into each optically active compound by HPLC using a chiral column. Compound (3b) can be converted to compound (1c) by the method of the above-mentioned Production Method 3. 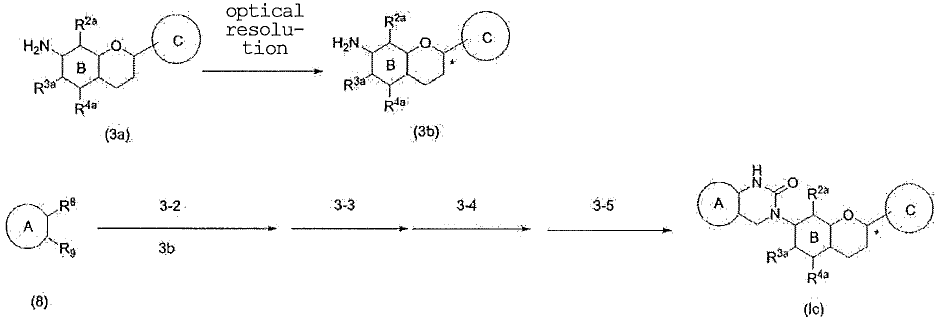 wherein the groups are each as defined above.

The aforementioned starting compound and/or the production intermediate for the compound (I) may form a salt. While the salt is not particularly limited as long as the reaction can be performed, examples thereof include those similar to the salts optionally formed by the compound (I) represented by the aforementioned formula (I), and the like.

As for the configuration isomers (E, Z forms) of compound (I), they can be isolated and purified when isomerization occurs, for example, according to a conventional separation means such as extraction, recrystallization, distillation, chromatography and the like to obtain a pure compound. In addition, the corresponding pure isomer can be obtained by isomerizing a double bond using heating, an acid catalyst, a transition metal complex, a metal catalyst, a radical catalyst, light irradiation or a strong base catalyst and the like, according to the method described in Jikken Kagaku Kouza (Courses in Experimental Chemistry) 14 (The Chemical Society of Japan ed.), pages 251 to 253, 4th Edition Jikken Kagaku Kouza 19 (The Chemical Society of Japan ed.), pages 273 to 274 or a method analogous thereto.

Compound (I) contains a stereoisomer depending to the kind of a substituent, and each stereoisomer and a mixture thereof are encompassed in the present invention.

Compound (I) may be a hydrate or a non-hydrate.

When desired, compound (I) can be synthesized by performing deprotection reaction, acylation reaction, alkylation reaction, hydrogenation reaction, oxidation reaction, reduction reaction, reaction of carbon chain extension, substituent exchange reaction singly or two or more thereof in combination.

When the objective product is obtained as a free form by the above-mentioned reaction, it can be converted to a salt according to a conventional method, or when the objective product is obtained as a salt, it can be converted to a free form or other salt according to a conventional method. The thus-obtained compound (I) can also be isolated and purified from a reaction mixture according to a known method such as phase transfer, concentration, solvent extraction, distillation, crystallization, recrystallization, chromatography and the like.

When compound (I) contains a configurational isomer, a diastereomer, a conformer and the like, each can be isolated according to the above-mentioned separation and purification methods, if desired. In addition, when compound (I) is racemic, d-form and 1-form can be isolated according to a conventional optical resolution.

In each of the above-mentioned reactions, when the compound has a functional group such as an amino group, a hydroxy group or a carboxyl group, the reaction can be carried out after a protecting group generally used in peptide chemistry and the like is introduced into these groups. By removing the protecting group as necessary after the reaction, the objective compound can be obtained.

Examples of the protecting group include formyl, C₁₋₆ alkyl-carbonyl (e.g., acetyl, propionyl etc.), phenylcarbonyl, C₁₋₆ alkoxy-carbonyl (e.g., methoxycarbonyl, ethoxycarbonyl etc.), phenyloxycarbonyl, C₇₋₁₀ aralkyloxy-carbonyl (e.g., benzyloxycarbonyl etc.), trityl, phthaloyl and the like, each of which is optionally substituted. Examples of the substituent include a halogen atom (e.g., fluorine, chlorine, bromine, iodine etc.), C₁₋₆ alkyl-carbonyl (e.g., acetyl, propionyl, valeryl etc.), nitro and the like. The number of substituents is, for example, 1 to 3.

The removal method of the protecting group can be carried out according to a method known per se (e.g., Wiley-Interscience, Inc., 2006 "Protective Groups in Organic Synthesis, 4^{th}Ed."(Theodora W. Greene, Peter G. M. Wuts), and for example, a method using acid, base, ultraviolet rays, hydrazine, phenylhydrazine, sodium N-methyldithiocarbamate, tetrabutylammonium fluoride, palladium acetate and the like, a reduction method, and the like can be employed.

The thus-obtained compound (I), other reaction intermediate therefor and starting compounds thereof can be isolated and purified from a reaction mixture according to a method known per se, for example, extraction, concentration, neutralization, filtration, distillation, recrystallization, column chromatography, thin layer chromatography, preparative high performance liquid chromatography (preparative HPLC), moderate-pressure preparative liquid chromatography (moderate-pressure preparative LC) and the like.

A salt of the compound represented by the formula (I) can be produced according to a method known *per se.* For example, when a free form of compound (I) is a basic compound, it can be produced by adding an inorganic acid or organic acid, or when a free form of compound (I) is an acidic compound, by adding an organic base or inorganic base.

When compound (I) contains an optical isomer, each optical isomer and a mixture thereof are encompassed in the scope of the present invention, and these isomers can be subjected to optical resolution or can be produced respectively, according to a method known per se, if desired.

When compound (I) contains a configurational isomer, a diastereomer, a conformer and the like, each can be isolated according to the above-mentioned separation and purification methods, if desired. In addition, when compound (I) is racemic, S-form and R-form can be isolated according to a conventional optical resolution.

When compound (I) contains a stereoisomer, each isomer and a mixture thereof are encompassed in the present invention.

Compound (I) may be used as a prodrug. A prodrug of compound (I) means a compound which is converted to compound (I) with a reaction due to an enzyme, an gastric acid, etc. under the physiological condition in the living body, that is, a compound which is converted to compound (I) by oxidation, reduction, hydrolysis, etc. due to an enzyme; a compound which is converted to compound (I) by hydrolysis etc. due to gastric acid, etc.

Examples of the prodrug of compound (I) include
(1) a compound obtained by subjecting amino in compound (I) to an acylation, alkylation or phosphorylation (e.g., a compound obtained by subjecting amino in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, ethoxycarbonylation, tert-butoxycarbonylation, acetylation or cyclopropylcarbonylation etc.);
(2) a compound obtained by subjecting hydroxy in compound (I) to an acylation, alkylation, phosphorylation or boration (e.g., a compound obtained by subjecting hydroxy in compound (I) to an acetylation, palmitoylation, propanoylation, pivaloylation, succinylation, fumarylation, alanylation or dimethylaminomethylcarbonylation);
(3) a compound obtained by subjecting carboxy in compound (I) to an esterification or amidation (e.g., a compound obtained by subjecting carboxy in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methyl amidation etc.) and the like. These compounds can be produced from compound (I) according to a method known per se.

A prodrug for compound (I) may also be one which is converted to compound (I) under a physiological condition, such as those described in IYAKUHIN no KAIHATSU, Development of Pharmaceuticals, Vol. 7, Design of Molecules, p. 163-198, Published by HIROKAWA SHOTEN, 1990.

In the present specification, compound (I) and a prodrug thereof are sometimes collectively abbreviated as "the compound of the present invention".

When compound (I) contains an isomer such as an optical isomer, a stereoisomer, a regioisomer or a rotamer, any one of them and a mixture thereof are also encompassed in compound (I). For example, when compound (I) contains an optical isomer, an optical isomer resolved from racemate is also encompassed in compound (I). Each of these isomers can be obtained as a single product by a synthesis means, and separation means (e.g., concentration, solvent extraction, column chromatography, recrystallization etc.), which are known per se.

Compound (I) may be a crystal, and the crystal form may be single or a mixture of crystal forms, both of which are encompassed in compound (I). The crystal can be produced by a crystallization method known per se.

Compound (I) may be a hydrate, a non-hydrate, a solvate or a non-solvate.

Compound (I) may be labeled with an isotope (e.g., ³H, ¹⁴C, ¹⁸F, ³⁵S, ¹²⁵I etc.) and the like.

Compound (I) also encompasses a deuterium conversion form wherein ¹H is converted to ²H(D).

Compound (I) may be a pharmaceutically acceptable cocrystal or cocrystal salt. Here, the cocrystal or cocrystal salt means a crystalline substance consisting of two or more particular substances which are solids at room temperature, each having different physical properties (e.g., structure, melting point, heat of melting, hygroscopicity, solubility, stability etc.). The cocrystal and cocrystal salt can be produced by cocrystallization method known per se.

Compound (I) may also be used as a PET tracer.

The compound of the present invention has low toxicity, and can be used as it is or in the form of a pharmaceutical composition by mixing with a pharmacologically acceptable carrier etc. to mammals (e.g., human, mouse, rat, rabbit, dog, cat, bovine, horse, swine, monkey) as an agent for the prophylaxis or treatment of various diseases mentioned below.

As pharmacologically acceptable carriers, various organic or inorganic carrier substances conventionally used as preparation materials can be used. These are incorporated as excipient, lubricant, binder and disintegrant for solid preparations, or solvent, solubilizing agent, suspending agent, isotonicity agent, buffer and soothing agent for liquid preparations, and the like, and preparation additives such as preservative, antioxidant, colorant, sweetening agent and the like can be added as necessary.

Preferable examples of the excipient include lactose, sucrose, D-mannitol, D-sorbitol, starch, gelatinated starch, dextrin, crystalline cellulose, low-substituted hydroxypropylcellulose, sodium carboxymethylcellulose, gum arabic, pullulan, light anhydrous silicic acid, synthesis aluminum silicate and magnesium alumino metasilicate.

Preferable examples of the lubricant include magnesium stearate, calcium stearate, talc and colloidal silica.

Preferable examples of the binder include gelatinated starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.

Preferable examples of the disintegrant include lactose, sucrose, starch, carboxymethylcellulose, calcium carboxymethylcellulose, croscarmellose sodium, sodium carboxymethyl starch, light anhydrous silicic acid and low-substituted hydroxypropylcellulose.

Preferable examples of the solvent include water for injection, physiological brine, Ringer's solution, alcohol, propylene glycol, polyethylene glycol, sesame oil, corn oil, olive oil and cottonseed oil.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, and polyoxyethylene hydrogenated castor oil.

Preferable examples of the isotonicity agent include sodium chloride, glycerol, D-mannitol, D-sorbitol and glucose.

Preferable examples of the buffer include buffers such as phosphate, acetate, carbonate, citrate and the like.

Preferable examples of the soothing agent include benzyl alcohol.

Preferable examples of the preservative include p-oxybenzoates, chlorobutanol, benzyl alcohol, phenethyl alcohol, dehydroacetic acid and sorbic acid.

Preferable examples of the antioxidant include sulfite and ascorbate.

Preferable examples of the colorant include aqueous water-soluble food tar colors (e.g., food colors such as Food Color Red Nos. 2 and 3, Food Color Yellow Nos. 4 and 5, Food Color Blue Nos. 1 and 2 and the like), water insoluble lake dyes (e.g., aluminum salt of the above-mentioned water-soluble food tar color) and natural dyes (e.g., β-carotene, chlorophyll, ferric oxide red).

Preferable examples of the sweetening agent include saccharin sodium, dipotassium glycyrrhizinate, aspartame and stevia.

Examples of the dosage form of the pharmaceutical composition include oral preparations such as tablet (including sugar-coated tablet, film-coated tablet, sublingual tablet, orally disintegrating tablet), capsules (including soft capsule, microcapsule), granule, powder, troche, syrup, emulsion, suspension, films (e.g., orally disintegrable films) and the like; and parenteral agents such as injection (e.g., subcutaneous injection, intravenous injection, intramuscular injection, intraperitoneal injection, drip infusion), external preparations (e.g., dermal preparation, ointment), suppository (e.g., rectal suppository, vaginal suppository), pellet, nasal preparation, pulmonary preparation (inhalant), eye drop and the like.

These can be respectively safely administered orally or parenterally (e.g., topically, rectally, intravenously administered).

These preparations may be a release control preparation (e.g., sustained-release microcapsule) such as an immediate-release preparation, a sustained-release preparation and the like.

The pharmaceutical composition can be produced according to a method conventionally used in the field of pharmaceutical formulation, for example, the method described in the Japanese Pharmacopoeia, and the like.

While the content of the compound of the present invention in the pharmaceutical composition varies depending on the dosage form, dose of the compound of the present invention and the like, it is for example, about 0.1 to 100 wt%.

During production of an oral preparation, coating may be applied as necessary for the purpose of masking of taste, enteric property or durability.

Examples of the coating base to be used for coating include sugar coating base, aqueous film coating base, enteric film coating base and sustained-release film coating base.

As the sugar coating base, sucrose is used. Moreover, one or more kinds selected from talc, precipitated calcium carbonate, gelatin, gum arabic, pullulan, carnauba wax and the like may be used in combination.

Examples of the aqueous film coating base include cellulose polymers such as hydroxypropyl cellulose, hydroxypropylmethyl cellulose, hydroxyethyl cellulose, methylhydroxyethyl cellulose etc.; synthetic polymers such as polyvinylacetal diethylaminoacetate, aminoalkyl methacrylate copolymer E [Eudragit E (trade name)], polyvinylpyrrolidone etc.; and polysaccharides such as pullulan etc.

Examples of the enteric film coating base include cellulose polymers such as hydroxypropylmethyl cellulose phthalate, hydroxypropylmethyl cellulose acetate succinate, carboxymethylethyl cellulose, cellulose acetate phthalate etc.; acrylic polymers such as methacrylic acid copolymer L [Eudragit L (trade name)], methacrylic acid copolymer LD [Eudragit L-30D55 (trade name)], methacrylic acid copolymer S [Eudragit S (trade name)] etc.; and naturally occurring substances such as shellac etc.

Examples of the sustained-release film coating base include cellulose polymers such as ethyl cellulose etc.; and acrylic polymers such as aminoalkyl methacrylate copolymer RS [Eudragit RS (trade name)], ethyl acrylate-methyl methacrylate copolymer suspension [Eudragit NE (trade name)] etc.

The above-mentioned coating bases may be used after mixing with two or more kinds thereof at appropriate ratios. For coating, for example, a light shielding agent such as titanium oxide, red ferric oxide and the like can be used.

Since compound (I) or a prodrug thereof (hereinafter to be abbreviated as the compound of the present invention) has a strong 5-lipoxygenase activating protein inhibitory action, it is useful as a prophylactic or therapeutic drug for the diseases developed (or diseases promoted to be developed) in association with leukotriene produced via the 5-lipoxygenase activating protein in mammals (e.g., human, monkey, cat, swine, horse, bovine, mouse, rat, guinea pig, dog, rabbit etc.).

The compound is useful for preventing or treating, for example, cardiac diseases (cardiac hypertrophy, acute heart failure and chronic heart failure including cardiac failure, cardiomyopathy, angina, myocarditis, arrhythmia, tachycardia, myocardial infarction, etc.), myocardial ischemia, venous insufficiency, post-myocardial infarction transition to heart failure, hypertension, cor pulmonale, arteriosclerosis including atherosclerosis (aneurysm, coronary arterial sclerosis, cerebral arterial sclerosis, peripheral artery disease, arteriosclerosis obliterans, chronic arterial occlusion etc.), intervention (percutaneous coronary angioplasty, stent placement, coronary angioscopy, intravascular ultrasound, coronary thrombolytic therapy, etc.)-and heart transplantation-related vascular thickening/occlusion/organ damages, vascular reocclusion/restenosis after bypass surgery, respiratory diseases (cold syndrome, pneumonia, asthma, chronic obstructive pulmonary disease, pulmonary hypertension, pulmonary thrombus/pulmonary embolism, etc.), bone disorders (bone fracture, osteosarcoma, myeloma, osteitis fibrosis, myelitis with rigidity, rheumatoid arthritis, gonarthrosis etc.), inflammatory diseases (retinopathy, nephropathy, nerve damage, arthritis such as rheumatoid arthritis, osteoarthritis, rheumatoid myelitis and periostitis, inflammation after surgery/trauma, reduction of swelling, pharyngitis, cystitis, atopic dermatitis, inflammatory enteric diseases such as Crohn's disease and ulcerative colitis, meningitis, inflammatory eye diseases, pulmonary sarcoidosis such as pneumonia, silicosis, pulmonary sarcoidosis and pulmonary tuberculosis, etc.), allergic diseases (allergic rhinitis, conjunctivitis, gastrointestinal allergy, pollen allergy, anaphylaxis, etc.), neurodegenerative diseases (Alzheimer's disease, Parkinson's disease, amyotrophic lateral sclerosis, AIDS encephalopathy, etc.), central nervous system damage (disorders such as cerebral hemorrhage and cerebral infarction and aftereffects and complications thereof, head injury, spinal damage, cerebral edema, etc.), dementia, mental disorders ((schizophrenia, depression, epilepsy, alcohol dependence etc.), Rett syndrome, Huntington's disease, spinal damage, neuropathic pain, ischemic peripheral circulation disorder, obstructive peripheral circulation disorder, occlusive thromboangiitis, diabetes (type 1 diabetes, type 2 diabetes, type 1.5 diabetes), diabetic complications (nerve damage, nephropathy, retinopathy, cataract, macroangiopathy, osteopenia, diabetic hyperosmolar diabetic coma, infectious diseases (respiratory infection; urinary infection, digestive tract infection, skin and soft tissue infection, lower limb infection, etc.), diabetic gangrene, xerostomia, deterioration in hearing, cerebrovascular damage, peripheral circulatory disorder, etc.), urinary incontinence, metabolic/nutritional disorders (obesity, hyperlipidemia, hypercholesterolemia, impaired glucose tolerance, etc.), insulin resistant syndrome, syndrome X, vesceral obesity syndrome, cerebrovascular damage (asymptomatic cerebrovascular damage, transient cerebral ischemia attack, stroke, cerebrovascular dementia, hypertensive encephalopathy, cerebral infarction, etc.), cerebral edema, cerebral circulatory disturbance, recurrence and aftereffects of cerebrovascular damages (neurological symptoms, mental symptoms, subjective symptoms, impairment of activities of daily living, etc.), kidney diseases (nephritis, glomerulonephritis, glomerulosclerosis, renal failure, thrombotic microangiopathy, diabetic nephropathy, nephrotic syndrome, hypertensive nephrosclerosis, complications of dialysis, organ damage including nephropathy by irradiation, etc.), ocular disorders (glaucoma, ocular hypertension, etc.), multiple organ failure, endothelial dysfunction, other circulatory diseases (ischemic cerebral circulatory disturbance, Raynaud's disease, Buerger's disease, etc.), connective tissue disorders (e.g., systemic erythematosus, scleroderma, polyarteritis, etc.), liver disorders (hepatitis and cirrhosis including chronic types, etc.), digestive disorders (gastritis, gastric ulcer, gastric cancer, disorder after gastric surgery, esophageal ulcer, pancreatitis, esophageal and gastric variceal rupture, etc.), hematological/hematopoietic disorders (erythrocytosis, vascular purpura, autoimmune hemolytic anemia, disseminated intravascular coagulation syndrome, multiple myelosis, etc.), solid tumor, tumors (malignant melanoma, malignant lymphoma, digestive organs (e.g., stomach, intestine, etc.) cancers, etc.), cancers and cachexia associated therewith, cancer metastases, cystitis, menopausal disorders, septicemia and the like. In particular, the compound is preferably used for preventing or treating arteriosclerosis, respiratory diseases, allergic diseases and inflammatory diseases. Here, the concept of preventing or treating atherosclerosis include: preventing and delaying further progression of severity of so-called atherothrombosis such as ischemic cardiac diseases resulting from atherosclerotic plaque rupture (unstable angina, acute myocardial infarction, acute heart failure, cardiac death) or strokes (including transient cerebral ischemia); preventing occurrence of cardiovascular events of patients having a high risk of developing cardiovascular events (patients with acute coronary artery disease, stroke patients, patients with peripheral artery disease, patients with metabolic disorder, patients with hypertension/obesity/diabetes/hyperlipidemia, etc.) based on anti-atherosclerotic effects; preventing recurrence of ischemic cardiac diseases; preventing primary onset of cardiovascular event; preventing or treating peripheral artery disease (intermittent claudication, pain at rest, ischemic ulcer, ischemic necrosis etc.); and the like.

Arteriosclerosis including atherosclerosis is preferably a peripheral artery disease.

The compound of the present invention may also be used for secondary prevention and delaying the progression of the above-mentioned various diseases (e.g., cardiovascular events such as myocardial infarction).

Since the compound of the prevent invention can continuously suppress prophlogistic leukotriene production for a prolonged time period, it can also be used for preventing or treating inflammatory diseases suggestively associated with prophlogistic eicosanoid, such as arteriosclerosis, asthma, chronic obstructive pulmonary disease, allergic airway hyperresponsiveness, fever, pain production, thrombosis, cerebral infarction, myocardial infarction, cancer, autoimmune encephalomyelitis, pain, renal failure, rheumatism, osteoarthritis, pruritus, atopic dermatitis, rhinitis, inflammatory enteric diseases and Crohn's disease. Furthermore, the compound may improve or suppress enhancement of disorder or abnormality of biological function or physiological action that is causative of various diseases associated with inflammatory reaction, and may be used for primary or secondary prevention and delaying the progression of a disease or a pathological condition resulting therefrom. Examples of such disorders or abnormalities of biological functions and physiological actions include facial flush, pain and itch of skin (including those associated with administration of nicotinic acid derivative preparation, prostacyclin preparation or the like), overactive bladder, disorder or abnormality of cerebral circulatory/renal circulatory autoregulation, circulatory disorder (e.g., peripheral circulation, cerebral circulation, microcirculation, etc.), disorder of blood-brain barrier, salt sensitivity, abnormality of coagulation or fibrinolytic system, abnormality of blood/hemocyte component property (e.g., sickle cell disease, enhanced platelet aggregation, abnormality of erythrocyte deformability, enhanced leukocyte viscosity, increase in blood viscosity, etc.), generation and increased activities of growth factors and cytokines (e.g., PDGF, VEGF, FGF, interleukin, TNF-α, MCP-1, etc.), production and increased invasion of inflammatory cells, increase in free radical generation, acceleration of fatty deposition, endothelial dysfunction, endothelial, cellular and organ damages, edema, morphology alteration of cell such as smooth muscle (morphology alteration into proliferative form or the like), production and enhanced functions of vasoactive substances and thrombus-inducing substances (e.g., catecholamine, endothelin, thromboxane A2, etc.), abnormal contraction of blood vessel or the like, metabolic abnormality (e.g., serum lipid abnormality, blood glucose abnormality, etc.), overgrowth of cell or the like, and angiogenesis (including abnormal angiopoiesis upon abnormal capillary net formation of outer membrane of atherosclerotic plaque).

The content of the compound of the present invention in a pharmaceutical composition is generally about 0.01 to about 99.9% by weight, preferably about 0.1 to about 50% by weight of the whole preparation.

The dosage of the compound of the present invention is determined by considering age, weight, general health condition, sex, diet, administration time, administration method, excretion rate, combination of drugs, and the level of the patient's disease under treatment, and/or other factors.

While the dose varies depending on the target disease, symptom, subject of administration, administration method and the like, for example, when the compound of the present invention is orally administered to an adult as a therapeutic agent for arteriosclerosis, a single dose is generally about 0.01 - 100 mg/kg body weight, preferably 0.01 - 10 mg/kg body weight, which is preferably administered in 1 to 3 portions per day.

The compound of the present invention can be administered for a long term depending on the level of the disease state.

The compound of the invention may be used in combination, for example, with a drug such as an anti-atherosclerotic agent, an anti-thrombotic agent, an anti-heart failure agent, an anti-arrhythmia agent, an anti-hypertensive agent, an agent for treating diabetes, an agent for treating diabetic complications, an HDL-raising agent, an anti-hyperlipidemia agent, an antiobesity agent, a diuretic, an anti-inflammatory agent, an antigout agent, a chemotherapeutic agent, an immunotherapeutic agent, an osteoporosis drug, an anti-dementia agent and the like (hereinafter, abbreviated as concomitant drugs). These concomitant drugs may be low-molecular compounds, or high-molecular proteins, polypeptides, antibodies, vaccines or the like.

Examples of the above-mentioned "anti-atherosclerotic agent" include Lp-PL A2 inhibitors (e.g., darapladib, rilapladib, etc.), sPLA2 inhibitors (e.g., varespladib), acyl-coenzyme A: cholesterol acyltransferase (ACAT) inhibitors (e.g., melinamide, avasimibe, eflucimibe, etc.), lipid-rich plaque regression drugs (e.g., compounds described in WO 02/06264, WO 03/059900, etc.), reconstituted HDL (e.g., CSL-111, etc.), CETP inhibitors (e.g., torcetrapib, anacetrapib, dalcetrapib, etc.), MMP inhibitors, chymase inhibitors, SPT inhibitors, interleukin-1β inhibitor (e.g., canakinumab, rilonacept, anakinra), ApoA-1 and related molecules thereof (e.g., ApoA-1 Milano, D-4F, L-4F, etc.).

Examples of the above-mentioned "anti-thrombotic agent" include blood coagulation inhibitors (e.g., heparin sodium, heparin calcium, warfarin calcium (warfarin), antithrombin drugs (e.g., argatroban, dabigatran), activated blood coagulation Factor Xa inhibitors (e.g., rivaroxaban, apixaban, edoxaban, YM-150, compounds described in WO 02/06234, WO 2004/048363, WO 2005/030740, WO 2005/058823, WO 2005/113504 and WO 2004/048363), etc.), thrombolytic drugs (e.g., tPA, urokinase, tisokinase, alteplase, nateplase, monteplase, pamiteplase), antiplatelet drugs (e.g., aspirin, sulfinpyrazone (Anturan), dipyridamole (Persantin), ticlopidine (Panaldine), cilostazol (Pletal), GPIIb/IIIa antagonists (e.g., ReoPro, etc.), clopidogrel, prasugrel, ticagrelor, E5555, SHC530348, ethyl icosapentate, beraprost sodium, sarpogrelate hydrochloride, etc.) and the like.

Examples of the above-mentioned "anti-heart failure agent" include inotropic agents (e.g., digitoxin, digoxin, methyldigoxin, lanatoside C, proscillaridin, etc.), α,β-stimulants (e.g., epinephrine, norepinephrine, isoproterenol, dopamine, docarpamine, dobutamine, denopamine, etc.), phosphodiesterase inhibitors (e.g., amrinone, milrinone, olprinone hydrochloride, etc.), calcium channel sensitivity augmenting agents (e.g., pimobendan, etc.), nitrate drugs (e.g., nitroglycerin, isosorbide nitrate, etc.), angiotensin-converting enzyme inhibitors (e.g., an angiotensin-converting enzyme inhibitor mentioned below, etc.), angiotensin II antagonist (e.g., angiotensin II antagonist mentioned below, etc.), β-blockers (e.g., β-blocker mentioned below, etc.), diuretics (e.g., diuretic mentioned below, etc.), renin inhibitor, ANPs, sGC-activating agents, myosin sensitivity augmenting agents, carperitide, ubidecarenone, vesnarinone, aminophylline and the like.

Examples of the above-mentioned "anti-arrhythmia agents" include sodium channel blockers (e.g., quinidine, procainamide, disopyramide, ajmaline, cibenzoline, lidocaine, diphenylhydantoin, mexiletine, propafenone, flecainide, pilsicainide, phenytoin, etc.), β-blockers (e.g., propranolol, alprenolol, bufetolol, oxprenolol, atenolol, acebutolol, metoprolol, bisoprolol, pindolol, carteolol, arotinolol, etc.), potassium channel blockers (e.g., amiodarone, etc.), calcium channel blockers (e.g., verapamil, diltiazem, etc.) and the like.

Examples of the above-mentioned "anti-hypertensive agent" include angiotensin-converting enzyme inhibitors (e.g., captopril, enalapril, delapril, etc.), angiotensin II antagonists (e.g., candesartan cilexetil, candesartan, azilsartan, azilsartan medoxomil, losartan, losartan potassium, eprosartan, valsartan, telmisartan, irbesartan, tasosartan, olmesartan, olmesartan medoxomil, etc.), calcium antagonists (e.g., manidipine, nifedipine, amlodipine, efonidipine, nicardipine, etc.), β-blockers (e.g., propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.), renin inhibitor (e.g., aliskiren, 1-(4-methoxybutyl)-N-(2-methylpropyl)-N-[(3S,5R)-5-(morpholin-4-ylcarbonyl)piperidin-3-yl]-1H-benzimidazole-2-carboxamide or a salt thereof etc.), clonidine and the like.

Examples of the above-mentioned "therapeutic agent for diabetes" include insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Netoglitazone (MCC-555), Rivoglitazone (CS-011), FK-614, compound described in WO 01/38325, Tesaglitazar (AZ-242), Ragaglitazar (NN-622), Muraglitazar (BMS-298585), Edaglitazone (BM-13-1258), Metaglidasen (MBX-102), Naveglitazar (LY-519818), MX-6054, LY-510929, AMG131 (T-131) or a salt thereof, THR-0921), α-glucosidase inhibitors (e.g., voglibose, acarbose, miglitol, emiglitate), biguanides (e.g., phenformin, metformin, buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof], dipeptidyl peptidase IV inhibitors (e.g., Vildagliptin (LAF237), P32/98, Sitagliptin (MK-431), alogliptin, P93/01, PT-100, Saxagliptin (BMS-477118), BI1356, GRC8200, MP-513, PF-00734200, PHX1149, SK-0403, ALS2-0426, TA-6666, TS-021, KRP-104, 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof), β3 agonists (e.g., AJ-9677), GPR40 agonist, GLP-1 receptor agonists [e.g., GLP-1, GLP-1MR agent, liraglutide, AC-2993 (exendin-4)), BIM-51077, Aib(8,35)hGLP-1(7,37)NH2, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095, dapagliflozin, remogliflozin), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists (e.g., compounds described in WO 01/25228, WO 03/42204, WO 98/44921, WO 98/45285, WO 99/22735 etc.), glucokinase activators (e.g., Ro-28-1675), ACC2 (acetyl-CoA carboxylase 2) inhibitor and the like.

Examples of the above-mentioned "therapeutic agents for diabetic complications" include aldose reductase inhibitors (e.g., Tolrestat, Epalrestat, zenarestat, Zopolrestat, minalrestat, Fidarestat, CT-112, ranirestat (AS-3201)), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), PKC inhibitors (e.g., ruboxistaurin mesylate), AGE inhibitors (e.g., ALT-946, pimagedine, N-phenacylthiazolium bromide (ALT-766), EXO-226, Pyridorin, Pyridoxamine), active oxygen scavengers (e.g., thioctic acid), cerebral vasodilators (e.g., tiapuride, mexiletine), somatostatin receptor agonists (BIM23190), apoptosis signal regulating kinase-1 (ASK-1) inhibitors and the like.

Examples of the above-mentioned "HDL-raising agent" include squalene synthetase inhibitors, CETP inhibitors (e.g., torcetrapib, anacetrapib, dalcetrapib, etc.), LPL activators, nicotinic drugs (e.g., nicomol, niceritrol), endothelial lipase inhibitors and the like.

Examples of the above-mentioned "anti-hyperlipidemia agent" include statin compounds as cholesterol synthesis inhibitors (e.g., cerivastatin, pravastatin, simvastatin, lovastatin, rosuvastatin, atorvastatin, fluvastatin, pitavastatin or salts thereof (e.g., sodium salt, etc.) etc.), squalene synthetase inhibitors or fibrate compounds with hypotriglyceride action (e.g., bezafibrate, clofibrate, simfibrate, clinofibrate, etc.), cholesterol absorption inhibitors (e.g., zetia), anion-exchange resins (e.g., cholestyramine), probucol, nicotinic drugs (e.g., nicomol, niceritrol), phytosterols (e.g., soysterol, [gamma]-oryzanol)), fish oil preparations (EPA, DHA, omega-3-acid ethyl ester 90 etc.), PPAR α-agonists, PPAR γ-agonists, PPAR [delta]-agonists, LXR agonists, FXR antagonists, FXR agonists, DGAT inhibitors, MGAT inhibitors, MTP inhibitors (e.g., lomitapide), nucleic acid drugs including ApoB antisense (e.g., mipomersen) or PCSK9 siRNA antisense oligonucleotides, antibody drugs containing anti-PCSK9 antibody and antigen binding fragment thereof and the like.

Examples of the above-mentioned "antiobesity agent" include monoamine uptake inhibitors (e.g., phentermine, sibutramine, mazindol, fluoxetine, tesofensine), serotonin 2C receptor agonists (e.g., lorcaserin), serotonin 6 receptor antagonists, histamine H3 receptor, GABA modulator (e.g., topiramate), neuropeptide Y antagonists (e.g., velneperit), cannabinoid receptor antagonists (e.g., rimonabant, taranabant), ghrelin antagonists, ghrelin receptor antagonists, ghrelin acylation enzyme inhibitors, opioid receptor antagonists (e.g., GSK-1521498), orexin receptor antagonists, melanocortin 4 receptor agonists, 11β-hydroxysteroid dehydrogenase inhibitors (e.g., AZD-4017), pancreatic lipase inhibitors (e.g., orlistat, cetilistat), β3 agonists (e.g., N-5984), diacylglycerol acyltransferase 1 (DGAT1) inhibitors, acetylCoA carboxylase (ACC) inhibitors, stearoyl-CoA desaturated enzyme inhibitors, microsomal triglyceride transfer protein inhibitors (e.g., R-256918), Na-glucose cotransporter inhibitors (e.g., JNJ-28431754, remogliflozin), NFκ inhibitors (e.g., HE-3286), PPAR agonists (e.g., GFT-505, DRF-11605), phosphotyrosine phosphatase inhibitors (e.g., sodium vanadate, Trodusquemin), GPR119 agonists (e.g., PSN821), glucokinase activators (e.g., AZD-1656), leptin, leptin derivatives (e.g., metreleptin), CNTF (ciliary neurotrophic factor), BDNF (brain-derived neurotrophic factor), cholecystokinin agonists, glucagon-like peptide-1 (GLP-1) preparations (e.g., animal GLP-1 preparations extracted from the pancreas of bovine or swine; human GLP-1 preparations genetically synthesized using *Escherichia coli* or yeast; fragments or derivatives of GLP-1 (e.g., exenatide, liraglutide)), amylin preparations (e.g., pramlintide, AC-2307), neuropeptide Y agonists (e.g., PYY3-36, derivatives of PYY3-36, obineptide, TM-30339, TM-30335), oxyntomodulin preparations: FGF21 preparations (e.g., animal FGF21 preparations extracted from the pancreas of bovine or swine; human FGF21 preparations genetically synthesized using *Escherichia coli* or yeast; fragments or derivatives of FGF21), anorexigenic agents (e.g., P-57) and the like.

Examples of the above-mentioned "diuretics" include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, poly5thiazide, methyclothiazide), antialdosterone preparations (e.g., spironolactone, eplerenone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the above-mentioned "anti-inflammatory agent" include nonsteroidal anti-inflammatory agents such as acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesylate, camostat mesylate, ulinastatin, colchicine, probenecid, sulfinpyrazone, benzbromarone, allopurinol, sodium aurothiomalate, sodium hyaluronate, sodium salicylate, morphine hydrochloride, salicylic acid, atropine, scopolamine, morphine, pethidine, levorphanol, ketoprofen, naproxen, oxymorphone and salts thereof, and the like.

Examples of the above-mentioned "antigout agent" include febuxostat, allopurinol, probenecid, colchicine, benzbromarone, febuxostat, citric salt and the like.

Examples of the above-mentioned chemotherapeutic agents include alkylating agents (e.g., cyclophosphamide, ifosfamide, etc.), metabolic antagonists (e.g., methotrexate, 5-fluorouracil, etc.), antitumor antibiotics (e.g., mitomycin, Adriamycin, etc.), plant-derived antitumor agents (e.g., vincristine, vindesine, Taxol, etc.), cisplatin, carboplatin, etoposide and the like. Of these, Furtulon or NeoFurtulon, which are 5-fluorouracil derivatives, and the like are preferable.

Examples of the above-mentioned "immunotherapeutic agents" include microorganism or bacterial components (e.g., muramyl dipeptide derivatives, Picibanil, etc.), polysaccharides having immunity potentiating activity (e.g., lentinan, schizophyllan, krestin, etc.), cytokines obtained by genetic engineering techniques (e.g., interferon, interleukin (IL), etc.), colony stimulating factors (e.g., granulocyte colony stimulating factor, erythropoietin, etc.) and the like, with preference given to interleukins such as IL-1, IL-2, IL-12 and the like.

Examples of the above-mentioned "therapeutic agents for osteoporosis" include alfacalcidol, calcitriol, elcaltonin, calcitonin salmon, estriol, ipriflavone, pamidronate disodium, alendronate sodium hydrate, incadronate disodium and the like.

Examples of the above-mentioned "antidementia agent" include tacrine, donepezil, rivastigmine, galanthamine and the like.

Moreover, examples of concomitant drugs include prostacyclin preparations/derivatives (e.g., beraprost, epoprostenol, iloprost, treprostinil, etc.), prostaglandin preparations/derivatives (e.g., enprostil, alprostadil, limaprost, misoprostol, ornoprostil, etc.), anti-asthma drugs (e.g., salmeterol, fluticasone, montelukast), rheumatoid arthritis agents (e.g., etanercept, infliximab, adalimumab), nerve regeneration promoters (e.g., Y-128, VX-853, prosaptide), antidepressants (e.g., desipramine, amitriptyline, imipramine), antiepilepsy drugs (e.g., lamotrigine), erectile dysfunction improvement agents (apomorphine, PDE5 inhibitors (e.g., citric acid sildenafil)), therapeutic agents for incontinence (e.g., flavoxate hydrochloride, oxybutynin hydrochloride, propiverine hydrochloride), therapeutic agents for dysuria (e.g., distigmine), acetylcholine receptor ligands (e.g., ABT-594), endothelin receptor antagonists (e.g., bosentan, ABT-627), monoamine uptake inhibitors (e.g., tramadol), narcotic analgesics (e.g., morphine), GABA receptor agonists (e.g., gabapentin), α₂ receptor agonists (e.g., clonidine), local analgesics (e.g., capsaicin), antianxiety drugs (e.g., benzothiazepines), dopamine agonists (e.g., apomorphine), interleukin-1β inhibitors (e.g., canakinumab, rilonacept, anakinra), midazolam, ketoconazole and the like.

The aforementioned concomitant drug is not restricted, and the compound of the present invention and the concomitant drug may be administered simultaneously, or may be administered at staggered times, to an administration subject. The dosage of the concomitant drug may be determined according to the dose clinically used, and can be appropriately selected depending on an administration subject, administration route, disease, combination and the like.

These concomitant drugs may be used in a mixture of two or more thereof in an appropriate ratio. In this case, the administration period of the compound of the present invention and the concomitant drugs is not limited as long as the compound of the present invention is combined with the concomitant drugs upon administration.

As such administration mode, the following methods can be mentioned: (1) The compound of the present invention and the concomitant drug are simultaneously formulated to give a single preparation which is administered. (2) The compound of the present invention and the concomitant drug are separately formulated to give two kinds of preparations which are administered simultaneously by the same administration route. (3) The compound of the present invention and the concomitant drug are separately formulated to give two kinds of preparations which are administered by the same administration route at staggered times. (4) The compound of the present invention and the concomitant drug are separately formulated to give two kinds of preparations which are administered simultaneously by the different administration routes. (5) The compound of the present invention and the concomitant drug are separately formulated to give two kinds of preparations which are administered by the different administration routes at staggered times (e.g., the compound of the present invention and the concomitant drug are administered in this order, or in the reverse order), and the like. The dose of the combination drug can be determined as appropriate based on the dose clinically employed. The proportion of the compound of the present invention and the concomitant drug can be appropriately determined depending on the administration subject, administration route, target disease, condition, combination and the like. When, for example, the administration subject is human, the concomitant drug is used in an amount of 0.0001-10000 parts by weight per 1 part by weight of the compound of the present invention.

The time of administration of the compound of the present invention and that of the concomitant drug are not limited, and they may be administered simultaneously or in a staggered manner to the administration subject. Furthermore, the compound of the present invention and the concomitant drug may be administered as two kinds of preparations containing each active ingredient, or a single preparation containing both active ingredients.

The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the subject of administration is human, for example, a concomitant drug can be used in 0.01 - 100 parts by weight relative to 1 part by weight of the compound of the present invention.

### Examples

The present invention is explained in detail in the following by referring to Examples, Experimental Examples and Formulation Examples. However, the examples do not limit the present invention and the present invention can be modified within the scope of the present invention.

The "room temperature" in the following Examples is generally about 10°C to about 35°C. The ratio for a mixed solvent is, unless otherwise specified, a volume mixing ratio and % means wt% unless otherwise specified.

In silica gel column chromatography, the indication of NH means use of aminopropylsilane-bonded silica gel. In HPLC (high performance liquid chromatography), the indication of C18 means use of octadecyl-bonded silica gel. The ratio of elution solvents is, unless otherwise specified, a volume mixing ratio.

In the following Examples, the following abbreviations are used.
THF: tetrahydrofuran
DMF: N,N-dimethylformamide
DMSO: dimethyl sulfoxide
ESI: electrospray method
APCI: atmospheric chemical ionization
[M+H]⁺: molecular ion peak
M: mol concentration
N: normal concentration
HPLC: high performance liquid chromatography
TFA: trifluoroacetic acid
DBU: 1, 8-diazabicyclo[5.4.0]undec-7-ene
CDI: 1,1'-carbonyldiimidazole
WSC: 1-ethyl-3-(3-dimethylaminopropyl)carbodiimide hydrochloride
HOBt: 1-hydroxybenzotriazole

¹H NMR (proton nuclear magnetic resonance spectrum) was measured by Fourier-transform type NMR. For the analysis, ACD/SpecManager (trade name) and the like were used. Peaks with very mild protons such as hydroxyl group, amino group and the like are not described.

MS (mass spectrum) was measured by LC/MS (liquid chromatography mass spectrometer). As the ionization method, ESI (ElectroSpray Ionization) method, or APCI (Atomospheric Pressure Chemical Ionization) method was used. The data indicates those found. Generally, molecular ion peak is observed; however, a peak after elimination of a tert-butoxycarbonyl group or tert-butyl group may be observed as a fragment ion. When the compound has a hydroxyl group (-OH), a peak after elimination of H₂O may be observed as a fragment ion. In the case of a salt, a molecular ion peak or fragment ion peak of free form is generally observed.

The elemental analytical value (Anal.) shows Calculated value (Calcd) and Found value (Found).

### Example 1

### 5-chloro-3-(6-chloro-2-(3-fluoropyridin-2-yl)-3,4-dihydro-2H-chromen-7-yl)-3,4-dihydropyrido[4,3-d]pyrimidin-2(1H)-one

### A) 3-acetamide-4-chlorophenyl acetate

To a solution (80 mL) of 3-amino-4-chlorophenol (48.5 g) in pyridine was added acetic anhydride (96.0 mL) under ice-cooling. The mixture was stirred at room temperature for 2 hr, and concentrated under reduced pressure. The obtained residue was crystallized from diisopropyl ether to give the title compound (62.2 g).
¹H NMR (400 MHz, DMSO-d₆) δ 2.11 (3H, s), 2.67 (3H, s), 6.97 (1H, dd, J = 8.4, 2.8 Hz), 7.51 (1H, d, J = 8.4 Hz), 7.61 (1H, s), 9.56 (1H, brs).

### B) N-(4-acetyl-2-chloro-5-hydroxyphenyl)acetamide

A mixture of 3-acetamide-4-chlorophenyl acetate (10.0 g), sodium chloride (2.6 g) and aluminum chloride (17.6 g) was stirred at 130°C for 4 hr, and cooled to room temperature. To the reaction mixture were added methanol (80 mL) and water (20 mL), and the mixture was stirred at room temperature for 1 hr. Water (350 mL) was further added and the mixture was stirred at room temperature. The obtained solid was collected by filtration, and washed with water to give the title compound (7.9 g).
¹H NMR (400 MHz, DMSO-d₆) δ 2.17 (3H, s), 2.66 (3H, s), 7.22 (1H, d, J = 1.6 Hz), 7.92 (1H, d, J = 1.6 Hz), 9.49 (1H, brs), 11.93 (1H, brs).

### C) (E)-N-(2-chloro-4-(3-(3-fluoropyridin-2-yl)acryloyl)-5-hydroxyphenyl)acetamide

To a solution of N-(4-acetyl-2-chloro-5-hydroxyphenyl)acetamide (20.0 g) and 3-fluoropyridine-2-carbaldehyde (13.2 g) in ethanol (300 mL) was added sodium hydroxide (10.5 g) under ice-cooling. The mixture was stirred at room temperature overnight, and then, water and 1N hydrochloric acid (350 mL) were added, and the mixture was stirred for 1 hr. The obtained solid was collected by filtration and washed with water to give the title compound (20. 9 g).
¹H NMR (400 MHz, DMSO-d₆) δ 2.20 (3H, s), 7.57-7.61 (1H, m), 7.82 (1H, d, J = 15.2 Hz), 7.85-7.89 (2H, m), 7.94 (1H, s), 8.27 (1H, d, J = 15.2 Hz), 8.57 (1H, d, J = 4.4 Hz), 9.50 (1H, brs), 11.73 (1H, brs).

### D) N-(6-chloro-2-(3-fluoropyridin-2-yl)-4-oxo-3,4-dihydro-2H-chromen-7-yl)acetamide

To a solution of (E)-N-(2-chloro-4-(3-(3-fluoropyridin-2-yl)acryloyl)-5-hydroxyphenyl)acetamide (5.35 g) in ethanol (245 mL) was added 1% potassium hydroxide ethanol (10.0 mL) solution at room temperature. The mixture was stirred at 50°C for 5 hr, and cooled to room temperature. The obtained solid was collected by filtration and washed with water to give the title compound (4.05 g).
¹H NMR (400 MHz, DMSO-d₆) δ 2.16 (3H, s), 3.03 (1H, dd, J = 17.2, 4.4 Hz), 3.36 (1H, dd, J = 17.2, 9.2 Hz), 6.12 (1H, dd, J = 9.2, 4.4 Hz), 7.54-7.58 (1H, m), 7.70 (1H, s), 7.76 (1H, s), 7.85 (1H, t, J = 9.2 Hz), 8.42 (1H, d, J = 4.8 Hz), 9.54 (1H, brs).

### E) N-(6-chloro-2-(3-fluoropyridin-2-yl)-3,4-dihydro-2H-chromen-7-yl)acetamide

To a solution of N-(6-chloro-2-(3-fluoropyridin-2-yl)-4-oxo-3,4-dihydro-2H-chromen-7-yl)acetamide (12.0 g) in trifluoroacetic acid (240 mL) was added triethylsilane (115 mL) at room temperature. The mixture was stirred at room temperature for 3 days and solvent was evaporated under reduced pressure. Water was added to the residue, and the mixture was extracted with ethyl acetate. The extract was washed with water, and dried over magnesium sulfate. The solvent was evaporated under reduced pressure and the residue was purified by silica gel column chromatography (ethyl acetate/hexane) to give the title compound (7.10 g).
¹H NMR (400 MHz, CDCl₃) δ 2.15-2.32 (4H, m), 2.38-2.47 (1H, m), 2.80-2.86 (1H, m), 2.92-3.00 (1H, m), 5.43 (1H, dd, J = 10.4, 1.2 Hz), 7.08 (1H, s), 7.30-7.34 (1H, m), 7.43-7.46 (2H, m), 7.96 (1H, brs), 8.47 (1H, d, J = 4.8 Hz).

### F) 6-chloro-2-(3-fluoropyridin-2-yl)chromane-7-amine

To a solution of N-(6-chloro-2-(3-fluoropyridin-2-yl)-3,4-dihydro-2H-chromen-7-yl)acetamide (7.10 g) in methanol (220 mL) was added 2N hydrochloric acid (111 mL) at room temperature. The mixture was heated under reflux overnight, and cooled to room temperature. The solvent was evaporated under reduced pressure and the residue was neutralized with 1N aqueous sodium hydroxide solution and extracted with ethyl acetate. The extract was washed with water, and dried over magnesium sulfate. The solvent was evaporated under reduced pressure and the residue was purified by silica gel column chromatography (ethyl acetate/hexane) to give the title compound (6.04 g).
¹H NMR (400 MHz, CDCl₃) δ 2.12-2.18 (1H, m), 2.33-2.43 (1H, m), 2.73-2.79 (1H, m), 2.87-2.95 (1H, m), 3.89 (2H, brs), 5.37-5.41 (1H, m), 6.34 (1H, s), 6.97 (1H, s), 7.26-7.32 (1H, m), 7.41-7.46 (1H, m), 8.47 (1H, m).

### G) N-(2-chloro-3-(((6-chloro-2-(3-fluoropyridin-2-yl)-3,4-dihydro-2H-chromen-7-yl)amino)methyl)pyridin-4-yl)-2,2-dimethylpropanamide

To a solution of N-(2-chloro-3-formylpyridin-4-yl)-2,2-dimethylpropanamide (1.55 g) and 6-chloro-2-(3-fluoropyridin-2-yl)chromane-7-amine (1.80 g) in toluene (60 mL) was added p-toluenesulfonic acid monohydrate (10 mg), and the mixture was heated under reflux for 4 hr. The reaction mixture was cooled to 0°C, and sodium borohydride (0.49 g) and ethanol (20 mL) were added. The reaction mixture was stirred at room temperature overnight, cooled to 0°C, saturated aqueous ammonium chloride solution was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (2.10 g).
MS (ESI+): [M+H]⁺503.2.

### H) 5-chloro-3-(6-chloro-2-(3-fluoropyridin-2-yl)-3,4-dihydro-2H-chromen-7-yl)-3,4-dihydropyrido[4,3-d]pyrimidin-2(1H)-one

A solution of N-(2-chloro-3-(((6-chloro-2-(3-fluoropyridin-2-yl)-3,4-dihydro-2H-chromen-7-yl)amino)methyl)pyridin-4-yl)-2,2-dimethylpropanamide (2.1 g) in 6N hydrochloric acid (50 mL) was heated under reflux for 5 hr. The reaction mixture was cooled to room temperature, neutralized with an aqueous sodium hydroxide solution, and extracted with ethyl acetate. The extract was washed with saturated brine, and dried over magnesium sulfate. The solvent was evaporated under reduced pressure. The residue was dissolved in THF (50 mL), and CDI (2.0 g) and DBU (1.9 mL) were added. The reaction mixture was stirred overnight at room temperature, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.7 g).
¹H NMR (300 MHz, DMSO-d₆) δ 2.11-2.42 (2H, m), 2.77-3.14 (2H, m), 4.44-4.61 (1H, m), 4.72-4.88 (1H, m), 5.51 (1H, dd, J = 9.5, 2.3 Hz), 6.80 (1H, d, J = 5.3 Hz), 7.07 (1H, s), 7.37 (1H, s), 7.54 (1H, dt, J = 8.5, 4.4 Hz), 7.81 (1H, t, J = 9.5 Hz), 8.10 (1H, d, J = 5.3 Hz), 8.47 (1H, d, J = 4.5 Hz), 10.24 (1H, brs). MS (ESI+): [M+H]⁺445.3.

### Example 2

### 5-chloro-3-((2S)-6-chloro-2-(3-fluoropyridin-2-yl)-3,4-dihydro-2H-chromen-7-yl)-3,4-dihydropyrido[4,3-d]pyrimidin-2(1H)-one

Racemate (394 mg) of 5-chloro-3-(6-chloro-2-(3-fluoropyridin-2-yl)-3,4-dihydro-2H-chromen-7-yl)-3,4-dihydropyrido[4,3-d]pyrimidin-2(1H)-one was separated by HPLC (column: CHIRALCEL OJ (MC001), 50 mmID×500 mmL, Daicel chemical industries Inc., mobile phase: ethanol) to give the title compound (184 mg) with a shorter retention time. The absolute configuration was determined by the X-ray crystal structure analysis method.
¹H NMR (300MHz, DMSO-d₆) δ 2.15-2.41 (2H, m), 2.81-3.08 (2H, m), 4.53 (1H, dd, J = 14.9, 3.2 Hz), 4.67-4.91 (1H, m), 5.52 (1H, dd, J = 9.7, 2.3 Hz), 6.80 (1H, d, J = 5.5 Hz), 7.07 (1H, s), 7.37 (1H, s), 7.54 (1H, dt, J = 8.5, 4.3 Hz), 7.82 (1H, ddd, J = 10.2, 8.7, 1.2 Hz), 8.10 (1H, d, J = 5.5 Hz), 8.48 (1H, d, J = 4.6 Hz), 10.27 (1H, s).
MS (ESI+): [M+H]⁺445.1.
[α]_{D}²⁰ +53.4 (c 0.49, DMSO)

### Example 3

### 5-chloro-3-((2R)-6-chloro-2-(3-fluoropyridin-2-yl)-3,4-dihydro-2H-chromen-7-yl)-3,4-dihydropyrido[4,3-d]pyrimidin-2(1H)-one

Racemate (394 mg) of 5-chloro-3-(6-chloro-2-(3-fluoropyridin-2-yl)-3,4-dihydro-2H-chromen-7-yl)-3,4-dihydropyrido[4,3-d]pyrimidin-2(1H)-one was separated by HPLC (column: CHIRALCEL OJ (MC001), 50 mmID×500 mmL, Daicel chemical industries Inc., mobile phase: ethanol) to give the title compound (162 mg) with a longer retention time. The absolute configuration was determined by the X-ray crystal structure analysis method.
¹H NMR (300MHz, DMSO-d₆) 5 2.11-2.41 (2H, m), 2.78-3.10 (2H, m), 4.53 (1H, dd, J = 14.8, 3.1 Hz), 4.71-4.89 (1H, m), 5.52 (1H, dd, J = 9.6, 2.2 Hz), 6.80 (1H, d, J = 5.5 Hz), 7.07 (1H, s), 7.37 (1H, s), 7.54 (1H, dt, J = 8.5, 4.4 Hz), 7.82 (1H, ddd, J = 10.3, 8.7, 1.2 Hz), 8.10 (1H, d, J = 5.4 Hz), 8.48 (1H, d, J = 4.5 Hz), 10.27 (1H, brs).
MS (ESI+): [M+H]⁺445.1.
[α]_{D}²⁰ -61.5 (c 0.48, DMSO)

### Example 4

### 3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

### A) 3-acetamide-4-methylphenyl acetate

A solution (40 mL) of 3-amino-4-methylphenol (16.1 g) in pyridine was added acetic anhydride (37.1 mL) under ice-cooling. The mixture was stirred at room temperature for 2 hr, concentrated under reduced pressure, and the obtained residue was crystallized from diisopropyl ether to give the title compound (25.9 g).
¹H NMR (400 MHz, DMSO-d₆) 5 2.19 (3H, s), 2.22 (3H, s), 2.28 (3H, s), 6.80 (1H, dd, J = 8.0, 2.4 Hz), 6.98 (1H, brs), 7.16 (1H, d, J = 8.0 Hz), 7.75 (1H, d, J= 2.4 Hz).

### B) N-(4-acetyl-5-hydroxy-2-methylphenyl)acetamide

A mixture of 3-acetamide-4-methylphenyl acetate (10.0 g), sodium chloride (2.8 g) and aluminum chloride (19.3 g) was stirred at 130°C for 4 hr, and cooled to room temperature. To the reaction mixture were added methanol (80 mL) and water (20 mL), and the mixture was stirred at room temperature for 1 hr. Furthermore, water (350 mL) was added, and the mixture was stirred at room temperature. The obtained solid was collected by filtration, and washed with water to give the title compound (7.7 g).
¹H NMR (400 MHz, DMSO-d₆) δ 2.10 (3H, s), 2.18 (3H, s), 2.54 (3H, s), 7.43 (1H, s), 7.67 (1H, s), 9.19 (1H, brs), 11.94 (1H, brs).

### C) (E)-N-(4-(3-(3-fluoropyridin-2-yl)acryloyl)-5-hydroxy-2-methylphenyl)acetamide

To a solution of N-(4-acetyl-5-hydroxy-2-methylphenyl)acetamide (20.0 g) and 3-fluoropyridine-2-carbaldehyde (13.2 g) in ethanol (400 mL) was added sodium hydroxide (11.6 g) under ice-cooling. The mixture was stirred at room temperature overnight, water and 1N hydrochloric acid (350 mL) were added, and the mixture was stirred for 1 hr. The obtained solid was collected by filtration and washed with water to give the title compound (23.1 g).
¹H NMR (400 MHz, DMSO-d₆) δ 2.12 (3H, s), 2.26 (3H, s), 7.57-7.62 (2H, m), 7.84-7.90 (3H, m), 8.30 (1H, d, J = 15.2 Hz), 8.58 (1H, d, J = 4.4 Hz), 9.24 (1H, brs), 11.05 (1H, brs).

### D) N-(2-(3-fluoropyridin-2-yl)-6-methyl-4-oxo-3,4-dihydro-2H-chromen-7-yl)acetamide

To a solution of (E)-N-(4-(3-(3-fluoropyridin-2-yl)acryloyl)-5-hydroxy-2-methylphenyl)acetamide (5.00 g) in ethanol (245 mL) was added 1% potassium hydroxide ethanol (10.0 mL) solution at room temperature. The mixture was stirred at 50°C for 5 hr, cooled to room temperature, and the obtained solid was collected by filtration and washed with water to give the title compound (3.60 g).
¹H NMR (300 MHz, DMSO-d₆) δ 2.11 (3H, s), 2.21 (3H, s), 2.93 (1H, dd, J = 17.0, 4.1 Hz), 3.40-3.52 (1H, m), 6.02 (1H, dd, J = 9.6, 2.8 Hz), 7.46 (1H, s), 7.51-7.57 (1H, m), 7.58 (1H, s), 7.77-7.89 (1H, m), 8.42 (1H, d, J = 4.5 Hz), 9.25 (1H, brs).

### E) 2-(3-fluoropyridin-2-yl)-6-methylchromane-7-amine

To a solution of N-(2-(3-fluoropyridin-2-yl)-6-methyl-4-oxo-3,4-dihydro-2H-chromen-7-yl)acetamide (10.6 g) in trifluoroacetic acid (200 mL) was added triethylsilane (108 mL) at room temperature, and the mixture was heated under reflux overnight. The reaction mixture was cooled to room temperature, and the solvent was evaporated under reduced pressure. Water was added to the residue, and the mixture was neutralized with 1N aqueous sodium hydroxide solution, and extracted with ethyl acetate. The extract was washed with water, and dried over magnesium sulfate. The solvent was evaporated under reduced pressure and the residue was purified by silica gel column chromatography (ethyl acetate/hexane). To the obtained compound was added 6N hydrochloric acid (150 mL), and the mixture was heated under reflux for 5 hr. The reaction mixture was cooled to room temperature, neutralized with 1N aqueous sodium hydroxide solution, and extracted with ethyl acetate. The extract was washed with water, and dried over magnesium sulfate. The solvent was evaporated under reduced pressure. The residue was crystallized from diisopropyl ether to give the title compound (6.2 g).
¹H NMR (400 MHz, DMSO-d₆) δ 1.96 (3H, s), 2.04-2.09 (1H, m), 2.20-2.30 (1H, m), 2.59-2.65 (1H, m), 2.75-2.83 (1H, m), 4.61 (2H, brs), 5.25 (1H, dd, J = 10.4, 2.4 Hz), 6.03 (1H, s), 6.63 (1H, s), 7.48-7.53 (1H, m), 7.75-7.80 (1H, m), 8.44-8.46 (1H, m).

### F) 4-chloro-2-(trifluoromethyl)nicotinic acid

To a solution of 2,2,6,6-tetramethylpiperidine (11.1 g) in THF (100 mL) was added dropwise 1.6 M n-butyllithium hexane solution (65.4 mL) at -78°C and the mixture was stirred at the same temperature for 30 min. Then a solution of 2-(trifluoromethyl)nicotinic acid (5.0 g) in THF (80 mL) was added dropwise at -78°C. After stirring at the same temperature for 20 min, the mixture was warmed to -50°C, and the mixture was further stirred for 1 hr. This solution was added dropwise to a solution of hexachloroethane (15.5 g) in THF (20 mL), which was separately stirring at -15°C, and the mixture was warmed to room temperature and stirred overnight. Water was added to the reaction mixture and the solvent was evaporated under reduced pressure. Water was added to the residue, diethyl ether was further added, and the obtained aqueous layer was neutralized with 1N hydrochloric acid, and extracted with ethyl acetate. The extract was washed with water, and dried over magnesium sulfate. The solvent was evaporated under reduced pressure to give the title compound. This was used without purification for the next step.
¹H NMR (300 MHz, DMSO-d₆) δ 8.06 (1H, d, J = 5.3 Hz), 8.80 (1H, d, J = 5.3 Hz).

### G) ethyl 4-chloro-2-(trifluoromethyl)nicotinate

To 4-chloro-2-(trifluoromethyl)nicotinic acid obtained in step F was added thionyl chloride (30 mL), and the mixture was heated under reflux for 2 hr. The solvent was evaporated under reduced pressure, ethanol (100 mL) was added to the obtained residue, and the mixture was heated under reflux for 2 hr. The solvent was evaporated under reduced pressure, and the obtained residue was purified by silica gel column chromatography (ethyl acetate/hexane) to give the title compound (4.6 g).
¹H NMR (300 MHz, CDCl₃) δ 1.41 (3H, t, J = 7.2 Hz), 4.48 (2H, q, J = 7.2 Hz), 7.60 (1H, d, J = 5.3 Hz), 8.66 (1H, d, J = 5.3 Hz).

### H) ethyl 4-azido-2-(trifluoromethyl)nicotinate

To a solution of ethyl 4-chloro-2-(trifluoromethyl)nicotinate (4.6 g) in DMF (40 mL) was added sodium azide (9.4 g), and the mixture was stirred at 50°C overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.5 g). ¹H NMR (300 MHz, CDCl₃) δ 1.39 (3H, t, J = 7.2 Hz), 4.44 (2H, q, J = 7.2 Hz), 7.30 (1H, d, J = 5.3 Hz), 8.69 (1H, d, J = 5.7 Hz).

### I) ethyl 4-amino-2-(trifluoromethyl)nicotinate

To a solution of ethyl 4-azido-2-(trifluoromethyl)nicotinate (1.5 g) in ethanol (50 mL) was added 10% palladium-carbon (0.2 g), and the mixture was stirred under a hydrogen atmosphere at room temperature for 2 hr. The insoluble material was removed by celite, the solvent was evaporated under reduced pressure, and the residue was purified by silica gel column chromatography (ethyl acetate/hexane) to give the title compound (0.88 g).
¹H NMR (300 MHz, CDCl₃) δ 1.39 (3H, t, J = 7.2 Hz), 4.39 (2H, q, J = 7.2 Hz), 5.48 (2H, brs), 6.69 (1H, d, J = 5.7 Hz), 8.25 (1H, d, J = 6.0 Hz).

### J) (4-amino-2-(trifluoromethyl)pyridin-3-yl)methanol

To a suspension of lithium aluminum hydride (139 mg) in THF (10 mL) was added a solution of ethyl 4-amino-2-(trifluoromethyl)nicotinate (429 mg) in THF (10 mL) under ice-cooling, and the mixture was stirred at room temperature overnight. The reaction mixture was ice-cooled, water was added, and the insoluble material was filtered off by using celite. The filtrate was extracted with ethyl acetate, and the extract was washed with saturated brine, and dried over magnesium sulfate. The solvent was evaporated under reduced pressure to give the title compound (345 mg).
¹H NMR (300 MHz, CDCl₃) δ4.84 (2H, s), 5.04 (2H, brs), 6.70 (1H, d, J = 5.7 Hz), 8.19 (1H, d, J = 5.7 Hz).

### K) 4-amino-2-(trifluoromethyl)nicotinaldehyde

To a solution of (4-amino-2-(trifluoromethyl)pyridin-3-yl)methanol (342 mg) in THF (7.0 mL) was added manganese dioxide (IV) (1.24 g), and the mixture was stirred at 60°C overnight. The reaction mixture was cooled to room temperature, and the insoluble material was filtered off by using celite. The filtrate was concentrated under reduced pressure, and the residue was crystallized from diisopropyl ether to give the title compound (328 mg).
¹H NMR (300 MHz, CDCl₃) δ 6.71 (1H, d, J = 5.7 Hz), 8.24 (1H, d, J = 6.0 Hz), 10.36 (1H, d, J = 1.5 Hz).

### L) 3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

To a solution of 4-amino-2-(trifluoromethyl)nicotinaldehyde (4.78 g) and 2-(3-fluoropyridin-2-yl)-6-methylchromane-7-amine (5.00 g) in toluene (80 mL) was added p-toluenesulfonic acid monohydrate (184 mg), and the mixture was heated at 70°C for 2 hr. The reaction mixture was cooled to room temperature, water was added, and the mixture was extracted with ethyl acetate. The extract was washed with saturated aqueous sodium hydrogen carbonate solution and saturated brine, and dried over magnesium sulfate. The solvent was evaporated under reduced pressure. The residue was dissolved in THF (100 mL) and the solution was added dropwise to an ice-cooled suspension of lithium aluminum hydride (1.47 g) in THF (100 mL). The reaction mixture was stirred under ice-cooling for 1 hr, water was added, and the insoluble material was filtered off by using celite. The filtrate was extracted with ethyl acetate, and the extract was washed with saturated brine, and dried over magnesium sulfate. The solvent was evaporated under reduced pressure. The residue was dissolved in THF (100 mL), and CDI (2.00 g) and DBU (1.90 mL) were added. The reaction mixture was stirred overnight at room temperature, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (8.00 g).
¹H NMR (300 MHz, DMSO-d₆) δ 2.05 (3H, s), 2.12-2.23 (1H, m), 2.24-2.42 (1H, m), 2.71-2.89 (1H, m), 2.89-3.15 (1H, m), 4.50-4.69 (1H, m), 5.02 (1H, d, J = 15.5 Hz), 5.35-5.52 (1H, m), 6.84-6.92 (1H, m), 7.00-7.13 (2H, m), 7.53 (1H, dt, J = 8.0, 4.1 Hz), 7.72-7.87 (1H, m), 8.39 (1H, d, J = 5.3 Hz), 8.47 (1H, d, J = 4.5 Hz), 10.30 (1H, brs).
MS (ESI+): [M+H]⁺459.4.

### Example 5

### 3-((2R)-2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

Racemate (450 mg) of 3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one was separated by SFC (column: CHIRALCEL ODH (KCOO3), 20 mmID×250 mmL, Daicel chemical industries Inc., mobile phase: carbon dioxide/ethanol = 770/230) to give the title compound (215 mg) with a shorter retention time. The absolute configuration was determined by X-ray structure analysis.
¹H NMR (300MHz, DMSO-d₆) δ 2.05 (3H, s), 2.13-2.41 (2H, m), 2.75-3.02 (2H, m), 4.58 (1H, d, J = 15.5 Hz), 5.02 (1H, d, J = 15.3 Hz), 5.43 (1H, d, J = 7.7 Hz), 6.86 (1H, s), 6.99-7.12 (2H, m), 7.40-7.58 (1H, m), 7.80 (1H, t, J = 9.4 Hz), 8.39 (1H, d, J = 5.4 Hz), 8.47 (1H, d, J = 4.6 Hz), 10.30 (1H, s).
MS (ESI+): [M+H]⁺459.4.
[α]_{D}²⁰ -51.6 (c 0.55, DMSO)

### Example 6

### 3-((2S)-2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

Racemate (450 mg) of 3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one was separated by SFC (column: CHIRALCEL ODH (KCOO3), 20 mmIDx250 mmL, Daicel chemical industries Inc., mobile phase: carbon dioxide/ethanol = 770/230) to give the title compound (200 mg) with a longer retention time. The absolute configuration was determined by X-ray structure analysis.
¹H NMR (300MHz, DMSO-d₆) δ 2.05 (3H, s), 2.13-2.40 (2H, m), 2.73-3.09 (2H, m), 4.58 (1H, d, J = 15.6 Hz), 5.02 (1H, d, J = 15.3 Hz), 5.43 (1H, d, J = 7.2 Hz), 6.86 (1H, s), 6.95-7.12 (2H, m), 7.53 (1H, dt, J = 8.2, 3.9 Hz), 7.80 (1H, t, J = 9.4 Hz), 8.40 (1H, d, J = 5.3 Hz), 8.47 (1H, d, J = 4.6 Hz), 10.30 (1H, s).
MS (ESI+) : [M+H]⁺459.4.
[α]_{D}²⁰ +50.5 (c 0.55, DMSO)

### Example 7

### 5-chloro-3-(6-chloro-2-(3-fluoropyridin-2-yl)-3,4-dihydro-2H-chromen-7-yl)-8-methyl-3,4-dihydropyrido[3,4-d]pyrimidin-2(1H)-one

### A) 3-azido-5-chloro-4-(dimethoxymethyl)pyridine

To a solution of 3,5-dichloroisonicotinaldehyde (7.5 g) in DMF (30 mL) was added sodium azide (3.1 g), and the mixture was stirred at 50°C for 1 hr. The reaction mixture was cooled to room temperature, water was added, and the mixture was extracted with ethyl acetate. The extract was washed with water and dried over magnesium sulfate. The solvent was evaporated under reduced pressure. The residue was dissolved in methanol (50 mL), p-toluenesulfonic acid monohydrate (0.8 g) was added, and the mixture was stirred at room temperature overnight. The reaction mixture was concentrated under reduced pressure and ethyl acetate was added to the residue. The solution was washed with saturated aqueous sodium hydrogen carbonate solution and then saturated brine, and dried over sodium sulfate. The solvent was evaporated under reduced pressure and the residue was purified by silica gel column chromatography (ethyl acetate/hexane) to give the title compound (5.8 g).
¹H NMR (300 MHz, CDCl₃) δ 3.49 (6H, s), 5.68 (1H, s), 8.36 (1H, s), 8.38 (1H, s).

### B) 5-chloro-4-(dimethoxymethyl)pyridin-3-amine

To a solution of cobalt bromide (0.7 g) in ethanol (75 mL) was added 2,2'-bipyridine (1.54 g) under ice-cooling, and sodium borohydride (1.4 g) was added at 5-10°C. To the reaction mixture was added a solution of 3-azido-5-chloro-4-(dimethoxymethyl)pyridine (7.5 g) in ethanol (30.0 mL), and the mixture was stirred at 0-5°C for 1 hr. To the reaction mixture were added acetic acid (10 mL) and then ethyl acetate. The solution was poured into water, neutralized with 2N aqueous sodium hydroxide solution. The organic layer was dried over sodium sulfate. The solvent was evaporated under reduced pressure and the residue was purified by silica gel column chromatography (ethyl acetate/hexane) to give the title compound (5.8 g).
¹H NMR (300 MHz, CDCl₃) δ 3.47 (6H, s), 4.71 (2H, brs), 5.73 (1H, s), 7.90 (1H, s), 7.91 (1H, s).

### C) 2-bromo-5-chloro-4-(dimethoxymethyl)pyridin-3-amine

To a solution of 5-chloro-4-(dimethoxymethyl)pyridin-3-amine (2.0 g) in acetic acid (40 mL) were added sodium acetate (2.4 g) and then bromine (0.5 mL) at 10°C. The mixture was stirred at room temperature for 2 hr. The reaction mixture was poured into 1N aqueous sodium hydroxide solution, and the mixture was extracted with ethyl acetate. The extract was washed with saturated aqueous sodium hydrogen carbonate solution, and then saturated brine, and dried over sodium sulfate. The solvent was evaporated under reduced pressure and the residue was purified by silica gel column chromatography (NH, ethyl acetate/hexane) to give the title compound (1.0 g) and 2,6-dibromo-5-chloro-4-(dimethoxymethyl)pyridin-3-amine (0.7 g) as a byproduct.
¹H NMR (300 MHz, CDCl₃) δ 3.48 (6H, s), 5.23 (2H, brs), 5.69 (1H, s), 7.69 (1H, s)

### D) 5-chloro-4-(dimethoxymethyl)-2-methylpyridin-3-amine

To a solution of 2-bromo-5-chloro-4-(dimethoxymethyl)pyridin-3-amine (2.00 g), methylboronic acid (0.51 g), tricyclohexylphosphine (0.25 g) and palladium(II) acetate (0.10 g) in toluene (40 mL) were added tripotassium phosphate (5.28 g) and water (2.0 mL), and the mixture was stirred under an argon atmosphere at 100°C for 23 hr. The reaction mixture was cooled to room temperature, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.10 g).
¹H NMR (300 MHz, CDCl₃) δ2.37 (3H, s), 3.48 (6H, s), 4.64 (2H, brs), 5.74 (1H, s), 7.85 (1H, s).

### E) 3-amino-5-chloro-2-methylisonicotinaldehyde

To a solution of 5-chloro-4-(dimethoxymethyl)-2-methylpyridin-3-amine (1.6 g) in THF (18 mL) was added 3N hydrochloric acid (17.4 mL), and the mixture was stirred at room temperature for 2 hr. The reaction mixture was cooled to 0°C, neutralized with saturated aqueous sodium hydrogen carbonate solution, and extracted with ethyl acetate. The extract was washed with saturated brine, and dried over magnesium sulfate. The solvent was evaporated under reduced pressure to give the title compound (1.20 g).
¹H NMR (300 MHz, CDCl₃) δ2.43 (3H, s), 6.41 (2H, brs), 7.86 (1H, s), 10.51 (1H, s).

### F) 5-chloro-4-(((6-chloro-2-(3-fluoropyridin-2-yl) 3,4-dihydro-2H-chromen-7-yl)amino)methyl)-2-methylpyridin-3-amine

To a solution of 3-amino-5-chloro-2-methylisonicotinaldehyde (1.20 g) and 6-chloro-2-(3-fluoropyridin-2-yl)chromane-7-amine (1.96 g) in toluene (18 mL) was added p-toluenesulfonic acid monohydrate (0.13 g), and the mixture was stirred under a nitrogen atmosphere at 100°C for 18 hr. After cooling to room temperature, the mixture was extracted with ethyl acetate and saturated aqueous sodium hydrogen carbonate solution. The extract was washed with saturated brine, and dried over magnesium sulfate. The solvent was evaporated under reduced pressure. The residue was dissolved in THF (18 mL) and ethanol (14 mL), and the mixture was cooled to 0°C, and sodium borohydride (1.06 g) was added. The reaction mixture was stirred at room temperature for 4.5 hr, cooled to 0°C, saturated aqueous sodium hydrogen carbonate solution was added, and the mixture was stirred at room temperature for 1 hr. The mixture was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (2.03 g).
¹H NMR (300 MHz, CDCl₃) δ 2.09-2.26 (1H, m), 2.30-2.51 (4H, m), 2.69-2.86 (1H, m), 2.86-3.04 (1H, m), 3.96 (1H, t, J = 5.3 Hz), 4.27 (2H, s), 4.37 (2H, d, J = 5.3 Hz), 5.44 (1H, d, J = 10.6 Hz), 6.47 (1H, s), 7.04 (1H, s), 7.32 (1H, dt, J = 8.6, 4.2 Hz), 7.40-7.52 (1H, m), 7.95 (1H, s), 8.49 (1H, d, J = 4.9 Hz).

### G) 5-chloro-3-(6-chloro-2-(3-fluoropyridin-2-yl)-3,4-dihydro-2H-chromen-7-yl)-8-methyl-3,4-dihydropyrido[3,4-d]pyrimidin-2(1H)-one

To a solution of 5-chloro-4-(((6-chloro-2-(3-fluoropyridin-2-yl)-3,4-dihydro-2H-chromen-7-yl)amino)methyl)-2-methylpyridin-3-amine (2.03 g) and CDI (2.66 g) in THF (45 mL) was added DBU (2.50 g) and the mixture was stirred at room temperature overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.70 g).
¹H NMR (300 MHz, CDCl₃) δ 2.14-2.30 (1H, m), 2.34-2.57 (4H, m), 2.83-3.14 (2H, m), 4.62-4.92 (2H, m), 5.37-5.54 (1H, m), 6.91-7.00 (1H, m), 7.20-7.28 (2H, m), 7.28-7.38 (1H, m), 7.40-7.51 (1H, m), 8.11 (1H, s), 8.48 (1H, d, J = 4.5 Hz).
MS (ESI+): [M+H]⁺458.9.

### Example 8

### 5-chloro-3-((2R)-6-chloro-2-(3-fluoropyridin-2-yl)-3,4-dihydro-2H-chromen-7-yl)-8-methyl-3,4-dihydropyrido[3,4-d]pyrimidin-2(1H)-one

Racemate (1500 mg) of 5-chloro-3-(6-chloro-2-(3-fluoropyridin-2-yl)-3,4-dihydro-2H-chromen-7-yl)-8-methyl-3,4-dihydropyrido[3,4-d]pyrimidin-2(1H)-one was separated by SFC (column: CHIRALCEL IA (MB001), 20 mmIDx250 mmL, Daicel chemical industries Inc., mobile phase: carbon dioxide/ethanol = 660/340) to give the title compound (794 mg) with a shorter retention time. The absolute configuration was determined by the X-ray crystal structure analysis method.
¹H NMR (300MHz, DMSO-d₆) δ 2.14-2.39 (2H, m), 2.42 (3H, s), 2.79-3.16 (2H, m), 4.48-4.67 (1H, m), 4.75-4.95 (1H, m), 5.52 (1H, dd, J = 9.7, 2.2 Hz), 7.08 (1H, s), 7.36 (1H, s), 7.54 (1H, dt, J = 8.5, 4.3 Hz), 7.82 (1H, ddd, J = 10.3, 8.6, 1.2 Hz), 8.09 (1H, s), 8.48 (1H, d, J = 4.4 Hz), 9.37 (1H, s). MS (ESI+): [M+H]⁺458.9.
[α]_{D}²⁰ -61.3 (c 0.47, DMSO)

### Example 9

### 5-chloro-3-((2S)-6-chloro-2-(3-fluoropyridin-2-yl)-3,4-dihydro-2H-chromen-7-yl)-8-methyl-3,4-dihydropyrido[3,4-d]pyrimidin-2(1H)-one

Racemate (1500 mg) of 5-chloro-3-(6-chloro-2-(3-fluoropyridin-2-yl)-3,4-dihydro-2H-chromen-7-yl)-8-methyl-3,4-dihydropyrido[3,4-d]pyrimidin-2(1H)-one was separated by SFC (column: CHIRALCEL IA (MB001), 20 mmID×250 mmL, Daicel chemical industries Inc., mobile phase: carbon dioxide/ethanol = 660/340) to give the title compound (770 mg) with a longer retention time. The absolute configuration was determined by the X-ray crystal structure analysis method.
¹H NMR (300MHz, DMSO-d₆) δ 2.13-2.38 (2H, m), 2.42 (3H, s), 2.77-3.10 (2H, m), 4.46-4.67 (1H, m), 4.76-4.95 (1H, m), 5.52 (1H, dd, J = 9.6, 2.1 Hz), 7.08 (1H, s), 7.36 (1H, s), 7.54 (1H, dt, J = 8.6, 4.4 Hz), 7.82 (1H, ddd, J = 10.3, 8.7, 1.2 Hz), 8.09 (1H, s), 8.48 (1H, d, J = 4.5 Hz), 9.37 (1H, s). MS (ESI+): [M+H]⁺458.9.
[α]_{D}²⁰ +61.5 (c 0.46, DMSO)

### Example 10

### 5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one

### A) 5-chloro-4-(dimethoxymethyl)-2-(3,5-dimethylisoxazol-4-yl)pyridin-3-amine

To a solution of 2-bromo-5-chloro-4-(dimethoxymethyl)pyridin-3-amine (1.5 g), 3,5-dimethyl-4-(4,4,5,5-tetramethyl-1,3,2-dioxaborolan-2-yl)isoxazole (1.5 g) and tetrakis(triphenylphosphine)palladium(0) (0.6 g) in DMF (35 mL) was added 2N aqueous sodium carbonate solution (8.0 mL), and the mixture was stirred under an argon atmosphere at 90°C for 2 hr. The reaction mixture was cooled to room temperature, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.6 g).
¹H NMR (300 MHz, CDCl₃) δ2.21 (3H, s), 2.35 (3H, s), 3.51 (6H, s), 4.71 (2H, brs), 5.79 (1H, s), 8.00 (1H, s).

### B) 5-chloro-2-(3,5-dimethylisoxazol-4-yl)-4-(((2-(3-fluoropyridin-2-yl)-6-methyl-3,4-dihydro-2H-chromen-7-yl)amino)methyl)pyridin-3-amine

To a solution of 5-chloro-4-(dimethoxymethyl)-2-(3,5-dimethylisoxazol-4-yl)pyridin-3-amine (1.56 g) and 2-(3-fluoropyridin-2-yl)-6-methylchromane-7-amine (1.49 g) in toluene (60 mL) was added p-toluenesulfonic acid monohydrate (0.50 g), and the mixture was stirred under a nitrogen atmosphere at 120°C for 1 hr. After cooling to room temperature, saturated aqueous sodium hydrogen carbonate solution was added, and the mixture was extracted with ethyl acetate. The extract was washed with saturated brine, and dried over magnesium sulfate. The solvent was evaporated under reduced pressure. The residue was dissolved in THF (45 mL) and ethanol (60 mL), cooled to 0°C, and sodium borohydride (0.79 g) was added. The reaction mixture was stirred at room temperature overnight, cooled to 0°C, saturated aqueous sodium hydrogen carbonate solution was added, and the mixture was stirred at room temperature for 1 hr. The mixture was extracted with ethyl acetate and the extract was washed with saturated brine, and dried over magnesium sulfate. The solvent was evaporated under reduced pressure and the residue was purified by silica gel column chromatography (ethyl acetate/hexane) to give the title compound (1.26 g).
¹H NMR (300 MHz, CDCl₃) δ 2.07 (3H, s), 2.12-2.22 (1H, m), 2.23 (3H, s), 2.35 (3H, s), 2.38-2.52 (1H, m), 2.71-2.84 (1H, m), 2.86-3.05 (1H, m), 3.30 (1H, brs), 4.39 (2H, brs), 4.43 (2H, s), 5.36-5.48 (1H, m), 6.47 (1H, s), 6.84 (1H, s), 7.31 (1H, dt, J = 8.4, 4.3 Hz), 7.45 (1H, ddd, J = 9.8, 8.5, 1.3 Hz), 8.11 (1H, s), 8.49 (1H, d, J = 4.9 Hz).

### C) 5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one

To a solution of 5-chloro-2-(3,5-dimethylisoxazol-4-yl)-4-(((2-(3-fluoropyridin-2-yl)-6-methyl-3,4-dihydro-2H-chromen-7-yl)amino)methyl)pyridin-3-amine (1.26 g) and triethylamine (1.29 g) in THF (36 mL) was added a solution of triphosgene (0.83 g) in THF (12 mL) at 0°C, and the mixture was stirred for 1 hr. Potassium carbonate (3.53 g) and acetonitrile (36 mL) were added, and the mixture was stirred at room temperature overnight, cooled to 0°C, and water was added. The mixture was extracted with ethyl acetate and the extract was washed with saturated brine, and dried over magnesium sulfate. The solvent was evaporated under reduced pressure and the residue was purified by silica gel column chromatography (ethyl acetate/hexane) to give the title compound (1.16 g).
¹H NMR (300 MHz, CDCl₃) δ 2.20 (4H, m), 2.25 (3H, s), 2.35-2.42 (3H, m), 2.42-2.57 (1H, m), 2.80-3.12 (2H, m), 4.64-4.97 (2H, m), 5.35-5.52 (1H, m), 6.46 (1H, s), 6.83-6.91 (1H, m), 7.05 (1H, s), 7.28-7.37 (1H, m), 7.38-7.53 (1H, m), 8.30 (1H, s), 8.41-8.55 (1H, m).
MS (ESI+): [M+H]⁺520.1.

### Example 11

### (-)-5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one

Racemate (1200 mg) of 5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one was separated by SFC (column: CHIRALCEL ODH (KCOO3), 20 mmID×250 mmL, Daicel chemical industries Inc., mobile phase: carbon dioxide/ethanol = 600/400) to give the title compound (517 mg) with a shorter retention time.
¹H NMR (300MHz, DMSO-d₆) δ 2.07 (3H, s), 2.10-2.22 (4H, m), 2.25-2.39 (4H, m), 2.73-3.02 (2H, m), 4.59 (1H, dd, J = 16.0, 3.8 Hz), 4.99 (1H, d, J = 16.1 Hz), 5.43 (1H, d, J = 9.6 Hz), 6.88 (1H, s), 7.03 (1H, s), 7.53 (1H, dt, J = 8.5, 4.3 Hz), 7.81 (1H, t, J = 9.1 Hz), 8.30 (1H, s), 8.43-8.56 (1H, m), 9.24 (1H, s).
MS (ESI+) : [M+H]⁺520.1.
[α]_{D}²⁰ -37.8 (c 0.46, DMSO)

### Example 12

### (+)-5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one

Racemate (1200 mg) of 5-chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidin-2(1H)-one was separated by SFC (column: CHIRALCEL ODH (KCOO3), 20 mmIDx250 mmL, Daicel chemical industries Inc., mobile phase: carbon dioxide/ethanol = 600/400) to give the title compound (534 mg) with a longer retention time.
¹H NMR (300MHz, DMSO-d₆) δ 2.07 (3H, s), 2.10-2.23 (4H, m), 2.26-2.40 (4H, m), 2.69-3.05 (2H, m), 4.59 (1H, dd, J = 15.7, 3.7 Hz), 4.99 (1H, d, J = 16.2 Hz), 5.43 (1H, d, J = 9.8 Hz), 6.88 (1H, s), 7.03 (1H, s), 7.53 (1H, dt, J = 8.3, 4.3 Hz), 7.81 (1H, t, J = 9.1 Hz), 8.30 (1H, s), 8.47 (1H, d, J = 4.5 Hz), 9.24 (1H, s).
MS (ESI+): [M+H]⁺520.1.
[α]_{D}²⁰ +47.6 (c 0.46, DMSO)

### Example 13

### 5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one

### A) 5-chloro-4-(dimethoxymethyl)-2-(1,3,5-trimethyl-1H-pyrazol-4-yl)pyridin-3-amine

To a solution of 2-bromo-5-chloro-4-(dimethoxymethyl)pyridin-3-amine (2.0 g), 1,3,5-trimethyl-4-(4,4,5,5-tetramethyl-1,3,2-dioxaborolan-2-yl)-1H-pyrazole (2.2 g) and tetrakis(triphenylphosphine)palladium (0) (0.82 g) in DMF (50 mL) was added 2N aqueous sodium carbonate solution (10.7 mL), and the mixture was stirred under an argon atmosphere at 100°C for 7.5 hr. The reaction mixture was cooled to room temperature, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (2.4 g). ¹H NMR (300 MHz, CDCl₃) δ 2.14 (6H, d, J = 1.1 Hz), 3.50 (6H, s), 3.76 (3H, s), 4.74 (2H, s), 5.79 (1H, s), 7.97 (1H, s).

### B) 5-chloro-4-(((2-(3-fluoropyridin-2-yl)-6-methyl-3,4-dihydro-2H-chromen-7-yl)amino)methyl)-2-(1,3,5-trimethyl-1H-pyrazol-4-yl)pyridin-3-amine

To a solution of 5-chloro-4-(dimethoxymethyl)-2-(1,3,5-trimethyl-1H-pyrazol-4-yl)pyridin-3-amine (2.34 g) and 2-(3-fluoropyridin-2-yl)-6-methylchromane-7-amine (2.14 g) in toluene (85 mL) was added p-toluenesulfonic acid monohydrate (0.43 g), and the mixture was stirred under a nitrogen atmosphere at 100°C for 1 hr. After cooling to room temperature, saturated aqueous sodium hydrogen carbonate solution was added, and the mixture was extracted with ethyl acetate. The extract was washed with saturated brine, and dried over magnesium sulfate. The solvent was evaporated under reduced pressure. The residue was dissolved in THF (64 mL) and ethanol (85 mL), and the solution was cooled to 0°C and sodium borohydride (1.50 g) was added. The reaction mixture was stirred at room temperature overnight, and cooled to 0°C. Saturated aqueous sodium hydrogen carbonate solution was added, and the mixture was stirred at room temperature for 1 hr. The mixture was extracted with ethyl acetate and the extract was washed with saturated brine, and dried over magnesium sulfate. The solvent was evaporated under reduced pressure and the residue was purified by silica gel column chromatography (ethyl acetate/hexane) to give the title compound (2.64 g). ¹H NMR (300 MHz, CDCl₃) δ 2.07 (3H, s), 2.16 (3H, s), 2.17 (3H, s), 2.19-2.25 (1H, m), 2.34-2.52 (1H, m), 2.72-2.85 (1H, m), 2.88-3.03 (1H, m), 3.33 (1H, brs), 3.77 (3H, s), 4.36 (2H, s), 4.42 (2H, s), 5.38-5.47 (1H, m), 6.48 (1H, s), 6.84 (1H, s), 7.30 (1H, dt, J = 8.4, 4.3 Hz), 7.39-7.50 (1H, m), 8.09 (1H, s), 8.49 (1H, dt, J = 4.7, 1.2 Hz).

### C) 5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one

To a solution of 5-chloro-4-(((2-(3-fluoropyridin-2-yl)-6-methyl-3,4-dihydro-2H-chromen-7-yl)amino)methyl)-2-(1,3,5-trimethyl-1H-pyrazol-4-yl)pyridin-3-amine (2.63 g) and triethylamine (2.62 g) in THF (36 mL) was added a solution of triphosgene (1.69 g) in THF (12 mL) at 0°C, and the mixture was stirred for 1 hr. Potassium carbonate (7.17 g) and acetonitrile (36 mL) were added, and the mixture was stirred at room temperature for 4 hr, and at 60°C for 1 hr. The mixture was cooled to 0°C, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (2.50 g). ¹H NMR (300 MHz, CDCl₃) δ 2.17 (3H, s), 2.18-2.21 (6H, m), 2.21-2.29 (1H, m), 2.35-2.57 (1H, m), 2.78-3.14 (2H, m), 3.78 (3H, s), 4.66-4.95 (2H, m), 5.43 (1H, dd, J = 10.6, 1.5 Hz), 6.56 (1H, s), 6.83-6.91 (1H, m), 7.04 (1H, s), 7.27-7.36 (1H, m), 7.39-7.50 (1H, m), 8.28 (1H, s), 8.40-8.53 (1H, m).
MS (ESI+) : [M+H]⁺533.1.

### Example 14

### (+)-5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one

Racemate (2500 mg) of 5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one was separated by SFC (column: CHIRALCEL ODH (KCOO3), 20 mmIDx250 mmL, Daicel chemical industries Inc., mobile phase: carbon dioxide/ethanol = 600/400) to give the title compound (1040 mg) with a shorter retention time.
¹H NMR (300MHz, DMSO-d₆) δ 2.00-2.12 (9H, m), 2.13-2.39 (2H, m), 2.69-3.07 (2H, m), 3.68 (3H, s), 4.57 (1H, dd, J = 16.0, 3.9 Hz), 4.98 (1H, d, J = 16.1 Hz), 5.43 (1H, d, J = 10.1 Hz), 6.88 (1H, s), 7.02 (1H, s), 7.53 (1H, dt, J = 8.4, 4.1 Hz), 7.80 (1H, t, J = 9.2 Hz), 8.26 (1H, s), 8.40 (1H, s), 8.47 (1H, d, J = 4.6 Hz).
MS (ESI+): [M+H]⁺533.1.
[α]_{D}²⁰ +45.6 (c 0.41, DMSO)

### Example 15

### (-)-5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one

Racemate (2500 mg) of 5-chloro-3-(2-(3-fluoropyridin-2-yl)-6-methyl-3,4-dihydro-2H-chromen-7-yl)-8-(1,3,5-trimethyl-1H-pyrazol-4-yl)-3,4-dihydropyrido[3,4-d]pyrimidin-2(1H)-one was separated by SFC (column: CHIRALCEL ODH (KCOO3), 20 mmIDx250 mmL, Daicel chemical industries Inc., mobile phase: carbon dioxide/ethanol = 600/400) to give the title compound (1050 mg) with a longer retention time.
¹H NMR (300MHz, DMSO-d₆) δ 1.99-2.12 (9H, m), 2.13-2.38 (2H, m), 2.72-3.04 (2H, m), 3.68 (3H, s), 4.57 (1H, dd, J = 16.1, 3.7 Hz), 4.98 (1H, d, J = 16.1 Hz), 5.43 (1H, d, J = 9.3 Hz), 6.88 (1H, s), 7.02 (1H, s), 7.53 (1H, dt, J = 8.5, 4.2 Hz), 7.80 (1H, t, J = 9.1 Hz), 8.26 (1H, s), 8.40 (1H, s), 8.47 (1H, d, J = 4.6 Hz).
MS (ESI+): [M+H]⁺533.1.
[α]_{D}²⁰ -38.3 (c 0.42, DMSO)

### Example 16

### 3-(6-chloro-2-(3-fluoropyridin-2-yl)-3,4-dihydro-2H-chromen-7-yl)-5-cyclopropyl-7-methoxy-3,4-dihydropyrido[4,3-d]pyrimidin-2(1H)-one

### A) tert-butyl 4-amino-2,6-dichloronicotinate

To a solution of tert-butyl 4-(tert-butoxycarbonylamino)-2,6-dichloronicotinate (38.0 g) in ethyl acetate (240 mL) was added 4N hydrogen chloride ethyl acetate solution (240 mL) and the mixture was stirred at room temperature. The reaction mixture was ice-cooled, neutralized with 4N aqueous sodium hydroxide solution, and extracted with ethyl acetate. The extract was washing with water, and dried over magnesium sulfate. The solvent was evaporated under reduced pressure. The residue was crystallized from diisopropyl ether to give the title compound (16.1 g). The mother liquor was purified by silica gel column chromatography (ethyl acetate/hexane) to give the title compound (7.3 g).
¹H NMR (300 MHz, CDCl₃) δ 1.60 (9H, s), 5.74 (2H, brs), 6.51 (1H, s).

### B) 4-amino-2,6-dichloronicotinaldehyde

To a suspension of lithium aluminum hydride (6.75 g) in THF (200 mL) was added dropwise a solution of tert-butyl 4-amino-2,6-dichloronicotinate (23.4 g) in THF (150 mL) under ice-cooling. The reaction mixture was stirred under ice-cooling for 1 hr, sodium sulfate decahydrate was added, and the insoluble material was filtered off by using celite. The filtrate was concentrated under reduced pressure, dissolved in a mixture of toluene (200 mL) and THF (150 mL), and manganese dioxide (IV) (38.7 g) was added. The mixture was stirred at 80°C for 3 hr and cooled to room temperature, and the insoluble material was filtered off by using celite. The filtrate was concentrated under reduced pressure to give the title compound (13.3 g).
¹H NMR (300 MHz, CDCl₃) δ 4.47-5.74 (1H, m), 6.55 (1H, s), 8.74 (1H, brs), 10.39 (1H, s).

### C) 4-amino-6-chloro-2-cyclopropylnicotinaldehyde

To a mixed solution of 4-amino-2,6-dichloronicotinaldehyde (4.0 g), cyclopropylboronic acid (1.9 g), tricyclohexylphosphine (0.6 g) and tripotassium phosphate (15.6 g) in toluene (64 mL)-water (3.2 mL) was added palladium(II) acetate (0.2 g) at room temperature. The mixture was stirred under microwave irradiation at 180°C for 5 hr, and the insoluble material was filtered off by using celite. The filtrate was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.2 g). ¹H NMR (300 MHz, CDCl₃) δ 1.01-1.08 (2H, m), 1.24-1.28 (2H, m), 2.35-2.48 (1H, m), 6.34 (1H, s), 10.62 (1H, s). MS (ESI+): [M+H]⁺197.2.

### D) 4-amino-2-cyclopropyl-6-methoxynicotinaldehyde

To a solution of 4-amino-6-chloro-2-cyclopropylnicotinaldehyde(519 mg), 5-(di-tert-butylphosphino)-1',3',5'-triphenyl-1'H-1,4'-bipyrazole (67 mg) and cesium carbonate (2580 mg) in methanol (12 mL) was added tris(dibenzylideneacetone)dipalladium(0) (97 mg) at room temperature. The mixture was stirred under microwave irradiation at 100°C for 1 hr, and the insoluble material was filtered off by using celite. The filtrate was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (261 mg).
¹H NMR (300 MHz, CDCl₃) δ 0.94-1.03 (2H, m), 1.23-1.30 (2H, m), 2.39-2.51 (1H, m), 3.85 (3H, s), 5.63 (1H, s), 10.53 (1H, s).
MS (ESI+): [M+H]⁺193.3.

### E) 3-(6-chloro-2-(3-fluoropyridin-2-yl)-3,4-dihydro-2H-chromen-7-yl)-5-cyclopropyl-7-methoxy-3,4-dihydropyrido[4,3-d]pyrimidin-2(1H)-one

To a solution of 4-amino-2-cyclopropyl-6-methoxynicotinaldehyde (2.5 g) in acetic acid (45 mL) was added 6-chloro-2-(3-fluoropyridin-2-yl)chromane-7-amine (4.2 g) at room temperature. The mixture was stirred at room temperature for 16 hr and cooled to 0°C, and sodium triacetoxyborohydride (8.3 g) was added. The mixture was stirred at room temperature for 16 hr, cooled to 0°C, water was added, and the mixture was extracted with ethyl acetate. The extract was washed with saturated aqueous sodium hydrogen carbonate solution and then saturated brine, and dried over magnesium sulfate. The solvent was evaporated under reduced pressure. The residue was dissolved in THF (30 mL), and CDI (6.3 g) and DBU (5.9 mL) were added at room temperature. The reaction mixture was stirred at 60°C for 16 hr, and water was added. The obtained solid was collected by filtration and washed with water to give the title compound (4.7 g). The filtrate was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.7 g).
¹H NMR (300MHz, DMSO-d₆) δ 0.78-0.98 (4H, m), 1.84 (1H, dq, J = 8.4, 4.1 Hz), 2.14-2.42 (2H, m), 2.75-3.09 (2H, m), 3.71 (3H, s), 4.57-4.74 (1H, m), 4.77-4.92 (1H, m), 5.50 (1H, d, J = 9.1 Hz), 5.93 (1H, s), 7.05 (1H, s), 7.35 (1H, s), 7.54 (1H, dt, J = 8.5, 4.4 Hz), 7.81 (1H, ddd, J = 10.3, 8.6, 1.2 Hz), 8.47 (1H, d, J = 4.1 Hz), 9.78 (1H, s).
MS (ESI+): [M+H]⁺481.1.

### Example 17

### 3-(6-chloro-2-(3-fluoropyridin-2-yl)-3,4-dihydro-2H-chromen-7-yl)-5-cyclopropyl-4,6-dihydropyrido[4,3-d]pyrimidine-2,7(1H,3H)-dione

A solution of 3-(6-chloro-2-(3-fluoropyridin-2-yl)-3,4-dihydro-2H-chromen-7-yl)-5-cyclopropyl-7-methoxy-3,4-dihydropyrido[4,3-d]pyrimidin-2(1H)-one (5.4 g) in 6N hydrochloric acid (135 mL) was stirred at 110°C for 2 days, cooled to 0°C, 8N aqueous sodium hydroxide solution was added, and the mixture was extracted with ethyl acetate. The extract was washed with saturated brine, and dried over magnesium sulfate. The solvent was evaporated under reduced pressure and the residue was purified by silica gel column chromatography (ethyl acetate/methanol) to give the title compound (4.8 g).
¹H NMR (300MHz, DMSO-d₆) δ 0.70-1.02 (4H, m), 1.67-1.83 (1H, m), 2.14-2.37 (2H, m), 2.78-3.03 (2H, m), 4.34-4.77 (2H, m), 5.36-5.58 (2H, m), 6.85-7.12 (1H, m), 7.34 (1H, s), 7.54 (1H, dt, J = 8.5, 4.4 Hz), 7.68-7.94 (1H, m), 8.47 (1H, d, J = 4.4 Hz), 9.79 (1H, s), 10.48 (1H, brs).
MS (ESI+): [M+H]⁺467.2.

### Example 18

### 3-(6-chloro-2-(3-fluoropyridin-2-yl)-3,4-dihydro-2H-chromen-7-yl)-5-cyclopropyl-7-(difluoromethoxy)-3,4-dihydropyrido[4,3-d]pyrimidin-2(1H)-one

To a mixed solution of 3-(6-chloro-2-(3-fluoropyridin-2-yl)-3,4-dihydro-2H-chromen-7-yl)-5-cyclopropyl-4,6-dihydropyrido[4,3-d]pyrimidine-2,7(1H,3H)-dione (4.8 g), sodium hydride (60%, oil)(0.5 g) and trimethylsilyl difluoro(fluorosulfonyl)acetate (5.1 g) in acetonitrile (150 mL)/DMF (75 mL) was added cesium fluoride (0.2 g) under ice-cooling. The reaction mixture was stirred under an argon atmosphere at room temperature for 5 hr, cooled to 0°C, saturated aqueous ammonium chloride solution was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and crystallized from ethyl acetate/methanol to give the title compound (1.3 g).
¹H NMR (300MHz, DMSO-d₆) δ 0.76-1.05 (4H, m), 1.73-1.94 (1H, m), 2.21 (2H, d, J = 6.3 Hz), 2.83-3.17 (2H, m), 4.61-4.82 (1H, m), 4.82-4.96 (1H, m), 5.51 (1H, d, J = 9.7 Hz), 6.11 (1H, s), 7.07 (1H, s), 7.28-7.40 (1H, m), 7.46-7.61 (2H, m), 7.73-7.92 (1H, m), 8.47 (1H, d, J = 3.5 Hz), 10.03 (1H, s).
MS (ESI+) : [M+H]⁺517.1.

### Example 19

### 3-((2R)-6-chloro-2-(3-fluoropyridin-2-yl)-3,4-dihydro-2H-chromen-7-yl)-5-cyclopropyl-7-(difluoromethoxy)-3,4-dihydropyrido[4,3-d]pyrimidin-2(1H)-one

Racemate (1267 mg) of 3-(6-chloro-2-(3-fluoropyridin-2-yl)-3,4-dihydro-2H-chromen-7-yl)-5-cyclopropyl-7-(difluoromethoxy)-3,4-dihydropyrido[4,3-d]pyrimidin-2(1H)-one was separated by SFC (column: CHIRALCEL ODH (KCOO3), 20 mmID×250 mmL, Daicel chemical industries Inc., mobile phase: carbon dioxide/isopropyl alcohol/acetonitrile = 700/150/150) to give the title compound (598 mg) with a shorter retention time. The absolute configuration was determined by the X-ray crystal structure analysis method.
¹H NMR (300MHz, DMSO-d₆) δ 0.77-0.98 (4H, m), 1.79-2.01 (1H, m), 2.11-2.41 (2H, m), 2.69-3.11 (2H, m), 4.61-4.80 (1H, m), 4.83-4.99 (1H, m), 5.51 (1H, d, J = 9.9 Hz), 6.11 (1H, s), 7.07 (1H, s), 7.26-7.41 (1H, m), 7.48-7.60 (2H, m), 7.71-7.89 (1H, m), 8.47 (1H, brs), 10.04 (1H, s).
MS (ESI+): [M+H]⁺517.2.
[α]_{D}²⁰ -59.3 (c 0.38, DMSO)

### Example 20

### 3-((2S)-6-chloro-2-(3-fluoropyridin-2-yl)-3,4-dihydro-2H-chromen-7-yl)-5-cyclopropyl-7-(difluoromethoxy)-3,4-dihydropyrido[4,3-d]pyrimidin-2(1H)-one

Racemate (1267 mg) of 3-(6-chloro-2-(3-fluoropyridin-2-yl)-3,4-dihydro-2H-chromen-7-yl)-5-cyclopropyl-7-(difluoromethoxy)-3,4-dihydropyrido[4,3-d]pyrimidin-2(1H)-one was separated by SFC (column: CHIRALCEL ODH (KCOO3), 20 mmID×250 mmL, Daicel chemical industries Inc., mobile phase: carbon dioxide/isopropyl alcohol/acetonitrile = 700/150/150) to give the title compound (615 mg) with a longer retention time. The absolute configuration was determined by the X-ray crystal structure analysis method.
¹H NMR (300MHz, DMSO-d₆) δ 0.77-1.00 (4H, m), 1.80-1.99 (1H, m), 2.11-2.40 (2H, m), 2.65-3.13 (2H, m), 4.55-4.79 (1H, m), 4.79-4.99 (1H, m), 5.51 (1H, d, J = 9.5 Hz), 6.11 (1H, s), 7.02-7.11 (1H, m), 7.31-7.37 (1H, m), 7.49-7.61 (2H, m), 7.71-7.89 (1H, m), 8.48 (1H, d, J = 1.8 Hz), 10.04 (1H, s).
MS (ESI+): [M+H]+517.2.
[α]_{D}²⁰ -53.1 (c 0.36, DMSO)

### Example 21

### 3-(2-(3-fluoropyridin-2-yl)-6-methyl-3,4-dihydro-2H-chromen-7-yl)-5-methoxy-3,4-dihydropyrido[4,3-d]pyrimidin-2(1H)-one

In the same manner as in Example 4, step L and using 4-amino-2-methoxynicotinaldehyde, the title compound was obtained.
MS (ESI+): [M+H]⁺421.3.

### Example 22

### 5-chloro-3-(2-(3-fluoropyridin-2-yl)-6-methyl-3,4-dihydro-2H-chromen-7-yl)-3,4-dihydropyrido[3,4-d]pyrimidin-2(1H)-one

### A) 3-azido-5-chloropyridine-4-carbonitrile

Under a nitrogen atmosphere, to a solution (20 mL) of 3,5-dichloropyridine-4-carbonitrile (3.07 g) in DMF was added sodium azide (1.38 g), and the mixture was stirred at 90°C overnight. Water was added to the reaction mixture, and the mixture was extracted with ethyl acetate. The obtained organic layer was washed with saturated brine, dried over anhydrous sodium sulfate, and the solvent was evaporated under reduced pressure. The residue was purified by silica gel column chromatography (ethyl acetate/hexane) to give the title compound (2.50 g).
¹H NMR (300 MHz, DMSO-d₆) δ 8.69 (1H, s), 8.88 (1H, s).

### B) 3-amino-5-chloropyridine-4-carbaldehyde

Under a nitrogen atmosphere at 0°C, to a solution of 3-azido-5-chloropyridine-4-carbonitrile (2.29 g) in THF (30 mL) was added sodium tetrahydroborate (1.40 g), and the mixture was stirred at room temperature for 2 hr. To the reaction mixture was added 1N hydrochloric acid, and the mixture was stirred for 30 min and neutralized with saturated aqueous sodium hydrogen carbonate solution. The obtained mixture was extracted with ethyl acetate, washed with saturated brine, and dried over anhydrous sodium sulfate. The solvent was evaporated under reduced pressure. To a solution of the residue in toluene (30 mL) was added manganese dioxide (IV) (6.20 g), and the mixture was heated under reflux for 3 hr while removing generated water by Dean-Stark apparatus. The reaction mixture was cooled to room temperature, and the insoluble material was removed by celite. The solvent was evaporated under reduced pressure and the residue was purified by silica gel column chromatography (ethyl acetate/hexane) to give the title compound (411 mg).
¹H NMR (300 MHz, DMSO-d₆) δ 7.63 (2H, brs), 7.78 (1H, s), 8.23 (1H, s), 10.34 (1H, s).

### C) 5-chloro-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 4, step L and using the compound obtained in the above-mentioned step B, the title compound was obtained.
MS (ESI+): [M+H]⁺425.3.

### Example 23

### 3-(2-(3-fluoropyridin-2-yl)-6-methyl-3,4-dihydro-2H-chromen-7-yl)-5-methoxy-3,4-dihydropyrido[3,4-d]pyrimidin-2(1H)-one

In the same manner as in Example 1, step G to H and using N-(4-formyl-5-methoxypyridin-3-yl)-2,2-dimethylpropanamide, the title compound was obtained.
MS (ESI+): [M+H]⁺421.4.

### Example 24

### 5-(difluoromethyl)-3-(2-(3-fluoropyridin-2-yl)-6-methyl-3,4-dihydro-2H-chromen-7-yl)-3,4-dihydropyrido[4,3-d]pyrimidin-2(1H)-one

### A) (3-methyl-4-nitropyridin-2-yl)methyl acetate

2,3-Dimethyl-4-nitropyridine 1-oxide (10.0 g) was added to acetic anhydride (100 mL). The reaction mixture was stirred at 100°C for 1.5 hr, and the solvent was evaporated under reduced pressure. Water was added to the residue, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (11.5 g).
¹H NMR (300 MHz, CDCl₃) δ 2.17 (3H, s), 5.35 (2H, s), 7.57 (1H, d, J = 5.3 Hz), 8.65 (1H, d, J = 5.3 Hz).

### B) 3-methyl-4-nitropyridin-2-yl)methanol

(3-Methyl-4-nitropyridin-2-yl)methyl acetate (11.5 g) was added to 1N aqueous sodium hydroxide solution (297 mL). The reaction mixture was stirred at room temperature for 2.5 hr, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (4.1 g).
MS (ESI+): [M+H]⁺169.1.

### C) 3-methyl-4-nitropyridine-2-carbaldehyde

To a solution of 3-methyl-4-nitropyridin-2-yl)methanol(4.1 g) in THF (80 mL) was added manganese dioxide (IV)(17.0 g). The reaction mixture was stirred at room temperature for 13 hr, and the insoluble material was filtered off by using celite. The filtrate was concentrated under reduced pressure to give the title compound (3.3 g).
MS (ESI+): [M+H]⁺167.1.

### D) 2-(difluoromethyl)-3-methyl-4-nitropyridine

To a solution of 3-methyl-4-nitropyridine-2-carbaldehyde (3.3 g) in toluene (66 mL) were added dropwise N,N-diethylaminosulfur trifluoride (4 mL) and ethanol (660 mL). The reaction mixture was stirred at room temperature for 4 hr, poured into 2N aqueous sodium hydroxide solution under ice-cooling, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (2.3 g).
¹H NMR (300 MHz, CDCl₃) δ 2.62 (3H, s), 6.80 (1H, d, J = 54.0 Hz), 7.70 (1H, d, J = 5.0 Hz), 8.67 (1H, d, J = 5.0 Hz).

### E) 3-(bromomethyl)-2-(difluoromethyl)-4-nitropyridine

To a solution of 2-(difluoromethyl)-3-methyl-4-nitropyridine (2.2 g) and N-bromosuccinimide (3.2 g) in trifluoromethylbenzene (45 mL) was added azodiisobutyronitrile (0.3 g). The reaction mixture was stirred under reflux for 2 hr, and N-bromosuccinimide (3.2 g) and azodiisobutyronitrile (0.3 g) were added. The reaction mixture was stirred under reflux for 2 hr, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (2.9 g).
¹H NMR (300 MHz, CDCl₃) δ 4.97 (2H, s), 6.68-7.11 (1H, m), 7.85 (1H, d, J = 5.3 Hz), 8.83 (1H, d, J = 4.9 Hz).

### F) N-((2-(difluoromethyl)-4-nitropyridin-3-yl)methyl)-2-(3-fluoropyridin-2-yl)-6-methylchromane-7-amine

To a solution of 3-(bromomethyl)-2-(difluoromethyl)-4-nitropyridine (227 mg) in N,N-dimethylacetamide (10 mL) was added 2-(3-fluoropyridin-2-yl)-6-methylchromane-7-amine (200 mg). The reaction mixture was stirred at 70°C for 1 hr, and 3-(bromomethyl)-2-(difluoromethyl)-4-nitropyridine (113 mg) was added. The reaction mixture was stirred at 70°C for 1 hr, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (239 mg).
MS (ESI+): [M+H]⁺445.4.

### G) 5-(difluoromethyl)-3-(2-(3-fluoropyridin-2-yl)-6-methyl-3,4-dihydro-2H-chromen-7-yl)-3,4-dihydropyrido[4,3-d]pyrimidin-2(1H)-one

To a solution of N-((2-(difluoromethyl)-4-nitropyridin-3-yl)methyl)-2-(3-fluoropyridin-2-yl)-6-methylchromane-7-amine (239 mg) and ammonium chloride (28 mg) in ethanol (3 mL)/water (3 mL) was added reduced iron (150 mg). The reaction mixture was stirred at 85°C for 1.5 hr, and the insoluble material was filtered off by using celite. The filtrate was concentrated under reduced pressure, and the residue was extracted with ethyl acetate. The extract was washed with saturated brine, and dried over magnesium sulfate. The solvent was evaporated under reduced pressure. The residue was dissolved in THF (6 mL), and CDI (258 mg) and DBU (238 µL) were added at room temperature. The reaction mixture was stirred at 50°C for 14 hr, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane), and recrystallized from ethyl acetate/hexane to give the title compound (56 mg).
MS (ESI+): [M+H]⁺441.2.

### Example 25

### 5-cyclopropyl-3-(2-(3-fluoropyridin-2-yl)-6-methyl-3,4-dihydro-2H-chromen-7-yl)-3,4-dihydropyrido[4,3-d]pyrimidin-2(1H)-one

### A) N-(2-cyclopropyl-3-formylpyridin-4-yl)-2,2-dimethylpropanamide

To a solution of N-(2-chloro-3-formylpyridin-4-yl)-2,2-dimethylpropanamide (600 mg), cyclopropylboronic acid (278 mg), tricyclohexylphosphine (70 mg) and tripotassium phosphate (1890 mg) in toluene (10 mL)/water (0.5 mL) was added palladium acetate (II) (28 mg) at room temperature. The reaction mixture was stirred under an argon atmosphere at 100°C for 22 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (460 mg). MS (ESI+): [M+H]⁺247.4.

### B) 5-(cyclopropyl)-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 1, step G to H and using the compound obtained in step A, the title compound was obtained.
MS (ESI+): [M+H]⁺431.2.

### Example 26

### 5-cyclopropyl-3-(2-(3-fluoropyridin-2-yl)-6-methyl-3,4-dihydro-2H-chromen-7-yl)-3,4-dihydropyrido[3,4-d]pyrimidin-2(1H)-one

### A) 3-cyclopropyl-4-methyl-5-nitropyridine

To a mixed solution of 3-bromo-4-methyl-5-nitropyridine (0.9 g), cyclopropylboronic acid (0.5 g), tricyclohexylphosphine (0.1 g) and tripotassium phosphate (2.8 g) in toluene (20 mL)/water (1 mL) was added palladium(II) acetate (0.05 g) at room temperature. The reaction mixture was stirred under an argon atmosphere at 100°C for 8 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (0.8 g). ¹H NMR (300 MHz, CDCl₃) δ 0.75 (2H, q, J = 5.1 Hz), 1.06-1.15 (2H, m), 1.89 (1H, tt, J = 8.5, 5.5 Hz), 2.63 (3H, s), 8.47 (1H, s), 8.87 (1H, s).

### B) 5-(cyclopropyl)-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 24, step E to G and using the compound obtained in step A, the title compound was obtained.
MS (ESI+): [M+H]⁺431.0.

### Example 27

### 5-ethyl-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 1, step G to H and using N-(2-ethyl-3-formylpyridin-4-yl)-2,2-dimethylpropanamide synthesized by a method similar to that in Example 25, step A, the title compound was obtained.
MS (ESI+) : [M+H]⁺419.2.

### Example 28

### 5-chloro-3-(2-(3-fluoropyridin-2-yl)-3,4-dihydro-2H-chromen-7-yl)-3,4-dihydropyrido[4,3-d]pyrimidin-2(1H)-one

In the same manner as in Example 1, step A to H and using 3-aminophenol, the title compound was obtained. MS (ESI+): [M+H]⁺411.4.

### Example 29

### 3-(2-(3-fluoropyridin-2-yl)-6-methyl-3,4-dihydro-2H-chromen-7-yl)-3,4-dihydropyrido[4,3-d]pyrimidin-2(1H)-one

To a mixed solution of 5-chloro-3-(2-(3-fluoropyridin-2-yl)-6-methyl-3,4-dihydro-2H-chromen-7-yl)-3,4-dihydropyrido[4,3-d]pyrimidin-2(1H)-one (200 mg), ethylboronic acid (47 mg), tricyclohexylphosphine (14 mg) and tripotassium phosphate (373 mg) in toluene (4 mL)/water (0.2 mL) was added palladium(II) acetate (6 mg) at room temperature. The reaction mixture was stirred under microwave irradiation at 200°C for 40 min, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (18 mg).
MS (ESI+): [M+H]⁺391.4.

### Example 30

### ethyl 3-(2-(3-fluoropyridin-2-yl)-6-methyl-3,4-dihydro-2H-chromen-7-yl)-2-oxo-1,2,3,4-tetrahydropyrido[4,3-d]pyrimidine-5-carboxylate

To a solution of 5-chloro-3-(2-(3-fluoropyridin-2-yl)-6-methyl-3,4-dihydro-2H-chromen-7-yl)-3,4-dihydropyrido[4,3-d]pyrimidin-2(1H)-one (640 mg), 1,1'-bis(diphenylphosphino)ferrocene (84 mg) and sodium acetate (248 mg) in ethanol (15 mL) was added palladium(II) acetate (17 mg) at room temperature. The reaction mixture was stirred under a carbon monoxide atmosphere at 100°C for 6 hr, and the insoluble material was filtered off by using celite. The filtrate was concentrated under reduced pressure, and the residue was purified by silica gel column chromatography (ethyl acetate/hexane) to give the title compound (308 mg).
MS (ESI+): [M+H]⁺463.1.

### Example 31

### 3-(2-(3-fluoropyridin-2-yl)-6-methyl-3,4-dihydro-2H-chromen-7-yl)-5-(2-methoxyethoxy)-3,4-dihydropyrido[3,4-d]pyrimidin-2(1H)-one

In the same manner as in Example 1, step G to H and using N-(4-formyl-5-(2-methoxyethoxy)pyridin-3-yl)-2,2-dimethylpropanamide, the title compound was obtained.
MS (ESI+) : [M+H]⁺421.4.

### Example 32

### N-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidine-5-carboxamide

To a mixed solution of ethyl 3-(2-(3-fluoropyridin-2-yl)-6-methyl-3,4-dihydro-2H-chromen-7-yl)-2-oxo-1,2,3,4-tetrahydropyrido[4,3-d]pyrimidine-5-carboxylate (365 mg) in THF (4 mL)/ethanol(4 mL) was added 1N aqueous sodium hydroxide solution (0.9 mL). The reaction mixture was stirred at room temperature for 3 hr, and 1N aqueous sodium hydroxide solution (0.2 mL) was added. The reaction mixture was concentrated under reduced pressure, ethyl acetate was added, and the aqueous phase was recovered. The aqueous phase was concentrated under reduced pressure, and the residue was dissolved in DMF (7 mL). Cyclopropylamine (54 mg), WSC (227 mg), HOBt (128 mg), and triethylamine (133 µL) were added, and the reaction mixture was stirred at room temperature for 4 hr. Cyclopropylamine (54 mg), WSC (227 mg), HOBt (128 mg), and triethylamine (133 µL) were added, and the reaction mixture was stirred at 50°C overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added saturated aqueous sodium hydrogen carbonate solution, and the mixture was extracted with ethyl acetate, dried over anhydrous magnesium sulfate, and concentrated under reduced pressure. The residue was recrystallized from ethyl acetate/hexane to give the title compound (61 mg).
¹H NMR (300 MHz, DMSO-d₆) δ 0.51-0.69 (4H, m), 2.05 (3H, s), 2.13-2.26 (1H, m), 2.25-2.40 (1H, m), 2.74-2.89 (2H, m), 2.91-3.03 (1H, m), 4.92-5.21 (2H, m), 5.44 (1H, d, J = 10.2 Hz), 6.82 (1H, s), 6.93 (1H, d, J = 5.3 Hz), 7.04 (1H, s), 7.45-7.59 (1H, m), 7.71-7.86 (1H, m), 8.27 (1H, d, J = 5.3 Hz), 8.48 (1H, d, J = 4.2 Hz), 8.68 (1H, d, J = 4.2 Hz), 10.0 (1H, s).

### Example 33

### 3-(2-(3-fluoropyridin-2-yl)-6-methyl-3,4-dihydro-2H-chromen-7-yl)-5-(2-hydroxypropan-2-yl)-3,4-dihydropyrido[4,3-d]pyrimidin-2(1H)-one

To a solution of ethyl 3-(2-(3-fluoropyridin-2-yl)-6-methyl-3,4-dihydro-2H-chromen-7-yl)-2-oxo-1,2,3,4-tetrahydropyrido[4,3-d]pyrimidine-5-carboxylate (250 mg) in THF (5 mL) was added methylmagnesium bromide THF solution (1 M, 2.2 mL). The reaction mixture was stirred at 0°C for 2 hr, and at room temperature for 2 hr. The reaction mixture was heated to 50°C, methylmagnesium bromide THF solution (1 M, 1.1 mL) was added and the mixture was stirred at 50°C for 2 hr. Water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the fraction with a shorter retention time was added saturated aqueous sodium hydrogen carbonate solution, and the mixture was extracted with ethyl acetate, dried over anhydrous magnesium sulfate, and concentrated under reduced pressure. The residue was recrystallized from ethyl acetate to give the title compound (50 mg).
MS (ESI+): [M+H]+449.3.

### Example 34

### 5-acetyl-3-(2-(3-fluoropyridin-2-yl)-6-methyl-3,4-dihydro-2H-chromen-7-yl)-3,4-dihydropyrido[4,3-d]pyrimidin-2(1H)-one

Saturated aqueous sodium hydrogen carbonate solution was added to an obtained fraction with a longer retention time, which was collected by HPLC (C18, mobile phase: water/acetonitrile (0.1% TFA-containing system)) in Example 33, and the mixture was extracted with ethyl acetate, dried over anhydrous magnesium sulfate, and concentrated under reduced pressure. The residue was recrystallized from ethyl acetate/hexane to give the title compound (40 mg).
MS (ESI+): [M+H]⁺433.3.

### Example 35

### 5-chloro-3-(2-(3-fluoropyridin-2-yl)-6-(trifluoromethyl)-3,4-dihydro-2H-chromen-7-yl)-3,4-dihydropyrido[4,3-d]pyrimidin-2(1H)-one

### A) N-[2-(3-fluoropyridin-2-yl)-6-iodo-3,4-dihydro-2H-chromen-7-yl]acetamide

Under a nitrogen atmosphere, to a solution of N-[2-(3-fluoropyridin-2-yl)-3,4-dihydro-2H-chromen-7-yl]acetamide (1.60 g) in ethanol (20 mL) were added silver sulfate (1.66 g) and iodine (1.35 g), and the mixture was stirred at room temperature overnight. Water was added to the reaction mixture, and the mixture was extracted with ethyl acetate. The obtained organic layer was washed with water and saturated brine, dried over anhydrous sodium sulfate, and the solvent was evaporated under reduced pressure. The residue was purified by silica gel column chromatography (ethyl acetate/hexane) to give the title compound (1.11 g).
MS (ESI+): [M+H]⁺413.2.

### B) N-[2-(3-fluoropyridin-2-yl)-6-(trifluoromethyl)-3,4-dihydro-2H-chromen-7-yl]acetamide

Under a nitrogen atmosphere, to a solution of N-[2-(3-fluoropyridin-2-yl)-6-iodo-3,4-dihydro-2H-chromen-7-yl]acetamide (300 mg) in DMF (5 mL) were added copper(I) iodide (170 mg) and methyl 2,2-difluoro-2-(fluorosulfonyl)acetate (0.46 mL) and the mixture was stirred at 80°C overnight. Water was added to the reaction mixture, and the mixture was extracted with ethyl acetate. The obtained organic layer was washed with water and saturated brine, dried over anhydrous sodium sulfate, and the solvent was evaporated under reduced pressure. The residue was purified by silica gel column chromatography (ethyl acetate/hexane) to give the title compound (80 mg).
MS (ESI+): [M+H]⁺355.3.

### C) 5-chloro-3-(2-(3-fluoropyridin-2-yl)-6-(trifluoromethyl)-3,4-dihydro-2H-chromen-7-yl)-3,4-dihydropyrido[4,3-d]pyrimidin-2(1H)-one

In the same manner as in Example 1, step F to H and using the compound obtained in step B, the title compound was obtained.
MS (ESI+) : [M+H]⁺479.0.

### Example 36

### 5-chloro-3-(2-(3-fluoropyridin-2-yl)-6-methyl-3,4-dihydro-2H-chromen-7-yl)-3,4-dihydropyrido[2,3-d]pyrimidin-2(1H)-one

In the same manner as in Example 1, step G to H and using N-(4-chloro-3-formylpyridin-2-yl)-2,2-dimethylpropanamide, the title compound was obtained.
MS (ESI+): [M+H]⁺425.3.

### Example 37

### 5-chloro-3-(2-(3-fluoropyridin-2-yl)-6-methyl-3,4-dihydro-2H-chromen-7-yl)-3,4-dihydropyrimido[4,5-d]pyrimidin-2(1H)-one

In the same manner as in Example 4, step L and using 4-amino-6-chloro-5-formylpyrimidine, the title compound was obtained.
MS (ESI+): [M+H]⁺426.2.

### Example 38

### 3-(2-(3-fluoropyridin-2-yl)-6-methyl-3,4-dihydro-2H-chromen-7-yl)-6-(2,2,2-trifluoroethyl)-4,6-dihydropyrido[4,3-d]pyrimidine-2,7(1H,3H)-dione

### A) 4-chloro-6-oxo-1-(2,2,2-trifluoroethyl)-1,6-dihydropyridine-3-carbaldehyde

To a solution of diketene (1.9 g) in toluene (50 mL) was added dropwise a solution (50 mL) of 2,2,2-trifluoroethylamine (2.0 g) in toluene over 10 min, and the mixture was stirred at room temperature for 48 hr. The solvent was evaporated under reduced pressure, and the obtained solid was washed with hexane to give crude 3-oxo-N-(2,2,2-trifluoroethyl)butanamide. Under a nitrogen atmosphere at 0°C, phosphorus oxychloride (15.5 mL) was added dropwise to anhydrous dimethylformamide (100 mL), and the mixture was stirred at the same temperature for 1 hr. The obtained reaction mixture was added dropwise a solution (100 mL) of crude 3-oxo-N-(2,2,2-trifluoroethyl)butanamide in dimethylformamide and the mixture was stirred at 80°C for 4 hr. To the reaction mixture was added saturated brine, and the mixture was extracted with ethyl acetate. The obtained organic layer was washed with water and saturated brine, dried over anhydrous sodium sulfate, and the solvent was evaporated under reduced pressure. The residue was purified by silica gel column chromatography (ethyl acetate/hexane) to give the title compound (380 mg).
¹H NMR (300 MHz, DMSO-d₆) δ 5.00 (2H, q), 6.83 (1H, s), 8.61 (1H, s), 9.84 (1H, s).

### B) 4-azido-6-oxo-1-(2,2,2-trifluoroethyl)-1,6-dihydropyridine-3-carbaldehyde

Under a nitrogen atmosphere, to an aqueous acetone solution (acetone/water = 4/3, 14 mL) of 4-chloro-6-oxo-1-(2,2,2-trifluoroethyl)-1,6-dihydropyridine-3-carbaldehyde (630 mg) was added sodium azide (210 mg) and the mixture was stirred at 45°C overnight. Water was added to the reaction mixture, and the mixture was extracted with ethyl acetate. The obtained organic layer was washed with water and saturated brine, dried over anhydrous sodium sulfate, and the solvent was evaporated under reduced pressure. The residue was purified by silica gel column chromatography (ethyl acetate/hexane) to give the title compound (320 mg).
¹H NMR (300 MHz, DMSO-d₆) δ 4.98 (2H, q), 6.44 (1H, s), 8.52 (1H, s), 9.75 (1H, s).

### C) 4-amino-6-oxo-1-(2,2,2-trifluoroethyl)-1,6-dihydropyridine-3-carbaldehyde

To a solution of 4-azido-6-oxo-1-(2,2,2-trifluoroethyl)-1,6-dihydropyridine-3-carbaldehyde (320 mg) in methanol (10 mL) was added (+/-)-camphorsulfonic acid (30 mg), and the mixture was stirred at room temperature overnight. The reaction mixture was neutralized with saturated aqueous sodium hydrogen carbonate solution, and extracted with ethyl acetate. The obtained organic layer was washed with water and saturated brine, dried over anhydrous sodium sulfate, and the solvent was evaporated under reduced pressure. An ethanol solution (5 mL) of the residue was added to a solution of cobalt borohydride in ethanol which was prepared by addition of 2,2'-bipyridyl (60 mg) and sodium borohydride (60 mg) to a solution of cobalt bromide (28 mg) in ethanol (5 mL) at 5-10°C (water bath). The mixture was stirred at 5-10°C (water bath) for 1 hr. To the reaction mixture was added acetic acid (5 mL), and the mixture was stirred at room temperature for 30 min. The solvent was evaporated under reduced pressure. The residue was neutralized with saturated aqueous sodium hydrogen carbonate solution, and extracted with ethyl acetate. The obtained organic layer was washed with saturated brine, dried over anhydrous sodium sulfate, and the solvent was evaporated under reduced pressure. To the residue was added 2N hydrochloric acid and the mixture was stirred at 60°C for 1 hr. The reaction mixture was neutralized with saturated aqueous sodium hydrogen carbonate solution, and extracted with ethyl acetate. The obtained organic layer was washed with water and saturated brine, dried over anhydrous sodium sulfate, and the solvent was evaporated under reduced pressure. The residue was purified by silica gel column chromatography (ethyl acetate/hexane) to give the title compound (160 mg).
¹H NMR (300 MHz, DMSO-d₆) δ 4.81 (2H, q, J = 9.3 Hz), 5.34 (1H, s), 7.23 (2H, s), 8.38 (1H, s), 9.52 (1H, s).

### D) 3-(2-(3-fluoropyridin-2-yl)-6-methyl-3,4-dihydro-2H-chromen-7-yl)-6-(2,2,2-trifluoroethyl)-4,6-dihydropyrido[4,3-d]pyrimidine-2,7(1H,3H)-dione

In the same manner as in Example 4, step L and using the compound obtained in step C, the title compound was obtained. MS (ESI+): [M+H]⁺489.1.

### Example 39

### 3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one

In the same manner as in Example 1, step G to H and using N-(2-cyclopropyl-3-formylpyridin-4-yl)-2,2-dimethylpropanamide and 6-chloro-2-(3-fluoropyridin-2-yl)chromane-7-amine, the title compound was obtained.
MS (ESI+) : [M+H]⁺451.3.

### Example 40

### 3-(2-(3-fluoropyridin-2-yl)-6-methyl-3,4-dihydro-2H-chromen-7-yl)-3,4-dihydropyrido[2,3-d]pyrimidin-2(1H)-one

Under a hydrogen (1 atm) atmosphere, to a solution of 5-chloro-3-[2-(3-fluoropyridin-2-yl)-6-methyl-3,4-dihydro-2H-chromen-7-yl]-3,4-dihydropyrido[2,3-d]pyrimidin-2(1H)-one (100 mg) in THF (6 mL) was added palladium hydroxide (50 mg), and the mixture was stirred at room temperature overnight. Palladium hydroxide was removed by celite. The solvent was evaporated under reduced pressure and the residue was purified by silica gel column chromatography (ethyl acetate/hexane) to give the title compound (10 mg).
MS (ESI+) : [M+H]⁺391.3.

### Example 41

### 5,7-dichloro-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 1, step G to H and using N-(2,6-dichloro-3-formylpyridin-4-yl)-2,2-dimethylpropanamide, the title compound was obtained.
MS (ESI+) : [M+H]⁺459.1.

### Example 42

### 5-cyclopropyl-3-(2-(3-fluoropyridin-2-yl)-6-methyl-3,4-dihydro-2H-chromen-7-yl)-7-(oxetan-3-yl)-3,4-dihydropyrido[4,3-d]pyrimidin-2(1H)-one

### A) N-(2-cyclopropyl-3-formyl-6-(oxetan-3-yl)pyridin-4-yl)-2,2-dimethylpropanamide

To a solution of N-(2-cyclopropyl-3-formylpyridin-4-yl)-2,2-dimethylpropanamide (600 mg), concentrated sulfuric acid (502 µL), 3-iodooxetane (896 mg) and iron(II) sulfate heptahydrate (203 mg) in DMSO (10 mL) was added dropwise 30% aqueous hydrogen peroxide solution (830 µL) at room temperature. After stirring at room temperature for 10 min, iron(II) sulfate heptahydrate (203 mg) was added, and the reaction mixture was stirred at room temperature for 20 min. Iron(II) sulfate heptahydrate (203 mg) and 30% aqueous hydrogen peroxide solution (830 µL) were added, and the reaction mixture was stirred at room temperature for 30 min. The reaction mixture was poured into saturated aqueous sodium hydrogen carbonate solution, and the mixture was extracted with ethyl acetate. The extract was washed with saturated brine, and dried over magnesium sulfate. The solvent was evaporated under reduced pressure. The residue was purified by silica gel column chromatography (ethyl acetate/hexane) to give the title compound (160 mg).
MS (ESI+) : [M+H]⁺303.4.

### B) 5-cyclopropyl-3-(2-(3-fluoropyridin-2-yl)-6-methyl-3,4-dihydro-2H-chromen-7-yl)-7-(oxetan-3-yl)-3,4-dihydropyrido[4,3-d]pyrimidin-2(1H)-one

In the same manner as in Example 1, step G to H, the title compound was obtained.
MS (ESI+) : [M+H]⁺487.4.

### Example 43

### 6-chloro-3-(2-(3-fluoropyridin-2-yl)-6-methyl-3,4-dihydro-2H-chromen-7-yl)-3,4-dihydropyrido[3,4-d]pyrimidin-2(1H)-one

### A) tert-butyl (6-chloro-4-(hydroxymethyl)pyridin-3-yl)carbamate

To a solution of 5-((tert-butoxycarbonyl)amino)-2-chloroisonicotinic acid (545 mg) in THF (15 mL) were added triethylamine (0.335 mL) and isobutyl chloroformate (0.285 mL) at room temperature. The reaction mixture was stirred at room temperature for 30 min, and the precipitated solid was filtered off. The residue was washed with THF (5 mL). To the obtained filtrate was added sodium borohydride (151 mg) suspended in water (0.6 mL) at room temperature. The mixture was stirred at room temperature for 90 min, saturated aqueous ammonium chloride solution was added, and the mixture was extracted with ethyl acetate. The extract was washed with saturated brine, and dried over sodium sulfate. The solvent was evaporated under reduced pressure and the residue was purified by silica gel column chromatography (ethyl acetate/hexane) to give the title compound (268 mg).
¹H NMR (400 MHz, DMSO-d₆) δ 1.38-1.66 (9H, s), 2.87 (1H, brs), 4.68 (2H, s), 7.16 (1H, s), 7.49 (1H, brs), 8.85 (1H, s).

### B) tert-butyl (6-chloro-4-formylpyridin-3-yl)carbamate

To a solution of tert-butyl (6-chloro-4-(hydroxymethyl)pyridin-3-yl)carbamate (268 mg) in THF (10.0 mL) was added manganese dioxide (IV) (450 mg), and the mixture was stirred at room temperature overnight. To the reaction mixture was added manganese dioxide (IV) (630 mg) and the mixture was stirred at room temperature for 6 hr, and at 50°C overnight. To the reaction mixture was added manganese dioxide (IV) (450 mg), and the mixture was stirred at 50°C overnight. The insoluble material was filtered off by using celite. The filtrate was concentrated under reduced pressure, and the residue was purified by silica gel column chromatography (ethyl acetate/hexane) to give the title compound (168 mg).
¹H NMR (300 MHz, CDCl₃) δ 1.55 (9H, s), 7.54 (1H, s), 9.61 (1H, s), 9.75 (1H, brs), 9.93 (1H, s).

### C) 6-chloro-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 1, step G to H and using the compound obtained in step B, the title compound was obtained.
MS (ESI+) : [M+H]⁺425.0.

### Example 44

### 5-chloro-3-(6-fluoro-2-(3-fluoropyridin-2-yl)-3,4-dihydro-2H-chromen-7-yl)-3,4-dihydropyrido[4,3-d]pyrimidin-2(1H)-one

In the same manner as in Example 1, step A to H and using 3-amino-4-fluorophenol, the title compound was obtained.
MS (ESI+): [M+H]⁺421.4.

### Example 45

### 3-(2-(3-fluoropyridin-2-yl)-6-methyl-3,4-dihydro-2H-chromen-7-yl)-3,4-dihydropyrido[3,2-d]pyrimidin-2(1H)-one

In the same manner as in Example 4, step L and using 3-amino-2-formylpyridine, the title compound was obtained.
MS (ESI+): [M+H]⁺391.1.

### Example 46

### 3-(2-(3-fluoropyridin-2-yl)-6-methyl-3,4-dihydro-2H-chromen-7-yl)-5-(1,3-oxazol-2-yl)-3,4-dihydropyrido[4,3-d]pyrimidin-2(1H)-one

To a solution of 5-chloro-3-(2-(3-fluoropyridin-2-yl)-6-methyl-3,4-dihydro-2H-chromen-7-yl)-3,4-dihydropyrido[4,3-d]pyrimidin-2(1H)-one (200 mg) and 2-(tributylstanyl)-1,3-oxazole (253 mg) in DMF (4 mL) was added 1,1'-bis(diphenylphosphino)ferrocene-palladium(II)dichloride-dichloromethane complex (38 mg) at room temperature. The reaction mixture was stirred under microwave irradiation at 200°C for 40 min, and the insoluble material was filtered off by using celite. Water was added to the filtrate, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate to give the title compound (18 mg).
MS (ESI+) : [M+H]⁺458.5.

### Example 47

### 7-chloro-3-(2-(3-fluoropyridin-2-yl)-6-methyl-3,4-dihydro-2H-chromen-7-yl)-3,4-dihydropyrido[4,3-d]pyrimidin-2(1H)-one

### A) (4-amino-6-chloropyridin-3-yl)methanol

To a solution of methyl 4,6-dichloronicotinate (1.5 g) in DMF (30 mL) was added sodium azide (710 mg), and the mixture was stirred at 50°C overnight. The reaction mixture was cooled to room temperature, water was added, and the mixture was extracted with ethyl acetate. The extract was washed with water and saturated brine, and dried over sodium sulfate. The solvent was evaporated under reduced pressure. To the residue was added hydroiodic acid (20 mL), and the mixture was stirred at room temperature for 2 hr. The reaction mixture was diluted with ethyl acetate, washed with saturated aqueous sodium thiosulfate solution and saturated brine, and dried over sodium sulfate. The solvent was evaporated under reduced pressure. The residue was dissolved in THF (54.0 mL) and a solution of lithium aluminum hydride (1164 mg) in THF (100 mL) was added dropwise at 0°C. The reaction mixture was stirred at 0°C for 10 min, sodium sulfate decahydrate was added, and the insoluble material was filtered off by using celite. The residue was purified by silica gel column chromatography (ethyl acetate/hexane) to give the title compound (359 mg).
¹H NMR (300 MHz, CDCl₃) δ 2.37 (1H, brs), 4.65 (2H, s), 4.91 (2H, brs), 6.56 (1H, s), 7.78 (1H, s).

### B) 7-chloro-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 4, step K to L and using the compound obtained in step A, the title compound was obtained.
MS (ESI+): [M+H]⁺425.3.

### Example 48

### 6-chloro-3-(2-(3-fluoropyridin-2-yl)-6-methyl-3,4-dihydro-2H-chromen-7-yl)-3,4-dihydropyrido[2,3-d]pyrimidin-2(1H)-one

### A) (2-amino-5-chloropyridin-3-yl)methanol

To a solution of ethyl 2-aminonicotinate (831 mg) in concentrated hydrochloric acid (5.0 mL) was added aqueous hydrogen peroxide solution (0.511 mL), and the mixture was stirred at 60°C for 2 hr. The reaction mixture was cooled to room temperature, adjusted to pH 8.0 with saturated aqueous sodium hydrogen carbonate solution, and the precipitated solid was filtered off. The residue was washed with water, suspended in toluene. The solvent was evaporated under reduced pressure. The residue was dissolved in THF (10.0 mL) solution, and added dropwise to a solution of lithium aluminum hydride (444 mg) in THF (20 mL) at 0°C. The reaction mixture was stirred at 0°C for 10 min, sodium sulfate decahydrate was added, and the insoluble material was filtered off with celite. The residue was purified by silica gel column chromatography (ethyl acetate/hexane) to give the title compound (312 mg).
¹H NMR (300 MHz, CDCl₃) δ 1.72-1.84 (1H, m), 4.60 (2H, d, J = 4.9 Hz), 4.97 (2H, brs), 7.31 (1H, d, J = 2.5 Hz), 7.98 (1H, d, J = 2.5 Hz).

### B) 6-chloro-3-(2-(3-fluoropyridin-2-yl)-6-methyl-3,4-dihydro-2H-chromen-7-yl)-3,4-dihydropyrido[2,3-d]pyrimidin-2(1H)-one

In the same manner as in Example 4, step K to L and using the compound obtained in step A, the title compound was obtained.
MS (ESI+): [M+H]⁺425.3.

### Example 49

### 5-cyclopropyl-3-(2-(3-fluoropyridin-2-yl)-6-methyl-3,4-dihydro-2H-chromen-7-yl)-7-(1-(1H-imidazol-1-ylcarbonyl)azetidin-3-yl)-3,4-dihydropyrido[4,3-d]pyrimidin-2(1H)-one

### A) tert-butyl 3-(6-cyclopropyl-4-((2,2-dimethylpropanoyl)amino)-5-formylpyridin-2-yl)azetidine-1-carboxylate

In the same manner as in Example 42, step A and using N-(2-cyclopropyl-3-formylpyridin-4-yl)-2,2-dimethylpropanamide, the title compound was obtained.
MS (ESI+): [M+H]⁺402.4.

### B) tert-butyl 3-(4-amino-6-cyclopropyl-5-(((2-(3-fluoropyridin-2-yl)-6-methyl-3,4-dihydro-2H-chromen-7-yl)amino)methyl)pyridin-2-yl)azetidine-1-carboxylate

In the same manner as in Example 1 , step G and using the compound obtained in step A, the title compound was obtained.
MS (ESI+): [M+H]⁺644.8.

### C) 5-cyclopropyl-3-(2-(3-fluoropyridin-2-yl)-6-methyl-3,4-dihydro-2H-chromen-7-yl)-7-(1-(1H-imidazol-1-ylcarbonyl)azetidin-3-yl)-3,4-dihydropyrido[4,3-d]pyrimidin-2(1H)-one

To a solution of tert-butyl 3-(4-amino-6-cyclopropyl-5-(((2-(3-fluoropyridin-2-yl)-6-methyl-3,4-dihydro-2H-chromen-7-yl)amino)methyl)pyridin-2-yl)azetidine-1-carboxylate (212 mg) in methanol (2 mL) was added sodium methoxide (89 mg), and the reaction mixture was stirred under reflux for 3 hr. 3N Hydrochloric acid (0.1 mL) was added, and the reaction mixture was stirred at 85°C for 20 min. Saturated aqueous sodium hydrogen carbonate solution was added and the mixture was extracted with ethyl acetate. The extract was washed with saturated brine, and dried over magnesium sulfate. The solvent was evaporated under reduced pressure. The residue was dissolved in THF (4 mL), and CDI (160 mg) and DBU (0.3 mL) were added. The reaction mixture was stirred at 50°C overnight, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added saturated aqueous sodium hydrogen carbonate solution, and the mixture was extracted with ethyl acetate, dried over anhydrous magnesium sulfate, and concentrated under reduced pressure to give the title compound (18 mg).
MS (ESI+): [M+H]⁺580.4.

### Example 50

### 5-cyclopropyl-3-(2-(3-fluoropyridin-2-yl)-6-methyl-3,4-dihydro-2H-chromen-7-yl)-3,4-dihydropyrido[2,3-d]pyrimidin-2(1H)-one

In the same manner as in Example 25, step A and using N-(4-chloro-3-formylpyridin-2-yl)-2,2-dimethylpropanamide, N-(4-cyclopropyl-3-formylpyridin-2-yl)-2,2-dimethylpropanamide was synthesized, and in the same manner as in Example 1, step G to H, the title compound was obtained.
MS (ESI+) : [M+H]⁺431.3.

### Example 51

### 5-chloro-3-(6-methyl-2-(pyridin-2-yl)-3,4-dihydro-2H-chromen-7-yl)-3,4-dihydropyrido[4,3-d]pyrimidin-2(1H)-one

### A) 6-methyl-2-(pyridin-2-yl)chromane-7-amine

In the same manner as in Example 1, step A to D and using 3-amino-4-methylphenol, the title compound was obtained.
MS (ESI+): [M+H]⁺241.4.

### B) 5-chloro-3-(6-methyl-2-(pyridin-2-yl)-3,4-dihydro-2H-chromen-7-yl)-3,4-dihydropyrido[4,3-d]pyrimidin-2(1H)-one

In the same manner as in Example 1, step G to H and using the compound obtained in step A, the title compound was obtained.
MS (ESI+): [M+H]⁺407.0.

### Example 52

### 5,7-dicyclopropyl-3-(2-(3-fluoropyridin-2-yl)-6-methyl-3,4-dihydro-2H-chromen-7-yl)-3,4-dihydropyrido[4,3-d]pyrimidin-2(1H)-one

### A) 4-amino-2,6-dicyclopropylnicotinaldehyde

In the same manner as in Example 25, step A, the title compound was obtained.
MS (ESI+): [M+H]⁺203.3.

### B) 5,7-dicyclopropyl-3-(2-(3-fluoropyridin-2-yl)-6-methyl-3,4-dihydro-2H-chromen-7-yl)-3,4-dihydropyrido[4,3-d]pyrimidin2(1H)-one

In the same manner as in Example 4, step L and using the compound obtained in step A, the title compound was obtained.
MS (ESI+): [M+H]⁺471.2.

### Example 53

### 5,8-dichloro-3-(6-chloro-2-(3-fluoropyridin-2-yl)-3,4-dihydro-2H-chromen-7-yl)-3,4-dihydropyrido[4,3-d]pyrimidin-2(1H)-one

### A) 4-amino-2,5-dichloronicotinaldehyde

To a solution of 4-amino-2-chloronicotinaldehyde (1.0 g) in DMF (5 mL) was added N-chlorosuccinimide (0.98 g) at room temperature. The reaction mixture was stirred at room temperature for 5 hr, water was added, precipitated solid was collected by filtration to give the title compound (0.91 g).
MS (ESI+): [M+H]⁺190.9.

### B) 5,8-dichloro-3-(6-chloro-2-(3-fluoropyridin-2-yl)-3,4-dihydro-2H-chromen-7-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺479.1.

### Example 54

### 8-chloro-3-(6-chloro-2-(3-fluoropyridin-2-yl)-3,4-dihydro-2H-chromen-7-yl)-3,4-dihydropyrido[3,4-d]pyrimidin-2(1H)-one

### A) 2-chloro-4-(((6-chloro-2-(3-fluoropyridin-2-yl)-3,4-dihydro-2H-chromen-7-yl)amino)methyl)pyridin-3-amine

To a suspension of lithium aluminum hydride (244 mg) in THF (10.0 mL) was added dropwise a solution of methyl 3-amino-2-chloroisonicotinate (300 mg) in THF (6.0 mL) at 0°C, and the mixture was stirred at 0°C for 10 min. To the reaction mixture was added sodium sulfate decahydrate, and the insoluble material was filtered off with celite, and the filtrate was concentrated under reduced pressure. The residue was dissolved in THF (4.0 mL) and toluene (12.0 mL), manganese dioxide (IV) (704 mg) was added, and the mixture was stirred at 50°C overnight. The reaction mixture was cooled to room temperature, and the insoluble material was filtered off by using celite. The filtrate was concentrated under reduced pressure. To a solution of the residue in toluene (7.7 mL) was added 6-chloro-2-(3-fluoropyridin-2-yl)chromane-7-amine (120 mg). p-Toluenesulfonic acid monohydrate (14.6 mg) was added, and the mixture was stirred under a nitrogen atmosphere at 100°C overnight. After cooling to room temperature, the mixture was extracted with ethyl acetate and saturated aqueous sodium hydrogen carbonate solution. The extract was washed with saturated brine, and dried over sodium sulfate. The solvent was evaporated under reduced pressure. To a suspension of lithium aluminum hydride (117 mg) in THF (5.0 mL) was added dropwise a solution of the residue in THF (2.0 mL) at 0°C, and the mixture was stirred at 0°C for 15 min. To the reaction mixture was added sodium sulfate decahydrate, and the insoluble material was filtered off with celite. The filtrate was concentrated under reduced pressure. The residue was purified by silica gel column chromatography (ethyl acetate/hexane) to give the title compound (194 mg).
¹H NMR (300 MHz, CDCl₃) δ 2.10-2.23 (1H, m), 2.30-2.46 (1H, m), 2.71-2.84 (1H, m), 2.86-3.01 (1H, m), 4.17-4.24 (2H, m), 4.27-4.36 (1H, m), 4.46 (2H, s), 5.36-5.45 (1H, m), 6.26 (1H, s), 7.01-7.07 (2H, m), 7.27-7.34 (1H, m), 7.44 (1H, ddd, J = 9.8, 8.3, 1.4 Hz), 7.77 (1H, d, J = 4.9 Hz), 8.47 (1H, dt, J = 4.4, 1.4 Hz).

### B) 8-chloro-3-(6-chloro-2-(3-fluoropyridin-2-yl)-3,4-dihydro-2H-chromen-7-yl)-3,4-dihydropyrido[3,4-d]pyrimidin-2(1H)-one

In the same manner as in Example 7, step G and using the compound obtained in step A, the title compound was obtained. MS (ESI+): [M+H]⁺445.3.

### Example 55

### 5-chloro-3-(6-chloro-2-(3-fluoropyridin-2-yl)-3,4-dihydro-2H-chromen-7-yl)-1-(2-methoxyethyl)-3,4-dihydropyrido[4,3-d]pyrimidin-2(1H)-one

To a solution of 5-chloro-3-(2-(3-fluoropyridin-2-yl)-6-chloro-3,4-dihydro-2H-chromen-7-yl)-3,4-dihydropyrido[4,3-d]pyrimidin-2(1H)-one (200 mg) in DMF (4 mL) was added sodium hydride (60%, oil) (27 mg), and the mixture was stirred at 0°C for 10 min. 1-Bromo-2-methoxyethane (94 mg) was added, and the mixture was stirred at room temperature for 1 hr, and at 50°C for 2 hr. Sodium hydride (60%, oil) (27 mg) and 1-bromo-2-methoxyethane (94 mg) were added, and the mixture was stirred at 50°C overnight. Water was added, and the mixture was filtered. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (48 mg).
MS (ESI+): [M+H]⁺503.3.

### Example 56

### ethyl 3-(6-chloro-2-(3-fluoropyridin-2-yl)-3,4-dihydro-2H-chromen-7-yl)-2-oxo-1,2,3,4-tetrahydropyrido[4,3-d]pyrimidine-5-carboxylate

In the same manner as in Example 30 and using 5-chloro-3-(6-chloro-2-(3-fluoropyridin-2-yl)-3,4-dihydro-2H-chromen-7-yl)-3,4-dihydropyrido[4,3-d]pyrimidin-2(1H)-one, the title compound was obtained.
MS (ESI+) : [M+H]⁺483.4.

### Example 57

3-(6-chloro-2-(3-fluoropyridin-2-yl)-3,4-dihydro-2H-chromen-7-yl)-5-(trifluoromethyl)-3,4-dihydropyrido[2,3-d]pyrimidin-2(1H)-one

### A) ethyl 2-chloro-4-(trifluoromethyl)nicotinate

To a solution of diisopropylamine (2.84 mL) in tetrahydrofuran (10.0 mL) was added n-hexane solution (12.6 mL) of n-butyllithium at -78°C. The reaction mixture was stirred at room temperature for 30 min and cooled to -78°C. To the reaction mixture was added dropwise a solution of 2-chloro-4-(trifluoromethyl)pyridine in THF (10.0 mL) at -78°C. The reaction mixture was stirred at -78°C for 90 min, and a solution of ethyl chloroformate (2.80 mL) in THF (10.0 mL) was added dropwise at -78°C. The reaction mixture was stirred at - 78°C for 1 hr, saturated aqueous sodium hydrogen carbonate solution was added, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (1.97 g).
¹H NMR (300 MHz, CDCl₃) δ 1.42 (3H, t, J = 7.1 Hz), 4.41-4.54 (2H, m), 7.53 (1H, d, J = 5.2 Hz), 8.63 (1H, d, J = 5.2 Hz).

### B) ethyl 2-((4-methoxybenzyl)amino)-4-(trifluoromethyl)nicotinate

To a solution of ethyl 2-chloro-4-(trifluoromethyl)nicotinate (750 mg) in DMSO (15 mL) were added triethylamine (0.618 mL) and 1-(4-methoxyphenyl)methanamine (0.574 mL), and the mixture was stirred at 70°C for 1 hr. The reaction mixture was cooled to 0°C, water was added to the reaction mixture, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (590 mg).
¹H NMR (300 MHz, CDCl₃) δ 1.35 (3H, t, J = 7.1 Hz), 3.80 (3H, s), 4.35 (2H, q, J = 7.1 Hz), 4.61 (2H, d, J = 5.2 Hz), 6.79-6.90 (4H, m), 7.00 (1H, brs), 7.27-7.30 (1H, m), 8.35 (1H, d, J = 4.9 Hz).

### C) 2-((4-methoxybenzyl)amino)-4-(trifluoromethyl)nicotinaldehyde

To a suspension of lithium aluminum hydride (97 mg) in THF (4.0 mL) was added dropwise a solution of ethyl 2-((4-methoxybenzyl)amino)-4-(trifluoromethyl)nicotinate (226 mg) in THF (2.4 mL) at 0°C, and the mixture was stirred at 0°C for 10 min. To the reaction mixture was added sodium sulfate decahydrate, and the insoluble material was filtered off with celite, and the filtrate was concentrated under reduced pressure. The residue was dissolved in THF (1.5 mL) and toluene (4.5 mL), manganese dioxide (IV) (267 mg) was added, and the mixture was stirred at 50°C overnight. To the reaction mixture was added manganese dioxide (IV) (802 mg), and the mixture was stirred at 70°C for 5 hr. The reaction mixture was cooled to room temperature, and the insoluble material was filtered off by using celite. The filtrate was concentrated under reduced pressure. The residue was purified by silica gel column chromatography (ethyl acetate/hexane) to give the title compound (130 mg).
¹H NMR (300 MHz, CDCl₃) δ 3.78-3.82 (3H, m), 4.74 (2H, d, J = 5.5 Hz), 6.82-6.92 (3H, m), 7.26-7.28 (1H, m), 7.28-7.31 (1H, m), 8.49 (1H, d, J = 4.9 Hz), 9.49 (1H, brs), 10.25 (1H, q, J = 2.1 Hz).

### D) 3-(((6-chloro-2-(3-fluoropyridin-2-yl)-3,4-dihydro-2H-chromen-7-yl)amino)methyl)-N-(4-methoxybenzyl)-4-(trifluoromethyl)pyridin-2-amine

To a solution of 2-((4-methoxybenzyl)amino)-4-(trifluoromethyl)nicotinaldehyde (199 mg) in toluene (5.0 mL) were added 6-chloro-2-(3-fluoropyridin-2-yl)chromane-7-amine (179 mg) and, p-toluenesulfonic acid monohydrate (48.8 mg), and the mixture was heated under reflux under a nitrogen atmosphere for 2 hr. After cooling to room temperature, the mixture was extracted with ethyl acetate and saturated aqueous sodium hydrogen carbonate solution. The extract was washed with saturated brine and dried over sodium sulfate. The solvent was evaporated under reduced pressure. To a suspension of lithium aluminum hydride (110 mg) in THF (4.0 mL) was added dropwise a solution of the residue in THF (3.0 mL) at 0°C, and the mixture was stirred at 0°C for 20 min. To the reaction mixture was added sodium sulfate decahydrate, and the insoluble material was filtered off with celite. The filtrate was concentrated under reduced pressure. The residue was purified by silica gel column chromatography (ethyl acetate/hexane) to give the title compound (199 mg).
¹H NMR (300 MHz, CDCl₃) δ 2.11-2.25 (1H, m), 2.30-2.51 (1H, m), 2.72-2.85 (1H, m), 2.86-3.03 (1H, m), 3.78 (3H, s), 3.88 (1H, t, J = 5.6 Hz), 4.20 (2H, d, J = 5.5 Hz), 4.61 (2H, d, J = 5.5 Hz), 5.40-5.47 (1H, m), 5.86 (1H, t, J = 5.6 Hz), 6.45 (1H, s), 6.76-6.87 (3H, m), 7.03 (1H, s), 7.19-7.25 (2H, m), 7.27-7.36 (1H, m), 7.45 (1H, ddd, J = 9.8, 8.4, 1.4 Hz), 8.25 (1H, d, J = 4.7 Hz), 8.48 (1H, dt, J = 4.6, 1.4 Hz).

### E) 3-(((6-chloro-2-(3-fluoropyridin-2-yl)-3,4-dihydro-2H-chromen-7-yl)amino)methyl)-4-(trifluoromethyl)pyridin-2-amine

A reaction mixture of 3-(((6-chloro-2-(3-fluoropyridin-2-yl)-3,4-dihydro-2H-chromen-7-yl)amino)methyl)-N-(4-methoxybenzyl)-4-(trifluoromethyl)pyridin-2-amine (199 mg) and trifluoroacetic acid (1.0 mL) was stirred at 70°C for 40 min, and cooled to room temperature. To the reaction mixture was added saturated aqueous sodium hydrogen carbonate solution, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (90 mg).
MS (ESI+): [M+H]⁺453.2.

### F) 3-(6-chloro-2-(3-fluoropyridin-2-yl)-3,4-dihydro-2H-chromen-7-yl)-5-(trifluoromethyl)-3,4-dihydropyrido[2,3-d]pyrimidin-2(1H)-one

In the same manner as in Example 7, step G and using the compound obtained in step E, the title compound was obtained.
MS (ESI+) : [M+H]⁺479.3.

### Example 58

### 3-(6-chloro-2-(3-fluoropyridin-2-yl)-3,4-dihydro-2H-chromen-7-yl)-5-(morpholin-4-yl)-3,4-dihydropyrido[4,3-d]pyrimidin-2(1H)-one

### A) 4-amino-2-(morpholin-4-yl)nicotinaldehyde

To a solution of 4-amino-2-chloronicotinaldehyde (200 mg) and triethylamine (1.8 mL) in DMF (4 mL) was added morpholine (445 mg), and the mixture was stirred at 120°C overnight. Water was added and the mixture was filtered. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (232 mg).
MS (ESI+): [M+H]⁺208.0.

### B) 3-(6-chloro-2-(3-fluoropyridin-2-yl)-3,4-dihydro-2H-chromen-7-yl)-5-(morpholin-4-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺496.1.

### Example 59

### 3-(6-chloro-2-(3-fluoropyridin-2-yl)-3,4-dihydro-2H-chromen-7-yl)-5-cyclopropyl-7-(morpholin-4-yl)-3,4-dihydropyrido[4,3-d]pyrimidin-2(1H)-one

### A) 4-amino-2-cyclopropyl-6-(morpholin-4-yl)nicotinaldehyde

To a mixed solution of 4-amino-2,6-dichloronicotinaldehyde (1000 mg), cyclopropylboronic acid (472 mg), tricyclohexylphosphine (147 mg) and tripotassium phosphate (3890 mg) in toluene (16 mL)/water (0.8 mL) was added palladium(II) acetate (55 mg) at room temperature. The reaction mixture was stirred under microwave irradiation at 180°C for 3 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane), and the obtained resultant product was dissolved in DMSO (5 mL). Triethylamine (1.1 mL) and morpholine (684 mg) were added, and the mixture was stirred at 120°C for 5 hr. Water was added and the mixture was filtered. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (165 mg).
MS (ESI+) : [M+H]⁺248.2.

### B) 3-(6-chloro-2-(3-fluoropyridin-2-yl)-3,4-dihydro-2H-chromen-7-yl)-5-cyclopropyl-7-(morpholin-4-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺536.0.

### Example 60

### 3-(6-chloro-2-(3-fluoropyridin-2-yl)-3,4-dihydro-2H-chromen-7-yl)-5-(1-methyl-1H-pyrazol-5-yl)-3,4-dihydropyrido[4,3-d]pyrimidin-2(1H)-one

### A) N-(3-formyl-2-(1-methyl-1H-pyrazol-5-yl)pyridin-4-yl)-2,2-dimethylpropanamide

To a mixture of N-(2-chloro-3-formylpyridin-4-yl)-2,2-dimethylpropanamide (481 mg), 1-methyl-5-(4,4,5,5-tetramethyl-1,3,2-dioxaborolan-2-yl)-1H-pyrazole (499 mg), tetrakis(triphenylphosphine)palladium(0) (92 mg), and 1,2-dimethoxyethane (20 mL) was added 2N aqueous sodium carbonate solution (4.0 mL), and the mixture was heated under reflux for 4 hr. The reaction mixture was cooled to room temperature, water was added, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (521 mg).
MS (ESI+) : [M+H]⁺287.2.

### B) 3-(6-chloro-2-(3-fluoropyridin-2-yl)-3,4-dihydro-2H-chromen-7-yl)-5-(1-methyl-1H-pyrazol-5-yl)-3,4-dihydropyrido[4,3-d]pyrimidin-2(1H)-one

In the same manner as in Example 1, step G to H and using the compound obtained in step A, the title compound was obtained.
MS (ESI+): [M+H]⁺491.4.

### Example 61

### 3-(6-chloro-2-(3-fluoropyridin-2-yl)-3,4-dihydro-2H-chromen-7-yl)-5-(2,2,2-trifluoroethoxy)-3,4-dihydropyrido[2,3-d]pyrimidin-2(1H)-one

### A) 2-amino-4-(2,2,2-trifluoroethoxy)nicotinaldehyde

To a solution of N-(4-chloro-3-formylpyridin-2-yl)-2,2-dimethylpropanamide (481 mg) in DMF (15 mL) were added 2,2,2-trifluoroethanol (0.359 mL) and potassium carbonate (553 mg) at room temperature, and the reaction mixture was stirred at 80°C for 2 days. Water was added to the reaction mixture, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (90 mg).
¹H NMR (300 MHz, CDCl₃) δ 4.47 (2H, q, J = 7.8 Hz), 6.13 (1H, d, J = 5.8 Hz), 8.17 (1H, d, J = 5.8 Hz), 10.40 (1H, s), (2H, hidden).

### B) 3-(6-chloro-2-(3-fluoropyridin-2-yl)-3,4-dihydro-2H-chromen-7-yl)-5-(2,2,2-trifluoroethoxy)-3,4-dihydropyrido[4,3-d]pyrimidin-2(1H)-one

In the same manner as in Example 4, step L and using the compound obtained in step A, the title compound was obtained. MS (ESI+) : [M+H]⁺509.3.

### Example 62

### 3-(6-chloro-2-(3-fluoropyridin-2-yl)-3,4-dihydro-2H-chromen-7-yl)-5-(1-methyl-1H-pyrazol-4-yl)-3,4-dihydropyrido[4,3-d]pyrimidin-2(1H)-one

In the same manner as in Example 60, step A, then Example 1, step G to H and using 1-methyl-5-(4,4,5,5-tetramethyl-1,3,2-dioxaborolan-2-yl)-1H-pyrazole, the title compound was obtained. MS (ESI+) : [M+H]⁺491.3.

### Example 63

### 3-(6-chloro-2-(3-fluoropyridin-2-yl)-3,4-dihydro-2H-chromen-7-yl)-5,7-dimethyl-3,4-dihydropyrido[4,3-d]pyrimidin-2(1H)-one

### A) 4-amino-2,6-dimethylnicotinaldehyde

To a mixed solution of 4-amino-2,6-dichloronicotinaldehyde (500 mg), methylboronic acid (627 mg), tricyclohexylphosphine (367 mg) and tripotassium phosphate (1945 mg) in toluene (10 mL)/water (0.5 mL) was added palladium(II) acetate (136 mg) at room temperature. The reaction mixture was stirred under microwave irradiation at 180°C for 40 min, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (212 mg).
MS (ESI+): [M+H]⁺151.0.

### B) 3-(6-chloro-2-(3-fluoropyridin-2-yl)-3,4-dihydro-2H-chromen-7-yl)-5,7-dimethyl-3,4-dihydropyrido[4,3-d]pyrimidin-2(1H)-one

In the same manner as in Example 4, step L and using the compound obtained in step A, the title compound was obtained.
MS (ESI+): [M+H]⁺439.1.

### Example 64

### 3-(6-chloro-2-(3-methoxypyridin-2-yl)-3,4-dihydro-2H-chromen-7-yl)-5-cyclopropyl-3,4-dihydropyrido[4,3-d]pyrimidin-2(1H)-one

To a solution of 3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one (265 mg) in methanol (5 mL) was added 28% sodium methoxide-methanol solution (794 mg). The reaction mixture was stirred under reflux overnight, 28% sodium methoxide-methanol solution (1111 mg) was added, and the mixture was stirred under reflux overnight. The reaction mixture was concentrated under reduced pressure, water was added, and the mixture was extracted with ethyl acetate. The extract was washed with saturated brine, and dried over magnesium sulfate. The solvent was evaporated under reduced pressure. The residue was recrystallized from ethyl acetate to give the title compound (230 mg).
MS (ESI+) : [M+H]⁺463.1.

### Example 65

### 8-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one

### A) 4-amino-5-chloro-2-cyclopropylnicotinaldehyde

In the same manner as in Example 25, step A and using 4-amino-2,5-dichloronicotinaldehyde, the title compound was obtained.
MS (ESI+) : [M+H]⁺197.3.

### B) 8-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one

In the same manner as in Example 4, step L and using the compound obtained in step A, the title compound was obtained.
MS (ESI+): [M+H]⁺485.2.

### Example 66

### 3-(6-chloro-2-(3-fluoropyridin-2-yl)-3,4-dihydro-2H-chromen-7-yl)-8-(trifluoromethyl)-3,4-dihydropyrido[3,4-d]pyrimidin-2(1H)-one

### A) ethyl 3-fluoro-2-(trifluoromethyl)isonicotinate

To a solution of 3-fluoro-2-(trifluoromethyl)isonicotinic acid (1064 mg) in ethanol (10.0 mL) was added concentrated sulfuric acid (0.3 mL) at room temperature, and the reaction mixture was heated under reflux for 5 hr. The solvent was evaporated under reduced pressure and the residue was diluted with ethyl acetate, washed with saturated aqueous sodium hydrogen carbonate solution and saturated brine and dried over sodium sulfate. The solvent was evaporated under reduced pressure and the residue was purified by silica gel column chromatography (ethyl acetate/hexane) to give the title compound (1050 mg).
¹H NMR (300 MHz, CDCl₃) δ 1.44 (3H, t, J = 7.1 Hz), 4.47 (2H, q, J = 7.1 Hz), 8.00 (1H, t, J = 4.9 Hz), 8.60 (1H, d, J = 4.9 Hz).

### B) 3-(6-chloro-2-(3-fluoropyridin-2-yl)-3,4-dihydro-2H-chromen-7-yl)-8-(trifluoromethyl)-3,4-dihydropyrido[3,4-d]pyrimidin-2(1H)-one

In the same manner as in Example 47, step A, and further, Example 4, step K to L and using the compound obtained in step A, the title compound was obtained.
MS (ESI+) : [M+H]⁺479.3.

### Example 67

### 3-(2-(3-fluoropyridin-2-yl)-6-methyl-3,4-dihydro-2H-chromen-7-yl)-5-isopropyl-3,4-dihydropyrido[4,3-d]pyrimidin-2(1H)-one

### A) N-(3-formyl-2-(prop-1-en-2-yl)pyridin-4-yl)-2,2-dimethylpropanamide

In the same manner as in Example 25, step A, the title compound was obtained.
MS (ESI+) : [M+H]⁺247.1.

### B) N-(3-formyl-2-isopropylpyridin-4-yl)-2,2-dimethylpropanamide

To a solution of N-(3-formyl-2-(prop-1-en-2-yl)pyridin-4-yl)-2,2-dimethylpropanamide (282 mg) in methanol (5 mL) was added 10% palladium-carbon (28 mg), and the reaction mixture was stirred under a hydrogen atmosphere at room temperature for 2 hr. The insoluble material was filtered off by using celite, and the filtrate was concentrated under reduced pressure to give the title compound (277 mg).
MS (ESI+): [M+H]⁺249.2.

### C) 3-(2-(3-fluoropyridin-2-yl)-6-methyl-3,4-dihydro-2H-chromen-7-yl)-5-isopropyl-3,4-dihydropyrido[4,3-d]pyrimidin-2(1H)-one

In the same manner as in Example 1, step G to H and using the compound obtained in step B, the title compound was obtained.
MS (ESI+): [M+H]⁺433.1.

### Example 68

### 3-(6-fluoro-2-(3-fluoropyridin-2-yl)-3,4-dihydro-2H-chromen-7-yl)-5-(trifluoromethyl)-3,4-dihydropyrido[4,3-d]pyrimidin-2(1H)-one

### A) 6-fluoro-2-(3-fluoropyridin-2-yl)chromane-7-amine

In the same manner as in Example 1, step A to D and using 3-amino-4-fluorophenol, the title compound was obtained. MS (ESI+): [M+H]⁺263.2.

### B) 3-(6-fluoro-2-(3-fluoropyridin-2-yl)-3,4-dihydro-2H-chromen-7-yl)-5-(trifluoromethyl)-3,4-dihydropyrido[4,3-d]pyrimidin-2(1H)-one

In the same manner as in Example 4, step L and using the compound obtained in step A, the title compound was obtained. MS (ESI+): [M+H]⁺463.4.

### Example 69

### 5-acetyl-3-(6-chloro-2-(3-fluoropyridin-2-yl)-3,4-dihydro-2H-chromen-7-yl)-3,4-dihydropyrido[4,3-d]pyrimidin-2(1H)-one

A product obtained by a method similar to that in Example 33 was purified by silica gel column chromatography (ethyl acetate/hexane) and separated by HPLC (C18, mobile phase: water/acetonitrile (0.1% TFA-containing system)). A saturated aqueous sodium hydrogen carbonate solution was added to a fraction with a longer retention time, and the mixture was extracted with ethyl acetate, dried over anhydrous magnesium sulfate, and concentrated under reduced pressure. The residue was recrystallized from ethyl acetate to give the title compound (7.5 mg).
MS (ESI+): [M+H]⁺453.1.

### Example 70

### methyl 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylate

### A) methyl 4-amino-6-cyclopropyl-5-formylpyridine-2-carboxylate

To a mixed solution of 4-amino-2,6-dichloronicotinaldehyde (2.0 g), cyclopropylboronic acid (0.9 g), tricyclohexylphosphine (0.3 g) and tripotassium phosphate (7.8 g) in toluene (32 mL)/water (2 mL) was added palladium(II) acetate (0.1 g) at room temperature. The reaction mixture was stirred under microwave irradiation at 180°C for 5 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane). The resultant product was dissolved in methanol (15 mL). 1,1'-Bis(diphenylphosphino)ferrocene (0.8 g), sodium acetate (1.4 g) and palladium(II) acetate (0.2 g) were added at room temperature, and the reaction mixture was stirred under a carbon monoxide atmosphere at 100°C for 10 hr. The insoluble material was filtered off with celite, and the filtrate was concentrated under reduced pressure. Water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.4 g).
MS (ESI+): [M+H]⁺221.3.

### B) methyl 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylate

In the same manner as in Example 4, step L and using the compound obtained in step A, the title compound was obtained.
MS (ESI+): [M+H]⁺509.3.

### Example 71

### 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid

To a mixed solution of methyl 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylate (308 mg) in THF (3 mL)/ethanol(3 mL) was added 1N aqueous sodium hydroxide solution (0.9 mL). The reaction mixture was stirred at room temperature for 2.5 hr, 1N hydrochloric acid was added to adjust to pH4, and the mixture was extracted with ethyl acetate. The extract was washed with saturated brine, and dried over magnesium sulfate. The solvent was evaporated under reduced pressure. The residue was recrystallized from ethyl acetate/ethanol to give the title compound (206 mg).
MS (ESI+): [M+H]⁺495.2.

### Example 72

### 7-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one

### A) 4-amino-6-chloro-2-cyclopropylnicotinaldehyde

To a mixed solution of 4-amino-2,6-dichloronicotinaldehyde (4.0 g), cyclopropylboronic acid (1.9 g), tricyclohexylphosphine (0.6 g) and tripotassium phosphate (15.6 g) in toluene (24 mL)/water (3 mL) was added palladium(II) acetate (0.2 g) at room temperature. The reaction mixture was stirred under microwave irradiation at 180°C for 5 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (1.2 g).
MS (ESI+): [M+H]⁺197.2.

### B) 7-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one

In the same manner as in Example 4, step L and using the compound obtained in step A, the title compound was obtained. MS (ESI+): [M+H]⁺485.0.

### Example 73

### 3-(6-chloro-2-(3-fluoropyridin-2-yl)-3,4-dihydro-2H-chromen-7-yl)-5-cyclopropyl-7-(hydroxymethyl)-3,4-dihydropyrido[4,3-d]pyrimidin-2(1H)-one

To a solution of methyl 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylate (100 mg) in THF (3 mL) was added lithium aluminum hydride at 0°C. The reaction mixture was stirred at 0°C for 1.5 hr, to the reaction mixture were added water (12 µL), 15% aqueous sodium hydroxide solution (12 µL) and water (36 µL) in this order, and the mixture was stirred at room temperature for 1 hr. The insoluble material was filtered off by using celite. The filtrate was concentrated under reduced pressure, and the residue was washed with water, and recrystallized from ethyl acetate/ethanol to give the title compound (58 mg).
MS (ESI+) : [M+H]⁺481.3.

### Example 74

### 3-(6-chloro-2-(3-fluoropyridin-2-yl)-3,4-dihydro-2H-chromen-7-yl)-5-cyclopropyl-7-(morpholin-4-ylcarbonyl)-3,4-dihydropyrido[4,3-d]pyrimidin-2(1H)-one

To a solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (30 mg), morpholine (58 µL), and HOBt (89 mg) in DMF (3 mL) was added WSC (128 mg), and the reaction mixture was stirred at room temperature overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (30 mg).
MS (ESI+): [M+H]⁺564.4.

### Example 75

### 3-(6-chloro-2-(3-fluoropyridin-2-yl)-3,4-dihydro-2H-chromen-7-yl)-5-cyclopropyl-7-(2-hydroxypropan-2-yl)-3,4-dihydropyrido[4,3-d]pyrimidin-2(1H)-one

By a method similar to that in Example 33, the title compound was obtained.
MS (ESI+) : [M+H]⁺509.2.

### Example 76

### 3-(6-chloro-2-(3-fluoropyridin-2-yl)-3,4-dihydro-2H-chromen-7-yl)-N,5-dicyclopropyl-2-oxo-1,2,3,4-tetrahydropyrido[4,3-d]pyrimidine-7-carboxamide

By a method similar to that in Example 74, the title compound was obtained.
MS (ESI+): [M+H]⁺534.4.

### Example 77

### 5-chloro-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 1 and using 6-methyl-2-(3-fluoropyridin-2-yl)chromane-7-amine, the title compound was obtained.
MS (ESI+): [M+H]⁺399.4.

### Example 78

### 3-(6-chloro-2-(3-fluoropyridin-2-yl)-3,4-dihydro-2H-chromen-7-yl)-5-(trifluoromethyl)-3,4-dihydropyrido[3,4-d]pyrimidin-2(1H)-one

### A) ethyl 3-fluoro-5-(trifluoromethyl)isonicotinate

By a method similar to that in Example 57, step A, the title compound was obtained.
¹H NMR (300 MHz, CDCl₃) δ 1.41 (3H, t, J = 7.1 Hz), 4.48 (2H, q, J = 7.1 Hz), 8.76 (1H, d, J = 0.5 Hz), 8.79 (1H, s).

### B) 3-(6-chloro-2-(3-fluoropyridin-2-yl)-3,4-dihydro-2H-chromen-7-yl)-5-(trifluoromethyl)-3,4-dihydropyrido[3,4-d]pyrimidin-2(1H)-one

In the same manner as in Example 47, step A, and further, Example 4, step K to L and using the compound obtained in step A, the title compound was obtained.
¹H NMR (300 MHz, CDCl₃) δ 2.17-2.33 (1H, m), 2.36-2.62 (1H, m), 2.84-3.16 (2H, m), 4.72-5.02 (2H, m), 5.40-5.57 (1H, m), 6.92-7.01 (1H, m), 7.27 (1H, s), 7.32 (1H, dt, J = 8.3, 4.2 Hz), 7.41-7.50 (1H, m), 8.29 (1H, s), 8.40-8.65 (3H, m).
MS (ESI+): [M+H]⁺479.3.

### Example 79

### 5-chloro-3-(6-chloro-2-(3-fluoropyridin-2-yl)-3,4-dihydro-2H-chromen-7-yl)-6,8-dimethyl-3,4-dihydropyrido[3,4-d]pyrimidin-2(1H)-one

### A) 2,6-dibromo-5-chloro-4-(dimethoxymethyl)pyridin-3-amine

In Example 7, step C, 2,6-dibromo-5-chloro-4-(dimethoxymethyl)pyridin-3-amine (500 mg) was obtained from a highly-polar fraction.
¹H NMR (300 MHz, CDCl₃) δ 3.48 (6H, s), 5.25 (2H, brs), 5.73 (1H, s).

### B) 5-chloro-4-(dimethoxymethyl)-2,6-dimethylpyridin-3-amine

Under ice-cooling, to a mixed solution of 2,6-dibromo-5-chloro-4-(dimethoxymethyl)pyridin-3-amine (300 mg), methylboronic acid (59.8 mg), tripotassium phosphate (618 mg) and tricyclohexylphosphine (23.3 mg) in toluene (5 mL) and water (0.25 mL) was added palladium(II) acetate (9.3 mg), and the mixture was heated at 100°C overnight. The reaction mixture was cooled to room temperature, and filtered. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (135 mg).
¹H NMR (300 MHz, CDCl₃) δ 2.27-2.43 (3H, m), 2.49 (3H, s), 3.48 (6H, s), 4.49 (2H, brs), 5.80 (1H, s).

### C) 5-chloro-3-(6-chloro-2-(3-fluoropyridin-2-yl)-3,4-dihydro-2H-chromen-7-yl)-6,8-dimethyl-3,4-dihydropyrido[3,4-d]pyrimidin-2(1H)-one

In the same manner as in Example 10, step B to C and using the compound obtained in step B, the title compound was obtained.
MS (ESI+): [M+H]⁺473.3.

### Example 80

### 5-chloro-3-(6-chloro-2-(3-fluoropyridin-2-yl)-3,4-dihydro-2H-chromen-7-yl)-8-methoxy-3,4-dihydropyrido[3,4-d]pyrimidin-2(1H)-one

### A) 5-chloro-4-(dimethoxymethyl)-2-methoxypyridin-3-amine

To a solution of 2-bromo-5-chloro-4-(dimethoxymethyl)pyridin-3-amine (200 mg) in methanol (4 mL) was added sodium methanolate (1.15 g), and the mixture was heated under reflux overnight. The solvent was evaporated under reduced pressure and the residue was diluted with water. The mixture was extracted with ethyl acetate. The extract was washed with saturated brine, and dried over magnesium sulfate. The solvent was evaporated under reduced pressure and the residue was crystallized from ethyl acetate to give the title compound (160 mg).
¹H NMR (300 MHz, CDCl₃) δ 3.46 (6H, s), 3.95 (3H, s), 4.87 (2H, brs), 5.68 (1H, s), 7.45 (1H, s).

### B) 5-chloro-3-(6-chloro-2-(3-fluoropyridin-2-yl)-3,4-dihydro-2H-chromen-7-yl)-8-methoxy-3,4-dihydropyrido[3,4-d]pyrimidin-2(1H)-one

In the same manner as in Example 10, step B to C and using the compound obtained in step A, the title compound was obtained.
MS (ESI+): [M+H]⁺475.3.

### Example 81

### 5-chloro-3-(6-chloro-2-(3-fluoropyridin-2-yl)-3,4-dihydro-2H-chromen-7-yl)-8-(morpholin-4-yl)-3,4-dihydropyrido[3,4-d]pyrimidin-2(1H)-one

### A) 5-chloro-4-(dimethoxymethyl)-2-morpholinopyridin-3-amine

To a solution of 2-bromo-5-chloro-4-(dimethoxymethyl)pyridin-3-amine (200 mg) in DMF (1 mL) was added morpholine (1.86 g), and the mixture was heated at 100°C for 60 hr, cooled to room temperature and poured into water. The mixture was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (190 mg).
¹H NMR (300 MHz, CDCl₃) δ 3.09 (4H, m), 3.47 (6H, s), 3.85 (4H, m), 4.92 (2H, brs), 5.70 (1H, s), 7.70 (1H, s).

### B) 5-chloro-3-(6-chloro-2-(3-fluoropyridin-2-yl)-3,4-dihydro-2H-chromen-7-yl)-8-(morpholin-4-yl)-3,4-dihydropyrido[3,4-d]pyrimidin-2(1H)-one

In the same manner as in Example 10, step B to C and using the compound obtained in step A, the title compound was obtained.
MS (ESI+): [M+H]⁺530.4.

### Example 82

### 5-chloro-N-cyclopropyl-3-(2-(3-fluoropyridin-2-yl)-6-methyl-3,4-dihydro-2H-chromen-7-yl)-2-oxo-1,2,3,4-tetrahydroquinazoline-7-carboxamide

### A) methyl 3-chloro-4-methyl-5-nitro-benzoate

To a mixed solution of methyl 3-amino-4-methyl-5-nitro-benzoate(2.0 g) in 1,4-dioxane(15.0 mL) and acetic acid (15.0 mL) was added dropwise an aqueous solution (10.0 mL) of sodium nitrite (690 mg) at 0°C. The reaction mixture was stirred for 2 hr, and added dropwise to a solution of copper(I) chloride (1.10 g) in concentrated hydrochloric acid (5.0 mL). The obtained reaction mixture was further stirred for 1 hr, poured into ice, and the mixture was extracted with ethyl acetate, dried over anhydrous magnesium sulfate, and concentrated under reduced pressure to give the title compound (1.42 g).
¹H NMR (300 MHz, CDCl₃) δ 2.49 (3H, s), 3.88 (3H, s), 8.21 (1H, s), 8.31 (1H, s).

### B) methyl 4-bromomethyl-3-chloro-5-nitro-benzoate

To a solution of methyl 3-chloro-4-methyl-5-nitrobenzoate (1.42 g) and N-bromosuccinimide (1.20 g) in carbon tetrachloride (20.0 mL) was added perbenzoic acid (20 mg), and the mixture was heated under reflux for 24 hr. The obtained reaction mixture was poured into ice, and the mixture was extracted with methylene chloride, washed with water, dried over anhydrous sodium sulfate and concentrated under reduced pressure to give the title compound (1.43 g).
¹H NMR (300 MHz, DMSO-d₆) δ 3.99 (3H, s), 4.88 (2H, s), 8.32 (1H, s), 8.47 (1H, s).

### C) methyl 5-chloro-N-cyclopropyl-3-(2-(3-fluoropyridin-2-yl)-6-methyl-3,4-dihydro-2H-chromen-7-yl)-2-oxo-1,2,3,4-tetrahydroquinazoline-7-carboxylate

In the same manner as in Example 24, step F to G and using the compound obtained in step B, the title compound was obtained.
¹H NMR (300 MHz, DMSO-d₆) δ 2.04 (3H, s), 2.14-2.20 (1H, m), 2.22-2.31 (1H, m), 2.78-2.81 (1H, m), 2.83-3.02 (1H, m), 3.82 (3H, s), 4.53-4.58 (1H, m), 4.87-4.91 (1H, m), 5.41 (1H, d, J = 1.6 Hz), 6.84 (1H, s), 7.01 (1H, s), 7.36-7.37 (1H, d, J = 2.0 Hz), 7.46-7.53 (2H, m), 7.76-7.81 (1H, m), 8.45-8.46 (1H, m), 9.85 (1H, s).

### D) 5-chloro-N-cyclopropyl-3-(2-(3-fluoropyridin-2-yl)-6-methyl-3,4-dihydro-2H-chromen-7-yl)-2-oxo-1,2,3,4-tetrahydroquinazoline-7-carboxylic acid

In the same manner as in Example 71 and using the compound obtained in step C, the title compound was obtained. MS (ESI+): [M+H]⁺467.9.

### E) 5-chloro-N-cyclopropyl-3-(2-(3-fluoropyridin-2-yl)-6-methyl-3,4-dihydro-2H-chromen-7-yl)-2-oxo-1,2,3,4-tetrahydroquinazoline-7-carboxamide

In the same manner as in Example 74 and using the compound obtained in step D, the title compound was obtained.
MS (ESI+) : [M+H]⁺506.2.

### Example 83

### 5-cyclopropyl-3-(2-(3-fluoropyridin-2-yl)-6-methyl-3,4-dihydro-2H-chromen-7-yl)-7-methyl-3,4-dihydropyrido[4,3-d]pyrimidin-2(1H)-one

### A) N-(2-cyclopropyl-3-formyl-6-methylpyridin-4-yl)-2,2-dimethylpropanamide

In the same manner as in Example 42, step A, the title compound was obtained.
MS (ESI+): [M+H]⁺261.4.

### B) 5-cyclopropyl-3-(2-(3-fluoropyridin-2-yl)-6-methyl-3,4-dihydro-2H-chromen-7-yl)-7-methyl-3,4-dihydropyrido[4,3-d]pyrimidin-2(1H)-one

In the same manner as in Example 1, step G to H and using the compound obtained in step A, the title compound was obtained.
MS (ESI+): [M+H]⁺445.4.

### Example 84

### 3-(6-chloro-2-(3-fluoropyridin-2-yl)-3,4-dihydro-2H-chromen-7-yl)-5-(2-hydroxypropan-2-yl)-3,4-dihydropyrido[4,3-d]pyrimidin-2(1H)-one

By a method similar to that in Example 33, the title compound was obtained.
MS (ESI+): [M+H]⁺469.3.

### Example 85

### 3-(6-chloro-2-(3-fluoropyridin-2-yl)-3,4-dihydro-2H-chromen-7-yl)-5-cyclopropyl-7-(1,3-oxazol-2-yl)-3,4-dihydropyrido[4,3-d]pyrimidin-2(1H)-one

By a method similar to that in Example 42, the title compound was obtained.
MS (ESI+): [M+H]⁺518.3.

### Example 86

### 3-(6-chloro-2-(3-fluoropyridin-2-yl)-3,4-dihydro-2H-chromen-7-yl)-5-cyclopropyl-7-(2-oxopyrrolidin-1-yl)-3,4-dihydropyrido[4,3-d]pyrimidin-2(1H)-one

### A) 4-amino-2-cyclopropyl-6-(2-oxopyrrolidin-1-yl)nicotinaldehyde

To a mixture of 4-amino-6-chloro-2-cyclopropylnicotinaldehyde (364 mg), 2-piperidinone (315 mg), (9,9-dimethyl-9H-xanthene-4,5-diyl)bis(diphenylphosphine) (161 mg), cesium carbonate (905 mg), and toluene (10 mL) was added tris(dibenzylideneacetone)dipalladium(0) (85 mg). The reaction mixture was stirred under microwave irradiation at 180°C for 40 min, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (99 mg). MS (ESI+): [M+H]⁺246.1.

### B) 3-(6-chloro-2-(3-fluoropyridin-2-yl)-3,4-dihydro-2H-chromen-7-yl)-5-cyclopropyl-7-(2-oxopyrrolidin-1-yl)-3,4-dihydropyrido[4,3-d]pyrimidin-2(1H)-one

In the same manner as in Example 4, step L and using the compound obtained in step A, the title compound was obtained. MS (ESI+) : [M+H]⁺534.4.

### Example 87

### 8-bromo-3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

### A) 4-amino-5-bromo-2-(trifluoromethyl)nicotinaldehyde

To a solution of 4-amino-2-(trifluoromethyl)nicotinaldehyde (662 mg) in DMF (5 mL) was added N-bromosuccinimide (713 mg) at room temperature. The reaction mixture was stirred at room temperature overnight, water was added, and the precipitated solid was collected by filtration to give the title compound (840 mg).
MS (ESI+): [M+H]⁺271.0.

### B) 8-bromo-3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one

In the same manner as in Example 4, step L and using the compound obtained in step A, the title compound was obtained.
MS (ESI+): [M+H]⁺539.1.

### Example 88

### 5-fluoro-3-(2-(3-fluoropyridin-2-yl)-6-methyl-3,4-dihydro-2H-chromen-7-yl)-3,4-dihydropyrido[3,4-d]pyrimidin-2(1H)-one

### A) ethyl 3,5-difluoroisonicotinate

In the same manner as in Example 57, step A, the title compound was produced.
¹H NMR (400 MHz, CDCl₃) δ 1.41 (3H, t, J = 7.2 Hz), 4.47 (2H, q, J = 7.2 Hz), 8.44 (2H, s).

### B) ethyl 3-azido-5-fluoroisonicotinate

To a solution of ethyl 3,5-difluoroisonicotinate (10.0 g) in DMF (50 mL) was added sodium azide (1.99 g), and the mixture was stirred at 95°C overnight. The reaction mixture was cooled to room temperature, and diluted with ethyl acetate. The diluted solution was washed with saturated brine, and dried over magnesium sulfate. The solvent was evaporated under reduced pressure and the residue was purified by silica gel column chromatography (ethyl acetate/hexane) to give the title compound (4.00 g).
¹H NMR (400 MHz, CDCl₃) δ 1.41 (3H, t, J = 7.2 Hz), 4. 45 (2H, q, J = 7.2 Hz), 8.34 (1H, s), 8.42 (1H, s).

### C) (3-amino-5-fluoropyridin-4-yl)methanol

To a solution of ethyl 3-azido-5-fluoroisonicotinate (2.5 g) in methanol (25.0 mL) was added 10% palladium-carbon (633 mg), and the mixture was stirred at room temperature under a hydrogen atmosphere for 3 hr. The insoluble material was filtered off using celite, and the filtrate was concentrated under reduced pressure. To a solution of the residue (2.19 g) in THF (25.0 mL) was added lithium aluminum hydride (496 mg) at 0°C, and the mixture was stirred at room temperature for 3 hr. Water was added to the reaction mixture, and the mixture was extracted with ethyl acetate. The extract was dried over magnesium sulfate. The solvent was evaporated under reduced pressure and the residue was purified by silica gel column chromatography (ethyl acetate/hexane) to give the title compound (1.08 g).
¹H NMR (400 MHz, DMSO-d₆) δ 4.80 (2H, d, J = 5.6 Hz), 5.19 (1H, t, J = 5.6 Hz),5.55 (2H, s), 7.66 (1H, s), 7.83 (1H, s).

### D) 5-fluoro-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 4, step K to L and using the compound obtained in step C, the title compound was obtained.
¹H NMR (300 MHz, CDCl₃) δ 2.12-2.29 (4H, m), 2.35-2.55 (1H, m), 2.81-3.13 (2H, m), 4.62-4.89 (2H, m), 5.35-5.51 (1H, m), 6.80-6.87 (1H, m), 7.04 (1H, s), 7.27-7.36 (1H, m), 7.39-7.50 (1H, m), 7.92 (1H, s), 7.99-8.08 (1H, m), 8.07-8.12 (1H, m), 8.42-8.52 (1H, m).

### Example 89

### methyl (2E)-3-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)acrylate

### A) methyl (2E)-3-(4-amino-6-cyclopropyl-5-formylpyridin-2-yl)acrylate

To a solution of 4-amino-6-chloro-2-cyclopropylnicotinaldehyde (475 mg), methyl acrylate (416 mg), biphenyl-2-yl(di-tert-butyl)phosphine (144 mg) and triethylamine (1.0 mL) in DMF (10 mL) was added palladium(II) acetate (54 mg). The reaction mixture was stirred under microwave irradiation at 150°C for 1 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (162 mg). MS (ESI+): [M+H]⁺247.3.

### B) methyl (2E)-3-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)acrylate

In the same manner as in Example 4, step L and using the compound obtained in step A, the title compound was obtained. MS (ESI+): [M+H]⁺515.3.

### Example 90

### 5,7-dicyclopropyl-3-(2-(3-fluoropyridin-2-yl)-3,4-dihydro-2H-chromen-7-yl)-3,4-dihydropyrido[4,3-d]pyrimidin-2(1H)-one

In the same manner as in Example 4, step L and using 4-amino-2,6-dicyclopropylnicotinaldehyde, the title compound was obtained.
MS (ESI+) : [M+H]⁺457.2.

### Example 91

### (2E)-3-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)acrylic acid

By a method similar to that in Example 71, the title compound was obtained.
MS (ESI+): [M+H]⁺501.4.

### Example 92

### (2E)-3-(3-(2-(3-fluoropyridin-2-yl)-6-methyl-3,4-dihydro-2H-chromen-7-yl)-2-oxo-5-(trifluoromethyl)-1,2,3,4-tetrahydropyrido[4,3-d]pyrimidine-8-yl)acrylic acid

### A) (E)-butyl 3-(3-(2-(3-fluoropyridin-2-yl)-6-methylchroman-7-yl)-2-oxo-5-(trifluoromethyl)-1,2,3,4-tetrahydropyrido[4,3-d]pyrimidine-8-yl)acrylate

A solution of 8-bromo-3-(2-(3-fluoropyridin-2-yl)-6-methyl-3,4-dihydro-2H-chromen-7-yl)-5-(trifluoromethyl)-3,4-dihydropyrido[4,3-d]pyrimidin-2(1H)-one (600 mg), butyl acrylate (0.80 mL), palladium(II) acetate (50 mg), tri-o-tolylphosphine (136 mg) and diisopropylethylamine (0.78 mL) in DMF (10 mL) was stirred under a nitrogen atmosphere at 120°C overnight. The reaction mixture was cooled to room temperature, and diluted with ethyl acetate. The diluted solution was washed with saturated brine, and dried over magnesium sulfate. The solvent was evaporated under reduced pressure and the residue was purified by silica gel column chromatography (ethyl acetate/hexane) to give the title compound (380 mg).
MS (ESI+): [M+H]⁺585.3.

### B) (2E)-3-(3-(2-(3-fluoropyridin-2-yl)-6-methyl-3,4-dihydro-2H-chromen-7-yl)-2-oxo-5-(trifluoromethyl)-1,2,3,4-tetrahydropyrido[4,3-d]pyrimidine-8-yl)acrylic acid

In the same manner as in Example 71 and using the compound obtained in step A, the title compound was obtained.
MS (ESI+): [M+H]⁺529.2.

### Example 93

### 5-cyclopropyl-3-(2-(3-fluoropyridin-2-yl)-6-methyl-3,4-dihydro-2H-chromen-7-yl)-7-(1-methyl-1H-pyrazol-5-yl)-3,4-dihydropyrido[4,3-d]pyrimidin-2(1H)-one

### A) 4-amino-2-cyclopropyl-6-(1-methyl-1H-pyrazol-5-yl)nicotinaldehyde

To a mixed solution of 4-amino-6-chloro-2-cyclopropylnicotinaldehyde (378 mg), 1-methyl-5-(4,4,5,5-tetramethyl-1,3,2-dioxaborolan-2-yl)-1H-pyrazole (480 mg), tricyclohexylphosphine (53.9 mg) and tripotassium phosphate (1.43 g) in toluene (6.5 mL)-water (0.325 mL) was added palladium(II) acetate (21.6 mg) at room temperature. The mixture was stirred under microwave irradiation at 150°C for 40 min. Water was added to the reaction mixture, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (148 mg).
MS (ESI+) : [M+H]⁺243.1.

### B) 5-cyclopropyl-3-(2-(3-fluoropyridin-2-yl)-6-methyl-3,4-dihydro-2H-chromen-7-yl)-7-(1-methyl-1H-pyrazol-5-yl)-3,4-dihydroquinazolin-2(1H)-one

In the same manner as in Example 4, step L and using the compound obtained in step A, the title compound was obtained.
MS (ESI+): [M+H]⁺511.3.

### Example 94

### 3-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)propanoic acid

To a solution of (2E)-3-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)acrylic acid (43 mg) in methanol (2 mL) was added palladium-carbon (9 mg). The reaction mixture was stirred under a hydrogen atmosphere at room temperature for 7 hr, and the insoluble material was filtered off by using celite. The filtrate was concentrated under reduced pressure, and the residue was recrystallized from ethyl acetate to give the title compound (20 mg).
MS (ESI+) : [M+H]⁺503.3.

### Example 95

### 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxamide

To a solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (100 mg) and HOBt/ammonium salt(37 mg) in DMF (2 mL) was added WSC (47 mg), and the reaction mixture was stirred at room temperature for 2 hr. Water was added to the reaction mixture, the resulting solid was recovered, purified by silica gel column chromatography (ethyl acetate/hexane) and recrystallized from ethyl acetate/hexane to give the title compound (58 mg).
MS (ESI+) : [M+H]⁺494.3.

### Example 96

### 5-cyclopropyl-7-ethyl-3-(2-(3-fluoropyridin-2-yl)-6-methyl-3,4-dihydro-2H-chromen-7-yl)-3,4-dihydropyrido[4,3-d]pyrimidin-2(1H)-one

### A) N-(2-cyclopropyl-6-ethyl-3-formylpyridin-4-yl)-2,2-dimethylpropanamide

By a method similar to that in Example 42, step A, the title compound was obtained.
MS (ESI+) : [M+H]⁺275.3.

### B) 5-cyclopropyl-7-ethyl-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 1, step G to H and using the compound obtained in step A, the title compound was obtained.
MS (ESI+) : [M+H]⁺459.4.

### Example 97

### 3-(6-chloro-2-(3-fluoropyridin-2-yl)-3,4-dihydro-2H-chromen-7-yl)-5-cyclopropyl-N-(2-methoxyethyl)-2-oxo-1,2,3,4-tetrahydropyrido[4,3-d]pyrimidine-7-carboxamide

By a method similar to that in Example 74, the title compound was obtained.
MS (ESI+): [M+H]⁺552.4.

### Example 98

### 3-(6-chloro-2-(3-fluoropyridin-2-yl)-3,4-dihydro-2H-chromen-7-yl)-5-cyclopropyl-N-(oxetan-3-yl)-2-oxo-1,2,3,4-tetrahydropyrido[4,3-d]pyrimidine-7-carboxamide

By a method similar to that in Example 74, the title compound was obtained.
MS (ESI+): [M+H]⁺550.3.

### Example 99

### 3-(6-chloro-2-(3-fluoropyridin-2-yl)-3,4-dihydro-2H-chromen-7-yl)-5-cyclopropyl-N-methyl-2-oxo-1,2,3,4-tetrahydropyrido[4,3-d]pyrimidine-7-carboxamide

By a method similar to that in Example 74, the title compound was obtained.
MS (ESI+): [M+H]⁺508.3.

### Example 100

### 3-(6-chloro-2-(3-fluoropyridin-2-yl)-3,4-dihydro-2H-chromen-7-yl)-5-cyclopropyl-7-(1-methyl-1H-pyrazol-4-yl)-3,4-dihydropyrido[4,3-d]pyrimidin-2(1H)-one

### A) 4-amino-2-cyclopropyl-6-(1-methyl-1H-pyrazol-4-yl)nicotinaldehyde

By a method similar to that in Example 93, step A, the title compound was obtained.
MS (ESI+): [M+H]⁺243.1.

### B) 3-(6-chloro-2-(3-fluoropyridin-2-yl)-3,4-dihydro-2H-chromen-7-yl)-5-cyclopropyl-7-(1-methyl-1H-pyrazol-4-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺531.4.

### Example 101

### 3-(6-chloro-2-(3-fluoropyridin-2-yl)-3,4-dihydro-2H-chromen-7-yl)-5-cyclopropyl-N-(2-hydroxyethyl)-2-oxo-1,2,3,4-tetrahydropyrido[4,3-d]pyrimidine-7-carboxamide

By a method similar to that in Example 74, the title compound was obtained.
MS (ESI+): [M+H]⁺538.3.

### Example 102

### 5-chloro-3-(6-chloro-2-(3-fluoropyridin-2-yl)-3,4-dihydro-2H-chromen-7-yl)-8-(1-methyl-1H-pyrazol-4-yl)-3,4-dihydropyrido[3,4-d]pyrimidin-2(1H)-one

### A) 8-bromo-5-chloro-3-(6-chloro-2-(3-fluoropyridin-2-yl)chroman-7-yl)-3,4-dihydropyrido[3,4-d]pyrimidin-2(1H)-one

By a method similar to that in Example 10, step B to C, the title compound was obtained.
¹H NMR (300 MHz, DMSO-d₆) δ 2.12-2.44 (2H, m), 2.79-3.13 (2H, m), 4.56-5.01 (2H, m), 5.52 (1H, d, J = 8.3 Hz), 7.08 (1H, s), 7.37 (1H, s), 7.54 (1H, dt, J = 8.4, 4.3 Hz), 7.82 (1H, t, J = 9.4 Hz), 8.11 (1H, s), 8.48 (1H, d, J = 4.2 Hz), 9.25 (1H, s).

### B) 5-chloro-3-(6-chloro-2-(3-fluoropyridin-2-yl)-3,4-dihydro-2H-chromen-7-yl)-8-(1-methyl-1H-pyrazol-4-yl)-3,4-dihydropyrido[3,4-d]pyrimidin-2(1H)-one

To a solution of 8-bromo-5-chloro-3-(6-chloro-2-(3-fluoropyridin-2-yl)chroman-7-yl)-3,4-dihydropyrido[3,4-d]pyrimidin-2(1H)-one (50 mg), 1-methyl-4-(4,4,5,5-tetramethyl-1,3,2-dioxaborolan-2-yl)-1H-pyrazole (30 mg), tetrakistriphenylphosphinepalladium(0) (11 mg) in DMF (1 mL) was added 2M aqueous sodium carbonate solution (0.14 mL), and the mixture was stirred under an argon atmosphere at 90°C for 2 hr. Water was added to the mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (28 mg).
MS (ESI+): [M+H]⁺524.9.

### Example 103

### 3-(6-chloro-2-(3-fluoropyridin-2-yl)-3,4-dihydro-2H-chromen-7-yl)-5-cyclopropyl-7-(5-oxo-4,5-dihydro-1,2,4-oxadiazol-3-yl)-3,4-dihydropyrido[4,3-d]pyrimidin-2(1H)-one

### A) 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile

A mixture of 7-chloro-3-(6-chloro-2-(3-fluoropyridin-2-yl)chroman-7-yl)-5-cyclopropyl-3,4-dihydropyrido[4,3-d]pyrimidin-2(1H)-one (200 mg), 1,1'-bis(diphenylphosphino)ferrocene (46.2 mg), zinc cyanide (33.9 mg), zinc (18.9 mg), tris(dibenzylidene)dipalladium(0) (37.7 mg), and DMF (4 mL) was stirred under an argon atmosphere at 120°C for 5 hr. The reaction mixture was cooled to room temperature, and diluted with ethyl acetate. The diluted solution was washed with saturated brine, and dried over magnesium sulfate. The solvent was evaporated under reduced pressure and the residue was purified by silica gel column chromatography (ethyl acetate/hexane) to give the title compound (196 mg).
MS (ESI+): [M+H]⁺476.2.

### B) 3-(6-chloro-2-(3-fluoropyridin-2-yl)-3,4-dihydro-2H-chromen-7-yl)-5-cyclopropyl-7-(5-oxo-4,5-dihydro-1,2,4-oxadiazol-3-yl)-3,4-dihydropyrido[4,3-d]pyrimidin-2(1H)-one

To a solution of 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile (195 mg) in DMSO (2 mL) were added hydroxylamine (142 mg) and sodium carbonate (217 mg), and the mixture was stirred at 60°C for 5 hr. The reaction mixture was cooled to room temperature, water was added, and the mixture was extracted with ethyl acetate. The extract was washed with saturated brine, and dried over magnesium sulfate. The solvent was evaporated under reduced pressure. To the residue was added THF (5 mL), and CDI (200 mg) and DBU (0.19 mL) were added. The reaction mixture was stirred for 2 hr at room temperature, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (189 mg).
MS (ESI+): [M+H]⁺535.2.

### Example 104

### 7-cyclopropyl-3-(2-(3-fluoropyridin-2-yl)-6-methyl-3,4-dihydro-2H-chromen-7-yl)-5-(1-methyl-1H-pyrazol-5-yl)-3,4-dihydropyrido[4,3-d]pyrimidin-2(1H)-one

### A) 4-amino-6-cyclopropyl-2-(1-methyl-1H-pyrazol-5-yl)nicotinaldehyde

By a method similar to that in Example 93, step A, the title compound was obtained.
MS (ESI+): [M+H]⁺243.0.

### B) 7-cyclopropyl-3-(2-(3-fluoropyridin-2-yl)-6-methyl-3,4-dihydro-2H-chromen-7-yl)-5-(1-methyl-1H-pyrazol-5-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺511.3.

### Example 105

### 3-(6-chloro-2-(3-fluoropyridin-2-yl)-3,4-dihydro-2H-chromen-7-yl)-5-cyclopropyl-N-(2-(methylsulfinyl)ethyl)-2-oxo-1,2,3,4-tetrahydropyrido[4,3-d]pyrimidine-7-carboxamide

To a solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (250 mg) in DMF (5.0 mL) was added 2-(methylsulfanyl)ethanamine (0.057 mL), WSC (94 mg) and HOBt (82 mg), and the mixture was stirred at room temperature for 3 hr. Water was added to the reaction mixture, and the precipitated solid was collected by filtration. The obtained residue was dissolved in DMF (4.5 mL), and m-chloroperbenzoic acid (86 mg) was added at 0°C. The reaction mixture was stirred under a nitrogen atmosphere at 0°C for 20 min. To the reaction mixture was added saturated aqueous sodium hydrogen carbonate solution, and the mixture was extracted with ethyl acetate. The extract was washed with saturated brine and dried over sodium sulfate. The solvent was evaporated under reduced pressure and the residue was purified by silica gel column chromatography (NH, methanol/ethyl acetate) and silica gel column chromatography (methanol/ethyl acetate), and crystallized from ethanol/hexane to give the title compound (85 mg).
MS (ESI+): [M+H]⁺585.1.

### Example 106

### 3-(6-chloro-2-(3-fluoropyridin-2-yl)-3,4-dihydro-2H-chromen-7-yl)-5-cyclopropyl-7-(1,1-dioxido-1,2-thiazolidin-2-yl)-3,4-dihydropyrido[4,3-d]pyrimidin-2(1H)-one

### A) 4-amino-2-cyclopropyl-6-(1,1-dioxido-1,2-thiazolidin-2-yl)nicotinaldehyde

By a method similar to that in Example 86, step A, the title compound was obtained.
MS (ESI+) : [M+H]⁺282.2.

### B) 3-(6-chloro-2-(3-fluoropyridin-2-yl)-3,4-dihydro-2H-chromen-7-yl)-S-cyclopropyl-7-(1,1-dioxido-1,2-thiazolidin-2-yl)-3,4-dihydropyrido[4,3-d]pyrimidin-2(1H)-one

In the same manner as in Example 4, step L and using the compound obtained in step A, the title compound was obtained.
MS (ESI+): [M+H]⁺570.2.

### Example 107

### 3-(2-(3-fluoropyridin-2-yl)-6-methyl-3,4-dihydro-2H-chromen-7-yl)-8-methoxy-5-(trifluoromethyl)-3,4-dihydropyrido[3,4-d]pyrimidin-2(1H)-one

### A) 3-chloro-5-(trifluoromethyl)isonicotinaldehyde

To a solution of diisopropylamine (5.79 mL) in THF (200 mL) was added n-butyllithium (25.8 mL) at -78°C, and the mixture was stirred at room temperature for 30 min and cooled to -78°C. To this solution was added dropwise a solution of 3-chloro-5-(trifluoromethyl)pyridine (5 g) in THF (25 mL). The solution was stirred at -78°C for 1 hr, and thereto was added dropwise a solution of DMF (4.43 g) in THF (25 mL). The solution was stirred at -78°C for 90 min, poured into a saturated aqueous sodium hydrogen carbonate solution, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (3.6 g).
¹H NMR (300 MHz, CDCl₃) δ 8.93 (2H, s), 10.44 (1H, d).

### B) 3-azido-4-(dimethoxymethyl)-5-(trifluoromethyl)pyridine

In the same manner as in Example 7, step A and using the compound obtained in step A, the title compound was obtained.
¹H NMR (300 MHz, CDCl₃) δ 3.50 (6H, s), 5.46-5.51 (1H, m), 8.53-8.82 (2H, m), 8.66 (1H, s), 8.67 (1H, s).

### C) 4-(dimethoxymethyl)-5-(trifluoromethyl)pyridin-3-amine

A suspension of 3-azido-4-(dimethoxymethyl)-5-(trifluoromethyl)pyridine (4.6 g) and 10% palladium carbon (700 mg) in methanol (50 mL) was stirred under a hydrogen atmosphere at room temperature for 2 hr. The reaction mixture was filtered, and the solvent of the filtrate was evaporated under reduced pressure. The residue was purified by silica gel column chromatography (ethyl acetate/hexane) to give the title compound (3.9 g).
¹H NMR (300 MHz, CDCl₃) δ 3.47 (6H, s), 4.77 (2H, brs), 5.45 (1H, s), 8.21 (1H, s), 8.23 (1H, s).

### D) 2-bromo-4-(dimethoxymethyl)-5-(trifluoromethyl)pyridin-3-amine

the compound obtained in step C, the title compound was obtained.
¹H NMR (300 MHz, CDCl₃) δ 3.48 (6H, s), 5.24-5.38 (2H, m), 5.43 (1H, d, J = 1.1 Hz), 7.97 (1H, s).

### E) 4-(dimethoxymethyl)-2-methoxy-5-(trifluoromethyl)pyridin-3-amine

In the same manner as in Example 81, step A and using the compound obtained in step D, the title compound was obtained.
¹H NMR (300 MHz, CDCl₃) δ 3.46 (6H, s), 4.02 (3H, s), 4.91 (2H, brs), 5.40 (1H, s), 7.79 (1H, s).
MS (ESI+): [M+H]⁺267.0.

### F) 3-(2-(3-fluoropyridin-2-yl)-6-methyl-3,4-dihydro-2H-chromen-7-yl)-8-methoxy-5-(trifluoromethyl)-3,4-dihydropyrido[3,4-d]pyrimidin-2(1H)-one

In the same manner as in Example 10, step B to C and using compound obtained in step E, the title compound was obtained.
MS (ESI+): [M+H]⁺488.9.

### Example 108

### 3-(6-chloro-2-(3-fluoropyridin-2-yl)-3,4-dihydro-2H-chromen-7-yl)-8-methoxy-5-(trifluoromethyl)-3,4-dihydropyrido[3,4-d]pyrimidin-2(1H)-one

By a method similar to that in Example 10, step B to C, the title compound was obtained.
MS (ESI+): [M+H]⁺ 508.9.

### Example 109

### 5-chloro-3-(6-chloro-2-(3-fluoropyridin-2-yl)-3,4-dihydro-2H-chromen-7-yl)-8-(4-hydroxypiperidin-1-yl)-3,4-dihydropyrido[3,4-d]pyrimidin-2(1H)-one

### A) 1-(3-amino-5-chloro-4-(dimethoxymethyl)pyridin-2-yl)piperidin-4-ol

By a method similar to that in Example 81, step A, the title compound was obtained.
MS (ESI+): [M+H]⁺302.0.

### B) 5-chloro-3-(6-chloro-2-(3-fluoropyridin-2-yl)-3,4-dihydro-2H-chromen-7-yl)-8-(4-hydroxypiperidin-1-yl)-3,4-dihydropyrido[3,4-d]pyrimidin-2(1H)-one

In the same manner as in Example 10, step B to C and using the compound obtained in step A, the title compound was obtained.
MS (ESI+): [M+H]⁺543.9.

### Example 110

### 3-(2-(3-fluoropyridin-2-yl)-6-methyl-3,4-dihydro-2H-chromen-7-yl)-8-(2-methoxyethoxy)-5-(trifluoromethyl)-3,4-dihydropyrido[4,3-d]pyrimidin-2(1H)-one

A mixture of 8-bromo-3-(2-(3-fluoropyridin-2-yl)-6-methylchroman-7-yl)-5-(trifluoromethyl)-3,4-dihydropyrido[4,3-d]pyrimidin-2(1H)-one (150 mg), ethylene glycol monomethyl ester (106 mg), racemate of 2-(di-t-butylphosphino)-1,1'-binaphthyl (33.4 mg), palladium(II) acetate (12.5 mg), cesium carbonate(227 mg), and toluene (5 mL) was stirred under a nitrogen atmosphere at 110°C overnight. The reaction mixture was cooled to room temperature, and diluted with ethyl acetate. The diluted solution was washed with saturated brine, and dried over magnesium sulfate. The solvent was evaporated under reduced pressure and the residue was purified by silica gel column chromatography (ethyl acetate/hexane) to give the title compound (12.6 mg).
MS (ESI+): [M+H]⁺533.3.

### Example 111

### 3-(6-chloro-2-(3-fluoropyridin-2-yl)-3,4-dihydro-2H-chromen-7-yl)-5-cyclopropyl-N-(1,3-dihydroxypropan-2-yl)-2-oxo-1,2,3,4-tetrahydropyrido[4,3-d]pyrimidine-7-carboxamide

To a solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (130 mg) in DMF (3.0 mL) were added 2-aminopropane-1,3-diol (28.7 mg), WSC (48.9 mg) and HOBt (42.6 mg), and the mixture was stirred at room temperature for 3 hr. Water was added to the reaction mixture, and the precipitated solid was collected by filtration. The filtrated solid was dissolved in ethyl acetate. The solvent was evaporated under reduced pressure, and the residue was crystallized from ethyl acetate/hexane to give the title compound (80 mg).
MS (ESI+): [M+H]⁺586.3.

### Example 112

### 3-(6-chloro-2-(3-fluoropyridin-2-yl)-3,4-dihydro-2H-chromen-7-yl)-5-cyclopropyl-7-((3-hydroxyazetidin-1-yl)carbonyl)-3,4-dihydropyrido[4,3-d]pyrimidin-2(1H)-one

By a method similar to that in Example 74, the title compound was obtained.
MS (ESI+): [M+H]⁺550.2.

### Example 113

### 3-(6-chloro-2-(3-fluoropyridin-2-yl)-3,4-dihydro-2H-chromen-7-yl)-5-cyclopropyl-7-(2-oxo-1,3-oxazolidin-3-yl)-3,4-dihydropyrido[4,3-d]pyrimidin-2(1H)-one

To a mixture of 7-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one (269 mg), 1,3-oxazolidin-2-one (58 mg), (9,9-dimethyl-9H-xanthene-4,5-diyl)bis(diphenylphosphine) (48 mg), cesium carbonate (271 mg), and toluene (4 mL) was added tris(dibenzylideneacetone)dipalladium(0) (25 mg). The reaction mixture was stirred under microwave irradiation at 180°C for 1 hr, and the insoluble material was filtered off by using celite. The filtrate was extracted with ethyl acetate, and the extract was washed with saturated brine, and dried over magnesium sulfate. The solvent was evaporated under reduced pressure. The residue was collected by HPLC (C18, mobile phase: water/acetonitrile (0.1% TFA-containing system)), and saturated aqueous sodium hydrogen carbonate solution was added to the obtained fraction. The mixture was extracted with ethyl acetate, dried over anhydrous magnesium sulfate, and concentrated under reduced pressure. The residue was recrystallized from ethyl acetate/diisopropyl ether to give the title compound (69 mg).
MS (ESI+): [M+H]⁺536.3.

### Example 114

### 3-(6-chloro-2-(3-fluoropyridin-2-yl)-3,4-dihydro-2H-chromen-7-yl)-5-cyclopropyl-N-(2-hydroxy-2-methylpropyl)-2-oxo-1,2,3,4-tetrahydropyrido[4,3-d]pyrimidine-7-carboxamide

By a method similar to that in Example 74, the title compound was obtained.
MS (ESI+) : [M+H]⁺566.2.

### Example 115

### 3-(6-chloro-2-(3-fluoropyridin-2-yl)-3,4-dihydro-2H-chromen-7-yl)-5-cyclopropyl-7-(1H-pyrazol-5-yl)-3,4-dihydropyrido[4,3-d]pyrimidin-2(1H)-one

### A) 3-(6-chloro-2-(3-fluoropyridin-2-yl)-3,4-dihydro-2H-chromen-7-yl)-5-cyclopropyl-7-(1-(tetrahydro-2H-pyran-2-yl)-1H-pyrazol-5-yl)-3,4-dihydropyrido[4,3-d]pyrimidin-2(1H)-one

By a method similar to that in Example 93, step A, the title compound was obtained.
¹H NMR (300 MHz, CDCl₃) δ 0.90-1.07 (2H, m), 1.08-1.24 (2H, m), 1.48-1.61 (1H, m), 1.66-1.85 (3H, m), 1.93-2.04 (1H, m), 2.06-2.15 (1H, m), 2.15-2.32 (1H, m), 2.35-2.59 (2H, m), 2.83-3.14 (2H, m), 3.59 (1H, t, J = 11.4 Hz), 3.98-4.08 (1H, m), 4.79-5.07 (2H, m), 5.38-5.55 (1H, m), 6.27 (1H, dd, J = 10.2, 1.9 Hz), 6.49 (1H, d, J = 1.9 Hz), 6.73 (1H, s), 6.97-7.03 (1H, m), 7.26 (1H, s), 7.33 (1H, dt, J = 8.3, 4.2 Hz), 7.39-7.51 (1H, m), 7.57 (1H, d, J = 1.9 Hz), 8.03 (1H, brs), 8.41-8.53 (1H, m).

### B) 3-(6-chloro-2-(3-fluoropyridin-2-yl)-3,4-dihydro-2H-chromen-7-yl)-5-cyclopropyl-7-(1H-pyrazol-5-yl)-3,4-dihydropyrido[4,3-d]pyrimidin-2(1H)-one

To a solution of 3-(6-chloro-2-(3-fluoropyridin-2-yl)-3,4-dihydro-2H-chromen-7-yl)-5-cyclopropyl-7-(1-(tetrahydro-2H-pyran-2-yl)-1H-pyrazol-5-yl)-3,4-dihydropyrido[4,3-d]pyrimidin-2(1H)-one (128 mg) in ethanol (3.0 mL) was added concentrated hydrochloric acid (0.3 mL). The reaction mixture was stirred at room temperature for 1 hr. To the reaction mixture was added saturated aqueous sodium hydrogen carbonate solution, and the mixture was extracted with ethyl acetate. The extract was washed with saturated brine, and dried over magnesium sulfate. The solvent was evaporated under reduced pressure. The residue was crystallized from ethanol/hexane to give the title compound (63 mg).
MS (ESI+): [M+H]⁺517.2.

### Example 116

### 3-(6-chloro-2-(3-fluoropyridin-2-yl)-3,4-dihydro-2H-chromen-7-yl)-5-cyclopropyl-7-(1H-pyrazol-4-yl)-3,4-dihydropyrido[4,3-d]pyrimidin-2(1H)-one

### A) 3-(6-chloro-2-(3-fluoropyridin-2-yl)-3,4-dihydro-2H-chromen-7-yl)-5-cyclopropyl-7-(1-(tetrahydro-2H-pyran-2-yl)-1H-pyrazol-5-yl)-3,4-dihydropyrido[4,3-d]pyrimidin-2(1H)-one

By a method similar to that in Example 93, step A and using 1-(tetrahydro-2H-pyran-2-yl)-4-(4,4,5,5-tetramethyl-1,3,2-dioxaborolan-2-yl)-1H-pyrazole and 7-chloro-3-(6-chloro-2-(3-fluoropyridin-2-yl)-3,4-dihydro-2H-chromen-7-yl)-5-cyclopropyl-3,4-dihydropyrido[4,3-d]pyrimidin-2(1H)-one, the title compound was obtained.
¹H NMR (300 MHz, CDCl₃) δ 0.90-1.07 (2H, m), 1.08-1.24 (2H, m), 1.48-1.61 (1H, m), 1.66-1.85 (3H, m), 1.93-2.04 (1H, m), 2.06-2.15 (1H, m), 2.15-2.32 (1H, m), 2.35-2.59 (2H, m), 2.83-3.14 (2H, m), 3.59 (1H, t, J = 11.4 Hz), 3.98-4.08 (1H, m), 4.79-5.07 (2H, m), 5.38-5.55 (1H, m), 6.27 (1H, dd, J = 10.2, 1.9 Hz), 6.49 (1H, d, J = 1.9 Hz), 6.73 (1H, s), 6.97-7.03 (1H, m), 7.26 (1H, s), 7.33 (1H, dt, J = 8.3, 4.2 Hz), 7.39-7.51 (1H, m), 7.57 (1H, d, J = 1.9 Hz), 8.03 (1H, brs), 8.41-8.53 (1H, m).

### B) 3-(6-chloro-2-(3-fluoropyridin-2-yl)-3,4-dihydro-2H-chromen-7-yl)-5-cyclopropyl-7-(1H-pyrazol-4-yl)-3,4-dihydropyrido[4,3-d]pyrimidin-2(1H)-one

To a solution of 3-(6-chloro-2-(3-fluoropyridin-2-yl)-3,4-dihydro-2H-chromen-7-yl)-5-cyclopropyl-7-(1-(tetrahydro-2H-pyran-2-yl)-1H-pyrazol-5-yl)-3,4-dihydropyrido[4,3-d]pyrimidin-2(1H)-one (128 mg) in ethanol (3.0 mL) was added concentrated hydrochloric acid (0.3 mL). The reaction mixture was stirred at room temperature for 1 hr. To the reaction mixture was added a saturated aqueous sodium hydrogen carbonate solution, and the mixture was extracted with ethyl acetate. The extract was washed with saturated brine, and dried over magnesium sulfate. The solvent was evaporated under reduced pressure. The residue was crystallized from ethanol/hexane to give the title compound (63 mg).
MS (ESI+): [M+H]⁺517.1.

### Example 117

### 3-(6-chloro-2-(3-fluoropyridin-2-yl)-3,4-dihydro-2H-chromen-7-yl)-5-cyclopropyl-7-(5-oxo-4,5-dihydro-1,3,4-oxadiazol-2-yl)-3,4-dihydropyrido[4,3-d]pyrimidin-2(1H)-one

To a solution of ethyl 3-(6-chloro-2-(3-fluoropyridin-2-yl)chroman-7-yl)-5-cyclopropyl-2-oxo-1,2,3,4-tetrahydropyrido[4,3-d]pyrimidine-7-carboxylate (63 mg) in ethanol (10 mL) was added hydrazine monohydrate (0.5 mL), and the mixture was stirred at 100°C overnight. The reaction mixture was concentrated under reduced pressure, to the obtained residue was added THF (10 mL), and CDI (58.6 mg) and DBU (0.054 mL) were added. The reaction mixture was stirred for 3 hr at room temperature, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (51 mg).
MS (ESI+) : [M+H]⁺535.1.

### Example 118

### 3-(6-chloro-2-(3-fluoropyridin-2-yl)-3,4-dihydro-2H-chromen-7-yl)-5-cyclopropyl-7-(5-thioxo-4,5-dihydro-1,2,4-oxadiazol-3-yl)-3,4-dihydropyrido[4,3-d]pyrimidin-2(1H)-one

To a solution of 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile (150 mg) in DMF (2 mL) were added hydroxylamine (110 mg) and sodium carbonate (167 mg), and the mixture was stirred at 60°C for 5 hr. The reaction mixture was cooled to room temperature, water was added, and the mixture was extracted with ethyl acetate. The extract was washed with saturated brine, and dried over magnesium sulfate. The solvent was evaporated under reduced pressure. To the residue was added THF (5 mL), and 1,1-thiocarbonylbis-1H-imidazole (169 mg) and DBU (0.143 mL) were added. The reaction mixture was stirred for 3 hr at room temperature, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (45 mg).
MS (ESI+): [M+H]⁺551.0.

### Example 119

### 3-(6-chloro-2-(3-fluoropyridin-2-yl)-3,4-dihydro-2H-chromen-7-yl)-5-cyclopropyl-7-(3-methyl-1H-1,2,4-triazol-5-yl)-3,4-dihydropyrido[4,3-d]pyrimidin-2(1H)-one

To a solution of 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile (50 mg) in methanol (2 mL) was added sodium methoxide (50 mg), and the mixture was stirred at 60°C for 3 hr. To the reaction mixture was added acethydrazide (46.7 mg), and the mixture was stirred at 90°C for 3 days. The reaction mixture was cooled, 1N hydrochloric acid was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (32 mg).
MS (ESI+): [M+H]⁺532.2.

### Example 120

### 3-(6-chloro-2-(3-fluoropyridin-2-yl)-3,4-dihydro-2H-chromen-7-yl)-5-cyclopropyl-7-(3-oxomorpholin-4-yl)-3,4-dihydropyrido[4,3-d]pyrimidin-2(1H)-one

By a method similar to that in Example 113, the title compound was obtained.
MS (ESI+): [M+H]⁺550.3.

### Example 121

### 3-(6-chloro-2-(3-fluoropyridin-2-yl)-3,4-dihydro-2H-chromen-7-yl)-5-cyclopropyl-7-(3-methyl-1,2,4-oxadiazol-5-yl)-3,4-dihydropyrido[4,3-d]pyrimidin-2(1H)-one

To a mixed solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (100 mg), N'-hydroxyethanimidamide (15 mg), and 2,4,6-tripropyl-1,3,5,2,4,6-trioxatriphosphinan 2,4,6-trioxide (321 mg) in DMF (2 mL)/ethyl acetate (2 mL) was added triethylamine (84 µL), and the reaction mixture was stirred under a nitrogen atmosphere at 85°C for 5 hr. 2,4,6-Tripropyl-1,3,5,2,4,6-trioxatriphosphinan 2,4,6-trioxide (321 mg) and triethylamine(84 µL) were added to the reaction mixture, and the mixture was stirred under a nitrogen atmosphere at 110°C overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (34 mg).
MS (ESI+) : [M+H]⁺533.3.

### Example 122

### 5-cyclopropyl-3-(2-(3-fluoropyridin-2-yl)-6-methyl-3,4-dihydro-2H-chromen-7-yl)-7-methoxy-3,4-dihydropyrido[4,3-d]pyrimidin-2(1H)-one

In the same manner as in Example 16, step E and using 4-amino-2-cyclopropyl-6-methoxynicotinaldehyde and 2-(3-fluoropyridin-2-yl)-6-methylchromane-7-amine, the title compound was obtained.
MS (ESI+) : [M+H]⁺461.5.

### Example 123

### 5-cyclopropyl-3-(2-(3-fluoropyridin-2-yl)-6-methyl-3,4-dihydro-2H-chromen-7-yl)-7-(4-(hydroxymethyl)-1H-pyrazol-1-yl)-3,4-dihydropyrido[4,3-d]pyrimidin-2(1H)-one

### A) ethyl 1-(4-amino-6-cyclopropyl-5-formylpyridin-2-yl)-1H-pyrazole-4-carboxylate

To a solution of 4-amino-6-chloro-2-cyclopropylnicotinaldehyde (198 mg) in DMF (4 mL) were added ethyl 1H-pyrazole-4-carboxylate (212 mg) and cesium carbonate (656 mg) at room temperature. The mixture was stirred under microwave irradiation at 145°C for 20 min. Water was added to the reaction mixture, and the mixture was extracted with ethyl acetate. The extract was washed with water and saturated brine and dried over sodium sulfate. The solvent was evaporated under reduced pressure and the residue was purified by silica gel column chromatography (ethyl acetate/hexane) to give the title compound (126 mg).
¹H NMR (300 MHz, CDCl₃) δ 1.06-1.15 (2H, m), 1.29-1.36 (2H, m), 1.39 (3H, t, J = 7.1 Hz), 2.47-2.59 (1H, m), 4.30-4.41 (2H, m), 6.98 (1H, s), 8.05 (1H, s), 8.90 (1H, s), 10.63 (1H, s), (2H, hidden).

### B) ethyl 1-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)-1H-pyrazole-4-carboxylate

In the same manner as in Example 4, step L and using the compound obtained in step A, the title compound was obtained.
MS (ESI+): [M+H]⁺569.3.

### C) 5-cyclopropyl-3-(2-(3-fluoropyridin-2-yl)-6-methyl-3,4-dihydro-2H-chromen-7-yl)-7-(4-(hydroxymethyl)-1H-pyrazol-1-yl)-3,4-dihydropyrido[4,3-d]pyrimidin-2(1H)-one

To a solution of lithium aluminum hydride (20 mg) in THF (1.0 mL) was added dropwise a solution of ethyl 1-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)-1H-pyrazole-4-carboxylate (74 mg) in THF (2.0 mL) at 0°C, and the mixture was stirred under a nitrogen atmosphere at 0°C for 30 min. To the reaction mixture was added sodium sulfate decahydrate, and the insoluble material was filtered off with celite. The filtrate was concentrated under reduced pressure. The residue was purified by NH silica gel column chromatography (methanol/ethyl acetate), and crystallized from ethyl acetate/hexane to give the title compound (19 mg).
MS (ESI+): [M+H]⁺527.2.

### Example 124

### 3-(6-chloro-2-(3-fluoropyridin-2-yl)-3,4-dihydro-2H-chromen-7-yl)-5-cyclopropyl-7-(1,3,4-oxadiazol-2-yl)-3,4-dihydropyrido[4,3-d]pyrimidin-2(1H)-one

To a mixed solution of 3-(6-chloro-2-(3-fluoropyridin-2-yl)-3,4-dihydro-2H-chromen-7-yl)-5-cyclopropyl-2-oxo-1,2,3,4-tetrahydropyrido[4,3-d]pyrimidine-7-carboxylic acid (100 mg), formic acid hydrazide (13 mg), and 2,4,6-tripropyl-1,3,5,2,4,6-trioxatriphosphinan 2,4,6-trioxide (321 mg) in DMF (1 mL)/ethyl acetate (1 mL) was added triethylamine (61 mg), and the reaction mixture was stirred under a nitrogen atmosphere at 110°C overnight. 2,4,6-Tripropyl-1,3,5,2,4,6-trioxatriphosphinan 2,4,6-trioxide (321 mg) and triethylamine (61 mg) were added to the reaction mixture, and the mixture was stirred under a nitrogen atmosphere at 120°C overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and crystallized from ethyl acetate/hexane to give the title compound (48 mg).
MS (ESI+) : [M+H]⁺519.1.

### Example 125

### 3-(6-chloro-2-(3-fluoropyridin-2-yl)-3,4-dihydro-2H-chromen-7-yl)-5-cyclopropyl-7-(2-oxopiperazin-1-yl)-3,4-dihydropyrido[4,3-d]pyrimidin-2(1H)-one

By a method similar to that in Example 113, the title compound was obtained.
MS (ESI+): [M+H]⁺549.1.

### Example 126

### 5-chloro-3-(6-chloro-2-(3-fluoropyridin-2-yl)-3,4-dihydro-2H-chromen-7-yl)-8-(3,5-dimethyl-1,2-oxazol-4-yl)-3,4-dihydropyrido[3,4-d]pyrimidin-2(1H)-one

By a method similar to that in Example 10, step B to C, the title compound was obtained.
MS (ESI+): [M+H]⁺540.0.

### Example 127

### 5-chloro-3-(6-chloro-2-(3-fluoropyridin-2-yl)-3,4-dihydro-2H-chromen-7-yl)-8-(3,6-dihydro-2H-pyran-4-yl)-3,4-dihydropyrido[3,4-d]pyrimidin-2(1H)-one

### A) 5-chloro-2-(3,6-dihydro-2H-pyran-4-yl)-4-(dimethoxymethyl)pyridin-3-amine

By a method similar to that in Example 10, step A, the title compound was obtained.
¹H NMR (300 MHz, CDCl₃) δ 2.52 (2H, dt, J = 4.9, 2.6 Hz), 3.50 (6H, s), 3.95 (2H, t, J = 5.5 Hz), 4.31 (2H, q, J = 2.9 Hz), 4.99 (2H, brs), 5.75 (1H, s), 6.11 (1H, m), 7.90 (1H, s).

### B) 5-chloro-3-(6-chloro-2-(3-fluoropyridin-2-yl)-3,4-dihydro-2H-chromen-7-yl)-8-(3,6-dihydro-2H-pyran-4-yl)-3,4-dihydropyrido[3,4-d]pyrimidin-2(1H)-one

In the same manner as in Example 10, step B to step C and using the compound obtained in step A, the title compound was obtained.
MS (ESI+): [M+H]⁺527.0

### Example 128

### 5-cyclopropyl-7-(difluoromethoxy)-3-(2-(3-fluoropyridin-2-yl)-6-methyl-3,4-dihydro-2H-chromen-7-yl)-3,4-dihydropyrido[4,3-d]pyrimidin-2(1H)-one

In the same manner as in Example 17 then Example 18 and using 5-cyclopropyl-3-(2-(3-fluoropyridin-2-yl)-6-methyl-3,4-dihydro-2H-chromen-7-yl)-7-methoxy-3,4-dihydropyrido[4,3-d]pyrimidin-2(1H)-one, the title compound was obtained.
MS (ESI+): [M+H]⁺497.3.

### Example 129

### 5-chloro-3-(2-(3-fluoropyridin-2-yl)-6-methyl-3,4-dihydro-2H-chromen-7-yl)-8-methyl-3,4-dihydropyrido[3,4-d]pyrimidin-2(1H)-one

In the same manner as in Example 7, step F to step G and using 6-chloro-2-(3-fluoropyridin-2-yl)chromane-7-amine, the title compound was obtained.
MS (ESI+): [M+H]⁺439.2

### Example 130

### 5-chloro-3-(6-chloro-2-(3-fluoropyridin-2-yl)-3,4-dihydro-2H-chromen-7-yl)-8-(thiomorpholin-4-yl)-3,4-dihydropyrido[3,4-d]pyrimidin-2(1H)-one

### A) 5-chloro-4-(dimethoxymethyl)-2-thiomorpholinopyridin-3-amine

By a method similar to that in Example 81, step A, the title compound was obtained.
MS (ESI+) : [M+H]⁺304.01.

### B) 5-chloro-3-(6-chloro-2-(3-fluoropyridin-2-yl)-3,4-dihydro-2H-chromen-7-yl)-8-(thiomorpholin-4-yl)-3,4-dihydropyrido[3,4-d]pyrimidin-2(1H)-one

In the same manner as in Example 10, step B to step C and using the compound obtained in step A, the title compound was obtained.
MS (ESI+) : [M+H]⁺546.2.

### Example 131

### 3-(6-chloro-2-(3-fluoropyridin-2-yl)-3,4-dihydro-2H-chromen-7-yl)-5-cyclopropyl-7-(2-oxoimidazolidin-1-yl)-3,4-dihydropyrido[4,3-d]pyrimidin-2(1H)-one

By a method similar to that in Example 113, the title compound was obtained.
MS (ESI+): [M+H]⁺535.1.

### Example 132

### 5-cyclopropyl-3-(2-(3-fluoropyridin-2-yl)-6-methyl-3,4-dihydro-2H-chromen-7-yl)-7-(1H-pyrazol-1-yl)-3,4-dihydropyrido[4,3-d]pyrimidin-2(1H)-one

By a method similar to that in Example 123, step A to B, the title compound was obtained.
MS (ESI+): [M+H]⁺497.1.

### Example 133

### 5-cyclopropyl-3-(2-(3-fluoropyridin-2-yl)-6-methyl-3,4-dihydro-2H-chromen-7-yl)-7-(4-methyl-1H-pyrazol-1-yl)-3,4-dihydropyrido[4,3-d]pyrimidin-2(1H)-one

In the same manner as in Example 123, step B and using 6-methyl-2-(3-fluoropyridin-2-yl)chromane-7-amine, the title compound was obtained.
MS (ESI+): [M+H]⁺511.2.

### Example 134

### 3-(6-chloro-2-(3-fluoropyridin-2-yl)-3,4-dihydro-2H-chromen-7-yl)-5-cyclopropyl-7-(5-oxo-4,5-dihydro-1,2,4-thiadiazol-3-yl)-3,4-dihydropyrido[4,3-d]pyrimidin-2(1H)-one

To a solution of 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile (150 mg) in DMSO (2 mL) were added hydroxylamine (110 mg) and sodium carbonate (167 mg), and the mixture was stirred at 60°C for 5 hr. The reaction mixture was cooled to room temperature, water was added, and the mixture was extracted with ethyl acetate. The extract was washed with saturated brine, and dried over magnesium sulfate. The solvent was evaporated under reduced pressure. To the residue was added THF (5 mL), and 1,1'-thioCDI (169 mg) and DBU (143 µL) were added. The reaction mixture was stirred at room temperature overnight, 1N hydrochloric acid was added, and the mixture was extracted with ethyl acetate. The extract was washed with saturated brine, and dried over magnesium sulfate. The solvent was evaporated under reduced pressure. The residue was dissolved in THF (5 mL), boron trifluoride diethyl etherate (200 µL) was added, and the mixture was stirred at room temperature for 1 hr. Water was added to the reaction mixture, and the mixture was extracted with ethyl acetate, and the extract was washed with saturated brine, and dried over magnesium sulfate. The solvent was evaporated under reduced pressure and the residue was purified by silica gel column chromatography (ethyl acetate/hexane) to give the title compound (21 mg). MS (ESI+): [M+H]⁺551.1.

### Example 135

### 1-(5-cyclopropyl-3-(2-(3-fluoropyridin-2-yl)-6-methyl-3,4-dihydro-2H-chromen-7-yl)-2-oxo-1,2,3,4-tetrahydropyrido[4,3-d]pyrimidin-7-yl)-1H-pyrazole-4-carboxamide

By a method similar to that in Example 95, the title compound was obtained.
MS (ESI+) : [M+H]⁺540.4.

### Example 136

### 3-(6-chloro-2-(3-fluoropyridin-2-yl)-3,4-dihydro-2H-chromen-7-yl)-5-cyclopropyl-7-(1,2,4-oxadiazol-3-yl)-3,4-dihydropyrido[4,3-d]pyrimidin-2(1H)-one

To a solution of 3-(6-chloro-2-(3-fluoropyridin-2-yl)chroman-7-yl)-5-cyclopropyl-2-oxo-1,2,3,4-tetrahydropyrido[4,3-d]pyrimidine-7-carbonitrile (150 mg) in DMSO (2 mL) were added hydroxylamine (110 mg) and sodium carbonate (167 mg), and the mixture was stirred at 60°C for 5 hr. The reaction mixture was cooled to room temperature, water was added, and the mixture was extracted with ethyl acetate. The extract was washed with saturated brine, and dried over magnesium sulfate. The solvent was evaporated under reduced pressure. To the residue were added trimethyl orthoformate (10 mL) and boron trifluoride diethyl etherate (2 drops). The reaction mixture was stirred at 60°C overnight, and the reaction mixture was concentrated under reduced pressure. Water was added to the obtained residue, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (31 mg). MS (ESI+): [M+H]⁺519.2.

### Example 137

### 3-(6-chloro-2-(3-fluoropyridin-2-yl)-3,4-dihydro-2H-chromen-7-yl)-5-cyclopropyl-7-(2-oxa-6-azaspiro[3.3]hept-6-ylcarbonyl)-3,4-dihydropyrido[4,3-d]pyrimidin-2(1H)-one

By a method similar to that in Example 74, the title compound was obtained.
MS (ESI+) : [M+H]⁺576.4.

### Example 138

### 5-cyclopropyl-3-(2-(3-fluoropyridin-2-yl)-6-methyl-3,4-dihydro-2H-chromen-7-yl)-7-(2-oxo-1,3-oxazolidin-3-yl)-3,4-dihydropyrido[4,3-d]pyrimidin-2(1H)-one

By a method similar to that in Example 113, the title compound was obtained.
MS (ESI+): [M+H]⁺516.2.

### Example 139

### 3-(6-chloro-2-(3-fluoropyridin-2-yl)-3,4-dihydro-2H-chromen-7-yl)-5-cyclopropyl-7-((3,3-difluoroazetidin-1-yl)carbonyl)-3,4-dihydropyrido[4,3-d]pyrimidin-2(1H)-one

By a method similar to that in Example 74, the title compound was obtained.
MS (ESI+): [M+H]⁺570.2.

### Example 140

### 5-cyclopropyl-3-(2-(3-fluoropyridin-2-yl)-6-methyl-3,4-dihydro-2H-chromen-7-yl)-7-(1H-1,2,4-triazol-1-yl)-3,4-dihydropyrido[4,3-d]pyrimidin-2(1H)-one

Using 4-amino-6-chloro-2-cyclopropylnicotinaldehyde as a starting material, and by a method similar to that in Example 123, step A to B, the title compound was obtained.
MS (ESI+) : [M+H]⁺498.2.

### Example 141

### 5-cyclopropyl-3-(2-(3-fluoropyridin-2-yl)-6-methyl-3,4-dihydro-2H-chromen-7-yl)-7-((1-methyl-1H-pyrazol-5-yl)oxy)-3,4-dihydropyrido[4,3-d]pyrimidin-2(1H)-one

### A) 4-amino-2-cyclopropyl-6-((1-methyl-1H-pyrazol-5-yl)oxy)nicotinaldehyde

To a solution of 4-amino-6-chloro-2-cyclopropylnicotinaldehyde (97 mg) in DMF (4 mL) were added 1-methyl-1H-pyrazol-5-ol (73 mg) and cesium carbonate (321 mg) at room temperature. The mixture was stirred under microwave irradiation at 145°C for 20 min. Water was added to the reaction mixture, and the mixture was extracted with ethyl acetate. The extract was washed with water and saturated brine and dried over sodium sulfate. The solvent was evaporated under reduced pressure and the residue was purified by silica gel column chromatography (ethyl acetate/hexane) to give the title compound (62 mg).
¹H NMR (300 MHz, CDCl₃) δ 0.91-1.01 (2H, m), 1.13-1.20 (2H, m), 2.43 (1H, tt, J = 8.1, 4.7 Hz), 3.83 (3H, s), 5.85 (1H, d, J = 0.5 Hz), 5.98 (1H, d, J = 2.2 Hz), 7.25-7.28 (1H, m), 10.54 (1H, s).

### B) 5-cyclopropyl-3-(2-(3-fluoropyridin-2-yl)-6-methyl-3,4-dihydro-2H-chromen-7-yl)-7-((1-methyl-1H-pyrazol-5-yl)oxy)-3,4-dihydropyrido[4,3-d]pyrimidin-2(1H)-one

In the same manner as in Example 123, step B and using the compound obtained in step A, the title compound was obtained.
MS (ESI+): [M+H]⁺527.3.

### Example 142

### 5-chloro-8-cyclopropyl-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 4, step L and using 3-amino-5-chloro-2-cyclopropyl-4-formylpyridine, the title compound was obtained.
MS (ESI+): [M+H]⁺465.2.

### Example 143

### 5-chloro-3-(6-chloro-2-(3-fluoropyridin-2-yl)-3,4-dihydro-2H-chromen-7-yl)-8-cyclopropyl-3,4-dihydropyrido[3,4-d]pyrimidin-2(1H)-one

In the same manner as in Example 4, step L and using 3-amino-5-chloro-2-cyclopropyl-4-formylpyridine, the title compound was obtained.
MS (ESI+): [M+H]⁺485.2.

### Example 144

### 5-cyclopropyl-3-(2-(3-fluoropyridin-2-yl)-6-methyl-3,4-dihydro-2H-chromen-7-yl)-7-(2-oxopyrrolidin-1-yl)-3,4-dihydropyrido[4,3-d]pyrimidin-2(1H)-one

By a method similar to that in Example 113, the title compound was obtained.
MS (ESI+): [M+H]⁺514.4.

### Example 145

### 3-(6-chloro-2-(3-fluoropyridin-2-yl)-3,4-dihydro-2H-chromen-7-yl)-5-cyclopropyl-7-((3-fluoroazetidin-1-yl)carbonyl)-3,4-dihydropyrido[4,3-d]pyrimidin-2(1H)-one

By a method similar to that in Example 74, the title compound was obtained.
MS (ESI+): [M+H]⁺552.2.

### Example 146

### 3-(6-chloro-2-(3-fluoropyridin-2-yl)-3,4-dihydro-2H-chromen-7-yl)-5-cyclopropyl-7-((3-methoxyazetidin-1-yl)carbonyl)-3,4-dihydropyrido[4,3-d]pyrimidin-2(1H)-one

By a method similar to that in Example 74, the title compound was obtained.
MS (ESI+): [M+H]⁺564.2.

### Example 147

### 3-(6-chloro-2-(3-fluoropyridin-2-yl)-3,4-dihydro-2H-chromen-7-yl)-5-cyclopropyl-N-(2,2-difluoroethyl)-2-oxo-1,2,3,4-tetrahydropyrido[4,3-d]pyrimidine-7-carboxamide

By a method similar to that in Example 74, the title compound was obtained.
MS (ESI+): [M+H]⁺558.2.

### Example 148

### 5-chloro-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

### A) 5-chloro-4-(dimethoxymethyl)-2-(1-methyl-1H-pyrazol-4-yl)pyridin-3-amine

By a method similar to that in Example 10, step A, the title compound was obtained.
¹H NMR (300 MHz, CDCl₃) δ 3.51 (6H, s), 3.96 (3H, s), 4.92 (2H, brs), 5.78 (1H, s), 7.81 (1H, s),7.93 (1H, s), 7.95 (1H, s).

### B) 5-chloro-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

In the same manner as in Example 10, step B to step C and using the compound obtained in step A, the title compound was obtained.
MS (ESI+): [M+H]⁺505.0.

### Example 149

### 3-(6-chloro-2-(3-fluoropyridin-2-yl)-3,4-dihydro-2H-chromen-7-yl)-5-cyclopropyl-2-oxo-N-(2,2,2-trifluoroethyl)-1,2,3,4-tetrahydropyrido[4,3-d]pyrimidine-7-carboxamide

By a method similar to that in Example 74, the title compound was obtained.
MS (ESI+): [M+H]⁺576.2.

### Example 150

### 3-(6-chloro-2-(3-fluoropyridin-2-yl)-3,4-dihydro-2H-chromen-7-yl)-5-cyclopropyl-N,N-dimethyl-2-oxo-1,2,3,4-tetrahydropyrido[4,3-d]pyrimidine-7-carboxamide

By a method similar to that in Example 74, the title compound was obtained.
MS (ESI+): [M+H]⁺522.4.

### Example 151

### 5-cyclopropyl-7-(3,5-dimethyl-1,2-oxazol-4-yl)-3-(2-(3-fluoropyridin-2-yl)-6-methyl-3,4-dihydro-2H-chromen-7-yl)-3,4-dihydropyrido[4,3-d]pyrimidin-2(1H)-one

Using 4-amino-6-chloro-2-cyclopropylnicotinaldehyde as a starting material and by a method similar to that in Example 123, step A to B, the title compound was obtained.
MS (ESI+) : [M+H]⁺526.2.

### Example 152

### 5-chloro-3-(6-chloro-2-(3-fluoropyridin-2-yl)-3,4-dihydro-2H-chromen-7-yl)-3,4-dihydropyrido[3,4-d]pyrimidin-2(1H)-one

Using 3-amino-5-chloroisonicotinaldehyde as a starting material and by a method similar to that in Example 4, step L, the title compound was obtained.
MS (ESI+): [M+H]⁺444.9.

### Example 153

### 5-chloro-3-(6-chloro-2-(3-fluoropyridin-2-yl)-3,4-dihydro-2H-chromen-7-yl)-8-(pyrrolidin-1-yl)-3,4-dihydropyrido[3,4-d]pyrimidin-2(1H)-one

### A) 5-chloro-4-(dimethoxymethyl)-2-(pyrrolidin-1-yl)pyridin-3-amine

By a method similar to that in Example 10, step A, the title compound was obtained.
¹H NMR (300 MHz, CDCl₃) δ 1.83-1.98 (4H, m), 3.26-3.34 (4H, m), 3.47 (6H, s), 4.73 (2H, brs), 5.71 (1H, s), 7.63 (1H, s).

### B) 5-chloro-3-(6-chloro-2-(3-fluoropyridin-2-yl)-3,4-dihydro-2H-chromen-7-yl)-8-(pyrrolidin-1-yl)-3,4-dihydropyrido[3,4-d]pyrimidin-2(1H)-one

In the same manner as in Example 10, step B to step C and using the compound obtained in step A, the title compound was obtained.
MS (ESI+): [M+H]⁺514.0.

### Example 154

### 5-cyclopropyl-3-(2-(3-fluoropyridin-2-yl)-6-methyl-3,4-dihydro-2H-chromen-7-yl)-7-(4-(trifluoromethyl)-1H-pyrazol-1-yl)-3,4-dihydropyrido[4,3-d]pyrimidin-2(1H)-one

Using 4-amino-6-chloro-2-cyclopropylnicotinaldehyde as a starting material and by a method similar to that in Example 123, step A to B, the title compound was obtained.
MS (ESI+): [M+H]⁺565.2.

### Example 155

### 5-chloro-3-(6-chloro-2-(3-fluoropyridin-2-yl)-3,4-dihydro-2H-chromen-7-yl)-8-(3-furyl)-3,4-dihydropyrido[3,4-d]pyrimidin-2(1H)-one

### A) 5-chloro-4-(dimethoxymethyl)-2-(furan-3-yl)pyridin-3-amine

By a method similar to that in Example 10, step A, the title compound was obtained.
MS (ESI+): [M+H]⁺269.0.

### B) 5-chloro-3-(6-chloro-2-(3-fluoropyridin-2-yl)-3,4-dihydro-2H-chromen-7-yl)-8-(3-furyl)-3,4-dihydropyrido[3,4-d]pyrimidin-2(1H)-one

In the same manner as in Example 10, step B to C and using the compound obtained in step A, the title compound was obtained.
MS (ESI+) : [M+H]⁺510.9.

### Example 156

### 5-chloro-3-(2-(3-fluoropyridin-2-yl)-6-methyl-3,4-dihydro-2H-chromen-7-yl)-8-methoxy-3,4-dihydropyrido[3,4-d]pyrimidin-2(1H)-one

### A) 5-chloro-4-(dimethoxymethyl)-2-methoxypyridin-3-amine

To a solution of 2-bromo-5-chloro-4-(dimethoxymethyl)pyridin-3-amine (0.20 g) in methanol (4 mL) was added sodium methylate (1.15 g), and the mixture was stirred at 90°C overnight. The solvent was evaporated under reduced pressure, water was added to the obtained residue, and the mixture was extracted with ethyl acetate. The extract was washed with saturated brine, dried over magnesium sulfate, and the solvent was evaporated. The residue was purified by silica gel column chromatography (ethyl acetate/hexane) to give the title compound (0.16 g).
MS (ESI+): [M+H]⁺233.0.

### B) 5-chloro-3-(2-(3-fluoropyridin-2-yl)-6-methyl-3,4-dihydro-2H-chromen-7-yl)-8-methoxy-3,4-dihydropyrido[3,4-d]pyrimidin-2(1H)-one

In the same manner as in Example 10, step B to C and using the compound obtained in step A, the title compound was obtained.
MS (ESI+): [M+H]⁺455.0.

### Example 157

### 5-chloro-3-(6-chloro-2-(3-fluoropyridin-2-yl)-3,4-dihydro-2H-chromen-7-yl)-2-oxo-1,2,3,4-tetrahydropyrido[3,4-d]pyrimidine-8-carbonitrile

To a solution of 8-bromo-5-chloro-3-(6-chloro-2-(3-fluoropyridin-2-yl)chroman-7-yl)-3,4-dihydropyrido[3,4-d]pyrimidin-2(1H)-one (60 mg) in DMF (1.6 mL) was added copper(I) cyanide (69.7 mg), and the mixture was stirred under a nitrogen atmosphere at 140°C for 4 hr. After cooling, the mixture was neutralized with aqueous sodium hydrogen carbonate solution, and extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (14.5 mg).
MS (ESI+): [M+H]⁺469.8.

### Example 158

### 5-chloro-3-(2-(3-fluoropyridin-2-yl)-6-methyl-3,4-dihydro-2H-chromen-7-yl)-8-(pyrrolidin-1-yl)-3,4-dihydropyrido[3,4-d]pyrimidin-2(1H)-one

By a method similar to that in Example 10, step B to step C, the title compound was obtained.
MS (ESI+) : [M+H]⁺494.0.

### Example 159

### 5-cyclopropyl-7-(2,2-difluoroethoxy)-3-(2-(3-fluoropyridin-2-yl)-6-methyl-3,4-dihydro-2H-chromen-7-yl)-3,4-dihydropyrido[4,3-d]pyrimidin-2(1H)-one

To a solution of 7-chloro-5-cyclopropyl-3-(2-(3-fluoropyridin-2-yl)-6-methyl-3,4-dihydro-2H-chromen-7-yl)-3,4-dihydropyrido[4,3-d]pyrimidin-2(1H)-one (300 mg), 5-(di-tert-butylphosphino)-1',3',5'-triphenyl-1'H-1,4'-bipyrazole (20 mg) and cesium carbonate (631 mg) in DMF (5 mL) was added tris(dibenzylideneacetone)dipalladium(0) (24 mg) at room temperature. The mixture was stirred under microwave irradiation at 100°C for 1 hr, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate, and concentrated under reduced pressure to give the title compound (98 mg).
MS (ESI+) : [M+H]⁺511.1.

### Example 160

### 5-chloro-3-(2-(3-fluoropyridin-2-yl)-6-methyl-3,4-dihydro-2H-chromen-7-yl)-8-(1-methyl-1H-pyrazol-5-yl)-3,4-dihydropyrido[3,4-d]pyrimidin-2(1H)-one

### A) 5-chloro-4-(dimethoxymethyl)-2-(1-methyl-1H-pyrazol-5-yl)pyridin-3-amine

To a solution of 2-bromo-5-chloro-4-(dimethoxymethyl)pyridin-3-amine (205 mg), 1-methyl-5-(4,4,5,5-tetramethyl-1,3,2-dioxaborolan-2-yl)-1H-pyrazole (197 mg), tetrakistriphenylphosphinepalladium(0) (84 mg) in DMF (4 mL) was added a 2N aqueous sodium carbonate solution (1.1 mL), and the mixture was stirred under an argon atmosphere at 90°C for 2 hr. The reaction mixture was cooled to room temperature, saturated aqueous sodium carbonate solution was added, and the mixture was extracted with ethyl acetate. The extract was washed with saturated brine, and dried over magnesium sulfate, and the solvent was evaporated. The residue was purified by silica gel column chromatography (ethyl acetate/hexane) to give the title compound (202 mg).
MS (ESI+): [M+H]⁺283.0.

### B) 5-chloro-3-(2-(3-fluoropyridin-2-yl)-6-methyl-3,4-dihydro-2H-chromen-7-yl)-8-(1-methyl-1H-pyrazol-5-yl)-3,4-dihydropyrido[3,4-d]pyrimidin-2(1H)-one

In the same manner as in Example 10, step B to C and using the compound obtained in step A, the title compound was obtained.
MS (ESI+) : [M+H]⁺505.0.

### Example 161

### 5-chloro-8-ethoxy-3-(2-(3-fluoropyridin-2-yl)-6-methyl-3,4-dihydro-2H-chromen-7-yl)-3,4-dihydropyrido[3,4-d]pyrimidin-2(1H)-one

### A) 5-chloro-4-(diethoxymethyl)-2-ethoxypyridin-3-amine

2-Bromo-5-chloro-4-(dimethoxymethyl)pyridin-3-amine (250 mg) was added to 20% sodium ethylate ethanol solution (3 g), and the mixture was stirred in a sealed tube at 80°C for 5 hr. Water was added to the mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (200 mg).
¹H NMR (300 MHz, CDCl₃) δ 1.20-1.32 (6H, m), 1.39 (3H, t, J = 7.0 Hz), 3.58 (2H, dq, J = 9.4, 7.1 Hz), 3.76 (2H, dq, J = 9.4, 7.1 Hz), 4.35 (2H, q, J = 7.1 Hz),4.91 (2H, brs), 5.83(1H, s), 7.42 (1H, s).

### B) 5-chloro-8-ethoxy-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 10, step B to step C and using the compound obtained in step A, the title compound was obtained.
MS (ESI+): [M+H]⁺469.0.

### Example 162

### 5-cyclopropyl-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

### A) 3-azido-5-bromo-4-(dimethoxymethyl)pyridine

To a solution of 3,5-dibromoisonicotinaldehyde (5.3 g) in DMF (26 mL) was added sodium azide (1.4 g), and the mixture was stirred at room temperature for 5 hr. The reaction mixture was cooled to room temperature, water was added, and the mixture was extracted with ethyl acetate. The extract was washed with water and dried over sodium sulfate. The solvent was evaporated under reduced pressure. The residue was dissolved in methanol (40 mL), and p-toluenesulfonic acid monohydrate (0.38 g) was added. The reaction mixture was stirred at 40°C for 18 hr, and the solvent was evaporated under reduced pressure. The residue was purified by silica gel column chromatography (ethyl acetate/hexane) to give the title compound (3.6 g).
¹H NMR (300 MHz, CDCl₃) δ 3.50 (6H, s), 5.66 (1H, s), 8.40 (1H, s), 8.50 (1H, s).

### B) 5-bromo-4-(dimethoxymethyl)pyridin-3-amine

Under ice-cooling, to a solution of cobalt bromide (0.3 g) in ethanol (35 mL) was added 2,2'-bipyridyl (0.6 g), and to the suspension was added sodium borohydride (1.5 g) while maintaining at 5-10°C. To the solution was added dropwise a solution of 3-azido-5-bromo-4-(dimethoxymethyl)pyridine (3.36 g) in ethanol(5 mL), and the mixture was stirred under ice-cooling for 15 min and acetic acid (3 mL) was added. The reaction mixture was diluted with ethyl acetate, and poured into water. The solution was neutralized with 2N aqueous sodium hydroxide solution, and the organic layer was partitioned and dried over sodium sulfate. The solvent was evaporated under reduced pressure and the residue was purified by silica gel column chromatography (ethyl acetate/hexane) to give the title compound (2.1 g).
¹H NMR (300 MHz, CDCl₃) δ 3.48 (6H, s), 4.71 (2H, brs), 5.69 (1H, s), 7.94 (1H, s), 8.03 (1H, s).

### C) 5-cyclopropyl-4-(dimethoxymethyl)pyridin-3-amine

To a solution of 5-bromo-4-(dimethoxymethyl)pyridin-3-amine (2.44 g), cyclopropylboronic acid (1.02 g), tricyclohexylphosphine (0.28 g), tripotassium phosphate (6.92 g), water (2.6 mL) in toluene (52 mL) was added palladium(II) acetate (110 mg), and the mixture was stirred under an argon atmosphere at 90°C for 2 hr. Water was added to the mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.27 g).
¹H NMR (300 MHz, CDCl₃) δ 0.65-0.81 (2H, m), 0.88-1.02 (2H, m), 1.79-1.97 (1H, m), 3.46 (6H, s), 4.56 (2H, brs), 5.90 (1H, s),7.76 (1H, s), 7.92 (1H, s).

### D) 2-bromo-5-cyclopropyl-4-(dimethoxymethyl)pyridin-3-amine

Under ice-cooling, to a mixture of 5-cyclopropyl-4-(dimethoxymethyl)pyridin-3-amine (1.23 g), sodium acetate (1.45 g), acetic acid (8 mL), and acetonitrile (8 mL) was added dropwise bromine (0.27 mL), and the mixture was stirred at 5°C for 2 hr. The reaction mixture was poured into a saturated aqueous sodium hydrogen carbonate solution, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (0.98 g).
¹H NMR (300 MHz, CDCl₃) δ 0.65-0.79 (2H, m), 0.91-1.03 (2H, m), 1.76-1.93 (1H, m), 3.47 (6H, s), 5.07 (2H, brs), 5.87 (1H, s),7.53 (1H, s).

### E) 5-cyclopropyl-4-(dimethoxymethyl)-2-(1-methyl-1H-pyrazol-4-yl)pyridin-3-amine

In the same manner as in Example 10, step A and using the compound obtained in step D, the title compound was obtained.
¹H NMR (300 MHz, CDCl₃) δ 0.67-0.81 (2H, m), 0.91-1.03 (2H, m), 1.82-1.97 (1H, m), 3.51 (6H, s), 3.95 (3H, s), 4.77 (2H, s), 5.95 (1H, s), 7.80 (1H, s),7.83 (1H, s), 7.94 (1H, s).

### F) 5-cyclopropyl-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

In the same manner as in Example 10, step B to step C and using the compound obtained in step E, the title compound was obtained.
MS (ESI+): [M+H]⁺511.1.

### Example 163

### 3-(6-chloro-2-(3-fluoropyridin-2-yl)-3,4-dihydro-2H-chromen-7-yl)-5-cyclopropyl-8-(1-methyl-1H-pyrazol-4-yl)-3,4-dihydropyrido[3,4-d]pyrimidin-2(1H)-one

By a method similar to that in Example 10, step B to step C, the title compound was obtained.
MS (ESI+): [M+H]⁺531.0.

### Example 164

### 5-chloro-3-(2-(3-fluoropyridin-2-yl)-6-methyl-3,4-dihydro-2H-chromen-7-yl)-8-(3-furyl)-3,4-dihydropyrido[3,4-d]pyrimidin-2(1H)-one

By a method similar to that in Example 10, step B to C, the title compound was obtained.
MS (ESI+) : [M+H]⁺491.0.

### Example 165

### 5-chloro-8-(dimethylamino)-3-(2-(3-fluoropyridin-2-yl)-6-methyl-3,4-dihydro-2H-chromen-7-yl)-3,4-dihydropyrido[3,4-d]pyrimidin-2(1H)-one

### A) 5-chloro-4-(dimethoxymethyl)-N,N-dimethylpyridine-2,3-diamine

A solution of 2-bromo-5-chloro-4-(dimethoxymethyl)pyridin-3-amine (0.20 g) and dimethylamine (1.51 g) in N-methylpyrrolidone (1.05 mL)solution was stirred at 110°C for 16 hr. Water was added to the mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.15 g).
MS (ESI+) : [M+H]⁺246.0.

### B) 5-chloro-8-(dimethylamino)-3-(2-(3-fluoropyridin-2-yl)-6-methyl-3,4-dihydro-2H-chromen-7-yl)-3,4-dihydropyrido[3,4-d]pyrimidin-2(1H)-one

In the same manner as in Example 10, step B to C and using the compound obtained in step A, the title compound was obtained.
MS (ESI+): [M+H]⁺468.2.

### Example 166

### 3-(6-chloro-2-(3-fluoropyridin-2-yl)-3,4-dihydro-2H-chromen-7-yl)-8-cyclopropyl-5-fluoro-3,4-dihydropyrido[3,4-d]pyrimidin-2(1H)-one

### A) 3,5-difluoroisonicotinaldehyde

Under a nitrogen atmosphere, to a solution of diisopropylamine (9.67 g) in THF (100 mL) was added dropwise n-butyllithium hexane solution (1.6 M, 59.7 mL) at -78°C. After stirring for 20 min, a solution of 3,5-difluoropyridine (10.00 g) in THF (50 mL) was added dropwise while maintaining at -78°C. After stirring for 1 hr, ethyl formate (17.5 mL) was added dropwise, and the mixture was warmed to 0°C over 2 hr with stirring. Water was added, and the reaction mixture was extracted with ethyl acetate. The organic layer was washed with saturated brine and dried over sodium sulfate. The solvent was evaporated under reduced pressure and the residue was purified by silica gel column chromatography (ethyl acetate/hexane) to give the title compound (5.50 g).
¹H NMR (300 MHz, CDCl₃) δ 8.56 (2H, s), 10.42 (1H, s).

### B) 3-azido-4-(dimethoxymethyl)-5-fluoropyridine

To a solution of 3,5-difluoroisonicotinaldehyde (5.28 g) in DMF (36 mL) was added sodium azide (2.64 g), and the mixture was heated at room temperature for 3 hr. Water was added, and the mixture was extracted with ethyl acetate. The extract was washed with water and dried over sodium sulfate. The solvent was evaporated under reduced pressure. The residue was dissolved in methanol (40 mL), and p-toluenesulfonic acid monohydrate (0.70 g) was added to the solution. The reaction mixture was stirred at 40°C for 18 hr and the solvent was evaporated under reduced pressure. To the residue was added aqueous sodium hydrogen carbonate solution, and the mixture was extracted with ethyl acetate. The extract was washed with water and dried over sodium sulfate. The solvent was evaporated under reduced pressure and the residue was purified by silica gel column chromatography (ethyl acetate/hexane) to give the title compound (6.10 g).
¹H NMR (300 MHz, CDCl₃) δ 3.47 (6H, s), 5.57 (1H, s), 8.24-8.31 (1H, m), 8.35 (1H, s).

### C) 4-(dimethoxymethyl)-5-fluoropyridin-3-amine

Under ice-cooling, to a solution of cobalt bromide (0.63 g) in ethanol (70 mL) was added 2,2'-bipyridyl (1.35 g), and to the suspension was added sodium borohydride (3.59 g) while maintaining at 5-10°C. To the solution was added dropwise a solution of 3-azido-4-(dimethoxymethyl)-5-fluoropyridine (6.10 g) in ethanol (5 mL), and the mixture was stirred under ice-cooling for 1 hr and acetic acid (3 mL) was added. The reaction mixture was diluted with ethyl acetate, and poured into water. The solution was neutralized with a saturated aqueous sodium hydrogen carbonate solution, and the organic layer was partitioned and dried over sodium sulfate. The solvent was evaporated under reduced pressure and the residue was purified by silica gel column chromatography (ethyl acetate/hexane) to give the title compound (3.55 g).
¹H NMR (300 MHz, CDCl₃) δ 3.46 (6H, s), 4.70 (2H, brs), 5.59 (1H, s), 7.80 (1H, d, J = 1.1 Hz), 7.86 (1H, s).

### D) 2-bromo-4-(dimethoxymethyl)-5-fluoropyridin-3-amine

Under ice-cooling, to a mixture of 4-(dimethoxymethyl)-5-fluoropyridin-3-amine (3.38 g), sodium acetate (4.47g), acetic acid (25 mL), and acetonitrile (25 mL) was added dropwise bromine (0.93 mL), and the mixture was stirred at 5°C for 2 hr. The reaction mixture was poured into a saturated aqueous sodium hydrogen carbonate solution, and the mixture was extracted with ethyl acetate. The extract was washed with saturated brine and dried over sodium sulfate. The solvent was evaporated under reduced pressure and the residue was purified by silica gel column chromatography (ethyl acetate/hexane) to give the title compound (0.79 g).
¹H NMR (300 MHz, CDCl₃) δ 3.46 (6H, s), 5.18 (2H, brs), 5.55 (1H, s),7.62 (1H, s).

### E) 2-cyclopropyl-4-(dimethoxymethyl)-5-fluoropyridin-3-amine

To a solution of 2-bromo-4-(dimethoxymethyl)-5-fluoropyridin-3-amine (200 mg), cyclopropylboronic acid (84 mg), tricyclohexylphosphine (21 mg), tripotassium phosphate (561 mg), water (0.2 mL) in toluene (4.2 mL) was added palladium(II) acetate (8.5 mg), and the mixture was stirred under an argon atmosphere at 90°C for 2 hr. Water was added to the mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (168 mg). ¹H NMR (300 MHz, CDCl₃) δ 0.85-0.95 (4H, m), 1.72-1.80 (1H, m), 3.47 (6H, s), 4.91 (2H, brs), 5.58 (1H, s),7.70 (1H, d, J = 0.8 Hz).

### F) 3-(6-chloro-2-(3-fluoropyridin-2-yl)-3,4-dihydro-2H-chromen-7-yl)-8-cyclopropyl-5-fluoro-3,4-dihydropyrido[3,4-d]pyrimidin-2(1H)-one

In the same manner as in Example 10, step B to step C and using compound obtained in step E, the title compound was obtained.
MS (ESI+): [M+H]⁺468.9.

### Example 167

### 5-fluoro-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

### A) 4-(dimethoxymethyl)-5-fluoro-2-(1-methyl-1H-pyrazol-4-yl)pyridin-3-amine

By a method similar to that in Example 10, step A, the title compound was obtained.
¹H NMR (300 MHz, CDCl₃) δ 3.49 (6H, s), 3.96 (3H, s), 4.88 (2H, brs), 5.61 (1H, s), 7.76 (1H, s),7.86 (1H, s), 7.89 (1H, s).

### B) 5-fluoro-3-(2-(3-fluoropyridin-2-yl)-6-methyl-3,4-dihydro-2H-chromen-7-yl)-8-(1-methyl-1H-pyrazol-4-yl)-3,4-dihydropyrido[3,4-d]pyrimidin-2(1H)-one

By a method similar to that in Example 10, step B to step C, the title compound was obtained.
MS (ESI+): [M+H]⁺489.0.

### Example 168

### 8-cyclopropyl-5-fluoro-3-(2-(3-fluoropyridin-2-yl)-6-methyl-3,4-dihydro-2H-chromen-7-yl)-3,4-dihydropyrido[3,4-d]pyrimidin-2(1H)-one

By a method similar to that in Example 10, step B to step C, the title compound was obtained.
MS (ESI+): [M+H]⁺449.0.

### Example 169

### 3-(6-chloro-2-(3-fluoropyridin-2-yl)-3,4-dihydro-2H-chromen-7-yl)-5-fluoro-8-(1-methyl-1H-pyrazol-4-yl)-3,4-dihydropyrido[3,4-d]pyrimidin-2(1H)-one

By a method similar to that in Example 10, step B to step C, the title compound was obtained.
MS (ESI+) : [M+H]⁺509.0.

### Example 170

### 3-(5-((2,6-difluorobenzyl)oxy)-2-methylphenyl)-3,4-dihydroquinazolin-2(1H)-one

### A) 5-((2,6-difluorobenzyl)oxy)-2-methylaniline

A mixture of 4-methyl-3-nitrophenol (1.00 g), 2-(bromomethyl)-1,3-difluorobenzene (1.35 g), potassium carbonate (0.90 g), and DMF (10 mL) was stirred at 70°C for 2 hr. Water was added to the reaction mixture, and the mixture was extracted with ethyl acetate. The obtained organic layer was washed with saturated brine, dried over anhydrous magnesium sulfate, and the solvent was evaporated under reduced pressure. The obtained residue was dissolved in ethanol (10 mL), water (10 mL), reduced iron (1.82 g) and ammonium chloride (0.35 g) were added, and the obtained mixture was heated under reflux for 30 min. The precipitate was removed by filtration, and the filtrate was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (1.44 g).
¹H NMR (300 MHz, DMSO-d₆) δ 1.97 (3H, s), 4.82 (2H, s), 4.96 (2H, s), 6.15 (1H, dd, J = 8.1, 2.5 Hz), 6.26 (1H, d, J = 2.7 Hz), 6.80 (1H, d, J = 8.3 Hz), 7.06-7.22 (2H, m), 7.38-7.63 (1H, m).

### B) 3-(5-((2,6-difluorobenzyl)oxy)-2-methylphenyl)-3,4-dihydroquinazolin-2(1H)-one

A mixture of 2-nitrobenzaldehyde (303 mg), 5-((2,6-difluorobenzyl)oxy)-2-methylaniline (500 mg), sodium triacetoxyborohydride (426 mg), and acetic acid (5 mL) was stirred at room temperature for 12 hr. To the reaction mixture were added ethyl acetate and water, the obtained mixture was added to a saturated aqueous sodium hydrogen carbonate solution, and the mixture was extracted with ethyl acetate. The obtained organic layer was washed with saturated brine, dried over anhydrous magnesium sulfate, and the solvent was evaporated under reduced pressure. The obtained residue was dissolved in ethanol (5 mL), water (5 mL), reduced iron (558 mg) and ammonium chloride (107 mg) were added, and the obtained mixture was heated under reflux for 6 hr. The precipitate was removed by filtration, and the filtrate was extracted with ethyl acetate. The obtained organic layer was washed with saturated brine, dried over anhydrous magnesium sulfate, and the solvent was evaporated under reduced pressure. The obtained residue was dissolved in THF (5 mL), CDI (391 mg) was added, and the obtained mixture was stirred at room temperature for 3 days. The reaction mixture was added to 1N hydrochloric acid, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) and recrystallized from THF/diisopropyl ether to give the title compound (209 mg).
¹H NMR (300 MHz, DMSO-d₆) δ 2.07 (3H, s), 4.51 (1H, d, J = 14.3 Hz), 4.87 (1H, d, J = 14.3 Hz), 5.09 (2H, s), 6.80-6.97 (3H, m), 7.03-7.26 (6H, m), 7.46-7.61 (1H, m), 9.47 (1H, s).

### Example 171

### methyl 3-(5-((2,6-difluorobenzyl)oxy)-2-methylphenyl)-2-oxo-1,2,3,4-tetrahydroquinazoline-7-carboxylate

By a method similar to that in Example 170, step B, the title compound was obtained.
¹H NMR (300 MHz, DMSO-d₆) δ2.07 (3H, s), 3.85 (3H, s), 4.62 (1H, d, J = 15.3 Hz), 4.95 (1H, d, J = 15.3 Hz), 5.09 (2H, s), 6.93 (1H, dd, J = 8.3, 2.7 Hz), 7.10 (1H, d, J = 2.7 Hz), 7.13-7.35 (4H, m), 7.39-7.66 (3H, m), 9.70 (1H, s).

### Example 172

### 3-(5-((2-fluorobenzyl)oxy)-2-methylphenyl)-3,4-dihydroquinazolin-2(1H)-one

By a method similar to that in Example 170, step A to B, the title compound was obtained.
MS (ESI+): [M+H]⁺363.1.

### Example 173

### 3-(5-((2-chlorobenzyl)oxy)-2-methylphenyl)-3,4-dihydroquinazolin-2(1H)-one

By a method similar to that in Example 170, step A to B, the title compound was obtained.
MS (ESI+): [M+H]⁺378.9.

### Example 174

### 3-(5-((4-chloro-2,6-difluorobenzyl)oxy)-2-methylphenyl)-3,4-dihydroquinazolin-2(1H)-one

By a method similar to that in Example 170, step A to B, the title compound was obtained.
MS (ESI+): [M+H]⁺415.0.

### Example 175

### 3-(2-methyl-5-(2-thienylmethoxy)phenyl)-3,4-dihydroquinazolin-2(1H)-one

By a method similar to that in Example 170, step A to B, the title compound was obtained.
MS (ESI+): [M+H]⁺350.9.

### Example 176

### methyl 3-(5-((2,6-difluorobenzyl)oxy)-2-(methoxycarbonyl)phenyl)-2-oxo-1,2,3,4-tetrahydroquinazoline-7-carboxylate

By a method similar to that in Example 170, step A to B, the title compound was obtained.
¹H NMR (300 MHz, DMSO-d₆) δ 3.66 (3H, s), 3.85 (3H, s), 4.86 (2H, brs), 5.22 (2H, s), 7.02-7.33 (5H, m), 7.41-7.66 (3H, m), 7.84 (1H, d, J = 8.7 Hz), 9.74 (1H, s).

### Example 177

### methyl 3-(5-((2,6-difluorobenzyl)oxy)-2-ethylphenyl)-2-oxo-1,2,3,4-tetrahydroquinazoline-7-carboxylate

By a method similar to that in Example 170, step A to B, the title compound was obtained.
¹H NMR (300 MHz, DMSO-d₆) δ 1.06 (3H, t, J = 7.6 Hz), 2.33-2.48 (2H, m), 3.85 (3H, s), 4.56 (1H, d, J = 15.3 Hz), 4.97 (1H, d, J = 15.3 Hz), 5.09 (2H, s), 6.90-7.40 (6H, m), 7.41-7.67 (3H, m), 9.71 (1H, s).

### Example 178

### 3-(5-((2,6-difluorobenzyl)oxy)-2-ethylphenyl)-7-(2-hydroxypropan-2-yl)-3,4-dihydroquinazolin-2(1H)-one

To a solution of methyl 3-(5-((2,6-difluorobenzyl)oxy)-2-ethylphenyl)-2-oxo-1,2,3,4-tetrahydroquinazoline-7-carboxylate (250 mg) in THF (5 mL) was added dropwise methylmagnesium bromide (1.0M THF solution, 5.53 mL) at 0°C, and the obtained mixture was stirred at room temperature for 10 min. Methylmagnesium bromide (1.0 M THF solution, 5.53 mL) was further added, and the obtained mixture was stirred at room temperature for 10 min. Methylmagnesium bromide (1.0M THF solution, 2.76 mL) was further added, and the obtained mixture was stirred at room temperature for 10 min. Methylmagnesium bromide (1.0M THF solution, 2.76 mL) was further added, and the obtained mixture was stirred at room temperature for 10 min. To the reaction mixture was added 1N hydrochloric acid, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (192 mg).
¹H NMR (300 MHz, DMSO-d₆) δ 1.07 (3H, t, J = 7.6 Hz), 1.40 (6H, s), 2.34-2.48 (2H, m), 4.41 (1H, d, J = 14.2 Hz), 4.86 (1H, d, J = 14.2 Hz), 4.98 (1H, s), 5.09 (2H, s), 6.90-7.11 (5H, m), 7.12-7.29 (3H, m), 7.42-7.64 (1H, m), 9.40 (1H, s).

### Example 179

### 3-(5-((2,6-difluorobenzyl)oxy)-2-ethylphenyl)-7-(hydroxymethyl)-3,4-dihydroquinazolin-2(1H)-one

To a suspension of lithium aluminum hydride (40 mg) in THF (5 mL) was added methyl 3-(5-((2,6-difluorobenzyl)oxy)-2-ethylphenyl)-2-oxo-1,2,3,4-tetrahydroquinazoline-7-carboxylate (190 mg) at 0°C by small portions, and the obtained mixture was stirred at 0°C for 2 hr. To the reaction mixture was added sodium sulfate decahydrate, and the precipitate was removed by filtration, and the obtained filtrate was concentrated under reduced pressure. The residue was purified by silica gel column chromatography (hexane/ethyl acetate) to give the title compound (152 mg).
¹H NMR (300 MHz, DMSO-d₆) δ 1.06 (3H, t, J = 7.6 Hz), 2.34-2.47 (2H, m), 4.33-4.50 (3H, m), 4.88 (1H, d, J = 14.4 Hz), 5.10 (2H, s), 5.17 (1H, t, J = 5.7 Hz), 6.76-6.89 (2H, m), 6.97 (1H, dd, J = 8.3, 2.7 Hz), 7.03-7.12 (2H, m), 7.12-7.29 (3H, m), 7.44-7.65 (1H, m), 9.48 (1H, s).

### Example 180

### 3-(5-((2,6-difluorobenzyl)oxy)-2-methylphenyl)-7-(2-hydroxypropan-2-yl)-3,4-dihydroquinazolin-2(1H)-one

By a method similar to that in Example 178, the title compound was obtained.
¹H NMR (300 MHz, DMSO-d₆) δ 1.39 (6H, s), 2.07 (3H, s), 4.47 (1H, d, J = 14.4 Hz), 4.84 (1H, d, J = 14.4 Hz), 4.97 (1H, brs), 5.09 (2H, s), 6.84-7.09 (5H, m), 7.10-7.26 (3H, m), 7.45-7.62 (1H, m), 9.40 (1H, s).

### Example 181

### 3-(5-((2,6-difluorobenzyl)oxy)-2-methylphenyl)-7-(hydroxymethyl)-3,4-dihydroquinazolin-2(1H)-one

By a method similar to that in Example 179, the title compound was obtained.
¹H NMR (300 MHz, DMSO-d₆) δ 2.06 (3H, s), 4.36-4.57 (3H, m), 4.86 (1H, d, J = 14.4 Hz), 5.01-5.31 (3H, m), 6.77-6.97 (3H, m), 6.99-7.12 (2H, m), 7.12-7.28 (3H, m), 7.42-7.65 (1H, m), 9.48 (1H, s).

### Example 182

### methyl 3-(2-(3-fluoropyridin-2-yl)-6-methyl-3,4-dihydro-2H-chromen-7-yl)-2-oxo-1,2,3,4-tetrahydroquinazoline-7-carboxylate

By a method similar to that in Example 170, the title compound was obtained.
MS (ESI+): [M+H]⁺447.2.

### Example 183

### 3-(2-(3-fluoropyridin-2-yl)-6-methyl-3,4-dihydro-2H-chromen-7-yl)-7-(morpholin-4-ylcarbonyl)-3,4-dihydroquinazolin-2(1H)-one

By a method similar to those in Example 71 to Example 74, the title compound was obtained.
MS (ESI+): [M+H]⁺503.4.

### Example 184

### N-cyclopropyl-3-(2-(3-fluoropyridin-2-yl)-6-methyl-3,4-dihydro-2H-chromen-7-yl)-2-oxo-1,2,3,4-tetrahydroquinazoline-7-carboxamide

By a method similar to those in Example 71 to Example 74, the title compound was obtained.
MS (ESI+): [M+H]⁺473.4.

### Example 185

### 3-(2-(3-fluoropyridin-2-yl)-6-methyl-3,4-dihydro-2H-chromen-7-yl)-7-(hydroxymethyl)-3,4-dihydroquinazolin-2(1H)-one

By a method similar to that in Example 179, the title compound was obtained.
MS (ESI+) : [M+H]⁺420.4.

### Example 186

### 3-(5-((2,6-difluorobenzyl)oxy)-2-methylphenyl)-6-(hydroxymethyl)-3,4-dihydroquinazolin-2(1H)-one

By a method similar to that in Example 179, the title compound was obtained.
MS (ESI+): [M+H]⁺411.0.

### Example 187

### 3-(5-((3-chloropyridin-2-yl)methoxy)-2-methylphenyl)-3,4-dihydroquinazolin-2(1H)-one

By a method similar to that in Example 170, step A to B, the title compound was obtained.
MS (ESI+): [M+H]⁺379.9.

### Example 188

### methyl 4-((2,6-difluorobenzyl)oxy)-2-(2-oxo-1,4-dihydroquinazolin-3(2H)-yl)benzoate

By a method similar to that in Example 170, step A to B, the title compound was obtained.
¹H NMR (300 MHz, DMSO-d₆) δ 3.66 (3H, s), 4.81 (2H, brs), 5.22 (2H, s), 6.74-6.97 (2H, m), 6.99-7.31 (6H, m), 7.46-7.66 (1H, m), 7.82 (1H, d, J = 8.7 Hz), 9.50 (1H, s).

### Example 189

### 3-(5-((3-fluoropyridin-2-yl)methoxy)-2-methylphenyl)-3,4-dihydroquinazolin-2(1H)-one

By a method similar to that in Example 170, step A to B, the title compound was obtained.
MS (ESI+): [M+H]⁺362.8.

### Example 190

### 4-((2,6-difluorobenzyl)oxy)-N-(2-hydroxy-2-methylpropyl)-2-(2-oxo-1,4-dihydroquinazolin-3(2H)-yl)benzamide

### A) 4-((2,6-difluorobenzyl)oxy)-2-(2-oxo-1,4-dihydroquinazolin-3(2H)-yl)benzoic acid

A mixture of methyl 4-((2,6-difluorobenzyl)oxy)-2-(2-oxo-1,4-dihydroquinazolin-3(2H)-yl)benzoate (1.00 g), THF (20 mL), methanol (10 mL), and 1N aqueous sodium hydroxide solution (10 mL) was stirred at room temperature for 2 hr, subsequently stirred at 50°C for 1 hr, and further at 70°C for 1 hr. The reaction mixture was neutralized with 1N hydrochloric acid at 0°C, and the mixture was extracted with ethyl acetate. The obtained organic layer was washed with saturated brine, dried over anhydrous magnesium sulfate, and the solvent was evaporated under reduced pressure. The residue was washed with diethyl ether/ethyl acetate to give the title compound (0.90 g).
¹H NMR (300 MHz, DMSO-d₆) δ 4.79 (2H, brs), 5.21 (2H, s), 6.77-6.96 (2H, m), 7.00-7.31 (6H, m), 7.46-7.67 (1H, m), 7.84 (1H, d, J = 8.7 Hz), 9.41 (1H, s), 12.49 (1H, brs).

### B) ethyl N-(4-((2,6-difluorobenzyl)oxy)-2-(2-oxo-1,4-dihydroquinazolin-3(2H)-yl)benzoyl)glycinate

A mixture of 4-((2,6-difluorobenzyl)oxy)-2-(2-oxo-1,4-dihydroquinazolin-3(2H)-yl)benzoic acid (800 mg), ethyl glycinate hydrochloride (408 mg), WSC (560 mg), HOBt (395 mg), triethylamine (815 µL), and DMF (10 mL) was stirred at room temperature for 3 hr. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (785 mg).
¹H NMR (300 MHz, DMSO-d₆) δ 1.15 (3H, t, J = 7.1 Hz), 3.86 (2H, d, J = 5.8 Hz), 4.05 (2H, q, J = 7.1 Hz), 4.78 (2H, s), 5.18 (2H, s), 6.75-6.94 (2H, m), 7.02-7.27 (6H, m), 7.46-7.62 (2H, m), 8.50 (1H, t, J = 5.8 Hz), 9.40 (1H, s).

### C) 4-((2,6-difluorobenzyl)oxy)-N-(2-hydroxy-2-methylpropyl)-2-(2-oxo-1,4-dihydroquinazolin-3(2H)-yl)benzamide

By a method similar to that in Example 178, the title compound was obtained.
¹H NMR (300 MHz, DMSO-d₆) δ 1.01 (6H, s), 3.09 (2H, d, J = 6.1 Hz), 4.36 (1H, s), 4.80 (2H, s), 5.18 (2H, s), 6.76-6.96 (2H, m), 7.00-7.28 (6H, m), 7.45-7.62 (2H, m), 7.80 (1H, t, J = 6.1 Hz), 9.46 (1H, s).

### Example 192

### 4-((2,6-difluorobenzyl)oxy)-2-(2-oxo-1,4-dihydroquinazolin-3(2H)-yl)-N-(pyridin-2-ylmethyl)benzamide

By a method similar to that in Example 190, step B, the title compound was obtained.
¹H NMR (300 MHz, DMSO-d₆) δ 4.43 (2H, d, J = 5.9 Hz), 4.83 (2H, s), 5.20 (2H, s), 6.67-6.99 (2H, m), 7.01-7.30 (7H, m), 7.36 (1H, d, J = 8.0 Hz), 7.45-7.74 (3H, m), 8.26-8.46 (1H, m), 8.68 (1H, t, J = 5.9 Hz), 9.46 (1H, s).

### Example 193

### 3-(5-((2,6-difluorobenzyl)oxy)-2-methylphenyl)-7-(methoxymethyl)-3,4-dihydroquinazolin-2(1H)-one

To a solution of 3-(5-((2,6-difluorobenzyl)oxy)-2-methylphenyl)-7-(hydroxymethyl)-3,4-dihydroquinazolin-2(1H)-one (250 mg) in methanol (5 mL) was added concentrated sulfuric acid (597 mg) at room temperature. The reaction mixture was stirred under microwave irradiation at 150°C for 20 min, and concentrated under reduced pressure. Water was added to the residue, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (44 mg).
MS (ESI+) : [M+H]⁺424.9.

### Example 194

### 3-(5-((2,6-difluorobenzyl)oxy)-2-methylphenyl)-8-(hydroxymethyl)-3,4-dihydroquinazolin-2(1H)-one

By a method similar to that in Example 179, the title compound was obtained.
MS (ESI+) : [M+H]⁺410.9.

### Example 195

### 3-(5-((2,6-difluorobenzyl)oxy)-2-methylphenyl)-5-(hydroxymethyl)-3,4-dihydroquinazolin-2(1H)-one

By a method similar to that in Example 179, the title compound was obtained.
MS (ESI+): [M+H]⁺411.0.

### Example 196

### 3-(5-((2,6-difluorobenzyl)oxy)-2-methylphenyl)-2-oxo-1,2,3,4-tetrahydroquinazoline-7-carboxamide

### A) 3-(5-((2,6-difluorobenzyl)oxy)-2-methylphenyl)-2-oxo-1,2,3,4-tetrahydroquinazoline-7-carboxylic acid

By a method similar to that in Example 71, the title compound was obtained.
MS (ESI+): [M+H]⁺425.3.

### B) 3-(5-((2,6-difluorobenzyl)oxy)-2-methylphenyl)-2-oxo-1,2,3,4-tetrahydroquinazoline-7-carboxamide

By a method similar to that in Example 74, the title compound was obtained.
MS (ESI+): [M+H]⁺424.4.

### Example 197

### 3-(5-((2,6-difluorobenzyl)oxy)-2-methylphenyl)-N-(2-hydroxyethyl)-2-oxo-1,2,3,4-tetrahydroquinazoline-7-carboxamide

To a solution of 3-(5-((2,6-difluorobenzyl)oxy)-2-methylphenyl)-2-oxo-1,2,3,4-tetrahydroquinazoline-7-carboxylic acid (250 mg) in DMF (5 mL) was added oxalyl chloride (111 µL) at 0°C, and the reaction mixture was stirred at room temperature for 3 hr. A solution of 2-aminoethanol (72 mg) and triethylamine (119 mg) in DMF (5 mL) was added to the reaction mixture, and the mixture was stirred at room temperature for 2 days. The reaction mixture was concentrated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (77 mg).
MS (ESI+): [M+H]⁺468.0.

### Example 198

### methyl 3-(5-((2,3-difluorobenzyl)oxy)-2-methylphenyl)-2-oxo-1,2,3,4-tetrahydroquinazoline-7-carboxylate

By a method similar to that in Example 170, step A to B, the title compound was obtained.
MS (ESI+): [M+H]⁺439.0.

### Example 199

### methyl 3-(5-((2,5-difluorobenzyl)oxy)-2-methylphenyl)-2-oxo-1,2,3,4-tetrahydroquinazoline-7-carboxylate

By a method similar to that in Example 170, step A to B, the title compound was obtained.
MS (ESI+): [M+H]⁺439.0.

### Example 200

### methyl 3-(5-((2,4-difluorobenzyl)oxy)-2-methylphenyl)-2-oxo-1,2,3,4-tetrahydroquinazoline-7-carboxylate

By a method similar to that in Example 170, step A to B, the title compound was obtained.
¹H NMR (300 MHz, DMSO-d₆) δ 2.07 (3H, s), 3.85 (3H, s), 4.60 (1H, d, J = 15.3 Hz), 4.95 (1H, d, J = 15.3 Hz), 5.08 (2H, s), 6.92 (1H, dd, J = 8.3, 2.6 Hz), 7.04-7.39 (5H, m), 7.44-7.57 (2H, m), 7.58-7.71 (1H, m), 9.69 (1H, s).

### Example 201

### 3-(5-((2,4-difluorobenzyl)oxy)-2-methylphenyl)-7-(2-hydroxypropan-2-yl)-3,4-dihydroquinazolin-2(1H)-one

By a method similar to that in Example 178, the title compound was obtained.
¹H NMR (300 MHz, DMSO-d₆) δ 1.39 (6H, s), 2.06 (3H, s), 4.46 (1H, d, J = 14.5 Hz), 4.83 (1H, d, J = 14.5 Hz), 4.98 (1H, s), 5.08 (2H, s), 6.81-7.08 (5H, m), 7.08-7.23 (2H, m), 7.25-7.41 (1H, m), 7.53-7.75 (1H, m), 9.39 (1H, s).

### Example 202

### 3-(5-((2,6-difluorobenzyl)oxy)-2-methylphenyl)-5-(2-hydroxypropan-2-yl)-3,4-dihydroquinazolin-2(1H)-one

By a method similar to that in Example 178, the title compound was obtained.
MS (ESI+): [M+H]⁺439.0.

### Example 203

### 3-(5-((2,3-difluorobenzyl)oxy)-2-methylphenyl)-7-(2-hydroxypropan-2-yl)-3,4-dihydroquinazolin-2(1H)-one

By a method similar to that in Example 178, the title compound was obtained.
MS (ESI+): [M+H]⁺439.0.

### Example 204

### 3-(5-((2,6-difluorobenzyl)oxy)-2-methylphenyl)-2-oxo-1,2,3,4-tetrahydroquinazoline-7-carbonitrile

To a solution of 3-(5-((2,6-difluorobenzyl)oxy)-2-methylphenyl)-2-oxo-1,2,3,4-tetrahydroquinazoline-7-carboxamide (400 mg) in DMF (8 mL) was added thionyl chloride (350 mg) at room temperature, and the reaction mixture was stirred at 80°C for 3 days. The reaction mixture was concentrated under reduced pressure, and the residue was dissolved in ethyl acetate, and poured into a saturated aqueous sodium hydrogen carbonate solution. The organic layer was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (80 mg).
MS (ESI+): [M+H]⁺406.4.

### Example 205

### 3-(5-((2,6-difluorobenzyl)oxy)-2-methylphenyl)-7-((3-hydroxypiperidin-1-yl)carbonyl)-3,4-dihydroquinazolin-2(1H)-one

By a method similar to that in Example 74, the title compound was obtained.
MS (ESI+): [M+H]⁺508.4.

### Example 206

### 7-(aminomethyl)-3-(5-((2,6-difluorobenzyl)oxy)-2-methylphenyl)-3,4-dihydroquinazolin-2(1H)-one

To a solution of 3-(5-((2,6-difluorobenzyl)oxy)-2-methylphenyl)-7-(hydroxymethyl)-3,4-dihydroquinazolin-2(1H)-one (230 mg) and triethylamine (74 mg) in THF (5 mL) was added methanesulfonyl chloride (71 mg) at 0°C, and the reaction mixture was stirred at room temperature for 4 hr. Triethylamine (74 mg) and methanesulfonyl chloride (71 mg) were added, and the reaction mixture was stirred at room temperature for 2 days. The reaction mixture was concentrated under reduced pressure, a saturated aqueous sodium hydrogen carbonate solution was added, and the mixture was extracted with ethyl acetate. The extract was washed with saturated brine, and dried over magnesium sulfate. The solvent was evaporated under reduced pressure. The residue was dissolved in 2M ammonia-methanol solution (5 mL), and the reaction mixture was stirred at room temperature for 5.5 hr. The reaction mixture was concentrated under reduced pressure, and water and ethyl acetate were added. The aqueous layer was recovered, and the solvent was evaporated under reduced pressure. To the residue was added methanol, and the insoluble material was filtered off by using celite. The filtrate was concentrated under reduced pressure, water was added to the residue, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and washed with ethyl acetate to give the title compound (5 mg).
MS (ESI+): [M+H]⁺410.5.

### Example 207

### N-((3-(5-((2,6-difluorobenzyl)oxy)-2-methylphenyl)-2-oxo-1,2,3,4-tetrahydroquinazolin-7-yl)carbonyl)-2-methylalanine

By a method similar to that in Example 74, the title compound was obtained.
MS (ESI+): [M+H]⁺510.4.

### Example 208

### 3-(5-((2,6-difluorobenzyl)oxy)-2-methylphenyl)-7-(5-oxo-4,5-dihydro-1,2,4-oxadiazol-3-yl)-3,4-dihydroquinazolin-2(1H)-one

### A) 3-(5-((2,6-difluorobenzyl)oxy)-2-methylphenyl)-N'-hydroxy-2-oxo-1,2,3,4-tetrahydroquinazoline-7-carboximidamide

To a solution of 3-(5-((2,6-difluorobenzyl)oxy)-2-methylphenyl)-2-oxo-1,2,3,4-tetrahydroquinazoline-7-carbonitrile (170 mg) and hydroxylamine hydrochloride (30 mg) in DMSO (4 mL) was added sodium hydrogen carbonate (71 mg), and the reaction mixture was stirred at 50°C overnight. Hydroxylamine hydrochloride (30 mg) and sodium hydrogen carbonate (71 mg) were added, and the reaction mixture was stirred at 50°C for 5 hr. Water was added to the reaction mixture, and the resulting solid was recovered and washed with ethanol/ethyl acetate to give the title compound (44 mg).
MS (ESI+): [M+H]⁺439.3.

### B) 3-(5-((2,6-difluorobenzyl)oxy)-2-methylphenyl)-7-(5-oxo-4,5-dihydro-1,2,4-oxadiazol-3-yl)-3,4-dihydroquinazolin-2(1H)-one

To a solution of 3-(5-((2,6-difluorobenzyl)oxy)-2-methylphenyl)-N'-hydroxy-2-oxo-1,2,3,4-tetrahydroquinazoline-7-carboximidamide (108 mg) and DBU (57 mg) in THF (3 mL) was added CDI (60 mg), and the reaction mixture was stirred at room temperature overnight. To the reaction mixture was acidified with 1N hydrochloric acid, and the resulting solid was recovered and recrystallized from ethyl acetate/hexane to give the title compound (36 mg).
MS (ESI+): [M+H]⁺465.4.

### Example 209

### 3-(5-((2,6-difluorobenzyl)oxy)-2-methylphenyl)-N-(methylsulfonyl)-2-oxo-1,2,3,4-tetrahydroquinazoline-7-carboxamide

To a solution of 3-(5-((2,6-difluorobenzyl)oxy)-2-methylphenyl)-2-oxo-1,2,3,4-tetrahydroquinazoline-7-carboxylic acid (200 mg) in DMF (3 mL) was added CDI (115 mg), and the reaction mixture was stirred at room temperature for 1 hr. A solution of methanesulfonamide (67 mg) and DBU (108 mg) in DMF (3 mL) was added to the reaction mixture, and the reaction mixture was stirred at 80°C for 6 hr. To the reaction mixture was added 1N hydrochloric acid, and the reaction mixture was concentrated under reduced pressure and extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (75 mg).
MS (ESI+): [M+H]⁺502.3.

### Example 210

### 3-(5-((2,6-difluorobenzyl)oxy)-2-methylphenyl)-7-(4,4-dimethyl-5-oxo-4,5-dihydro-1,3-oxazol-2-yl)-3,4-dihydroquinazolin-2(1H)-one

To a solution of N-((3-(5-((2,6-difluorobenzyl)oxy)-2-methylphenyl)-2-oxo-1,2,3,4-tetrahydroquinazolin-7-yl)carbonyl)-2-methylalanine (72 mg) in pyridine(2 mL) was added WSC (54 mg), and the reaction mixture was stirred at room temperature overnight. The reaction mixture was concentrated under reduced pressure, water was added, and the mixture was extracted with ethyl acetate. The extract was washed with saturated brine, and dried over magnesium sulfate. The solvent was evaporated under reduced pressure. The residue was recrystallized from ethyl acetate/hexane to give the title compound (21 mg).
MS (ESI+) : [M+H]⁺492.4.

### Example 211

### methyl 3-(2-methyl-5-((2-methyl-1,3-thiazol-4-yl)methoxy)phenyl)-2-oxo-1,2,3,4-tetrahydroquinazoline-7-carboxylate

To a solution of methyl 3-(5-hydroxy-2-methylphenyl)-2-oxo-1,2,3,4-tetrahydroquinazoline-7-carboxylate (400 mg) and 4-(chloromethyl)-2-methyl-1,3-thiazole hydrochloride in DMF (8 mL) was added potassium carbonate (531 mg), and the reaction mixture was stirred at room temperature for 19 hr, and then at 80°C for 3 hr. The reaction mixture was concentrated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethanol/ethyl acetate/hexane to give the title compound (272 mg).
MS (ESI+): [M+H]⁺424.3.

### Example 212

### 3-(2-bromo-5-((3-fluoropyridin-2-yl)methoxy)phenyl)-3,4-dihydroquinazolin-2(1H)-one

By a method similar to that in Example 170, step A to B, the title compound was obtained.
MS (ESI+): [M+H]⁺427.7.

### Example 213

### 3-(2-cyclopropyl-5-((3-fluoropyridin-2-yl)methoxy)phenyl)-3,4-dihydroquinazolin-2(1H)-one

### A) 2-cyclopropyl-5-(3-fluoropyridin-2-yl)methoxy)aniline

A mixed solution of 2-bromo-5-(3-fluoropyridin-2-yl)methoxy)nitrobenzene (2.0 g), cyclopropylboronic acid (0.7 g), palladium(II) acetate (0.07 g), tricyclohexylphosphine (0.17 g) and tripotassium phosphate (4.7 g) in toluene (28 mL)/water (1.4 mL) was stirred under an argon atmosphere at 100°C overnight. The reaction mixture was cooled to room temperature, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane). The obtained resultant product was dissolved in ethanol (27 mL), water (3 mL), reduced iron (1.7 g) and calcium chloride (0.65 g) were added, and the obtained mixture was heated under reflux overnight. The precipitate was removed by filtration, and the filtrate was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (1.7 g).
MS (ESI+): [M+H]⁺258.8.

### B) 3-(2-cyclopropyl-5-((3-fluoropyridin-2-yl)methoxy)phenyl)-3,4-dihydroquinazolin-2(1H)-one

In the same manner as in Example 170, step B and using the compound obtained in step A, the title compound was obtained.
MS (ESI+): [M+H]⁺389.9.

### Example 214

### 3-(5-((3-fluoropyridin-2-yl)methoxy)-2-(trifluoromethyl)phenyl)-3,4-dihydroquinazolin-2(1H)-one

By a method similar to that in Example 170, step A to B, the title compound was obtained.
MS (ESI+): [M+H]⁺418.0.

### Example 215

### 3-(5-(1-(3-fluoropyridin-2-yl)ethoxy)-2-methylphenyl)-3,4-dihydroquinazolin-2(1H)-one

By a method similar to that in Example 170, step A to B, the title compound was obtained.
¹H NMR (300 MHz, DMSO-d₆) δ 1.65 (3H, d, J = 6.4 Hz), 2.01 (3H, s), 4.32-4.57 (1H, m), 4.60-4.94 (1H, m), 5.73 (1H, q, J = 6.4 Hz), 6.69-6.99 (4H, m), 7.03-7.23 (3H, m), 7.39-7.53 (1H, m), 7.62-7.83 (1H, m), 8.36-8.54 (1H, m), 9.43 (1H, brs).

### Example 216

### methyl 3-(5-((3-fluoropyridin-2-yl)methoxy)-2-methylphenyl)-2-oxo-1,2,3,4-tetrahydroquinazoline-7-carboxylate

To a solution of methyl 3-(5-hydroxy-2-methylphenyl)-2-oxo-1,2,3,4-tetrahydroquinazoline-7-carboxylate (500 mg), (3-fluoropyridin-2-yl)methanol (407 mg) and tri-tert-butylphosphine (809 mg) in THF (10 mL) was added 1,1'-(azodicarbonyl)dipiperidine (1009 mg), and the reaction mixture was stirred under microwave irradiation at 100°C for 1 hr. The reaction mixture was concentrated under reduced pressure, a saturated aqueous sodium hydrogen carbonate solution was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane), and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate, and concentrated under reduced pressure to give the title compound (9 mg).
MS (ESI+): [M+H]⁺422.3.

### Example 217

### 3-(5-((2,6-difluorobenzyl)oxy)-2-methylphenyl)-8-(2-hydroxypropan-2-yl)-3,4-dihydroquinazolin-2(1H)-one

By a method similar to that in Example 178, the title compound was obtained.
MS (ESI+) : [M+H]⁺438.9.

### Example 218

### 3-(2-chloro-5-((2,6-difluorobenzyl)oxy)phenyl)-7-(hydroxymethyl)-3,4-dihydroquinazolin-2(1H)-one

By a method similar to that in Example 179, the title compound was obtained.
MS (ESI+) : [M+H]⁺430.8.

### Example 219

### methyl 4-((3-fluoropyridin-2-yl)methoxy)-2-(2-oxo-1,4-dihydroquinazolin-3(2H)-yl)benzoate

To a solution of (3-fluoropyridin-2-yl)methanol (0.50 g) and methanesulfonyl chloride (0.37 mL) in THF (5 mL) was added dropwise triethylamine (1.1 mL) at 0°C, and the obtained mixture was stirred at 0°C for 1.5 hr. Water was added to the reaction mixture, and the mixture was extracted with ethyl acetate. The obtained organic layer was washed with saturated brine, dried over anhydrous magnesium sulfate, and the solvent was evaporated under reduced pressure. The obtained residue was dissolved in DMF (10 mL), and methyl 4-hydroxy-2-(2-oxo-1,4-dihydroquinazolin-3(2H)-yl)benzoate (1.17 g) and potassium carbonate (1.36 g) were added. The obtained mixture was stirred at 70°C overnight. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (0.96 g).
¹H NMR (300 MHz, DMSO-d₆) δ 3.66 (3H, s), 4.79 (2H, brs), 5.34 (2H, d, J = 1.9 Hz), 6.79-6.96 (2H, m), 7.02-7.25 (4H, m), 7.46-7.63 (1H, m), 7.72-7.90 (2H, m), 8.40-8.54 (1H, m), 9.48 (1H, s).

### Example 220

### 3-(2-ethyl-5-((3-fluoropyridin-2-yl)methoxy)phenyl)-3,4-dihydroquinazolin-2(1H)-one

By a method similar to that in Example 170, step A to B, the title compound was obtained.
MS (ESI+): [M+H]⁺378.2.

### Example 221

### N-cyclopropyl-4-((3-fluoropyridin-2-yl)methoxy)-2-(2-oxo-1,4-dihydroquinazolin-3(2H)-yl)benzamide

### A) (3-fluoropyridin-2-yl)methyl methanesulfonate

To a solution of (3-fluoropyridin-2-yl)methanol (230 mg) and triethylamine (74 mg) in THF (5 mL) was added methanesulfonyl chloride (71 mg) at 0°C, and the reaction mixture was stirred at 0°C for 2 hr. The reaction mixture was concentrated under reduced pressure, a saturated aqueous sodium hydrogen carbonate solution was added, and the mixture was extracted with ethyl acetate. The extract was washed with saturated brine, and dried over magnesium sulfate. The solvent was evaporated under reduced pressure to give the title compound.
MS (ESI+) : [M+H]⁺206.2.

### B) methyl 2-amino-4-hydroxybenzoate

4-Methoxy-2-nitrobenzoic acid (24.0 g) was dissolved in 25% hydrogen bromide acetic acid solution (480 mL), and the reaction mixture was stirred at 150°C for 23 hr. The reaction mixture was concentrated under reduced pressure, and the residue was dissolved in methanol (500 mL). Concentrated sulfuric acid (12.0 g) was added under water-cooling, and the reaction mixture was heated under reflux overnight. The reaction mixture was concentrated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and dissolved in THF (250 mL). Imidazole (4.9 g) and N,N-dimethyl-4-aminopyridine (1 piece) were added, tert-butyl(chloro)dimethylsilane (10.9 g) was added and the mixture was stirred at room temperature overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and dissolved in ethanol (170 mL) and water (170 mL). Ammonium chloride (2.9 g) and reduced iron (15.2 g) were added, and the reaction mixture was stirred at 85°C for 3 days. The insoluble material was filtered off with celite, the filtrate was concentrated under reduced pressure, and the residue was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (10.6 g).
MS (ESI+) : [M+H]⁺168.2.

### C) methyl 2-amino-4-((tert-butyl(dimethyl)silyl)oxy)benzoate

To a solution of methyl 2-amino-4-hydroxybenzoate (10.6 g), imidazole (3.9 g) and N,N-dimethyl-4-aminopyridine (1 piece) in THF (200 mL) was added tert-butyl(chloro)dimethylsilane (8.6 g) at room temperature. The reaction mixture was stirred at room temperature for 6 hr, tert-butyl(chloro)dimethylsilane (2.1 g) and imidazole (1.0 g) were added, and the mixture was stirred at room temperature for 1 hr and 60°C for 1 hr. The reaction mixture was ice-cooled, and the resulting solid was filtered off. The filtrate was concentrated under reduced pressure, water was added to the residue and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (11.4 g).
MS (ESI+) : [M+H]⁺282.4.

### D) methyl 4-((tert-butyl(dimethyl)silyl)oxy)-2-(2-oxo-1,4-dihydroquinazolin-3(2H)-yl)benzoate

To a solution of methyl 2-amino-4-((tert-butyl(dimethyl)silyl)oxy)benzoate (15.4 g) and 2-nitrobenzaldehyde (5.5 g) in acetic acid (300 mL) was added sodium triacetoxyborohydride (11.6 g). The reaction mixture was stirred at 60°C for 12 hr, water was added, and the mixture was extracted with ethyl acetate. The extract was washed with saturated brine, and dried over magnesium sulfate. The solvent was evaporated under reduced pressure. The residue was dissolved in a mixed solution of ethanol(75 mL)/water (75 mL), and ammonium chloride (1.0 g) and reduced iron (5.0 g) were added. The reaction mixture was stirred at 80°C for 3 hr, and the insoluble material was filtered off by using celite. The filtrate was concentrated under reduced pressure, and the residue was extracted with ethyl acetate. The extract was washed with saturated brine, and dried over magnesium sulfate. The solvent was evaporated under reduced pressure. The residue was dissolved in THF (140 mL), and CDI (3.5 g) was added at room temperature. The reaction mixture was stirred at room temperature for 1 hr, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (7.3 g).
MS (ESI+) : [M+H]⁺413.03.

### E) methyl 4-hydroxy-2-(2-oxo-1,4-dihydroquinazolin-3(2H)-yl)benzoate

To a solution of methyl 4-((tert-butyl(dimethyl)silyl)oxy)-2-(2-oxo-1,4-dihydroquinazolin-3(2H)-yl)benzoate (7.3 g) in THF (100 mL) was added 1.0 M tetrabutylammonium fluoride/THF solution (44.4 mL) under ice-cooling. The reaction mixture was stirred at room temperature for 2 hr, concentrated under reduced pressure, and the residue was poured into 1N hydrochloric acid. The resulting solid was recovered by filtration, and washed with ethyl acetate to give the title compound (3.5 g).
MS (ESI+): [M+H]⁺ 299.3.

### F) 4-((3-fluoropyridin-2-yl)methoxy)-2-(2-oxo-1,4-dihydroquinazolin-3(2H)-yl)benzoic acid

To a solution of methyl 4-hydroxy-2-(2-oxo-1,4-dihydroquinazolin-3(2H)-yl)benzoate (500 mg) and (3-fluoropyridin-2-yl)methyl methanesulfonate (413 mg) in DMF (10 mL) was added potassium carbonate (580 mg), and the reaction mixture was stirred at 85°C for 6 hr. To the reaction mixture were added water and ethyl acetate, and the insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) and dissolved in a mixed solution of THF (3 mL)/methanol (3 mL). 1N Aqueous sodium hydroxide solution (0.8 mL) was added, and the reaction mixture was stirred at 45°C for 16 hr, 1N aqueous sodium hydroxide solution (1.6 mL) was added, and the reaction mixture was stirred at 60°C for 2 hr. The reaction mixture was concentrated under reduced pressure, water and ethyl acetate were added, and the mixture was acidified with 1N hydrochloric acid. The resulting solid was recovered by filtration to give the title compound (255 mg).
MS (ESI+): [M+H]⁺394.3.

### G) N-cyclopropyl-4-((3-fluoropyridin-2-yl)methoxy)-2-(2-oxo-1,4-dihydroquinazolin-3(2H)-yl)benzamide

By a method similar to that in Example 74, the title compound was obtained.
MS (ESI+) : [M+H]⁺433.4.

### Example 222

### 3-(2-chloro-5-((2,6-difluorobenzyl)oxy)phenyl)-7-(2-hydroxypropan-2-yl)-3,4-dihydroquinazolin-2(1H)-one

By a method similar to that in Example 178, the title compound was obtained.
MS (ESI+): [M+H]⁺458.8.

### Example 223

### 3-(5-((2,6-difluorobenzyl)oxy)-2-methylphenyl)-3,4-dihydropyrido[3,2-d]pyrimidin-2(1H)-one

By a method similar to that in Example 170, step B, the title compound was obtained.
MS (ESI+): [M+H]⁺381.9.

### Example 224

### 4-((3-fluoropyridin-2-yl)methoxy)-2-(2-oxo-1,4-dihydroquinazolin-3(2H)-yl)benzonitrile

### A) 4-((3-fluoropyridin-2-yl)methoxy)-2-(2-oxo-1,4-dihydroquinazolin-3(2H)-yl)benzoic acid

By a method similar to that in Example 190, step A, the title compound was obtained.
¹H NMR (300 MHz, DMSO-d₆) δ 4.78 (2H, brs), 5.33 (2H, d, J = 1.9 Hz), 6.72-6.98 (2H, m), 7.01-7.24 (4H, m), 7.41-7.63 (1H, m), 7.70-7.95 (2H, m), 8.33-8.57 (1H, m), 9.39 (1H, s), 12.49 (1H, brs).

### B) 4-((3-fluoropyridin-2-yl)methoxy)-2-(2-oxo-1,4-dihydroquinazolin-3(2H)-yl)benzonitrile

A mixture of 4-((3-fluoropyridin-2-yl)methoxy)-2-(2-oxo-1,4-dihydroquinazolin-3(2H)-yl)benzoic acid (725 mg), WSC (497 mg), HOBt/ammonia complex (765 mg), triethylamine (513 µL) and DMF (10 mL) was stirred at room temperature overnight. Water was added to the reaction mixture, and the mixture was extracted with ethyl acetate. The obtained organic layer was washed with saturated brine, dried over anhydrous magnesium sulfate, and the solvent was evaporated under reduced pressure. The obtained residue was mixed with pyridine (296 µL) and THF (5 mL), and a solution of trifluoroacetic anhydride (305 µL) in THF (2 mL) was added to the obtained mixture at 0°C. The obtained mixture was stirred at 0°C for 1 hr, a solution of pyridine (592 µL) and trifluoroacetic anhydride (710 µL) in THF (1 mL) was further added, and the obtained mixture was stirred at room temperature for 1 hr. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (418 mg).
¹H NMR (300 MHz, DMSO-d₆) δ 4.84 (2H, s), 5.38 (2H, d, J = 1.5 Hz), 6.82-7.03 (2H, m), 7.09-7.30 (3H, m), 7.39 (1H, d, J = 2.6 Hz), 7.48-7.67 (1H, m), 7.74-7.90 (2H, m), 8.44-8.54 (1H, m), 9.84 (1H, s).

### Example 225

### butyl (2E)-3-(4-((3-fluoropyridin-2-yl)methoxy)-2-(2-oxo-1,4-dihydroquinazolin-3(2H)-yl)phenyl)acrylate

A solution of 3-(2-bromo-5-((3-fluoropyridin-2-yl)methoxy)phenyl)-3,4-dihydroquinazolin-2(1H)-one (1.0 g), butyl acrylate (1.7 mL), palladium(II) acetate (0.5 g), tri-o-tolylphosphine (1.4 g) and diisopropylethylamine (1.6 mL) in DMF (10 mL) was stirred under a nitrogen atmosphere at 120°C for 30 hr. The reaction mixture was cooled to room temperature, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.80 g).
MS (ESI+): [M+H]⁺476.0.

### Example 226

### 7-(2-hydroxypropan-2-yl)-3-(2-methyl-5-((2-methyl-1,3-thiazol-4-yl)methoxy)phenyl)-3,4-dihydroquinazolin-2(1H)-one

By a method similar to that in Example 178, the title compound was obtained.
MS (ESI+) : [M+H]⁺424.4.

### Example 227

### 3-(5-((3-fluoropyridin-2-yl)methoxy)-2-methylphenyl)-7-((3-hydroxypiperidin-1-yl)carbonyl)-3,4-dihydroquinazolin-2(1H)-one

By a method similar to that in Example 74, the title compound was obtained.
MS (ESI+): [M+H]⁺491.3.

### Example 228

### butyl 3-(4-((3-fluoropyridin-2-yl)methoxy)-2-(2-oxo-1,4-dihydroquinazolin-3(2H)-yl)phenyl)propanoate

Butyl (2E)-3-(4-((3-fluoropyridin-2-yl)methoxy)-2-(2-oxo-1,4-dihydroquinazolin-3(2H)-yl)phenyl)acrylate (0.6 g) was dissolved in THF (20 mL), palladium/carbon (0.2 g) was added, and the obtained mixture was stirred under a hydrogen atmosphere at room temperature overnight. The precipitate was removed by filtration, and the solvent was evaporated under reduced pressure. The residue was purified by silica gel column chromatography (hexane/ethyl acetate) to give the title compound (0.5 g).
MS (ESI+) : [M+H]⁺478.0.

### Example 229

### 3-(4-((3-fluoropyridin-2-yl)methoxy)-2-(2-oxo-1,4-dihydroquinazolin-3(2H)-yl)phenyl)propanoic acid

By a method similar to that in Example 71, the title compound was obtained.
MS (ESI+): [M+H]⁺422.0.

### Example 230

### 3-(5-((3-fluoropyridin-2-yl)methoxy)-2-(3-hydroxy-3-methylbutyl)phenyl)-3,4-dihydroquinazolin-2(1H)-one

By a method similar to that in Example 178, the title compound was obtained.
MS (ESI+): [M+H-H20]⁺418.0.

### Example 231

### 3-(2-chloro-5-((3-fluoropyridin-2-yl)methoxy)phenyl)-3,4-dihydroquinazolin-2(1H)-one

By a method similar to that in Example 170, step A to B, the title compound was obtained.
MS (ESI+): [M+H]⁺384.1.

### Example 232

### methyl 3-(2-methyl-5-(pyridin-2-ylmethoxy)phenyl)-2-oxo-1,2,3,4-tetrahydroquinazoline-7-carboxylate

By a method similar to that in Example 216, the title compound was obtained.
MS (ESI+): [M+H]⁺404.5.

### Example 233

### 3-(5-((2,6-difluorobenzyl)oxy)-2-fluorophenyl)-7-(2-hydroxypropan-2-yl)-3,4-dihydroquinazolin-2(1H)-one

By a method similar to that in Example 178, the title compound was obtained.
MS (ESI+): [M+H]⁺443.3.

### Example 234

### 3-(5-((2,6-difluorobenzyl)oxy)-2-fluorophenyl)-7-(hydroxymethyl)-3,4-dihydroquinazolin-2(1H)-one

By a method similar to that in Example 179, the title compound was obtained.
MS (ESI+): [M+H]⁺414.9.

### Example 235

### 3-(5-((2,6-difluorobenzyl)oxy)-2-methylphenyl)-3,4-dihydropyrido[4,3-d]pyrimidin-2(1H)-one

By a method similar to that in Example 170, step B, the title compound was obtained.
MS (ESI+): [M+H]⁺381.8.

### Example 236

### 6-(5-((2,6-difluorobenzyl)oxy)-2-methylphenyl)-2-methyl-2,4,6,7-tetrahydro-5H-pyrazolo[4,3-d]pyrimidin-5-one

By a method similar to that in Example 24, step F to G, the title compound was obtained.
MS (ESI+): [M+H]⁺385.0.

### Example 237

### methyl 3-(2-methyl-5-(1,3-thiazol-2-ylmethoxy)phenyl)-2-oxo-1,2,3,4-tetrahydroquinazoline-7-carboxylate

By a method similar to that in Example 216, the title compound was obtained.
MS (ESI+): [M+H]⁺410.3.

### Example 238

### 3-(5-((2,6-difluorobenzyl)oxy)-2-(trifluoromethyl)phenyl)-7-(2-hydroxypropan-2-yl)-3,4-dihydroquinazolin-2(1H)-one

By a method similar to that in Example 178, the title compound was obtained.
MS (ESI+) : [M+H]⁺492.8.

### Example 239

### 3-(5-((2,6-difluorobenzyl)oxy)-2-(trifluoromethyl)phenyl)-7-(hydroxymethyl)-3,4-dihydroquinazolin-2(1H)-one

By a method similar to that in Example 179, the title compound was obtained.
MS (ESI+): [M+H]⁺464.7.

### Example 240

### 3-(5-((2,6-difluorobenzyl)oxy)-2-methylphenyl)-3,4-dihydropyrido[3,4-d]pyrimidin-2(1H)-one

By a method similar to that in Example 170, step B, the title compound was obtained.
MS (ESI+) : [M+H]⁺382.2.

### Example 241

### methyl 3-(2-methyl-5-((5-methyl-1,3-thiazol-2-yl)methoxy)phenyl)-2-oxo-1,2,3,4-tetrahydroquinazoline-7-carboxylate

### A) methyl 3-(5-(2-amino-2-thioxoethoxy)-2-methylphenyl)-2-oxo-1,2,3,4-tetrahydroquinazoline-7-carboxylate

To a solution of methyl 3-(5-hydroxy-2-methylphenyl)-2-oxo-1,2,3,4-tetrahydroquinazoline-7-carboxylate (1.0 g) in DMF (10 mL) were added bromoacetonitrile (420 mg) and potassium carbonate (570 mg), and the mixture was stirred at room temperature for 4 hr. The insoluble material was removed by filtration, and the solvent was evaporated under reduced pressure. The residue was purified by silica gel column chromatography (NH silica, ethyl acetate) to give a crude product (430 mg). To the obtained crude product (430 mg) were added o,o-diethyl dithiophosphate (460 mg) and 4N hydrogen chloride ethyl acetate solution (10 mL), and the mixture was stirred at room temperature overnight. To the residue was added saturated aqueous sodium hydrogen carbonate solution, and the mixture was extracted with ethyl acetate. The obtained organic layer was washed with saturated brine, dried over anhydrous sodium sulfate, and the solvent was evaporated under reduced pressure. The residue was purified by silica gel column chromatography (NH silica, ethyl acetate) to give the title compound (70 mg).
MS (ESI+): [M+H]⁺386.3.

### B) methyl 3-(2-methyl-5-((5-methyl-1,3-thiazol-2-yl)methoxy)phenyl)-2-oxo-1,2,3,4-tetrahydroquinazoline-7-carboxylate

To a solution of methyl 3-(5-(2-amino-2-thioxoethoxy)-2-methylphenyl)-2-oxo-1,2,3,4-tetrahydroquinazoline-7-carboxylate (70 mg) in acetic acid (3 mL) was added 2-chloropropionaldehyde dimethylacetal (130 mg), and the mixture was stirred at 100°C overnight. Water was added to the reaction mixture, and the mixture was extracted with ethyl acetate. The obtained organic layer was washed with saturated brine, dried over anhydrous sodium sulfate, and the solvent was evaporated under reduced pressure. The residue was purified by column chromatography (ethyl acetate/hexane) and HPLC, and recrystallized from ethyl acetate/hexane to give the title compound (12 mg).
MS (ESI+): [M+H]⁺424.3.

### Example 242

### 5-chloro-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+) : [M+H]⁺398.3.

### Example 243

### 6-chloro-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+) : [M+H]⁺398.3.

### Example 244

### 7-chloro-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+): [M+H]⁺398.3.

### Example 245

### 3-(5-((3-fluoropyridin-2-yl)methoxy)-2-propylphenyl)-3,4-dihydroquinazolin-2(1H)-one

### A) 3-fluoro-2-((3-nitro-4-propylphenoxy)methyl)pyridine

To a solution of 2-((4-bromo-3-nitrophenoxy)methyl)-3-fluoropyridine (1.5 g), n-propylboronic acid (0.8 g), potassium carbonate (1.9 g) and iron oxide(I) (2.7 g) in THF (30 mL) was added 1,1'-bis(diphenylphosphino)ferrocene-palladium(II)dichloride-dichloromethane complex (0.7 g) at room temperature. The reaction mixture was stirred under reflux for 9 hr, and the insoluble material was filtered off by using celite. Water was added to the filtrate, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (0.2 g).
MS (ESI+): [M+H]⁺291.4.

### B) 5-((3-fluoropyridin-2-yl)methoxy)-2-propylaniline

To a solution of 3-fluoro-2-((3-nitro-4-propylphenoxy)methyl)pyridine (154 mg) and ammonium chloride (28 mg) in ethanol (2 mL)/water (2 mL) was added reduced iron (148 mg). The reaction mixture was stirred at 85°C for 3 hr, and the insoluble material was filtered off by using celite. The filtrate was concentrated under reduced pressure, and the residue was extracted with ethyl acetate. The extract was washed with saturated brine, and dried over magnesium sulfate. The solvent was evaporated under reduced pressure to give the title compound (139 mg).
MS (ESI+): [M+H]⁺261.4.

### C) 3-(5-((3-fluoropyridin-2-yl)methoxy)-2-propylphenyl)-3,4-dihydroquinazolin-2(1H)-one

By a method similar to that in Example 170, step B, the title compound was obtained.
MS (ESI+): [M+H]⁺392.5.

### Example 246

### methyl 3-(5-((4-chloro-3-fluoropyridin-2-yl)methoxy)-2-methylphenyl)-2-oxo-1,2,3,4-tetrahydroquinazoline-7-carboxylate

By a method similar to that in Example 216, the title compound was obtained.
MS (ESI+): [M+H]⁺456.2.

### Example 247

### 3-(5-((3,5-difluoropyridin-2-yl)methoxy)-2-methylphenyl)-3,4-dihydroquinazolin-2(1H)-one

By a method similar to that in Example 170, step A to B, the title compound was obtained.
MS (ESI+): [M+H]⁺382.0.

### Example 248

### ethyl 5-(4-((3-fluoropyridin-2-yl)methoxy)-2-(2-oxo-1,4-dihydroquinazolin-3(2H)-yl)phenyl)pentanoate

By a method similar to those in Example 225 and Example 228, the title compound was obtained.
MS (ESI+): [M+H]⁺478.1.

### Example 249

### methyl 3-(2-methyl-5-((4-methyl-1,3-thiazol-2-yl)methoxy)phenyl)-2-oxo-1,2,3,4-tetrahydroquinazoline-7-carboxylate

To a solution of methyl 3-(5-(2-amino-2-thioxoethoxy)-2-methylphenyl)-2-oxo-1,2,3,4-tetrahydroquinazoline-7-carboxylate (60 mg) in ethanol (5 mL) was added 2-chloroacetone (144 mg), and the mixture was stirred at 80°C overnight. Water was added to the reaction mixture, and the mixture was extracted with ethyl acetate. The obtained organic layer was washed with saturated brine, dried over anhydrous sodium sulfate, and the solvent was evaporated under reduced pressure. The residue was purified by HPLC and recrystallized from methanol/diisopropyl ether/hexane to give the title compound (28 mg).
MS (ESI+): [M+H]⁺424.3.

### Example 250

### 6-(5-((3-fluoropyridin-2-yl)methoxy)-2-methylphenyl)-2-methyl-2,4,6,7-tetrahydro-5H-pyrazolo[4,3-d]pyrimidin-5-one

By a method similar to that in Example 24, step F to G, the title compound was obtained.
MS (ESI+) : [M+H]⁺368.5.

### Example 251

### 5-(4-((3-fluoropyridin-2-yl)methoxy)-2-(2-oxo-1,4-dihydroquinazolin-3(2H)-yl)phenyl)pentanoic acid

By a method similar to that in Example 71, the title compound was obtained.
¹H NMR (300 MHz, DMSO-d₆) δ 1.30-1.60 (4H, m), 2.02-2.17 (2H, m), 2.40 (2H, d), 4.43 (1H, d, J = 14.3 Hz), 4.88 (1H, d, J = 14.3 Hz), 5.21 (2H, s), 6.77-7.02 (3H, m), 7.02-7.31 (4H, m), 7.54 (1H, dt, J = 8.4, 4.3 Hz), 7.80 (1H, t, J = 9.2 Hz), 8.47 (1H, d, J = 3.8 Hz), 9.47 (1H, brs), 11.93 (1H, brs).

### Example 252

### 3-(5-((3-fluoropyridin-2-yl)methoxy)-2-methylphenyl)-2-oxo-1,2,3,4-tetrahydroquinazolin-7-yl 4-methylbenzenesulfonate

### A) 4-formyl-3-nitrophenyl 4-methylbenzenesulfonate

To a solution of 4-methyl-3-nitrophenol (10.0 g) and potassium carbonate (22.9 g) in acetone (200 mL) was added 4-methylbenzenesulfonyl chloride (13.9 g) at room temperature, and the reaction mixture was heated under reflux for 4 hr. The reaction mixture was concentrated under reduced pressure, and the resulting solid was recovered by filtration. The filtrate was washed with water and saturated brine, and dried over magnesium sulfate. The solvent was evaporated under reduced pressure. The residue and the recovered solid were washed with ethyl acetate/diisopropyl ether/hexane, and dissolved in trifluoromethylbenzene (200 mL). To the reaction mixture were added N-bromosuccinimide (12.9 g) and azodiisobutyronitrile (1.1 g). The reaction mixture was heated under reflux overnight, and N-bromosuccinimide (3.2 g) and azodiisobutyronitrile (0.3 g) were added. The reaction mixture was heated under reflux for 2 hr, concentrated under reduced pressure, and water and ethyl acetate were added. The resulting solid was filtered off with celite, and the filtrate was extracted with ethyl acetate. The extract was washed with saturated brine, and dried over magnesium sulfate. The solvent was evaporated under reduced pressure and the residue was purified by silica gel column chromatography (ethyl acetate/hexane) and dissolved in acetonitrile (500 mL). Molecular sieves 4Å (80 g) and N-methylmorpholine N-oxide (20.7 g) were added to the reaction mixture, and the mixture was stirred at room temperature for 15 hr. The insoluble material was filtered off with celite, and the filtrate was concentrated under reduced pressure. The residue was purified by silica gel column chromatography (ethyl acetate/hexane) to give the title compound (6.8 g).
¹H NMR (300 MHz, CDCl3) δ 2.59 (3H, s), 7.14 - 7.26 (1H, m), 7.31 - 7.39 (1H, m), 7.61 (1H, d, J = 2.3 Hz), 8.00 - 8.14 (4H, m), 10.15 (1H, s).

### B) 3-(5-((3-fluoropyridin-2-yl)methoxy)-2-methylphenyl)-2-oxo-1,2,3,4-tetrahydroquinazolin-7-yl 4-methylbenzenesulfonate

By a method similar to that in Example 170, step B, the title compound was obtained.
MS (ESI+): [M+H]⁺534.2.

### Example 253

### 8-chloro-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+) : [M+H]⁺398.3.

### Example 254

### 3-(2-benzyl-5-((2,6-difluorobenzyl)oxy)phenyl)-7-(2-hydroxypropan-2-yl)-3,4-dihydroquinazolin-2(1H)-one

By a method similar to that in Example 178, the title compound was obtained.
MS (ESI+): [M+H]⁺514.8.

### Example 255

### 3-(2-benzyl-5-((2,6-difluorobenzyl)oxy)phenyl)-7-(hydroxymethyl)-3,4-dihydroquinazolin-2(1H)-one

By a method similar to that in Example 179, the title compound was obtained.
MS (ESI+): [M+H]⁺486.8.

### Example 256

### 3-(5-((3-fluoropyridin-2-yl)methoxy)-2-methylphenyl)-3,4-dihydropyrido[2,3-d]pyrimidin-2(1H)-one

Using 2-aminonicotinaldehyde as a starting material and by a method similar to that in Example 4, step L, the title compound was obtained.
MS (ESI+): [M+H]⁺365.3.

### Example 257

### 3-(5-((2,6-difluorobenzyl)oxy)-2-(2-phenylethyl)phenyl)-7-(hydroxymethyl)-3,4-dihydroquinazolin-2(1H)-one

By a method similar to that in Example 179, the title compound was obtained.
MS (ESI+): [M+H]⁺501.1.

### Example 258

### 3-(2-bromo-5-((3-fluoropyridin-2-yl)methoxy)phenyl)-1-(4-methoxybenzyl)-3,4-dihydroquinazolin-2(1H)-one

To a solution of 3-(2-bromo-5-((3-fluoropyridin-2-yl)methoxy)phenyl)-3,4-dihydroquinazolin-2(1H)-one (1.0 g) in DMF (30 mL) was added sodium hydride (60%, oil) (0.1 g) under ice-cooling, and the mixture was stirred at 0°C for 1 hr. To the reaction solution was added p-methoxybenzyl chloride (0.35 mL), and the mixture was stirred at 0°C for 3 hr. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (1.1 g).
MS (ESI+): [M+H]⁺548.4.

### Example 259

### 3-(5-((3-fluoropyridin-2-yl)methoxy)-2-methylphenyl)-3,4-dihydropyrido[3,4-d]pyrimidin-2(1H)-one

Using 3-aminoisonicotinaldehyde as a starting material and by a method similar to that in Example 4, step L, the title compound was obtained.
MS (ESI+): [M+H]⁺365.3.

### Example 260

### 3-(5-((2,6-difluorobenzyl)oxy)-2-(2-phenylethyl)phenyl)-7-(2-hydroxypropan-2-yl)-3,4-dihydroquinazolin-2(1H)-one

By a method similar to that in Example 178, the title compound was obtained.
MS (ESI+): [M+H]⁺528.9.

### Example 261

### N-cyclopropyl-3-(5-((3-fluoropyridin-2-yl)methoxy)-2-methylphenyl)-2-oxo-1,2,3,4-tetrahydroquinazoline-7-carboxamide

In the same manner as in Example 74, the title compound was obtained.
MS (ESI+): [M+H]⁺447.4.

### Example 262

### 3-(5-((3-fluoropyridin-2-yl)methoxy)-2-(hydroxymethyl)phenyl)-3,4-dihydroquinazolin-2(1H)-one

By a method similar to that in Example 179, the title compound was obtained.
MS (ESI+): [M+H-H2O]⁺361.9.

### Example 263

### 6-(5-((3-fluoropyridin-2-yl)methoxy)-2-methylphenyl)-2-(2,2,2-trifluoroethyl)-2,4,6,7-tetrahydro-5H-pyrazolo[4,3-d]pyrimidin-5-one

By a method similar to that in Example 24, step F to G, the title compound was obtained.
MS (ESI+): [M+H]⁺436.1.

### Example 264

### 6-(2-bromo-5-((3-fluoropyridin-2-yl)methoxy)phenyl)-2-methyl-2,4,6,7-tetrahydro-5H-pyrazolo[4,3-d]pyrimidin-5-one

By a method similar to that in Example 24, step F to G, the title compound was obtained.
MS (ESI+): [M+H]⁺432.1.

### Example 265

### 6-(5-((3-chloropyridin-2-yl)methoxy)-2-methylphenyl)-2-methyl-2,4,6,7-tetrahydro-5H-pyrazolo[4,3-d]pyrimidin-5-one

By a method similar to that in Example 24, step F to G, the title compound was obtained.
MS (ESI+): [M+H]⁺384.1.

### Example 266

### 3-(5-((3-fluoropyridin-2-yl)methoxy)-2-methylphenyl)-N-methyl-2-oxo-N-propyl-1,2,3,4-tetrahydroquinazoline-7-carboxamide

By a method similar to that in Example 74, the title compound was obtained.
MS (ESI+): [M+H]⁺463.3.

### Example 267

### 3-(2-bromo-5-(pyrimidin-2-ylmethoxy)phenyl)-3,4-dihydroquinazolin-2(1H)-one

By a method similar to that in Example 170, step A to B, the title compound was obtained.
¹H NMR (300 MHz, DMSO-d₆) δ 4.46-4.65 (1H, m), 4.70-4.90 (1H, m), 5.31 (2H, s), 6.78-6.97 (3H, m), 7.08-7.22 (2H, m), 7.25 (1H, d, J = 3.0 Hz), 7.46-7.52 (1H, m), 7.57 (1H, d, J = 8.7 Hz), 8.85 (2H, d, J = 4.9 Hz), 9.54 (1H, s).

### Example 268

### 3-(5-((3-fluoropyridin-2-yl)methoxy)-2-methylphenyl)-3,4-dihydropyrido[4,3-d]pyrimidin-2(1H)-one

By a method similar to that in Example 170, step B, the title compound was obtained.
MS (ESI+): [M+H]⁺365.1.

### Example 269

### 5-chloro-3-(5-((3-fluoropyridin-2-yl)methoxy)-2-methylphenyl)-1-methyl-3,4-dihydroquinazolin-2(1H)-one

To a mixture of sodium hydride (60%, oil)(18 mg) in DMF (3 mL) was added 5-chloro-3-(5-((3-fluoropyridin-2-yl)methoxy)-2-methylphenyl)-3,4-dihydroquinazolin-2(1H)-one (150 mg) at 0°C, and the reaction mixture was stirred at 0°C for 30 min. To the reaction mixture was added methyl iodide (24 µL) at 0°C, and the mixture was stirred at 0°C for 1.5 hr, and at room temperature for 8 hr. Water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate, and concentrated under reduced pressure to give the title compound (20 mg).
MS (ESI+): [M+H]⁺412.2.

### Example 270

### 3-(5-((3-fluoropyridin-2-yl)methoxy)-2-methylphenyl)-N-isopropyl-2-oxo-1,2,3,4-tetrahydroquinazoline-7-carboxamide

By a method similar to that in Example 74, the title compound was obtained.
MS (ESI+): [M+H]⁺449.2.

### Example 271

### 3-(5-((3-fluoropyridin-2-yl)methoxy)-2-methylphenyl)-2-oxo-N-(pyridin-2-yl)-1,2,3,4-tetrahydroquinazoline-7-carboxamide

By a method similar to that in Example 74, the title compound was obtained.
MS (ESI+) : [M+H]⁺484.3.

### Example 272

### 3-(5-((3-fluoropyridin-2-yl)methoxy)-2-methylphenyl)-3,4-dihydropyrido[3,2-d]pyrimidin-2(1H)-one

Using tert-butyl (2-formylpyridin-3-yl)carbamate as a starting material and by a method similar to that in Example 1, step G to H, the title compound was obtained.
¹H NMR (300 MHz, CDCl₃) δ 2.16 (3H, s), 3.92 (2H, brs), 4.31 (2H, s), 4.80 (1H, brs), 5.25 (2H, d, J = 2.3 Hz), 6.39 (1H, dd, J = 8.1, 2.4 Hz), 6.52 (1H, d, J = 2.4 Hz), 6.93-7.02 (2H, m), 7.03-7.11 (1H, m), 7.26-7.35 (1H, m), 7.38-7.49 (1H, m), 7.99-8.09 (1H, m), 8.42-8.51 (1H, m).

### Example 273

### 3-(5-((3-fluoropyridin-2-yl)methoxy)-2-methylphenyl)-2-oxo-N-phenyl-1,2,3,4-tetrahydroquinazoline-7-carboxamide

In the same manner as in Example 74, the title compound was obtained.
MS (ESI+) : [M+H]⁺483.2.

### Example 274

### 5-chloro-3-(2-methyl-5-(pyrimidin-2-ylmethoxy)phenyl)-3,4-dihydroquinazolin-2(1H)-one

By a method similar to that in Example 170, the title compound was obtained.
MS (ESI+) : [M+H]⁺381.1.

### Example 275

### 3-(5-((3-fluoropyridin-2-yl)methoxy)-2-methylphenyl)-7-(trifluoromethyl)-3,4-dihydroquinazolin-2(1H)-one

By a method similar to that in Example 170, step B, the title compound was obtained.
MS (ESI+): [M+H]⁺432.2.

### Example 276

### 2-(cyclopropylmethyl)-6-(5-((3-fluoropyridin-2-yl)methoxy)-2-methylphenyl)-2,4,6,7-tetrahydro-5H-pyrazolo[4,3-d]pyrimidin-5-one

By a method similar to that in Example 24, step F to G, the title compound was obtained.
MS (ESI+): [M+H]⁺408.4.

### Example 277

### 3-(2-bromo-5-((3-fluoropyridin-2-yl)methoxy)phenyl)-5-chloro-3,4-dihydroquinazolin-2(1H)-one

By a method similar to that in Example 170, step B, the title compound was obtained.
MS (ESI+): [M+H]⁺462.1.

### Example 278

### 2-(5-chloro-2-oxo-1,4-dihydroquinazolin-3(2H)-yl)-4-((3-fluoropyridin-2-yl)methoxy)benzonitrile

To a solution of 3-(2-bromo-5-((3-fluoropyridin-2-yl)methoxy)phenyl)-5-chloro-3,4-dihydroquinazolin-2(1H)-one (234 mg), zinc cyanide (119 mg) in DMF (5 mL) was added tetrakis(triphenylphosphine)palladium(0) (118 mg), and the reaction mixture was stirred under a nitrogen atmosphere and under microwave irradiation at 200°C for 20 min. The insoluble material was filtered off by using celite. The filtrate was extracted with ethyl acetate, and the extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate and concentrated under reduced pressure. The residue was washed with ethyl acetate/hexane/ethanol to give the title compound (47 mg).
MS (ESI+) : [M+H]⁺409.0.

### Example 279

### 5-chloro-3-(5-((3-fluoropyridin-2-yl)methoxy)-2-methylphenyl)-3,4-dihydropyrido[4,3-d]pyrimidin-2(1H)-one

Using N-(2-chloro-3-formylpyridin-4-yl)-2,2-dimethylpropanamide as a starting material and by a method similar to that in Example 1, step G to H, the title compound was obtained.
MS (ESI+): [M+H]⁺399.2.

### Example 280

### N-cyclopropyl-3-(4-((3-fluoropyridin-2-yl)methoxy)-2-(2-oxo-1,4-dihydroquinazolin-3(2H)-yl)phenyl)propanamide

By a method similar to that in Example 74, the title compound was obtained.
MS (ESI+): [M+H]⁺461.1.

### Example 281

### 5-fluoro-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+): [M+H]⁺382.3.

### Example 282

### 5-bromo-3-(5-((3-fluoropyridin-2-yl)methoxy)-2-methylphenyl)-3,4-dihydroquinazolin-2(1H)-one

By a method similar to that in Example 170, step B, the title compound was obtained.
MS (ESI+): [M+H]⁺442.3.

### Example 283

### 5-chloro-3-(5-((3-fluoropyridin-2-yl)methoxy)-2-methylphenyl)-1-(2-methoxyethyl)-3,4-dihydroquinazolin-2(1H)-one

By a method similar to that in Example 269, the title compound was obtained.
MS (ESI+) : [M+H]⁺456.4.

### Example 284

### 5-chloro-l-(cyclopropylmethyl)-3-(5-((3-fluoropyridin-2-yl)methoxy)-2-methylphenyl)-3,4-dihydroquinazolin-2(1H)-one

By a method similar to that in Example 269, the title compound was obtained.
MS (ESI+): [M+H]⁺452.3.

### Example 285

### (5-chloro-3-(5-((3-fluoropyridin-2-yl)methoxy)-2-methylphenyl)-2-oxo-3,4-dihydroquinazolin-1(2H)-yl)acetic acid

### A) ethyl 2-(5-chloro-3-(5-((3-fluoropyridin-2-yl)methoxy)-2-methylphenyl)-2-oxo-3,4-dihydroquinazolin-1(2H)-yl)acetate

By a method similar to that in Example 269, the title compound was obtained.
MS (ESI+): [M+H]⁺484.1.

### B) (5-chloro-3-(5-((3-fluoropyridin-2-yl)methoxy)-2-methylphenyl)-2-oxo-3,4-dihydroquinazoline-1(2H)-yl)acetic acid

By a method similar to that in Example 71, the title compound was obtained.
MS (ESI+): [M+H]⁺456.1.

### Example 286

### 2-(5-chloro-3-(5-((3-fluoropyridin-2-yl)methoxy)-2-methylphenyl)-2-oxo-3,4-dihydroquinazolin-1(2H)-yl)acetamide

By a method similar to that in Example 74, the title compound was obtained.
MS (ESI+) : [M+H]⁺455.2.

### Example 287

### 5-chloro-3-(2-chloro-4-fluoro-5-((3-fluoropyridin-2-yl)methoxy)phenyl)-3,4-dihydroquinazolin-2(1H)-one

By a method similar to that in Example 170, step A to B, the title compound was obtained.
¹H NMR (300 MHz, DMSO-d₆) δ 4.47-4.66 (1H, m), 4.75-4.91 (1H, m), 5.23-5.43 (2H, m), 6.77-6.86 (1H, m), 6.98-7.08 (1H, m), 7.16-7.30 (1H, m), 7.50-7.65 (2H, m), 7.70 (1H, d, J = 8.7 Hz), 7.78-7.88 (1H, m), 8.40-8.52 (1H, m), 9.86 (1H, s).

### Example 288

### 3-(5-((3-fluoropyridin-2-yl)methoxy)-2-methylphenyl)-7-(morpholin-4-ylcarbonyl)-3,4-dihydroquinazolin-2(1H)-one

By a method similar to that in Example 74, the title compound was obtained.
MS (ESI+): [M+H]⁺477.2.

### Example 289

### 3-(2-(cyclohexylmethyl)-5-((2,6-difluorobenzyl)oxy)phenyl)-7-(2-hydroxypropan-2-yl)-3,4-dihydroquinazolin-2(1H)-one

By a method similar to that in Example 178, the title compound was obtained.
MS (ESI+): [M+H]⁺521.4.

### Example 290

### 3-(2-(cyclohexylmethyl)-5-((2,6-difluorobenzyl)oxy)phenyl)-7-(hydroxymethyl)-3,4-dihydroquinazolin-2(1H)-one

By a method similar to that in Example 179, the title compound was obtained.
MS (ESI+): [M+H]⁺493.1.

### Example 291

### 5-chloro-3-(5-((3-fluoropyridin-2-yl)methoxy)-2-methylphenyl)-1-methyl-3,4-dihydroquinazolin-2(1H)-one

By a method similar to that in Example 269, the title compound was obtained.
¹H NMR (300MHz, DMSO-d₆) δ 2.03 (3H, s), 3.26 (3H, s), 4.54 (1H, d, J = 14.8 Hz), 4.75-5.03 (1H, m, J = 14.8 Hz), 5.22 (2H, s), 6.95 (1H, dd, J = 8.3, 2.7 Hz), 7.02 (1H, d, J = 8.3 Hz), 7.08-7.25 (3H, m), 7.31-7.43 (1H, m), 7.54 (1H, dt, J = 8.5, 4.4 Hz), 7.80 (1H, ddd, J = 10.0, 8.5, 1.1 Hz), 8.46 (1H, dt, J = 4.5, 1.5 Hz).

### Example 292

### 5-chloro-3-(5-((3-fluoropyridin-2-yl)methoxy)-2-methylphenyl)-2-oxo-1,2,3,4-tetrahydroquinazoline-7-carboxylic acid

### A) methyl 3-(5-((3-fluoropyridin-2-yl)methoxy)-2-methylphenyl)-2-oxo-1,2,3,4-tetrahydroquinazoline-7-carboxylate

By a method similar to that in Example 170, step B, the title compound was obtained.
MS (ESI+) : [M+H]⁺456.2.

### B) 5-chloro-3-(5-((3-fluoropyridin-2-yl)methoxy)-2-methylphenyl)-2-oxo-1,2,3,4-tetrahydroquinazoline-7-carboxylic acid

By a method similar to that in Example 71, the title compound was obtained.
MS (ESI+) : [M+H]⁺442.3.

### Example 293

### 3-(5-((2,6-difluorobenzyl)oxy)-2-(2,2-dimethylpropyl)phenyl)-7-(2-hydroxypropan-2-yl)-3,4-dihydroquinazolin-2(1H)-one

By a method similar to that in Example 178, the title compound was obtained.
MS (ESI+): [M+H]⁺495.4.

### Example 294

### 3-(5-((2,6-difluorobenzyl)oxy)-2-(2,2-dimethylpropyl)phenyl)-7-(hydroxymethyl)-3,4-dihydroquinazolin-2(1H)-one

By a method similar to that in Example 179, the title compound was obtained.
MS (ESI+): [M+H]⁺467.4.

### Example 295

### methyl 2-((5-chloro-3-(5-((3-fluoropyridin-2-yl)methoxy)-2-methylphenyl)-2-oxo-3,4-dihydroquinazolin-1(2H)-yl)methyl)benzoate

By a method similar to that in Example 269, the title compound was obtained.
MS (ESI+) : [M+H]⁺546.2.

### Example 296

### 2-((5-chloro-3-(5-((3-fluoropyridin-2-yl)methoxy)-2-methylphenyl)-2-oxo-3,4-dihydroquinazolin-1(2H)-yl)methyl)benzoic acid

By a method similar to that in Example 71, the title compound was obtained.
MS (ESI+): [M+H]⁺532.3.

### Example 297

### 3-(5-((3-fluoropyridin-2-yl)methoxy)-2-methylphenyl)-5-(trifluoromethyl)-3,4-dihydroquinazolin-2(1H)-one

By a method similar to that in Example 170, step B, the title compound was obtained.
MS (ESI+): [M+H]⁺432.0.

### Example 298

### 3-(5-((3-fluoropyridin-2-yl)methoxy)-2-methylphenyl)-2-oxo-1,2,3,4-tetrahydroquinazoline-5-carbonitrile

By a method similar to that in Example 278, the title compound was obtained.
¹H NMR (300 MHz, d6-DMSO) δ 2.09 (3H, s), 4.65 (1H, d, J = 15.5 Hz), 5.05 (1H, d, J = 15.5 Hz), 5.32 (2H, s), 6.96 (1H, dd, J = 8.3, 2.7 Hz), 7.09 - 7.24 (3H, m), 7.40 (2H, d, J = 3.8 Hz), 7.53 (1H, dt, J = 8.3, 4.3 Hz), 7.80 (1H, t, J = 9.3 Hz), 8.46 (1H, d, J = 9.3 Hz), 9.86 (1H, s).

### Example 299

### 5-chloro-N-cyclopropyl-3-(5-((3-fluoropyridin-2-yl)methoxy)-2-methylphenyl)-2-oxo-1,2,3,4-tetrahydroquinazoline-7-carboxamide

By a method similar to that in Example 74, the title compound was obtained.
MS (ESI+): [M+H]⁺481.3.

### Example 300

### 3-(2-fluoro-5-((3-fluoropyridin-2-yl)methoxy)phenyl)-3,4-dihydroquinazolin-2(1H)-one

By a method similar to that in Example 170, step A to B, the title compound was obtained.
MS (ESI+) : [M+H]⁺368.0.

### Example 301

### 7-bromo-3-(5-((3-fluoropyridin-2-yl)methoxy)-2-(trifluoromethyl)phenyl)-3,4-dihydroquinazolin-2(1H)-one

By a method similar to that in Example 170, step A to B, the title compound was obtained.
MS (ESI+): [M+H]⁺496.0.

### Example 302

### N-cyclopropyl-3-(5-((3-fluoropyridin-2-yl)methoxy)-2-methylphenyl)-N-methyl-2-oxo-1,2,3,4-tetrahydroquinazoline-7-carboxamide

By a method similar to that in Example 74, the title compound was obtained.
MS (ESI+) : [M+H]⁺461.3.

### Example 303

### 3-(5-((3-fluoropyridin-2-yl)methoxy)-2-methylphenyl)-2-oxo-N-(2,2,2-trifluoroethyl)-1,2,3,4-tetrahydroquinazoline-7-carboxamide

By a method similar to that in Example 74, the title compound was obtained.
MS (ESI+): [M+H]⁺489.2.

### Example 304

### 7-chloro-3-(5-((3-fluoropyridin-2-yl)methoxy)-4-iodo-2-methylphenyl)-3,4-dihydroquinazolin-2(1H)-one

### A) N-(4-chloro-2-nitrobenzyl)-5-((3-fluoropyridin-2-yl)methoxy)-2-methylaniline

A mixture of 4-chloro-2-nitrobenzaldehyde (370 mg), 5-((3-fluoropyridin-2-yl)methoxy)-2-methylaniline (464 mg), p-toluenesulfonic acid monohydrate (5 mg) and toluene (25 mL) was heated under reflux for 3 hr. The mixture was allowed to cool to room temperature and diluted with ethanol (10 mL). The mixture was cooled to 0°C, sodium borohydride (150 mg) was added, and the mixture was stirred at room temperature for 15 hr. The mixture was diluted with water, and extracted with ethyl acetate. The combined organic layer was washed with saturated brine, dried over anhydrous magnesium sulfate, and concentrated under reduced pressure. The residue was purified by silica gel column chromatography (hexane/ethyl acetate) to give the title compound (638 mg).
¹H NMR (300 MHz, DMSO-d₆) δ 2.10 (3H, s), 4.62 (2H, d, J = 5.7 Hz), 4.99 (2H, d, J = 1.9 Hz), 5.77 (1H, t, J = 5.7 Hz), 5.87 (1H, d, J = 2.7 Hz), 6.21 (1H, dd, J = 8.1, 2.5 Hz), 6.89 (1H, d, J = 8.3 Hz), 7.39-7.52 (2H, m), 7.64-7.78 (2H, m), 8.14 (1H, d, J = 2.3 Hz), 8.32-8.38 (1H, m).

### B) N-(4-chloro-2-nitrobenzyl)-5-((3-fluoropyridin-2-yl)methoxy)-4-iodo-2-methylaniline

To a mixture of N-(4-chloro-2-nitrobenzyl)-5-((3-fluoropyridin-2-yl)methoxy)-2-methylaniline(500 mg), silver sulfate (252 mg) and ethanol (10 mL) was added iodine (316 mg) at room temperature, and the mixture was stirred at room temperature for 3 hr. The mixture was diluted with water, and extracted with ethyl acetate. The combined organic layer was washed with saturated brine, dried over anhydrous magnesium sulfate, and concentrated under reduced pressure. The residue was purified by silica gel column chromatography (hexane/ethyl acetate) to give the title compound (400 mg).
¹H NMR (300 MHz, DMSO-d₆) δ 2.07 (3H, s), 4.64 (2H,d, J = 6.0 Hz), 5.01 (2H, d, J = 1.5 Hz), 5.98 (1H, t, J = 6.0 Hz), 6.03 (1H, s), 7.29 (1H, s), 7.38-7.46 (2H, m), 7.64 (1H, dd, J = 10.0, 1.4 Hz), 7.71 (1H, dd, J = 8.3, 2.3 Hz), 8.17 (1H, d, J = 2.3 Hz), 8.24 (1H, dd, J = 6.1, 1.4 Hz).

### C) N-(2-amino-4-chlorobenzyl)-5-((3-fluoropyridin-2-yl)methoxy)-4-iodo-2-methylaniline

A mixture of N-(4-chloro-2-nitrobenzyl)-5-((3-fluoropyridin-2-yl)methoxy)-4-iodo-2-methylaniline (400 mg), reduced iron (195 mg), calcium chloride (84 mg), water (4 mL) and ethanol (20 mL) was heated under reflux for 3 hr. The mixture was allowed to cool to room temperature and filtered off with celite. The filtrate was diluted with ethyl acetate. The obtained organic layer was washed with saturated brine, dried over anhydrous magnesium sulfate, and concentrated under reduced pressure. The residue was purified by silica gel column chromatography (hexane/ethyl acetate) to give the title compound (300 mg).
¹H NMR (300 MHz, DMSO-d₆) δ 2.02 (3H, s), 4.13 (2H, d, J = 5.7 Hz), 5.07 (2H, d, J = 1.9 Hz), 5.40 (2H, brs), 5.70 (1H, t, J = 5.7 Hz), 6.25 (1H, s), 6.44 (1H, dd, J = 8.1, 2.1 Hz), 6.65 (1H, d, J = 2.1 Hz), 7.01 (1H, d, J = 7.9 Hz), 7.24 (1H, s), 7.43-7.52 (1H, m), 7.69-7.77 (1H, m), 8.35-8.40 (1H, m).

### D) 7-chloro-3-(5-((3-fluoropyridin-2-yl)methoxy)-4-iodo-2-methylphenyl)-3,4-dihydroquinazolin-2(1H)-one

A mixture of N-(2-amino-4-chlorobenzyl)-5-((3-fluoropyridin-2-yl)methoxy)-4-iodo-2-methylaniline (300 mg), CDI (195 mg), DBU (183 mg) and acetonitrile (10 mL) was heated under reflux for 2 hr. The mixture was allowed to cool to room temperature and the solvent was evaporated under reduced pressure. The residue was purified by silica gel column chromatography (hexane/ethyl acetate) to give the title compound (150 mg).
MS (ESI+): [M+H]⁺524.0.

### Example 305

### 3-(5-((3-fluoropyridin-2-yl)methoxy)-2-methylphenyl)-5-hydroxy-3,4-dihydroquinazolin-2(1H)-one

To a solution of 5-bromo-3-(5-((3-fluoropyridin-2-yl)methoxy)-2-methylphenyl)-3,4-dihydroquinazolin-2(1H)-one (700 mg), 4,4,4',4',5,5,5',5'-octamethyl-2,2'-bi-1,3,2-dioxaborolane (482 mg), potassium acetate (465 mg) in DMF (15 mL) was added palladium(II) acetate (36 mg) at 90°C, and the reaction mixture was stirred under an argon atmosphere at 90°C for 20 hr. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and dissolved in THF (6 mL)/acetone(6 mL)/water (6 mL). Oxone (1070 mg) was added, and the mixture was stirred at room temperature overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate and concentrated under reduced pressure. The residue was recrystallized from ethyl acetate to give the title compound (33 mg).
MS (ESI+): [M+H]⁺380.3.

### Example 306

### 3-(5-((3-fluoropyridin-2-yl)methoxy)-2-(pyrrolidin-1-ylmethyl)phenyl)-3,4-dihydroquinazolin-2(1H)-one

### A) 3-(2-formyl-5-((3-fluoropyridin-2-yl)methoxy)phenyl)-3,4-dihydroquinazolin-2(1H)-one

To a solution of 3-(5-((3-fluoropyridin-2-yl)methoxy)-2-(hydroxymethyl)phenyl)-3,4-dihydroquinazolin-2(1H)-one (1.20 g), DMSO (2.0 mL) and triethylamine (2.0 mL) in dichloromethane (24 mL) was added sulfur trioxide-pyridine complex (1.51 g) under ice-cooling. The mixture was stirred at room temperature for 2 hr, water was added to the reaction mixture, and the mixture was extracted with ethyl acetate. The obtained organic layer was washed with saturated brine, dried over anhydrous sodium sulfate, and the solvent was evaporated under reduced pressure to give the title compound (1.18 g).
¹H NMR (300 MHz, CDCl₃) δ 4.60-4.98 (2H, m), 5.35 (2H, d, J = 1.6 Hz), 6.75 (1H, d, J = 8.0 Hz), 6.99-7.08 (4H, m), 7.13 (1H, dd, J = 8.4, 2.4 Hz), 7.22-7.27 (1H, m), 7.34-7.39 (1H, m), 7.46-7.51 (1H, m), 7.93 (1H, d, J = 8.4 Hz), 8.47-8.48 (1H, m), 9.99 (1H, s)

### B) 3-(5-((3-fluoropyridin-2-yl)methoxy)-2-(pyrrolidin-1-ylmethyl)phenyl)-3,4-dihydroquinazolin-2(1H)-one

To a solution of 3-(2-formyl-5-((3-fluoropyridin-2-yl)methoxy)phenyl)-3,4-dihydroquinazolin-2(1H)-one (174 mg) and pyrrolidine (65 mg) in dichloromethane (2.0 mL) was added acetic acid (33 mg), and the mixture was stirred at room temperature for 2 hr. Sodium triacetoxyborohydride (147 mg) was added and the mixture was stirred overnight at room temperature. The reaction mixture was diluted with dichloromethane, and 1N aqueous sodium hydroxide solution was added. The mixture was dried over magnesium sulfate, and filtered. The solvent was evaporated under reduced pressure and the residue was purified by silica gel column chromatography (ethyl acetate/hexane) to give the title compound (141 mg).
MS (ESI+): [M+H]⁺433.2.

### Example 307

### 3-(5-((3-fluoropyridin-2-yl)methoxy)-2-methylphenyl)-5-methoxy-3,4-dihydropyrido[4,3-d]pyrimidin-2(1H)-one

Using 4-amino-2-methoxynicotinaldehyde as a starting material and by a method similar to that in Example 4, step L, the title compound was obtained.
MS (ESI+) : [M+H]⁺395.3.

### Example 308

### 3-(5-((3-fluoropyridin-2-yl)methoxy)-2-methylphenyl)-5-methoxy-3,4-dihydroquinazolin-2(1H)-one

To a solution of 3-(5-((3-fluoropyridin-2-yl)methoxy)-2-methylphenyl)-5-hydroxy-3,4-dihydroquinazolin-2(1H)-one (77 mg) and potassium carbonate (57 mg) in acetone (4 mL) was added dimethyl sulfate (20 µL) at 40°C, and the reaction mixture was stirred at 80°C for 12 hr. Potassium carbonate (57 mg), dimethyl sulfate (20 µL) and DMSO (2 mL) were added and the mixture was stirred at 80°C for 2 days. The reaction mixture was concentrated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate and concentrated under reduced pressure. The residue was recrystallized from ethyl acetate/hexane to give the title compound (10 mg).
MS (ESI+): [M+H]⁺394.2.

### Example 309

### 6-(5-((2,6-difluorobenzyl)oxy)-2-methylphenyl)-1,4,6,7-tetrahydro-5H-pyrazolo[4,3-d]pyrimidin-5-one

By a method similar to that in Example 24, step F to G, the title compound was obtained.
MS (ESI+) : [M+H]⁺370.8.

### Example 310

### 3-(5-((3-fluoropyridin-2-yl)methoxy)-2-methylphenyl)-4,6-dihydropyrido[4,3-d]pyrimidine-2,5(1H,3H)-dione

To a solution of 3-(5-((3-fluoropyridin-2-yl)methoxy)-2-methylphenyl)-5-methoxy-3,4-dihydropyrido[4,3-d]pyrimidin-2(1H)-one (200 mg) in acetonitrile (10 mL) was added trimethylsilyl iodide (505 mg) at 0°C, and the reaction mixture was stirred at 50°C for 2 hr. DMSO (2 mL) and trimethylsilyl iodide (505 mg) were added to the reaction mixture, and the mixture was stirred at 90°C for 12 hr. Water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added a saturated aqueous sodium hydrogen carbonate solution, and the mixture was extracted with ethyl acetate, dried over anhydrous magnesium sulfate and concentrated under reduced pressure. The residue was recrystallized from ethanol to give the title compound (22 mg).
MS (ESI+) : [M+H]⁺381.4.

### Example 311

### 5-(5-((2,6-difluorobenzyl)oxy)-2-methylphenyl)-1-methyl-1,4,5,7-tetrahydro-6H-pyrazolo[3,4-d]pyrimidin-6-one

By a method similar to that in Example 24, step F to G, the title compound was obtained.
MS (ESI+): [M+H]⁺384.8.

### Example 312

### 3-(2-((cyclopropylamino)methyl)-5-((3-fluoropyridin-2-yl)methoxy)phenyl)-3,4-dihydroquinazolin-2(1H)-one

By a method similar to that in Example 306, step B, the title compound was obtained.
MS (ESI+) : [M+H]⁺419.2.

### Example 313

### 3-(5-((3-fluoropyridin-2-yl)methoxy)-2-(morpholin-4-ylmethyl)phenyl)-3,4-dihydroquinazolin-2(1H)-one

By a method similar to that in Example 306, step B, the title compound was obtained.
MS (ESI+) : [M+H]⁺449.2.

### Example 314

### 3-(5-((3-fluoropyridin-2-yl)methoxy)-2-((4-propylpiperazin-1-yl)methyl)phenyl)-3,4-dihydroquinazolin-2(1H)-one

By a method similar to that in Example 306, step B, the title compound was obtained.
MS (ESI+): [M+H]⁺ 490.2.

### Example 315

### 5-bromo-3-(5-((3-fluoropyridin-2-yl)methoxy)-2-methylphenyl)-3,4-dihydropyrido[3,4-d]pyrimidin-2(1H)-one

### A) 3-bromo-4-methyl-5-nitropyridine 1-oxide

A mixture of 3-bromo-4-methyl-5-nitropyridine (500 mg), 30% aqueous hydrogen peroxide solution (10 mL) and acetic anhydride (10 mL) was stirred at 70°C for 3 hr. The reaction mixture was concentrated under reduced pressure, and added to a mixture of ethyl acetate and water. The mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (188 mg).
¹H NMR (300 MHz, DMSO-d₆) δ 2.43 (3H, s), 8.87-8.98 (2H, m).

### B) 3-bromo-4-(bromomethyl)-5-nitropyridine 1-oxide

A mixture of 3-bromo-4-methyl-5-nitropyridine 1-oxide (188 mg), N-bromosuccinimide (172 mg), azobisisobutyronitrile (1.3 mg), and trifluorobenzene (5 mL) was stirred at 100°C for 3 hr. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (121 mg).
¹H NMR (300 MHz, DMSO-d₆) δ 4.72 (2H, s), 8.97 (1H, d, J = 1.9 Hz), 9.04 (1H, d, J = 1.5 Hz).

### C) 5-bromo-3-(5-((3-fluoropyridin-2-yl)methoxy)-2-methylphenyl)-3,4-dihydropyrido[3,4-d]pyrimidin-2(1H)-one

By a method similar to that in Example 24, step F to G, the title compound was obtained.
¹H NMR (300 MHz, DMSO-d₆) δ 2.09 (3H, s), 4.52 (1H, d, J = 16.0 Hz), 4.89 (1H, d, J = 16.0 Hz), 5.22 (2H, s), 6.96 (1H, dd, J = 8.3, 2.6 Hz), 7.11-7.31 (2H, m), 7.42-7.62 (1H, m), 7.71-7.87 (1H, m), 8.07 (1H, s), 8.27 (1H, s), 8.39-8.53 (1H, m), 9.91 (1H, s).

### Example 316

### 3-(5-((2,6-difluorobenzyl)oxy)-2-(pyridin-2-ylmethyl)phenyl)-7-(2-hydroxypropan-2-yl)-3,4-dihydroquinazolin-2(1H)-one

By a method similar to that in Example 178, the title compound was obtained.
MS (ESI+): [M+H]⁺516.0.

### Example 317

### 3-(5-((2,6-difluorobenzyl)oxy)-2-(pyridin-2-ylmethyl)phenyl)-7-(hydroxymethyl)-3,4-dihydroquinazolin-2(1H)-one

By a method similar to that in Example 179, the title compound was obtained.
MS (ESI+): [M+H]⁺487.8.

### Example 318

### 5-chloro-3-(5-((3-fluoropyridin-2-yl)methoxy)-2-methylphenyl)-3,4-dihydropyrimido[4,5-d]pyrimidin-2(1H)-one

Using 4-amino-6-chloro-5-formylpyrimidine as a starting material and by a method similar to that in Example 4, step L, the title compound was obtained.
MS (ESI+): [M+H]⁺400.0.

### Example 319

### 3-(5-((3-fluoropyridin-2-yl)methoxy)-2-methylphenyl)-5-methoxy-3,4-dihydropyrimido[4,5-d]pyrimidin-2(1H)-one

To a solution of 5-chloro-3-(5-((3-fluoropyridin-2-yl)methoxy)-2-methylphenyl)-3,4-dihydropyrimido[4,5-d]pyrimidin-2(1H)-one (200 mg) in methanol (3 mL) was added 28% sodium methoxide methanol solution (193 mg). The reaction mixture was stirred at 0°C for 1 hr, N,N-dimethylacetamide (3 mL) was added, and the reaction mixture was stirred at 60°C for 4 hr. The reaction mixture was concentrated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and collected by HPLC (C18, mobile phase: water/acetonitrile (0.1% TFA-containing system)). To the obtained fraction was added saturated aqueous sodium hydrogen carbonate solution, and the mixture was extracted with ethyl acetate, dried over anhydrous magnesium sulfate and concentrated under reduced pressure. The residue was recrystallized from ethanol to give the title compound (14 mg).
MS (ESI+): [M+H]⁺396.1.

### Example 320

### 5-cyclopropyl-3-(5-((3-fluoropyridin-2-yl)methoxy)-2-methylphenyl)-3,4-dihydropyrido[4,3-d]pyrimidin-2(1H)-one

Using N-(2-cyclopropyl-3-formylpyridin-4-yl)-2,2-dimethylpropanamide as a starting material and by a method similar to that in Example 1, step G to H, the title compound was obtained.
MS (ESI+): [M+H]⁺405.4.

### Example 321

### 3-(5-((3-fluoropyridin-2-yl)methoxy)-2-methylphenyl)-2-oxo-1,2,3,4-tetrahydropyrido[4,3-d]pyrimidine-5-carbonitrile

By a method similar to that in Example 278, the title compound was obtained.
MS (ESI+): [M+H]⁺390.4.

### Example 322

### 3-(5-((3-fluoropyridin-2-yl)methoxy)-2-methylphenyl)-2-oxo-1,2,3,4-tetrahydropyrido[4,3-d]pyrimidine-5-carboxamide

In the operation of Example 321, the title compound was obtained as a byproduct.
MS (ESI+): [M+H]⁺408.3.

### Example 323

### 3-(5-((3-fluoropyridin-2-yl)methoxy)-2-methylphenyl)-5-methyl-3,4-dihydropyrido[4,3-d]pyrimidin-2(1H)-one

Using N-(3-formyl-2-methylpyridin-4-yl)-2,2-dimethylpropanamide as a starting material and by a method similar to that in Example 1, step G to H, the title compound was obtained.
MS (ESI+) : [M+H]⁺379.3.

### Example 324

### 3-(4-((2,6-difluorobenzyl)oxy)pyridin-2-yl)quinazoline-2,4(1H,3H)-dione

### A) N-(4-chloropyridin-2-yl)-2-nitrobenzamide

A mixture of 2-nitrobenzoyl chloride (1.03 mL), 4-chloropyridin-2-amine (1.00 g), triethylamine (1.30 mL), and THF (10 mL) was stirred at room temperature for 2 hr. To the reaction mixture were added ethyl acetate and water, and the mixture was extracted with ethyl acetate. The obtained organic layer was washed with saturated brine, dried over anhydrous magnesium sulfate, and the solvent was evaporated under reduced pressure. The obtained residue was dissolved in a mixture of THF (10 mL), ethanol (10 mL), and 4N aqueous sodium hydroxide solution (10 mL), and the obtained mixture was stirred at room temperature for 15 hr. The reaction mixture was extracted with ethyl acetate, the obtained organic layer was washed with saturated brine, dried over anhydrous magnesium sulfate, and the solvent was evaporated under reduced pressure. The residue was washed with diethyl ether to give the title compound (0.75 g).
¹H NMR (300 MHz, DMSO-d₆) δ 7.34 (1H, dd, J = 5.3, 1.9 Hz), 7.70-7.81 (2H, m), 7.82-7.93 (1H, m), 8.11-8.20 (1H, m), 8.25 (1H, s), 8.36 (1H, d, J = 5.3 Hz), 11.51 (1H, s).

### B) 3-(4-chloropyridin-2-yl)quinazoline-2,4(1H,3H)-dione

A mixture of N-(4-chloropyridin-2-yl)-2-nitrobenzamide (750 mg), reduced iron (760 mg), ammonium chloride (145 mg), ethanol (10 mL), and water (10 mL) was heated under reflux for 5 hr. The precipitate was removed by filtration, and the filtrate was extracted with ethyl acetate. The obtained organic layer was washed with saturated brine, dried over anhydrous magnesium sulfate, and the solvent was evaporated under reduced pressure. The obtained residue was dissolved in THF (10 mL), CDI (527 mg) was added, and the obtained mixture was heated under reflux for 15 hr. The reaction mixture was concentrated under reduced pressure, and the obtained residue was washed with diethyl ether to give the title compound (285 mg).
¹H NMR (300 MHz, DMSO-d₆) δ 7.17-7.35 (2H, m), 7.60-7.85 (3H, m), 7.95 (1H, d, J = 7.9 Hz), 8.61 (1H, d, J = 5.7 Hz), 11.71 (1H, brs).

### C) 3-(4-((2,6-difluorobenzyl)oxy)pyridin-2-yl)quinazoline-2,4(1H,3H)-dione

A mixture of (2,6-difluorophenyl)methanol (1 mL) and sodium hydride (60%, oil) (34 mg) was stirred at room temperature for 30 min. 3-(4-Chloropyridin-2-yl)quinazoline-2,4(1H,3H)-dione (115 mg) was added, and the obtained mixture was stirred at 150°C for 4 hr. The reaction mixture was purified by silica gel column chromatography (hexane/ethyl acetate) to give the title compound (31 mg).
¹H NMR (300 MHz, DMSO-d₆) δ 5.24 (2H, s), 7.14-7.31 (6H, m), 7.51-7.65 (1H, m),7.66-7.79 (1H, m), 7.87-8.06 (1H, m), 8.45 (1H, d, J = 5.7 Hz), 11.65 (1H, s).

### Example 325

### 3-(3-((2,6-difluorobenzyl)oxy)phenyl)-1H-pyrrolo[3,2-d]pyrimidine-2,4(3H,5H)-dione

Under an argon atmosphere, to a mixture of ethyl 3-amino-1H-pyrrole-2-carboxylate (500 mg), triethylamine (1.35 mL), and toluene (15 mL) was added triphosgene (337 mg) at -78°C, and the obtained mixture was stirred at -78°C for 30 min and then at room temperature for 3 hr. To the reaction mixture were added 3-((2,6-difluorobenzyl)oxy)aniline (762 mg) and triethylamine (0.677 mL), and the obtained mixture was stirred at room temperature overnight. To the reaction mixture were added ethyl acetate and 1N hydrochloric acid, and the mixture was extracted with ethyl acetate. The obtained organic layer was washed with saturated brine, dried over anhydrous magnesium sulfate, and the solvent was evaporated under reduced pressure. The obtained residue was mixed with 20% sodium ethoxide ethanol solution (2 mL) and ethanol (20 mL), and the obtained mixture was heated under reflux for 1 hr. To the reaction mixture were added ethyl acetate and 1N hydrochloric acid, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate/methanol) and washed with ethyl acetate to give the title compound (359 mg).
¹H NMR (300 MHz, DMSO-d₆) δ 5.09 (2H, s), 5.81-5.97 (1H, m), 6.87 (1H, d, J = 8.1 Hz), 6.93-7.00 (1H, m), 7.08 (1H, dd, J = 8.1, 2.7 Hz), 7.14-7.28 (3H, m), 7.33-7.44 (1H, m), 7.46-7.64 (1H, m), 11.20 (1H, s), 11.99 (1H, brs).

### Example 326

### 3-(2-(2,6-difluorophenyl)-3,4-dihydro-2H-chromen-7-yl)quinazoline-2,4(1H,3H)-dione

By a method similar to that in Example 325, the title compound was obtained.
¹H NMR (400 MHz, DMSO-d₆) δ 2.13-2.24 (1H, m), 2.33-2.47 (1H, m), 2.89 (1H, dd, J = 16.6, 3.7 Hz), 3.10 (1H, ddd), 5.42-5.50 (1H, m), 6.76 (1H, d, J = 1.7 Hz), 6.81 (1H, dd, J = 7.8, 2.0 Hz), 7.13-7.26 (5H, m), 7.46-7.56 (1H, m), 7.65-7.73 (1H, m), 7.93 (1H, d, J = 7.1 Hz), 11.53 (1H, s).

### Example 327

### 3-(2-(3-fluoropyridin-2-yl)-6-methyl-3,4-dihydro-2H-chromen-7-yl)quinazoline-2,4(1H,3H)-dione

By a method similar to that in Example 325, the title compound was obtained.
MS (ESI+): [M+H]⁺404.3.

### Example 328

### 3-(2-(3-fluoropyridin-2-yl)-6-methyl-3,4-dihydro-2H-chromen-7-yl)-1-(2-methoxyethyl)quinazoline-2,4(1H,3H)-dione

By a method similar to that in Example 269, the title compound was obtained.
MS (ESI+): [M+H]⁺462.2.

### Example 329

### 3-(2-(2,6-difluorophenyl)-3,4-dihydro-2H-chromen-7-yl)-1-methylquinazoline-2,4(1H,3H)-dione

By a method similar to that in Example 269, the title compound was obtained.
¹H NMR (400 MHz, CDCl₃) δ 2.08-2.17 (1H, m), 2.52-2.70 (1H, m), 2.87-2.99 (1H, m), 3.09 (1H, ddd), 3.64 (3H, s), 5.45 (1H, dd), 6.75 (1H, d, J = 2.0 Hz), 6.79 (1H, dd, J = 8.1, 2.0 Hz), 6.85-6.96 (2H, m), 7.20-7.35 (4H, m), 7.68-7.77 (1H, m), 8.26 (1H, dd, J = 7.8, 1.2 Hz).

### Example 330

### 3-(3-((2,6-difluorobenzyl)oxy)phenyl)pyrido[3,4-d]pyrimidine-2,4(1H,3H)-dione

By a method similar to that in Example 325, the title compound was obtained.
¹H NMR (400 MHz, DMSO-d₆) δ 5.10 (2H, s), 6.97 (1H, d, J = 7.8 Hz), 7.04-7.10 (1H, m), 7.14 (1H, dd, J = 8.3, 2.4 Hz), 7.16-7.27 (2H, m), 7.44 (1H, t, J = 8.1 Hz), 7.55 (1H, quin), 7.81 (1H, d, J = 4. 9 Hz) , 8.44 (1H, d, J = 4.9 Hz), 8.64 (1H, s), 11.85 (1H, s).

### Example 331

### 3-(3-((2,6-difluorobenzyl)oxy)phenyl)-1-methylpyrido[3,4-d]pyrimidine-2,4(1H,3H)-dione

By a method similar to that in Example 269, the title compound was obtained.
¹H NMR (400 MHz, CDCl₃) δ 3.73 (3H, s), 5.12 (2H, s), 6.86-6.99 (4H, m), 7.08-7.16 (1H, m), 7.29-7.40 (1H, m), 7.46 (1H, t, J = 8.1 Hz), 8.06 (1H, d, J = 4.9 Hz), 8.61 (1H, d, J = 4.9 Hz), 8.82 (1H, s).

### Example 334

### 6-(5-((2,6-difluorobenzyl)oxy)-2-methylphenyl)-2-(morpholin-4-yl)[1,3]thiazolo[4,5-d]pyrimidine-5,7(4H,6H)-dione

By a method similar to that in Example 325, the title compound was obtained.
¹H NMR (300 MHz, DMSO-d₆) δ 1.96 (3H, s), 3.51-3.65 (4H, m), 3.66-3.80 (4H, m), 5.05 (2H, s), 6.93 (1H, d, J = 2.6 Hz), 7.00 (1H, dd, J = 8.3, 2.6 Hz), 7.09-7.31 (3H, m), 7.46-7.63 (1H, m), 12.32 (1H, s).

### Example 335

### methyl 3-(5-((2,6-difluorobenzyl)oxy)-2-methylphenyl)-2,4-dioxo-1,2,3,4-tetrahydroquinazoline-7-carboxylate

By a method similar to that in Example 325, the title compound was obtained.
MS (ESI+) : [M+H]⁺452.9.

### Example 336

### 3-(5-((2,6-difluorobenzyl)oxy)-2-methylphenyl)-7-(2-hydroxypropan-2-yl)quinazoline-2,4(1H,3H)-dione

By a method similar to that in Example 178, the title compound was obtained.
MS (ESI+): [M+H]⁺452.9.

### Example 337

### 3-(5-((3-fluoropyridin-2-yl)methoxy)-2-methylphenyl)quinazoline-2,4(1H,3H)-dione

By a method similar to that in Example 325, the title compound was obtained.
¹H NMR (300 MHz, DMSO-d₆) δ 1.97 (3H, s), 5.18 (2H, d, J = 1.9 Hz), 6.94-7.10 (2H, m), 7.17-7.33 (3H, m), 7.45-7.60 (1H, m), 7.63-7.86 (2H, m), 7.95 (1H, d, J = 8.3 Hz), 8.37-8.53 (1H, m), 11.60 (1H, s).

### Example 338

### 3-(5-((3-fluoropyridin-2-yl)methoxy)-2-methylphenyl)pyrido[3,2-d]pyrimidine-2,4(1H,3H)-dione

### A) 3-amino-N-(5-((3-fluoropyridin-2-yl)methoxy)-2-methylphenyl)pyridine-2-carboxamide

A mixture of 3-aminopyridine-2-carboxylic acid (297 mg), 5-((3-fluoropyridin-2-yl)methoxy)-2-methylaniline (500 mg), WSC (619 mg), HOBt (436 mg), triethylamine (450 µL) and DMF (5 mL) was stirred at room temperature overnight. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (349 mg).
¹H NMR (300 MHz, DMSO-d₆) δ 2.24 (3H, s), 5.20 (2H, d, J = 1.9 Hz), 6.77 (1H, dd, J = 8.5, 2.8 Hz), 6.94 (2H, brs), 7.09-7.41 (3H, m), 7.43-7.62 (1H, m), 7.69-7.98 (3H, m), 8.38-8.53 (1H, m), 10.19 (1H, s).

### B) 3-(5-((3-fluoropyridin-2-yl)methoxy)-2-methylphenyl)pyrido[3,2-d]pyrimidine-2,4(1H,3H)-dione

A mixture of 3-amino-N-(5-((3-fluoropyridin-2-yl)methoxy)-2-methylphenyl)pyridine-2-carboxamide (120 mg), CDI (83 mg), DBU (76 µL), and THF (2 mL) was stirred at 70°C for 2 hr. CDI (83 mg) and DBU (76 µL) were further added, and the obtained mixture was stirred at 70°C overnight. The reaction mixture was concentrated under reduced pressure and purified by silica gel column chromatography (ethyl acetate/hexane). The obtained residue was further purified by HPLC
(water/acetonitrile) to give the title compound (62.6 mg).
¹H NMR (300 MHz, DMSO-d₆) δ 1.99 (3H, s), 5.19 (2H, d, J = 1.9 Hz), 6.97-7.11 (2H, m), 7.28 (1H, d, J = 8.3 Hz), 7.48-7.59 (1H, m), 7.62-7.87 (3H, m), 8.40-8.49 (1H, m), 8.53 (1H, dd, J = 4.1, 1.5 Hz), 11.68 (1H, s).

### Example 339

### 3-(5-((3-fluoropyridin-2-yl)methoxy)-2-methylphenyl)pyrido[3,4-d]pyrimidine-2,4(1H,3H)-dione

By a method similar to that in Example 338, step A to B, the title compound was obtained.
MS (ESI+): [M+H]⁺379.3.

### Example 340

### 5-chloro-3-(5-((3-fluoropyridin-2-yl)methoxy)-2-methylphenyl)quinazoline-2,4(1H,3H)-dione

By a method similar to that in Example 338, step A to B, the title compound was obtained.
MS (ESI+): [M+H]⁺386.3.

### Example 341

### 3-(5-((3-fluoropyridin-2-yl)methoxy)-2-methylphenyl)-7-nitroquinazoline-2,4(1H,3H)-dione

By a method similar to that in Example 325, the title compound was obtained.
MS (ESI+): [M+H]⁺423.2.

### Example 342

### 6-chloro-3-(5-((3-fluoropyridin-2-yl)methoxy)-2-methylphenyl)pyrido[3,4-d]pyrimidine-2,4(1H,3H)-dione

### A) tert-butyl (6-chloro-4-((5-((3-fluoropyridin-2-yl)methoxy)-2-methylphenyl)carbamoyl)pyridin-3-yl)carbamate

To a solution of 5-((tert-butoxycarbonyl)amino)-2-chloroisonicotinic acid (0.4 g), 5-((3-fluoropyridin-2-yl)methoxy)-2-methylaniline (0.3 g), WSC (0.4 g) and triethylamine (0.2 mL) in DMF (6 mL) was added HOBt (0.2 g), and the mixture was stirred at room temperature for 16 hr. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate to give the title compound (0.5 g).
MS (ESI+): [M+H]⁺487.1.

### B) 6-chloro-3-(5-((3-fluoropyridin-2-yl)methoxy)-2-methylphenyl)pyrido[3,4-d]pyrimidine-2,4(1H,3H)-dione

tert-Butyl (6-chloro-4-((5-((3-fluoropyridin-2-yl)methoxy)-2-methylphenyl)carbamoyl)pyridin-3-yl)carbamate (0.5 g) was dissolved in 4N hydrogen chloride ethyl acetate solution (2.4 mL), and ethanol (1 mL) and THF (1 mL) were added. The reaction mixture was stirred at room temperature for 4 hr. 1N Aqueous sodium hydroxide solution was added, and the mixture was extracted with ethyl acetate. The extract was washed with saturated brine, and dried over magnesium sulfate. The solvent was evaporated under reduced pressure. The residue was dissolved in THF (7 mL), CDI (0.5 g) and DBU (0.4 mL) were added at room temperature. The reaction mixture was stirred at room temperature for 12 hr. The solvent was evaporated under reduced pressure, water was added, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) and recrystallized from ethyl acetate/hexane to give the title compound (0.1 g).
MS (ESI+): [M+H]⁺413.2.

### Example 343

### 3-(5-((3-fluoropyridin-2-yl)methoxy)-2-methylphenyl)pyrido[2,3-d]pyrimidine-2,4(1H,3H)-dione

By a method similar to that in Example 338, the title compound was obtained.
MS (ESI+): [M+H]⁺379.3.

### Example 344

### 3-(2-chloro-5-((3-fluoropyridin-2-yl)methoxy)phenyl)quinazoline-2,4(1H,3H)-dione

By a method similar to that in Example 325, the title compound was obtained.
MS (ESI+): [M+H]⁺398.0.

### Example 345

### 3-(2-fluoro-5-((3-fluoropyridin-2-yl)methoxy)phenyl)quinazoline-2,4(1H,3H)-dione

By a method similar to that in Example 325, the title compound was obtained.
MS (ESI+): [M+H]⁺382.1.

### Example 346

### 3-(3-((3-fluoropyridin-2-yl)methoxy)phenyl)quinazoline-2,4(1H,3H)-dione

By a method similar to that in Example 325, the title compound was obtained.
MS (ESI+) : [M+H]⁺364.1.

### Example 347

### 8-chloro-3-(5-((3-fluoropyridin-2-yl)methoxy)-2-methylphenyl)quinazoline-2,4(1H,3H)-dione

By a method similar to that in Example 325, the title compound was obtained.
MS (ESI+): [M+H]⁺412.1.

### Example 348

### 7-amino-3-(5-((3-fluoropyridin-2-yl)methoxy)-2-methylphenyl)quinazoline-2,4(1H,3H)-dione

By a method similar to that in Example 245, step B, the title compound was obtained.
MS (ESI+) : [M+H]⁺393.4.

### Example 349

### 7-(cyclopropylmethoxy)-3-(5-((3-fluoropyridin-2-yl)methoxy)-2-methylphenyl)quinazoline-2,4(1H,3H)-dione

By a method similar to that in Example 325, the title compound was obtained.
MS (ESI+): [M+H]⁺448.1.

### Example 350

### N-(3-(5-((3-fluoropyridin-2-yl)methoxy)-2-methylphenyl)-2,4-dioxo-1,2,3,4-tetrahydroquinazolin-7-yl)cyclopropanecarboxamide

By a method similar to that in Example 74, the title compound was obtained.
MS (ESI+): [M+H]⁺461.2.

### Example 351

### 7-chloro-3-(5-((3-fluoropyridin-2-yl)methoxy)-2-methylphenyl)quinazoline-2,4(1H,3H)-dione

By a method similar to that in Example 325, the title compound was obtained.
MS (ESI+): [M+H]⁺412.1.

### Example 352

### 3-(5-((3-fluoropyridin-2-yl)methoxy)-2-methylphenyl)-7-(2-methoxyethoxy)-1-(2-methoxyethyl)quinazoline-2,4(1H,3H)-dione

By a method similar to that in Example 328, the title compound was obtained.
MS (ESI+): [M+H]⁺510.2.

### Example 353

### 8-(cyclopropylmethoxy)-3-(5-((3-fluoropyridin-2-yl)methoxy)-2-methylphenyl)quinazoline-2,4(1H,3H)-dione

By a method similar to that in Example 325, the title compound was obtained.
MS (ESI+): [M+H]⁺448.1.

### Example 354

### 3-(5-((3-fluoropyridin-2-yl)methoxy)-2-methylphenyl)-8-(2-methoxyethoxy)quinazoline-2,4(1H,3H)-dione

By a method similar to that in Example 325, the title compound was obtained.
MS (ESI+): [M+H]⁺452.1.

### Example 355

### 6-chloro-3-(5-((3-fluoropyridin-2-yl)methoxy)-2-methylphenyl)quinazoline-2,4(1H,3H)-dione

By a method similar to that in Example 325, the title compound was obtained.
MS (ESI+) : [M+H]⁺412.1.

### Example 356

### 3-(5-((3-fluoropyridin-2-yl)methoxy)-2-methylphenyl)-7-(2-methoxyethoxy)quinazoline-2,4(1H,3H)-dione

By a method similar to that in Example 325, the title compound was obtained.
MS (ESI+): [M+H]⁺452.2.

### Example 357

### 3-(5-((3-fluoropyridin-2-yl)methoxy)-2-methylphenyl)-7-(trifluoromethyl)quinazoline-2,4(1H,3H)-dione

By a method similar to that in Example 325, the title compound was obtained.
MS (ESI+) : [M+H]⁺446.3.

### Example 358

### 6-(2-bromo-5-((3-fluoropyridin-2-yl)methoxy)phenyl)-2-(cyclopropylmethyl)-2H-pyrazolo[4,3-d]pyrimidine-5,7(4H,6H)-dione

By a method similar to that in Example 325, the title compound was obtained.
MS (ESI+): [M+H]⁺486.1.

### Example 359

### 5-fluoro-3-(5-((3-fluoropyridin-2-yl)methoxy)-2-methylphenyl)quinazoline-2,4(1H,3H)-dione

By a method similar to that in Example 338, the title compound was obtained.
MS (ESI+) : [M+H]⁺396.2.

### Example 360

### 6-(2-bromo-5-((3-fluoropyridin-2-yl)methoxy)phenyl)-2-(cyclopropylmethyl)-2,4,6,7-tetrahydro-5H-pyrazolo[4,3-d]pyrimidin-5-one

By a method similar to that in Example 325, the title compound was obtained.
MS (ESI+): [M+H]⁺472.1.

### Example 361

### 3-(5-((3-fluoropyridin-2-yl)methoxy)-2-methylphenyl)-2,4-dioxo-1,2,3,4-tetrahydroquinazoline-7-carboxylic acid

By a method similar to that in Example 71, the title compound was obtained.
MS (ESI+): [M+H]⁺420.2.

### Example 362

### N-(3-(5-((3-fluoropyridin-2-yl)methoxy)-2-methylphenyl)-2,4-dioxo-1,2,3,4-tetrahydroquinazolin-7-yl)cyclopropanesulfonamide

By a method similar to that in Example 74, the title compound was obtained.
MS (ESI+) : [M+H]⁺497.3.

### Example 363

### N-cyclopropyl-3-(5-((3-fluoropyridin-2-yl)methoxy)-2-methylphenyl)-2,4-dioxo-1,2,3,4-tetrahydroquinazoline-7-carboxamide

By a method similar to that in Example 74, the title compound was obtained.
MS (ESI+): [M+H]⁺461.3.

### Example 364

### 5-chloro-3-(2-chloro-4-fluoro-5-((3-fluoropyridin-2-yl)methoxy)phenyl)quinazoline-2,4(1H,3H)-dione

### A) 2-chloro-N-(2-chloro-4-fluoro-5-((3-fluoropyridin-2-yl)methoxy)phenyl)-6-nitrobenzamide

A mixture of 2-chloro-6-nitrobenzoic acid (500 mg), oxalyl chloride (0.319 mL), DMF (3 drops), and THF (5 mL) was stirred at 0°C for 1 hr. The reaction mixture was concentrated under reduced pressure. To the obtained residue were added N,N-dimethylacetamide (5 mL) and 2-chloro-4-fluoro-5-((3-fluoropyridin-2-yl)methoxy)aniline (671 mg) at 0°C, and the obtained mixture was stirred at room temperature overnight. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (345 mg).
¹H NMR (300 MHz, DMSO-d₆) δ 5.34 (2H, d, J = 1.9 Hz), 7.45-7.64 (2H, m), 7.65-7.88 (3H, m), 8.00-8.09 (1H, m), 8.20-8.33 (1H, m), 8.38-8.54 (1H, m), 10.46 (1H, s).

### B) 5-chloro-3-(2-chloro-4-fluoro-5-((3-fluoropyridin-2-yl)methoxy)phenyl)quinazoline-2,4(1H,3H)-dione

By a method similar to that in Example 324, step B, the title compound was obtained.
¹H NMR (300 MHz, DMSO-d₆) δ 5.26 (2H, d, J = 1.9 Hz), 7.18-7.35 (2H, m), 7.50-7.75 (4H, m), 7.77-7.89 (1H, m), 8.37-8.52 (1H, m), 11.88 (1H, s).

### Example 365

### 3-(5-((3-fluoropyridin-2-yl)methoxy)-2-methylphenyl)-5-methoxyquinazoline-2,4(1H,3H)-dione

By a method similar to that in Example 338, the title compound was obtained.
1H NMR (300 MHz, DMSO-d₆) δ 1.95 (3H, s), 3.81 (3H, s), 5.17 (2H, d, J = 1.89 Hz), 6.78 (2H, d, J = 8.0 Hz), 6.93 (1H, d, J = 2.7 Hz), 7.02 (1H, d, J = 2.7 Hz), 7.24 (1H, d, J = 8.7 Hz), 7.46 - 7.69 (2H, m), 7.80 (1H, d, J = 9.3 Hz), 8.46 (1H, d, J = 4.9 Hz), 11.42 (1H, s).

### Example 366

### 3-(5-((3-fluoropyridin-2-yl)methoxy)-2-methylphenyl)-5-(2-methoxyethoxy)quinazoline-2,4(1H,3H)-dione

### A) methyl 2-fluoro-6-nitrobenzoate

To a mixture of 2-fluoro-6-nitrobenzoic acid (5.75 g), methanol (100 mL) and toluene (50 mL) was added dropwise (diazomethyl)(trimethyl)silane (19 mL) at room temperature and the obtained mixture was stirred at room temperature for 4 days. The reaction mixture was concentrated under reduced pressure, and the obtained residue was purified by silica gel column chromatography (hexane/ethyl acetate) to give the title compound (2.59 g).
¹H NMR (300 MHz, DMSO-d₆) δ 3.92 (3H, s), 7.79-7.99 (2H, m), 8.12 (1H, dd, J = 6.8, 2.6 Hz).

### B) N-(5-((3-fluoropyridin-2-yl)methoxy)-2-methylphenyl)-2-(2-methoxyethoxy)-6-nitrobenzamide

A mixture of methyl 2-fluoro-6-nitrobenzoate (1.29 g), 2-methoxyethanol (5 mL), potassium carbonate (985 mg), and DMF (5 mL) was stirred at 80°C overnight. Water was added to the reaction mixture, and the mixture was extracted with ethyl acetate. The obtained organic layer was washed with saturated brine, dried over anhydrous magnesium sulfate, and the solvent was evaporated under reduced pressure. The obtained residue was mixed with THF (15 mL), methanol (15 mL), and 1N aqueous sodium hydroxide solution (15 mL), and the obtained mixture was stirred at 70°C overnight. The reaction mixture was acidified with 1N hydrochloric acid, concentrated under reduced pressure, and extracted with ethyl acetate. The obtained organic layer was washed with saturated brine, dried over anhydrous magnesium sulfate, and the solvent was evaporated under reduced pressure. The obtained mixture residue, 5-((3-fluoropyridin-2-yl)methoxy)-2-methylaniline (910 mg), WSC (1.13 g), HOBt (795 mg), triethylamine (820 µL), and DMF (15 mL) were stirred at room temperature overnight. Water was added to the reaction mixture, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (220 mg).
¹H NMR (300 MHz, DMSO-d₆) δ 2.20 (3H, s), 3.25 (3H, s), 3.60-3.73 (2H, m), 4.20-4.32 (2H, m), 5.19 (2H, d, J = 1.9 Hz), 6.79-6.93 (1H, m), 7.04-7.23 (2H, m), 7.46-7.69 (3H, m), 7.71-7.86 (2H, m), 8.39-8.52 (1H, m), 9.90 (1H, s).

### C) 3-(5-((3-fluoropyridin-2-yl)methoxy)-2-methylphenyl)-5-(2-methoxyethoxy)quinazoline-2,4(1H,3H)-dione

Under a hydrogen atmosphere, a mixture of N-(5-((3-fluoropyridin-2-yl)methoxy)-2-methylphenyl)-2-(2-methoxyethoxy)-6-nitrobenzamide (220 mg), palladium/carbon (22 mg), methanol (10 mL), and THF (10 mL) was stirred at room temperature for 3 hr. The precipitate was removed by filtration, and the filtrate was concentrated under reduced pressure. The obtained residue was mixed with CDI (156 mg), DBU (143 µL), and THF (5 mL), and the obtained mixture was stirred at 70°C for 3 hr. To the reaction mixture was added 1N hydrochloric acid, and the mixture was extracted with ethyl acetate. The obtained organic layer was washed with saturated brine, dried over anhydrous magnesium sulfate, and the solvent was evaporated under reduced pressure. The residue was purified by silica gel column chromatography (hexane/ethyl acetate) to give the title compound (18 mg).
MS (ESI+) : [M+H]⁺452.0.

### Example 367

### 5-(cyclopropylmethoxy)-3-(5-((3-fluoropyridin-2-yl)methoxy)-2-methylphenyl)quinazoline-2,4(1H,3H)-dione

By a method similar to that in Example 366, step B to C, the title compound was obtained.
MS (ESI+) : [M+H]⁺448.1.

### Example 368

### 6-bromo-5-chloro-3-(2-chloro-5-((3-fluoropyridin-2-yl)methoxy)phenyl)quinazoline-2,4(1H,3H)-dione

By a method similar to that in Example 324, the title compound was obtained.
MS (ESI+) : [M+H]⁺511.9.

### Example 369

### 3-(5-((3-fluoropyridin-2-yl)methoxy)-2-methylphenyl)pyrido[4,3-d]pyrimidine-2,4(1H,3H)-dione

By a method similar to that in Example 338, the title compound was obtained.
MS (ESI+): [M+H]⁺379.0.

### Example 370

### 3-(5-((3-fluoropyridin-2-yl)methoxy)-2-methylphenyl)-5-(trifluoromethyl)quinazoline-2,4(1H,3H)-dione

By a method similar to that in Example 338, the title compound was obtained.
MS (ESI+): [M+H]⁺446.3.

### Example 371

### 3-(5-((3-bromopyridin-2-yl)methoxy)-2-methylphenyl)quinazoline-2,4(1H,3H)-dione

By a method similar to that in Example 338, the title compound was obtained.
MS (ESI+) : [M+H]⁺438.2.

### Example 372

### 3-(6-chloro-2-(3-fluoropyridin-2-yl)-3,4-dihydro-2H-chromen-7-yl)quinazoline-2,4(1H,3H)-dione

By a method similar to that in Example 325, the title compound was obtained.
MS (ESI+): [M+H]⁺424.1.

### Example 373

### 5-chloro-3-((2R)-6-chloro-2-(3-fluoropyridin-2-yl)-3,4-dihydro-2H-chromen-7-yl)-8-methyl-3,4-dihydropyrido[3,4-d]pyrimidin-2(1H)-one

### A) (2R)-6-chloro-2-(3-fluoropyridin-2-yl)chromane-7-amine

Racemate (1000 mg) of 6-chloro-2-(3-fluoropyridin-2-yl)chromane-7-amine was separated by HPLC (column: CHIRALPAK AD (LF001), 50 mmID×500 mmL, Daicel chemical industries Inc., mobile phase: hexane/2-propanol =500/500) to give the title compound (893 mg) with a shorter retention time.
MS (ESI+) : [M+H]⁺279.2.

### B) 5-chloro-3-((2R)-6-chloro-2-(3-fluoropyridin-2-yl)-3,4-dihydro-2H-chromen-7-yl)-8-methyl-3,4-dihydropyrido[3,4-d]pyrimidin-2(1H)-one

To a solution of 3-amino-5-chloro-2-methylisonicotinaldehyde (390 mg) and (2R)-6-chloro-2-(3-fluoropyridin-2-yl)chromane-7-amine (490 mg) in toluene (15 mL) was added p-toluenesulfonic acid monohydrate (17 mg), and the mixture was stirred at 70°C for 2 hr. After cooling to room temperature, ethyl acetate and saturated aqueous sodium hydrogen carbonate solution were added and the mixture was extracted. The extract was washed with saturated brine, and dried over magnesium sulfate. The solvent was evaporated under reduced pressure. To a suspension of lithium aluminum hydride (133 mg) in THF (10 mL) was added dropwise a solution of the residue in THF (20 mL) at 0°C, and the mixture was stirred at 0°C for 1 hr. To the reaction mixture was added sodium sulfate decahydrate, and the insoluble material was filtered off with celite. The filtrate was concentrated under reduced pressure. The residue was dissolved in THF (45 mL), CDI (860 mg) and DBU (0.8 mL) were added, and the mixture was stirred at room temperature overnight. Water was added to the reaction mixture, and the mixture was extracted with ethyl acetate. The extract was washed with saturated brine, and dried over magnesium sulfate. The solvent was evaporated under reduced pressure and the residue was purified by silica gel column chromatography (ethyl acetate/hexane) to give the title compound (410 mg).
The optical purity and absolute configuration of the compound were determined by the analysis by HPLC using a chiral column and comparison with the compound of Example 8.
¹H NMR (300MHz, DMSO-d₆) δ 2.11-2.38 (2H, m), 2.42 (3H, s), 2.76-3.12 (2H, m), 4.56 (1H, brs), 4.76-4.99 (1H, m), 7.54 (1H, dt, J = 8.6, 4.2 Hz), 7.81 (1H, ddd, J = 10.3, 8.6, 1.1 Hz), 8.09 (1H, s), 8.48 (1H, d, J = 4.5 Hz), 9.35 (1H, brs).
MS (ESI+): [M+H]⁺459.1.

Example compounds produced according to the above-mentioned method or a method analogous thereto are shown in the following Tables.

**[Table 1-1]**

| Ex. No. | structure |
|---|---|
| 1 | 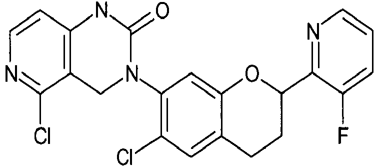 |
| 2 | 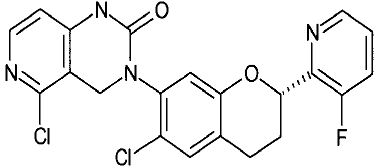 |
| 3 | 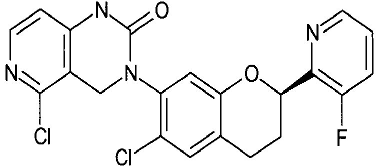 |
| 4 | 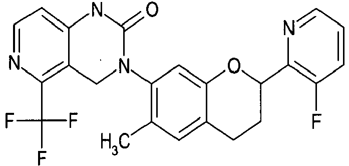 |
| 5 | 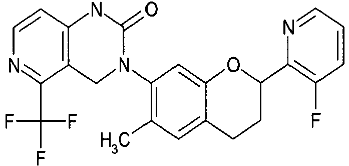 |
| 6 | 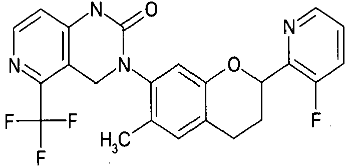 |
| 7 | 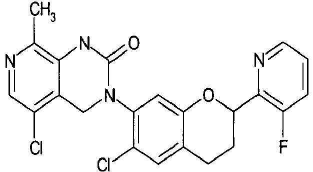 |
| 8 | 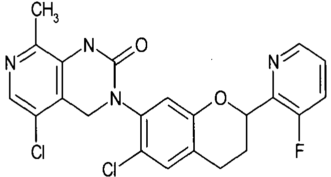 |
| 9 | 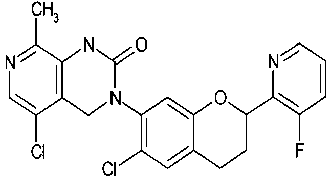 |
| 10 | 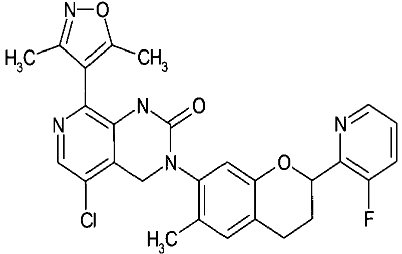 |
| 11 | 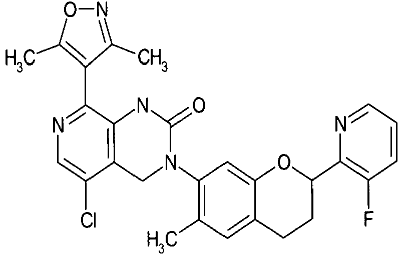 |
| 12 | 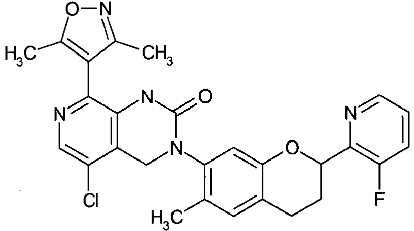 |
| 13 | 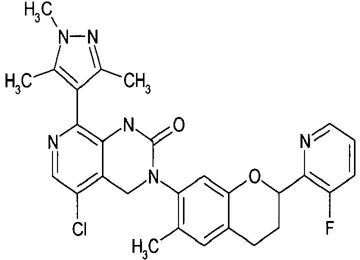 |
| 14 | 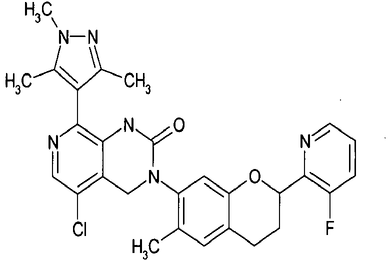 |
| 15 | 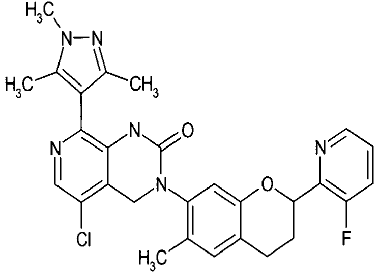 |

**[Table 1-2]**

| | |
|---|---|
| 16 | 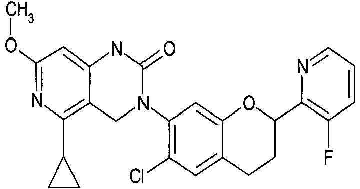 |
| 17 | 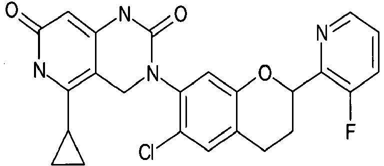 |
| 18 | 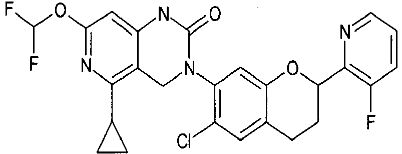 |
| 19 | 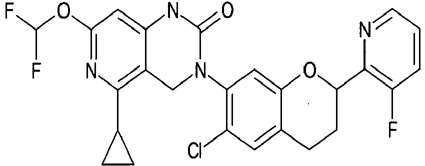 |
| 20 | 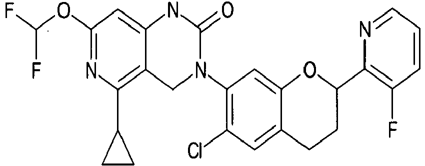 |
| 21 | 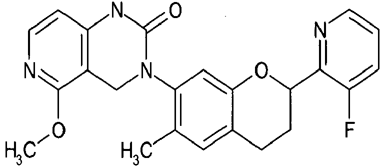 |
| 22 | 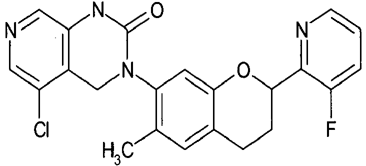 |
| 23 | 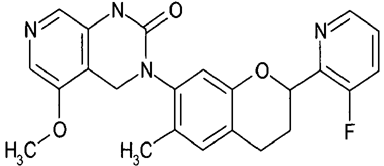 |
| 24 | 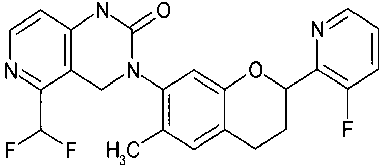 |
| 25 | 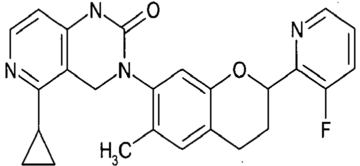 |
| 26 | 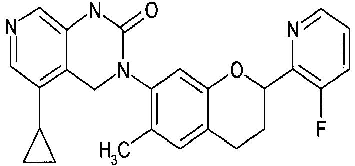 |
| 27 | 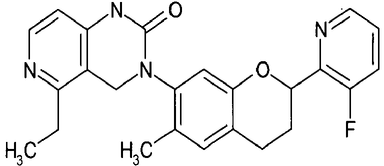 |
| 28 | 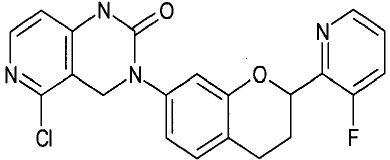 |
| 29 | 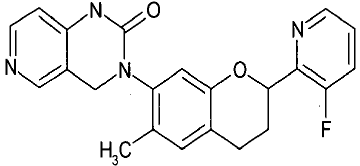 |
| 30 | 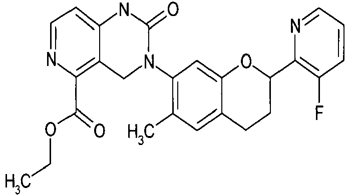 |
| 31 | 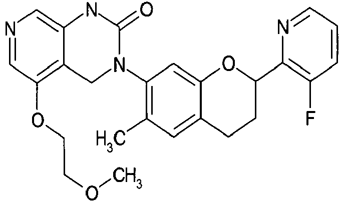 |

**[Table 1-3]**

| | |
|---|---|
| 32 | 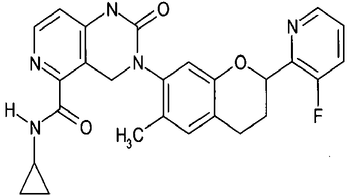 |
| 33 | 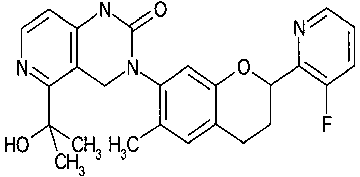 |
| 34 | 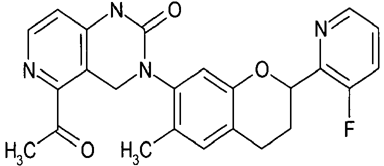 |
| 35 | 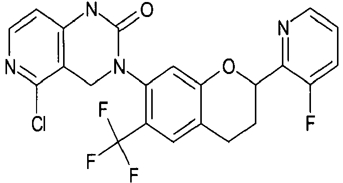 |
| 36 | 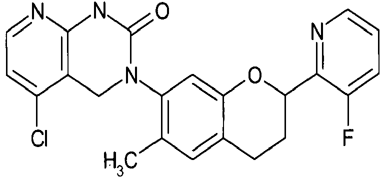 |
| 37 | 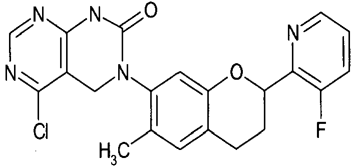 |
| 38 | 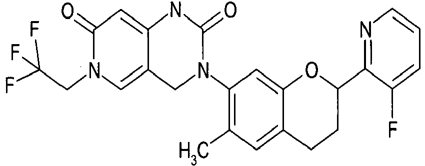 |
| 39 | 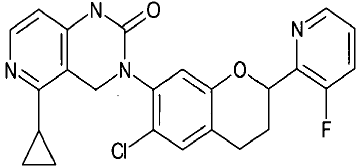 |
| 40 | 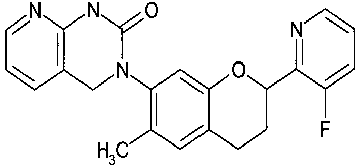 |
| 41 | 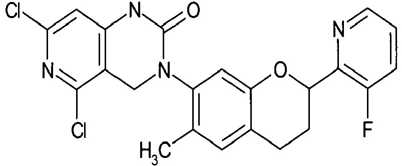 |
| 42 | 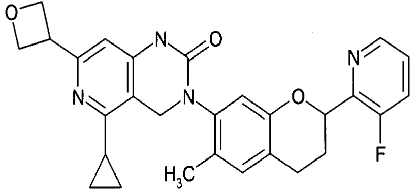 |
| 43 | 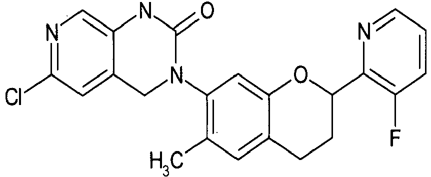 |
| 44 | 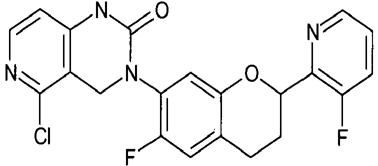 |
| 45 | 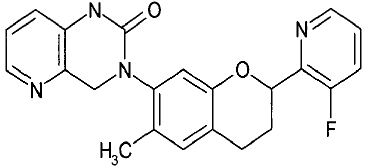 |
| 46 | 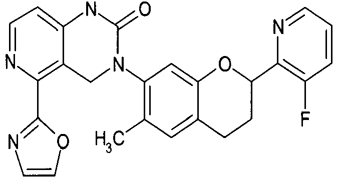 |
| 47 | 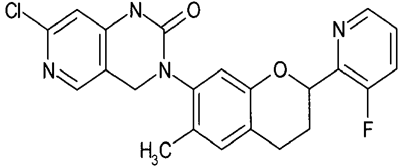 |

**[Table 1-4]**

| | |
|---|---|
| 48 | 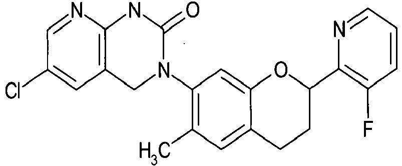 |
| 49 | 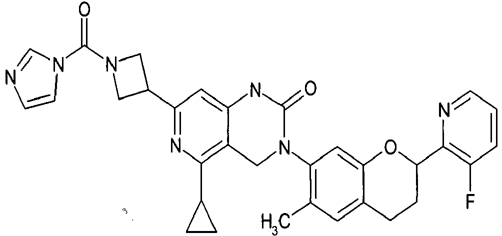 |
| 50 | 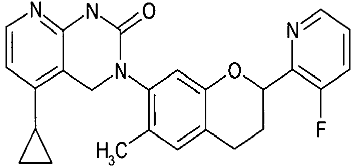 |
| 51 | 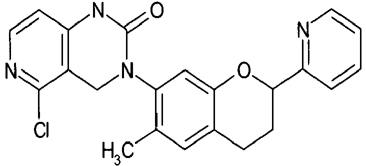 |
| 52 | 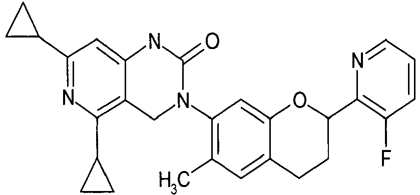 |
| 53 | 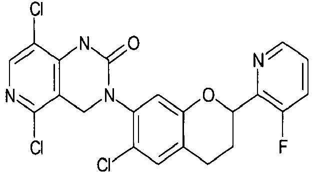 |
| 54 | 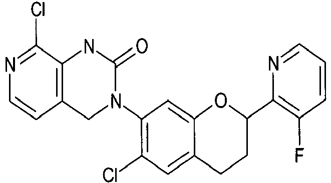 |
| 55 | 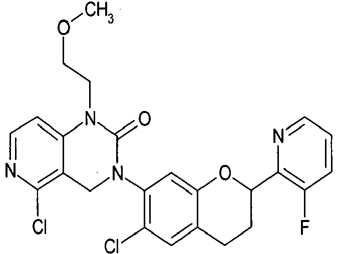 |
| 56 | 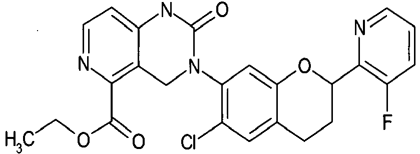 |
| 57 | 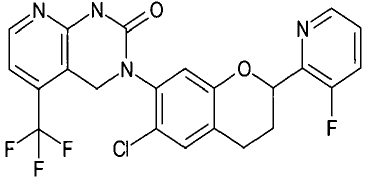 |
| 58 | 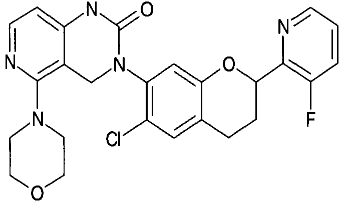 |
| 59 | 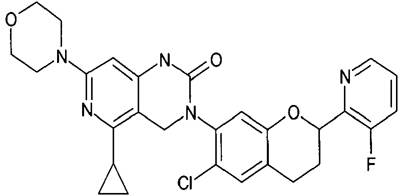 |
| 60 | 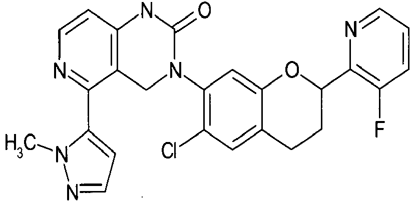 |
| 61 | 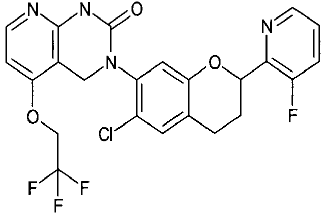 |
| 62 | 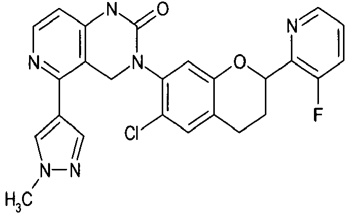 |
| 63 | 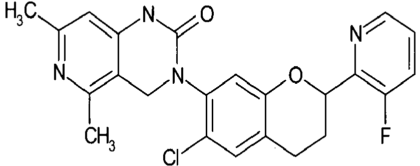 |

**[Table 1-5]**

| | |
|---|---|
| 64 | 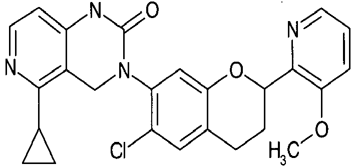 |
| 65 | 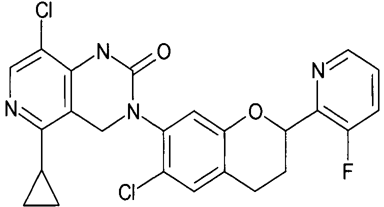 |
| 66 | 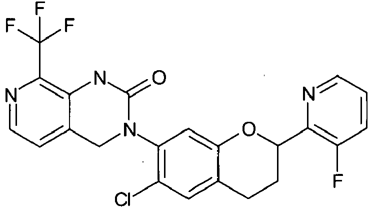 |
| 67 | 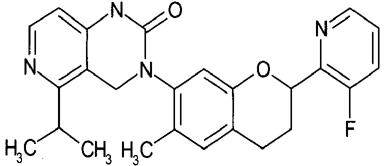 |
| 68 | 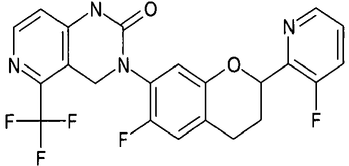 |
| 69 | 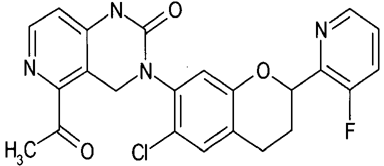 |
| 70 | 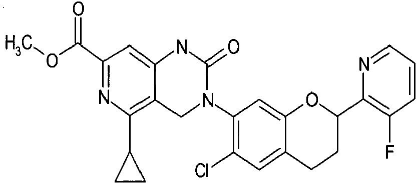 |
| 71 | 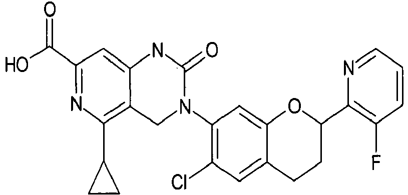 |
| 72 | 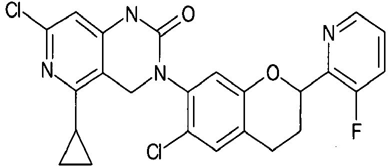 |
| 73 | 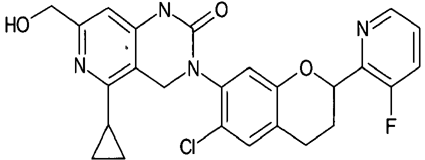 |
| 74 | 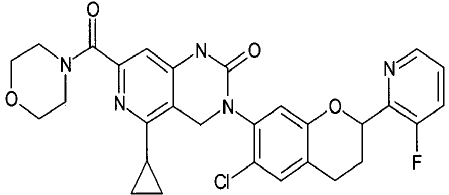 |
| 75 | 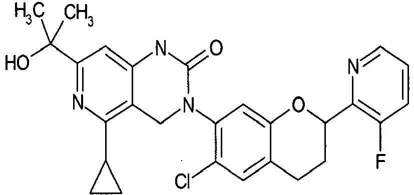 |
| 76 | 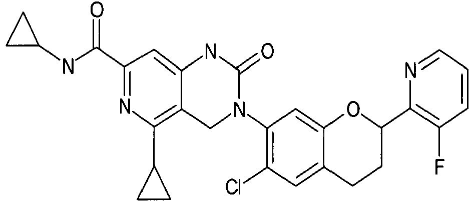 |
| 77 | 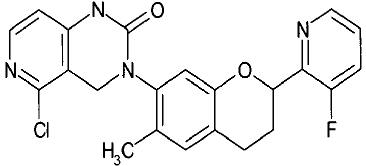 |
| 78 | 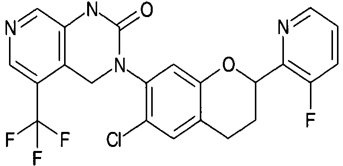 |
| 79 | 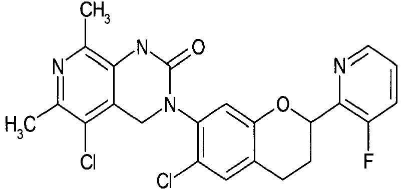 |

**[Table 1-6]**

| | |
|---|---|
| 80 | 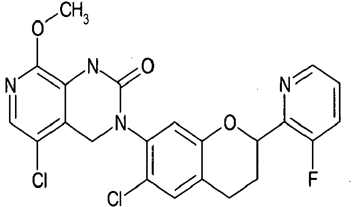 |
| 81 | 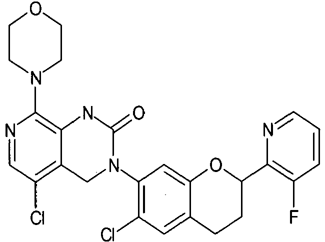 |
| 82 | 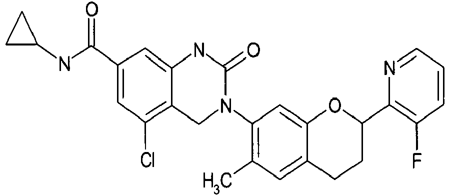 |
| 83 | 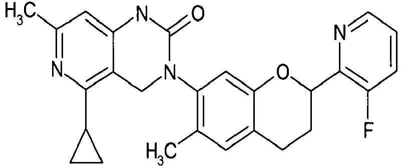 |
| 84 | 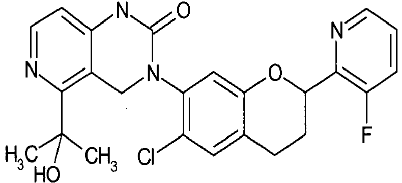 |
| 85 | 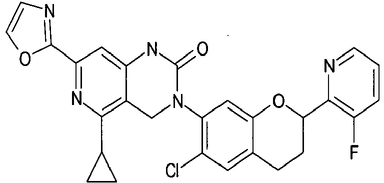 |
| 86 | 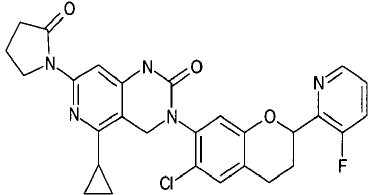 |
| 87 | 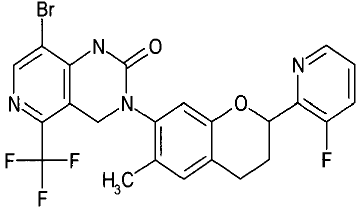 |
| 88 | 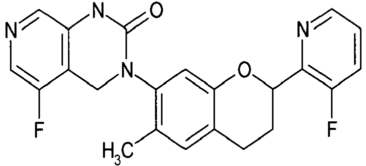 |
| 89 | 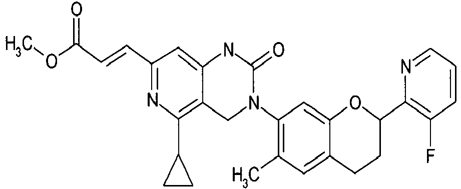 |
| 90 | 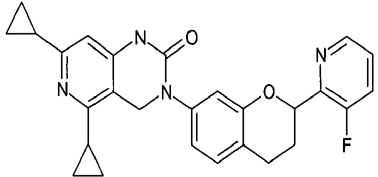 |
| 91 | 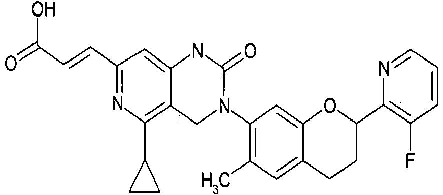 |
| 92 | 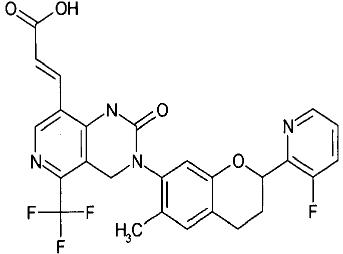 |
| 93 | 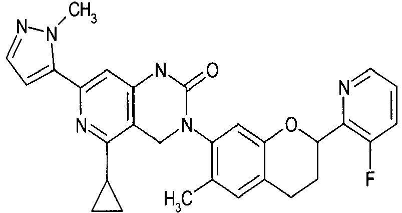 |
| 94 | 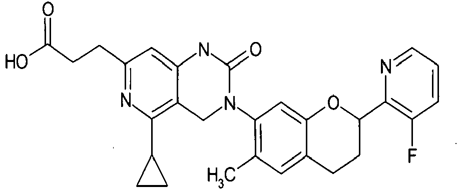 |
| 95 | 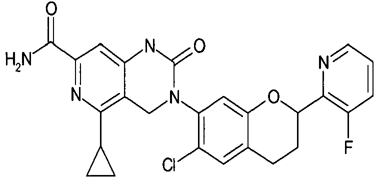 |

**[Table 1-7]**

| | |
|---|---|
| 96 | 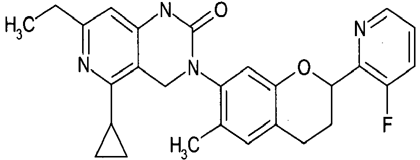 |
| 97 | 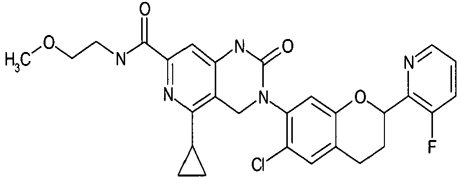 |
| 98 | 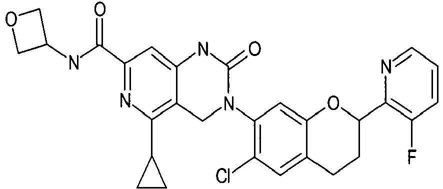 |
| 99 | 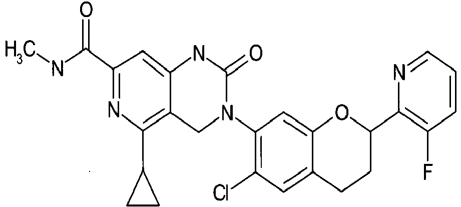 |
| 100 | 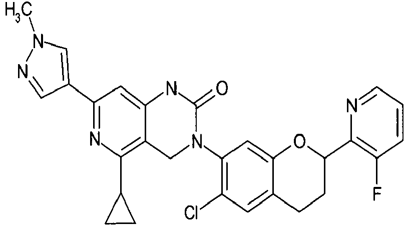 |
| 101 | 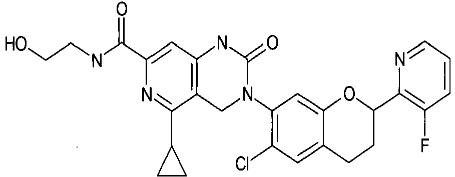 |
| 102 | 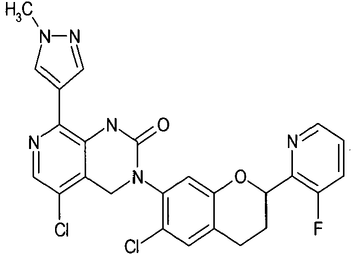 |
| 103 | 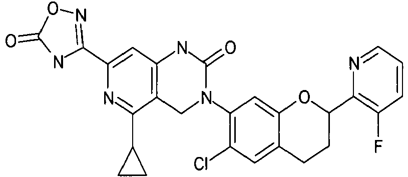 |
| 104 | 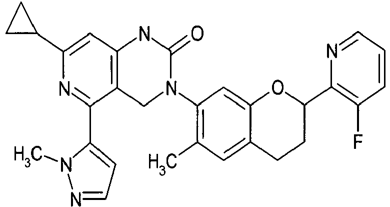 |
| 105 | 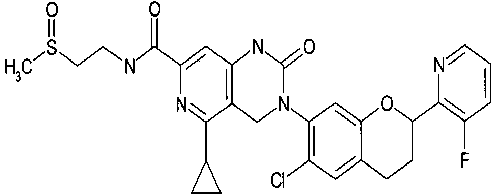 |
| 106 | 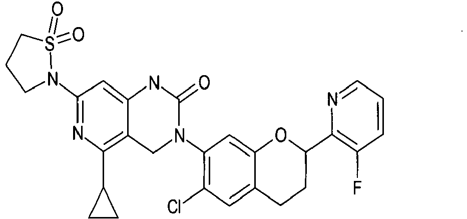 |
| 107 | 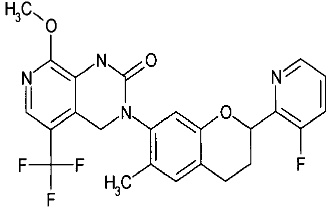 |
| 108 | 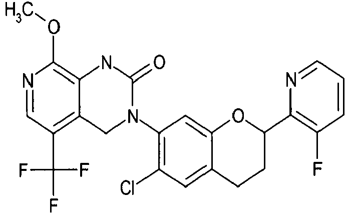 |
| 109 | 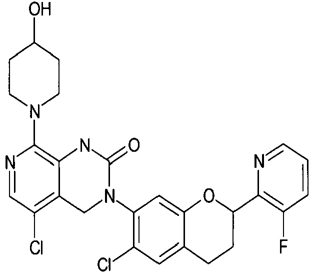 |
| 110 | 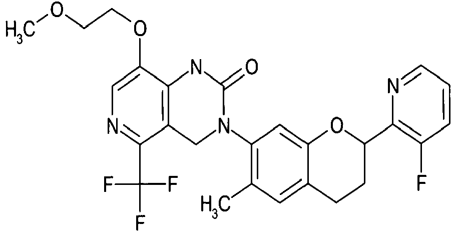 |
| 111 | 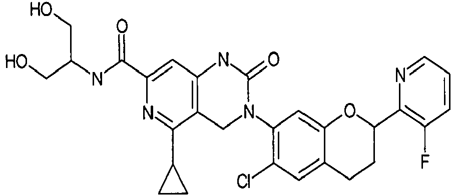 |

**[Table 1-8]**

| | |
|---|---|
| 112 | 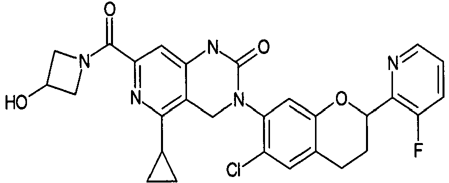 |
| 113 | 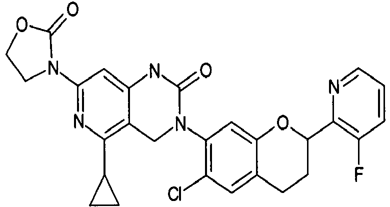 |
| 114 | 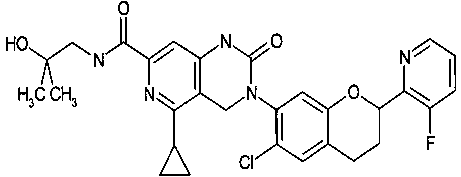 |
| 115 | 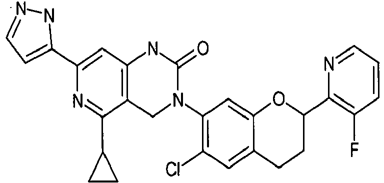 |
| 116 | 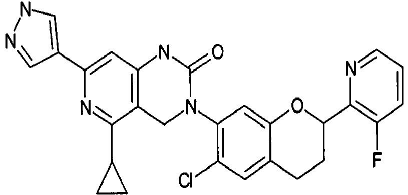 |
| 117 | 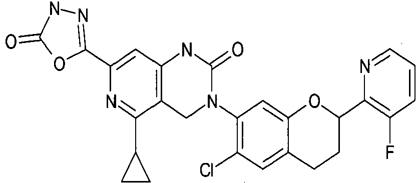 |
| 118 | 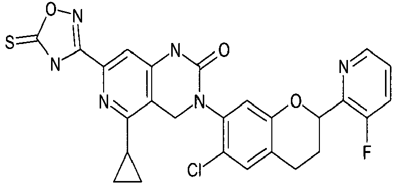 |
| 119 | 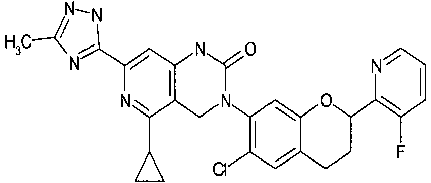 |
| 120 | 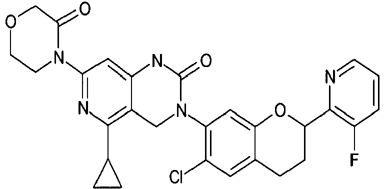 |
| 121 | 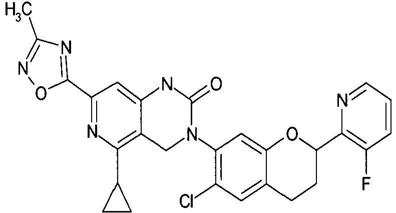 |
| 122 | 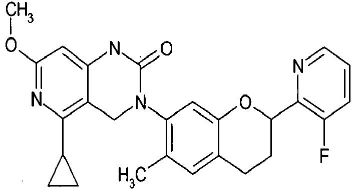 |
| 123 | 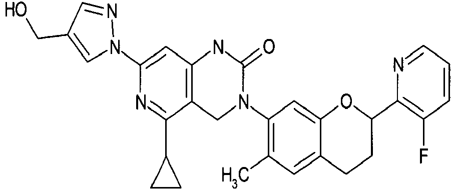 |
| 124 | 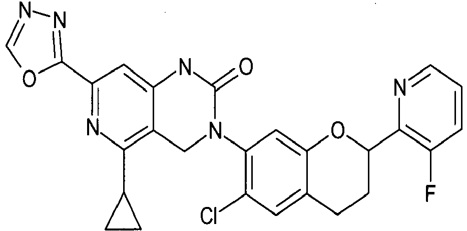 |
| 125 | 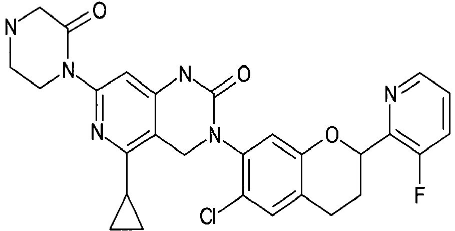 |
| 126 | 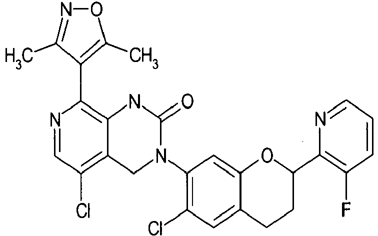 |
| 127 | 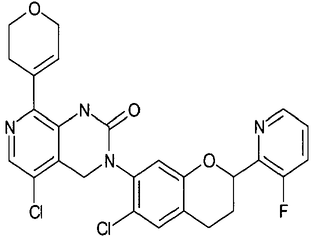 |

**[Table 1-9]**

| | |
|---|---|
| 128 | 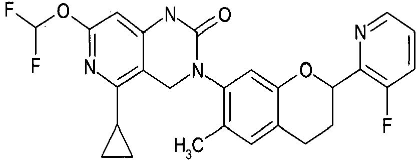 |
| 129 | 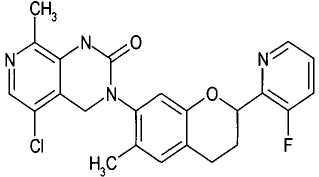 |
| 130 | 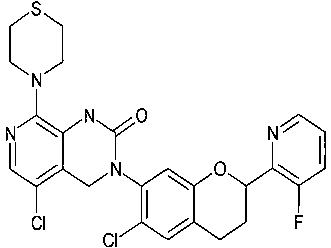 |
| 131 | 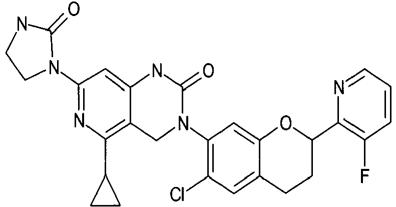 |
| 132 | 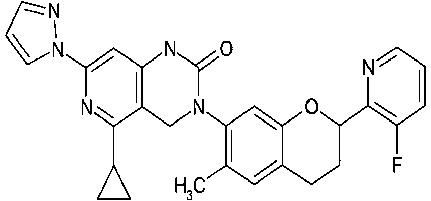 |
| 133 | 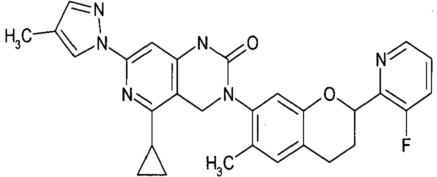 |
| 134 | 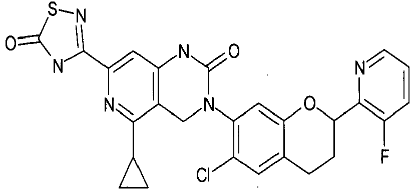 |
| 135 | 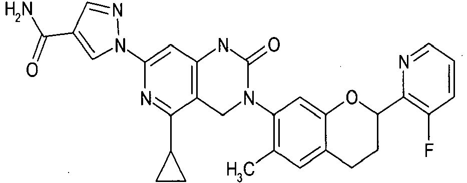 |
| 136 | 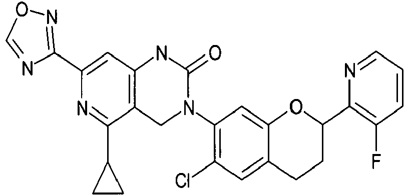 |
| 137 | 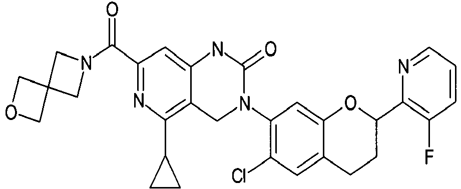 |
| 138 | 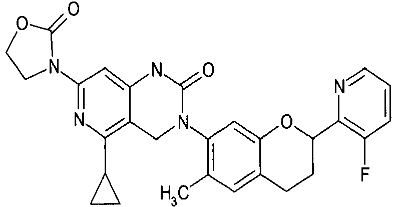 |
| 139 | 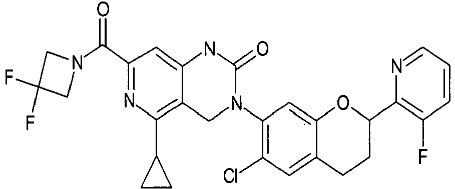 |
| 140 | 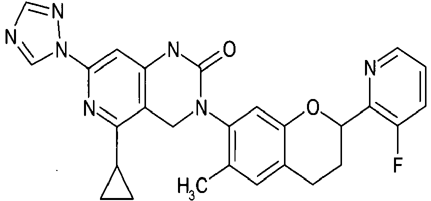 |
| 141 | 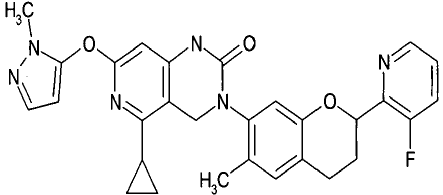 |
| 142 | 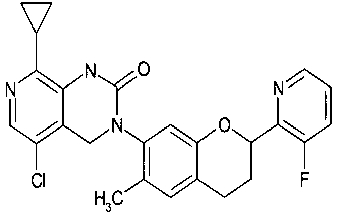 |
| 143 | 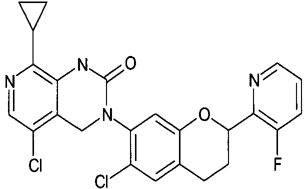 |

**[Table 1-10]**

| | |
|---|---|
| 144 | 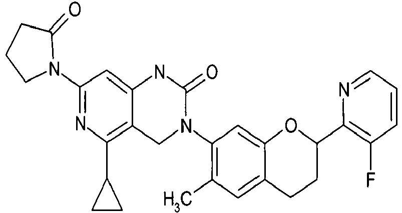 |
| 145 | 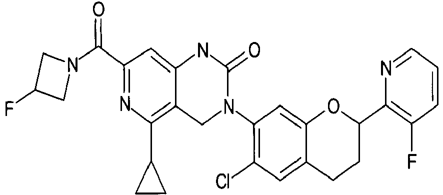 |
| 146 | 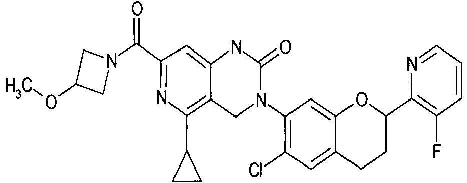 |
| 147 | 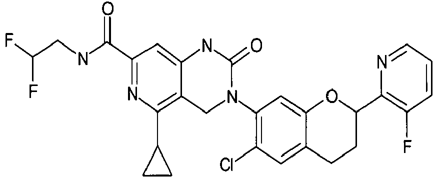 |
| 148 | 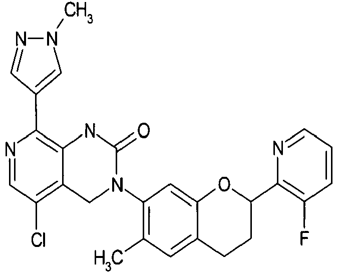 |
| 149 | 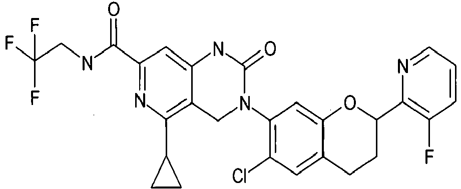 |
| 150 | 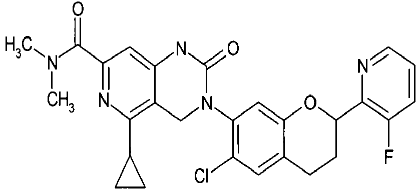 |
| 151 | 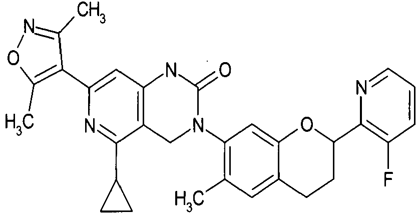 |
| 152 | 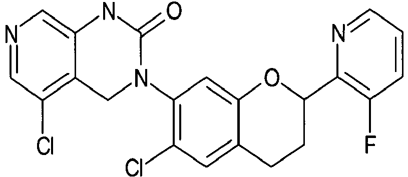 |
| 153 | 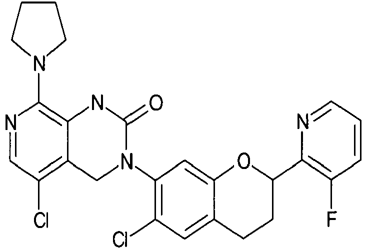 |
| 154 | 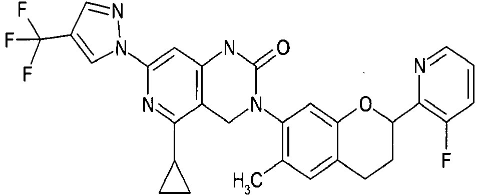 |
| 155 | 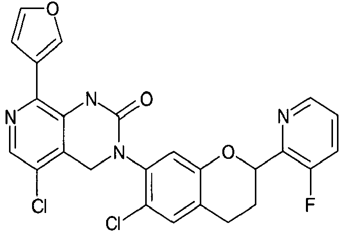 |
| 156 | 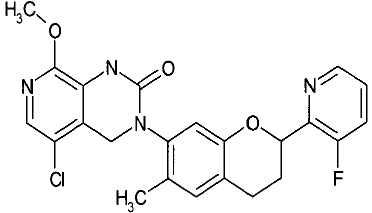 |
| 157 | 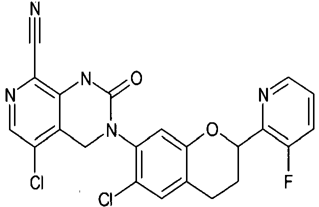 |
| 158 | 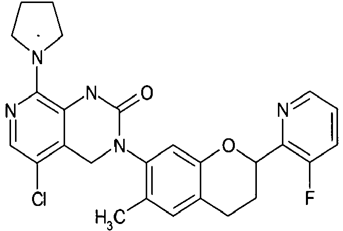 |
| 159 | 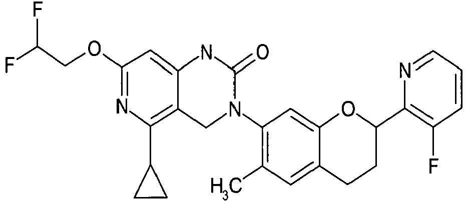 |

**[Table 1-11]**

| | |
|---|---|
| 160 | 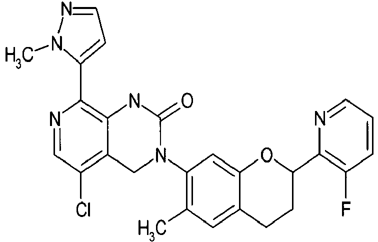 |
| 161 | 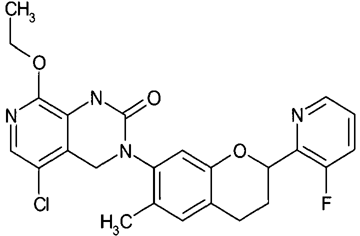 |
| 162 | 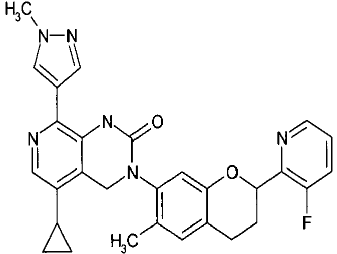 |
| 163 | 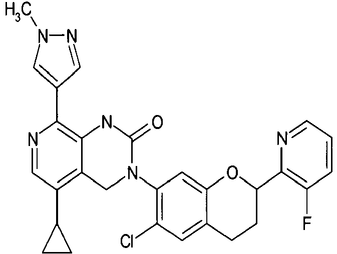 |
| 164 | 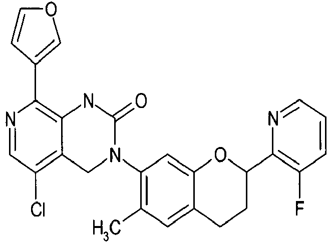 |
| 165 | 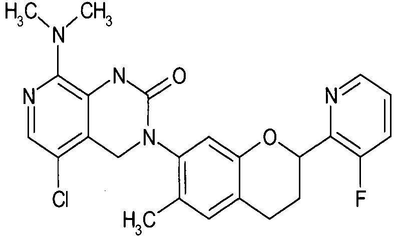 |
| 166 | 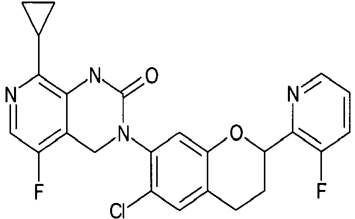 |
| 167 | 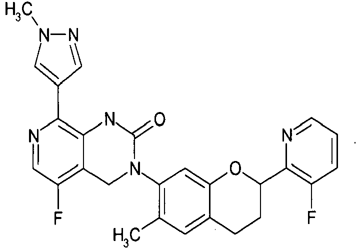 |
| 168 | 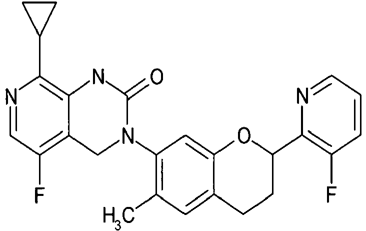 |
| 169 | 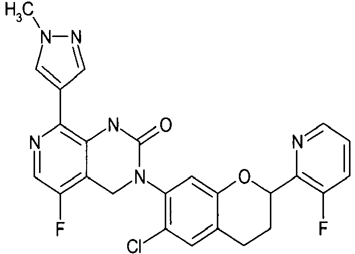 |
| 170 | 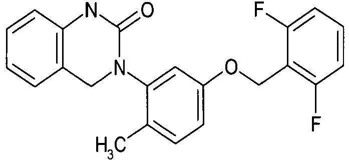 |
| 171 | 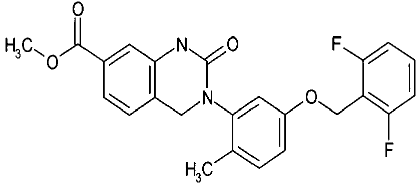 |
| 172 | 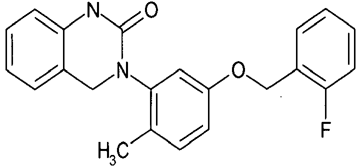 |
| 173 | 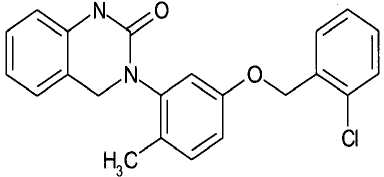 |
| 174 | 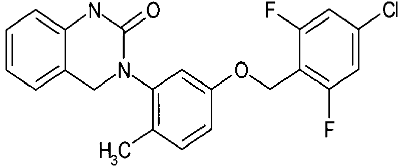 |
| 175 | 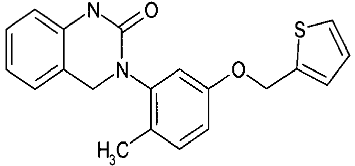 |

**[Table 1-12]**

| | |
|---|---|
| 176 | 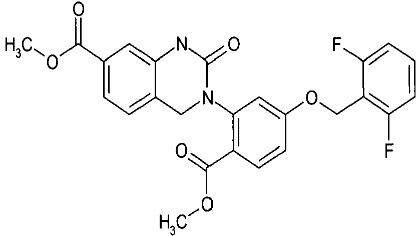 |
| 177 | 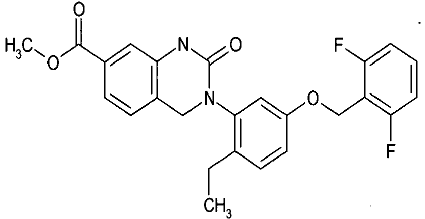 |
| 178 | 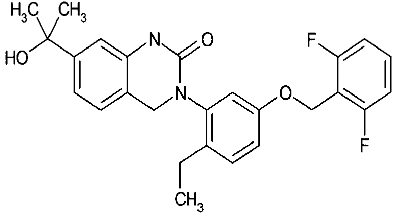 |
| 179 | 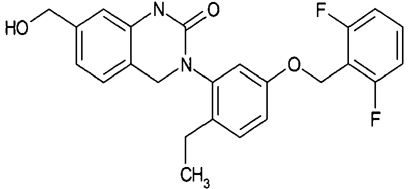 |
| 180 | 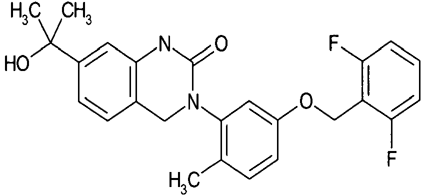 |
| 181 | 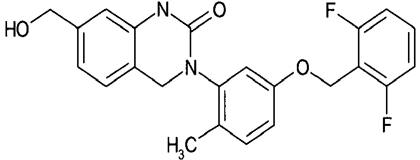 |
| 182 | 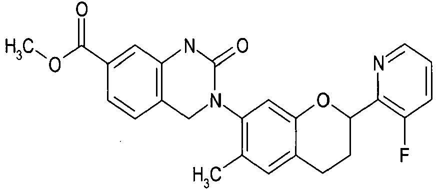 |
| 183 | 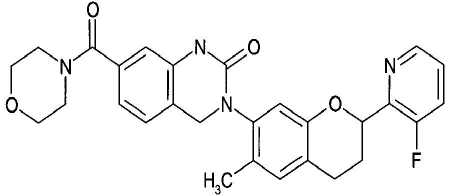 |
| 184 | 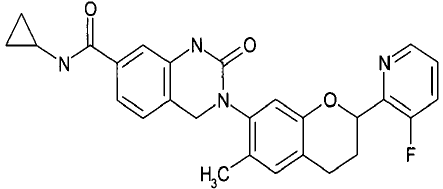 |
| 185 | 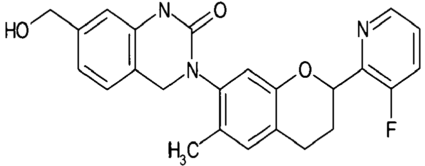 |
| 186 | 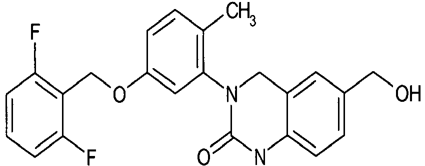 |
| 187 | 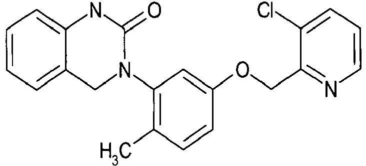 |
| 188 | 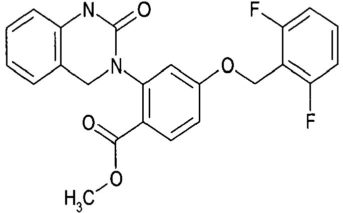 |
| 189 | 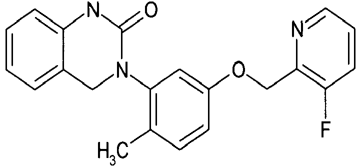 |
| 190 | 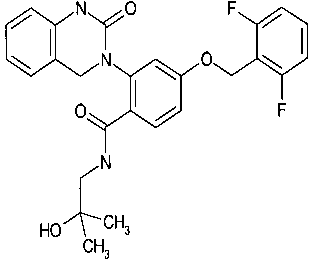 |
| 192 | 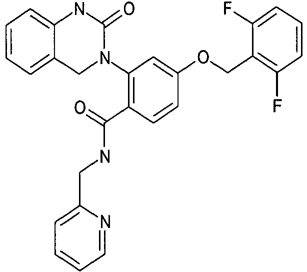 |

**[Table 1-13]**

| | |
|---|---|
| 193 | 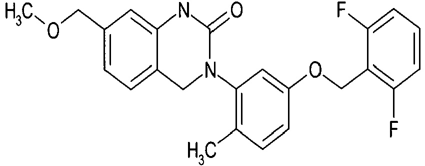 |
| 194 | 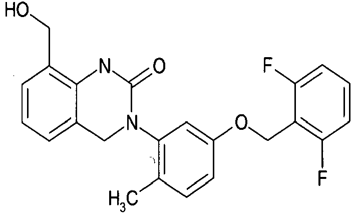 |
| 195 | 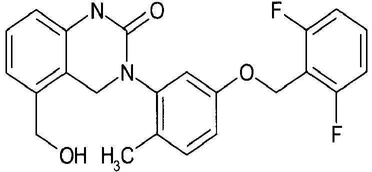 |
| 196 | 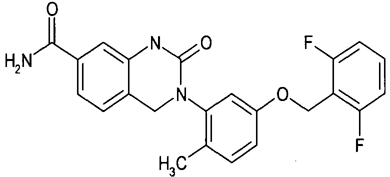 |
| 197 | 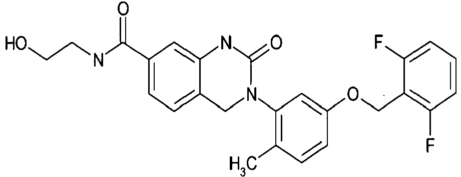 |
| 198 | 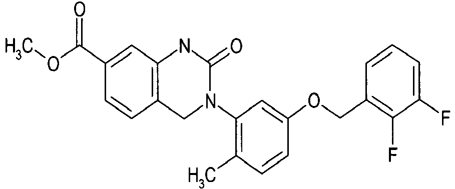 |
| 199 | 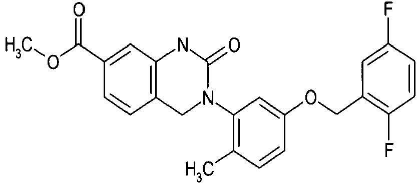 |
| 200 | 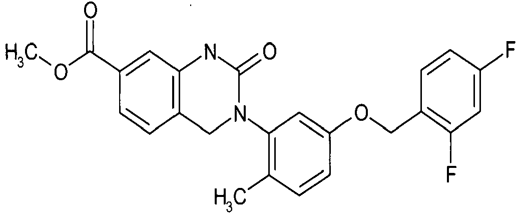 |
| 201 | 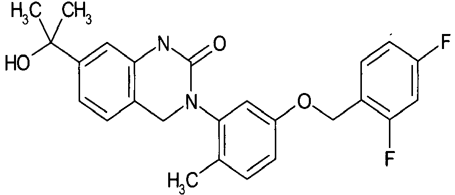 |
| 202 | 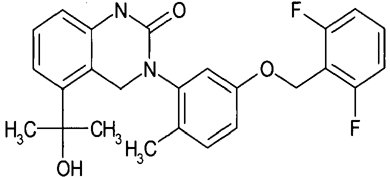 |
| 203 | 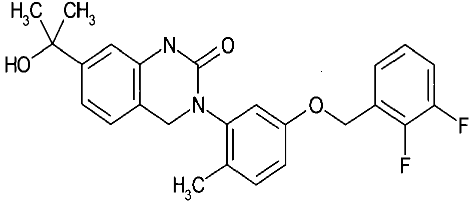 |
| 204 | 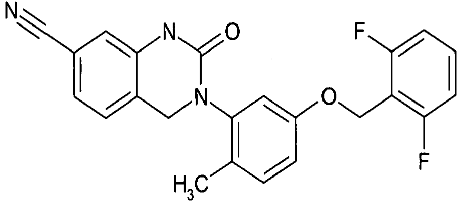 |
| 205 | 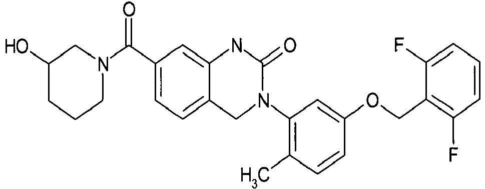 |
| 206 | 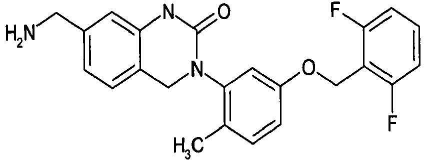 |
| 207 | 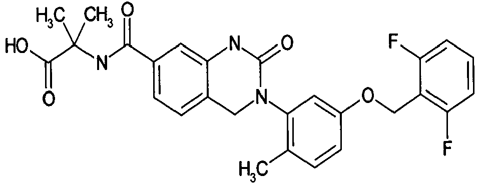 |
| 208 | 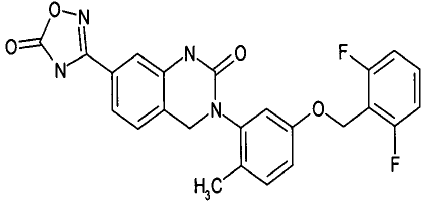 |

**[Table 1-14]**

| | |
|---|---|
| 209 | 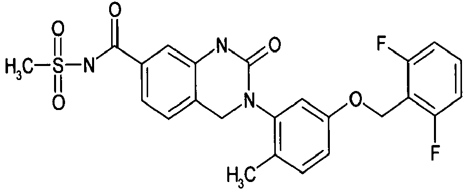 |
| 210 | 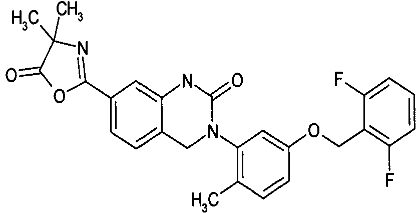 |
| 211 | 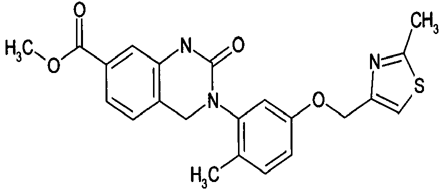 |
| 212 | 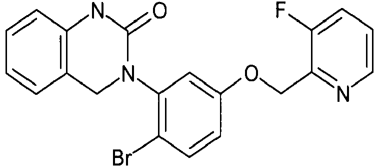 |
| 213 | 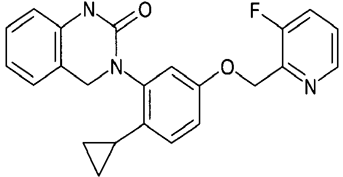 |
| 214 | 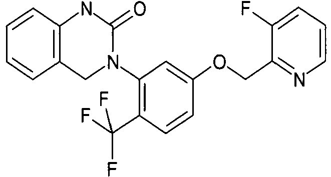 |
| 215 | 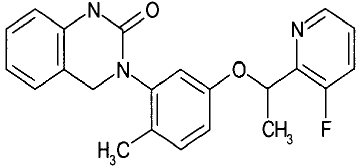 |
| 216 | 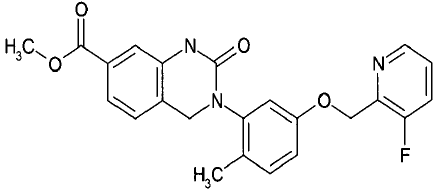 |
| 217 | 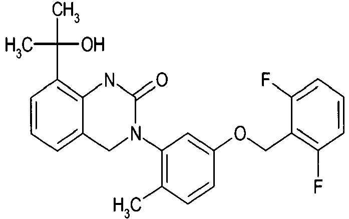 |
| 218 | 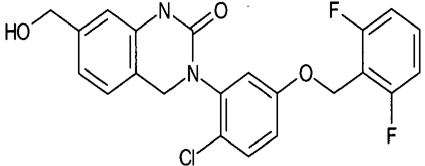 |
| 219 | 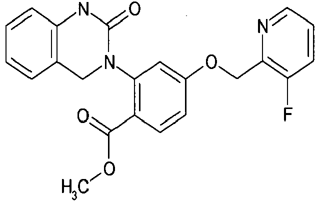 |
| 220 | 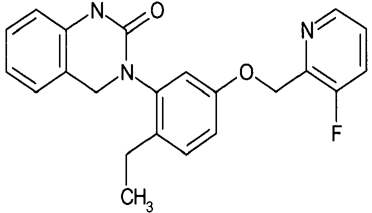 |
| 221 | 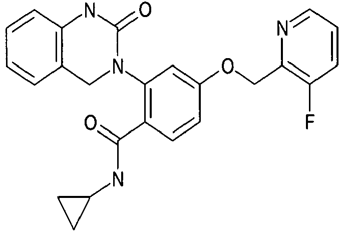 |
| 222 | 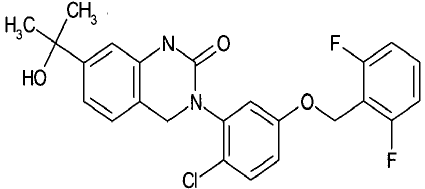 |
| 223 | 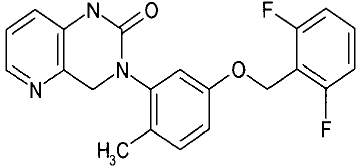 |
| 224 | 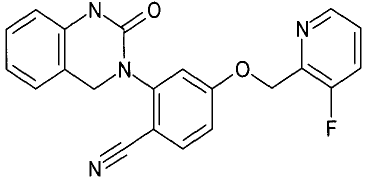 |

**[Table 1-15]**

| | |
|---|---|
| 225 | 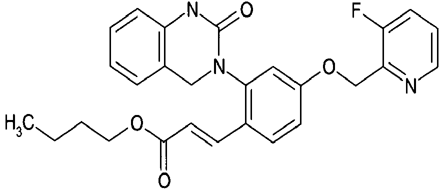 |
| 226 | 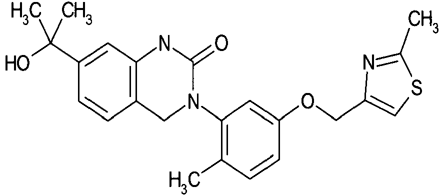 |
| 227 | 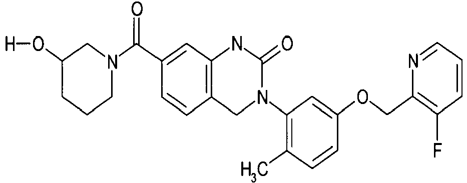 |
| 228 | 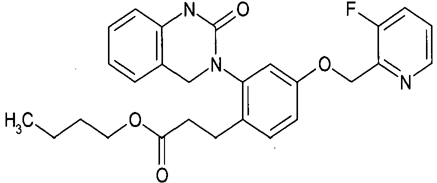 |
| 229 | 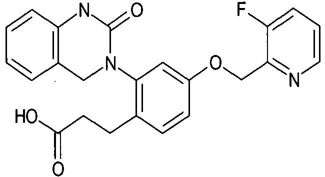 |
| 230 | 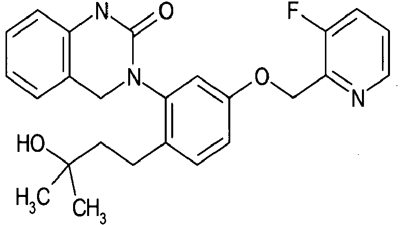 |
| 231 | 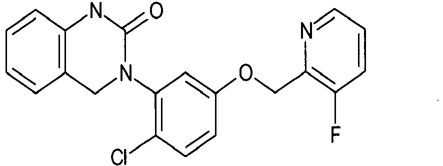 |
| 232 | 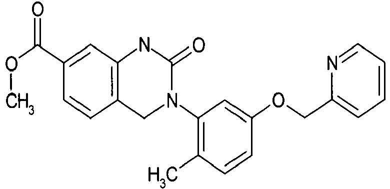 |
| 233 | 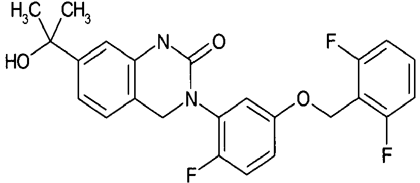 |
| 234 | 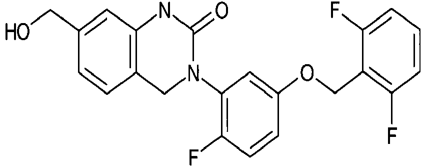 |
| 235 | 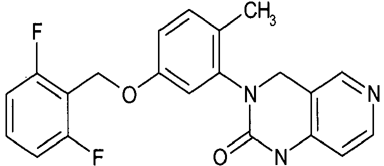 |
| 236 | 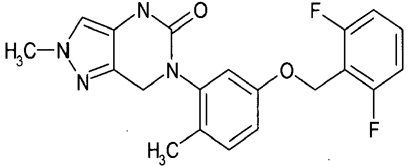 |
| 237 | 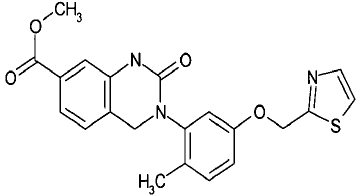 |
| 238 | 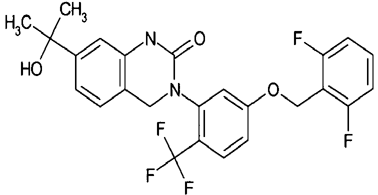 |
| 239 | 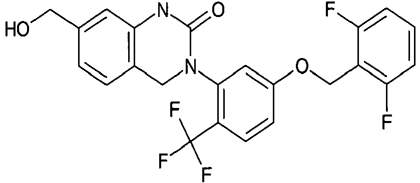 |
| 240 | 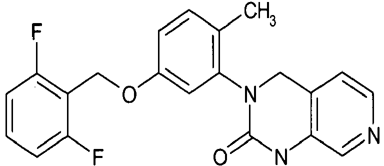 |

**[Table 1-16]**

| | |
|---|---|
| 241 | 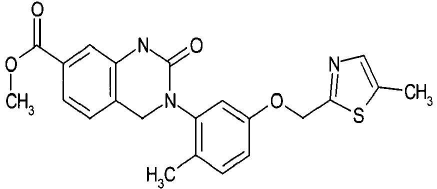 |
| 242 | 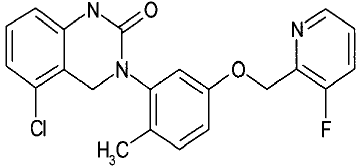 |
| 243 | 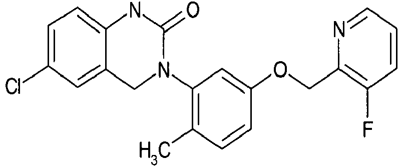 |
| 244 | 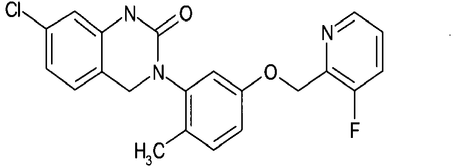 |
| 245 | 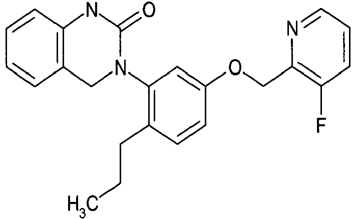 |
| 246 | 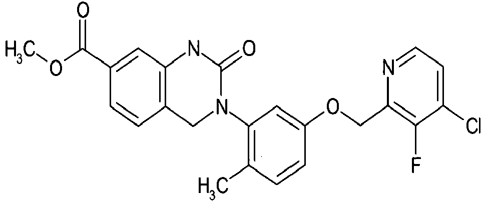 |
| 247 | 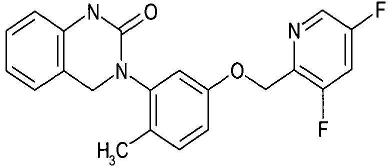 |
| 248 | 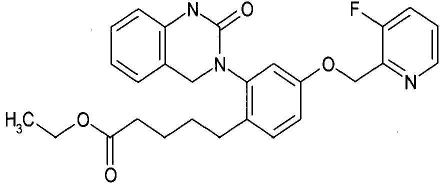 |
| 249 | 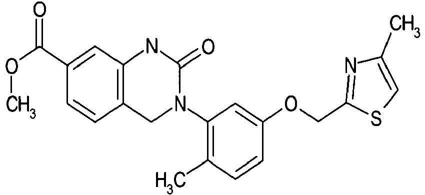 |
| 250 | 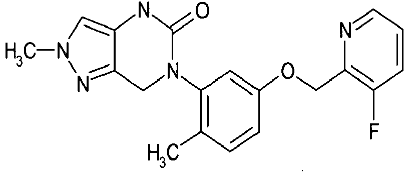 |
| 251 | 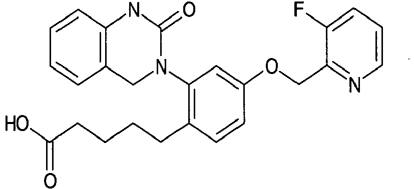 |
| 252 | 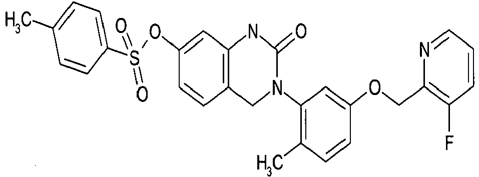 |
| 253 | 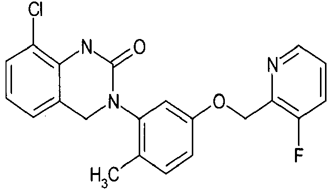 |
| 254 | 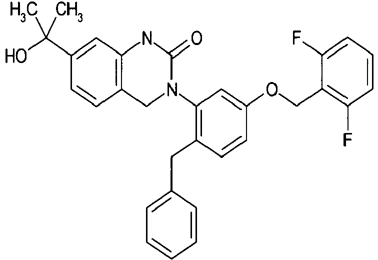 |
| 255 | 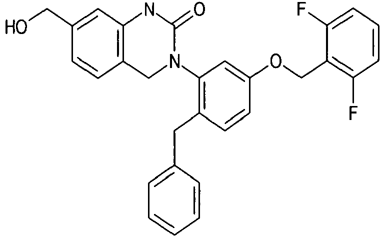 |
| 256 | 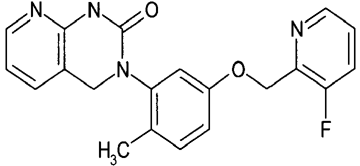 |

**[Table 1-17]**

| | |
|---|---|
| 257 | 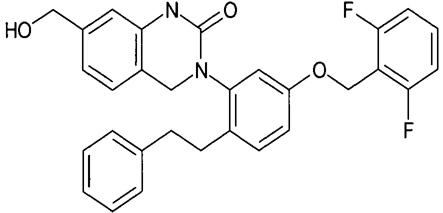 |
| 258 | 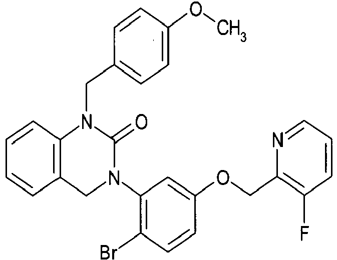 |
| 259 | 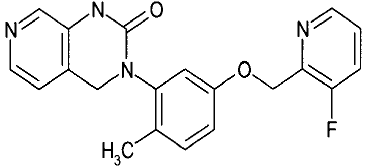 |
| 260 | 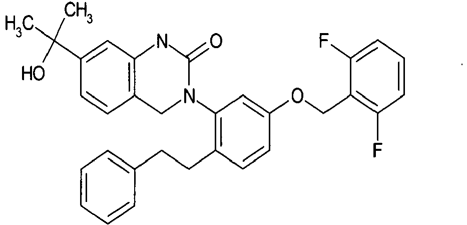 |
| 261 | 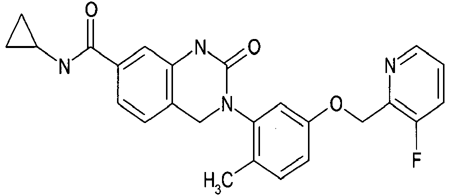 |
| 262 | 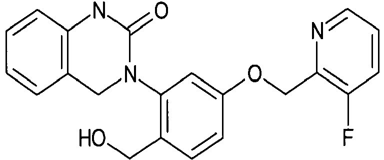 |
| 263 | 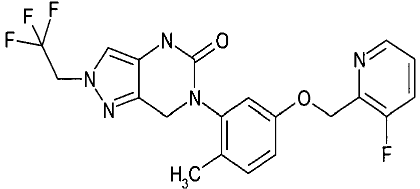 |
| 264 | 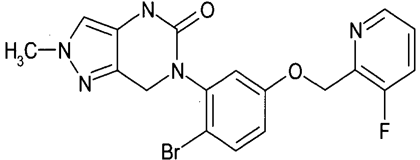 |
| 265 | 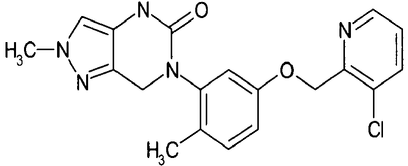 |
| 266 | 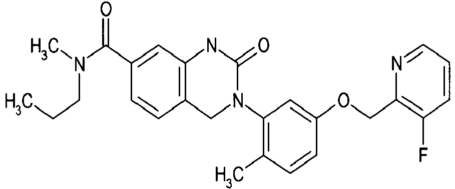 |
| 267 | 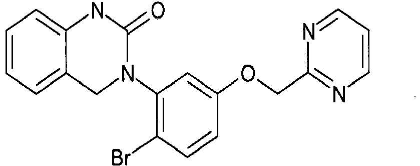 |
| 268 | 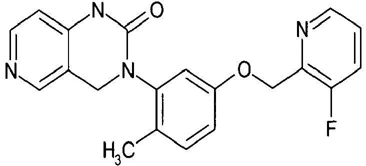 |
| 269 | 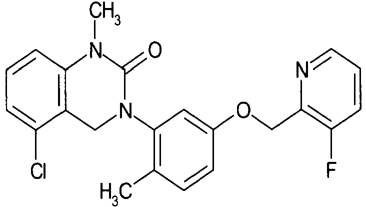 |
| 270 | 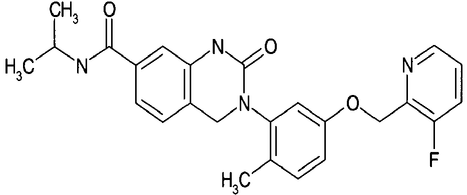 |
| 271 | 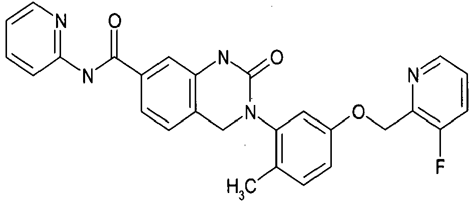 |
| 272 | 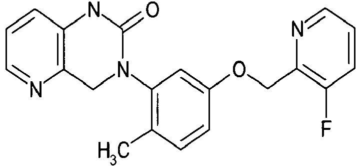 |

**[Table 1-18]**

| | |
|---|---|
| 273 | 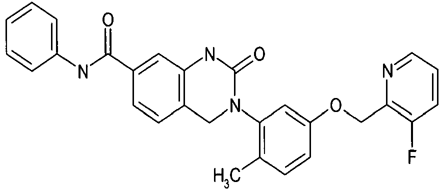 |
| 274 | 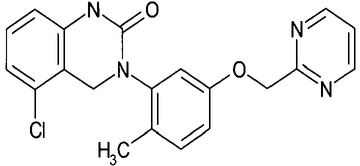 |
| 275 | 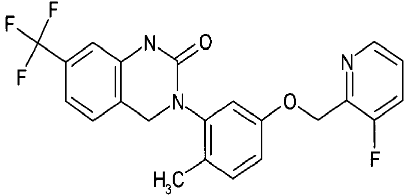 |
| 276 | 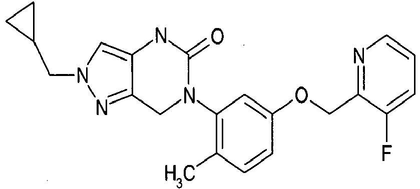 |
| 277 | 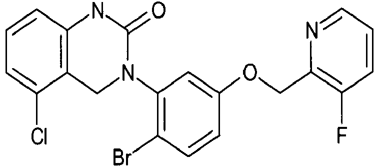 |
| 278 | 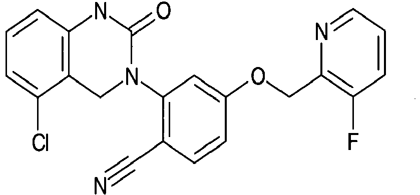 |
| 279 | 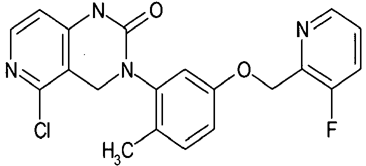 |
| 280 | 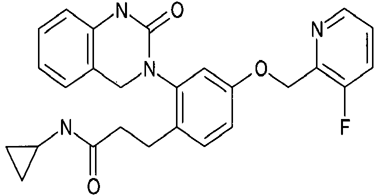 |
| 281 | 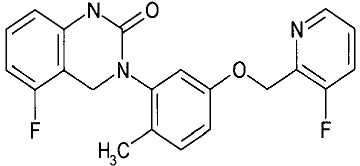 |
| 282 | 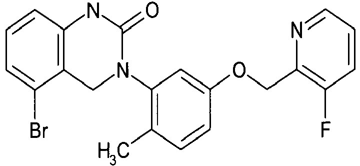 |
| 283 | 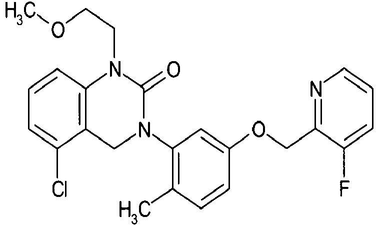 |
| 284 | 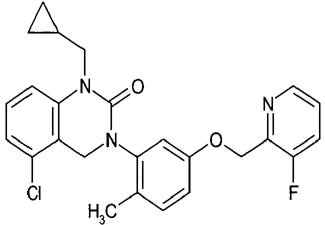 |
| 285 | 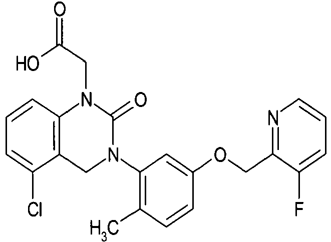 |
| 286 | 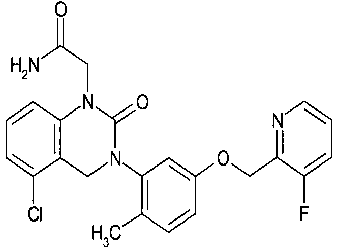 |
| 287 | 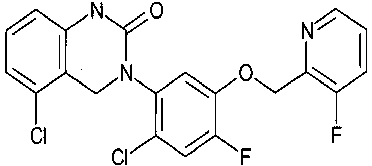 |
| 288 | 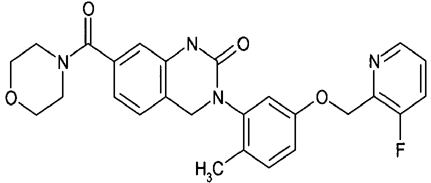 |

**[Table 1-19]**

| | |
|---|---|
| 289 | 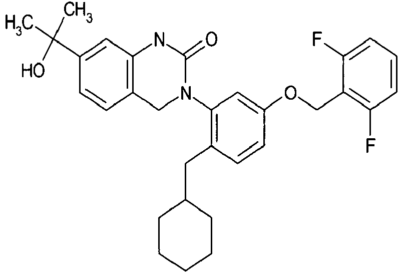 |
| 290 | 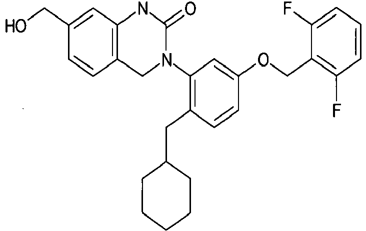 |
| 291 | 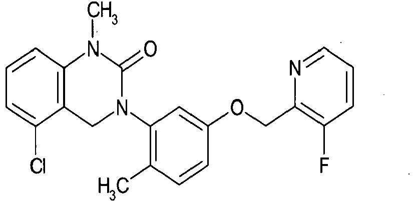 |
| 292 | 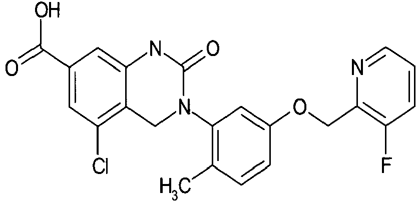 |
| 293 | 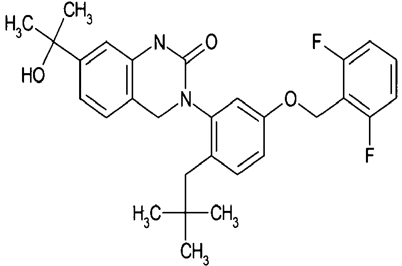 |
| 294 | 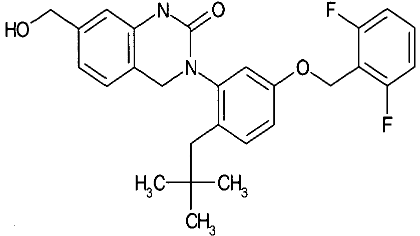 |
| 295 | 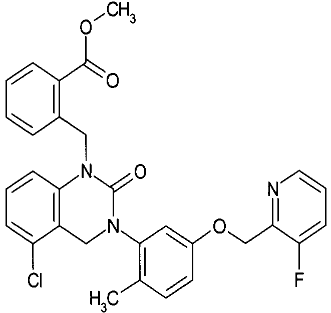 |
| 296 | 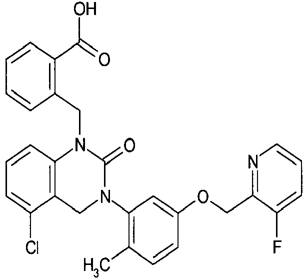 |
| 297 | 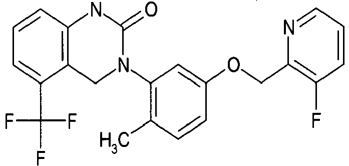 |
| 298 | 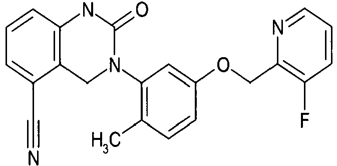 |
| 299 | 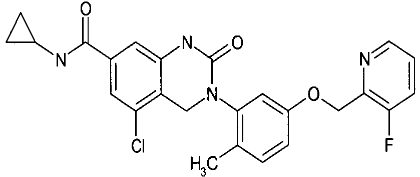 |
| 300 | 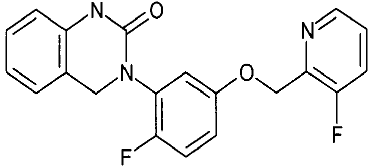 |
| 301 | 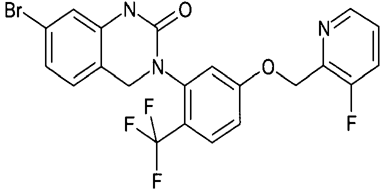 |
| 302 | 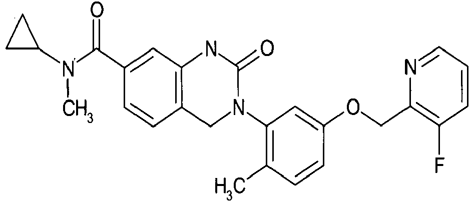 |
| 303 | 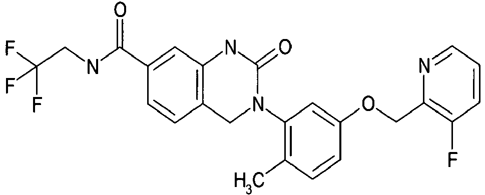 |
| 304 | 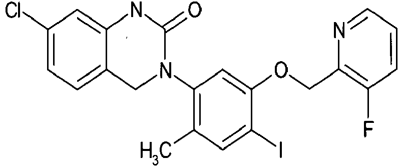 |

**[Table 1-20]**

| | |
|---|---|
| 305 | 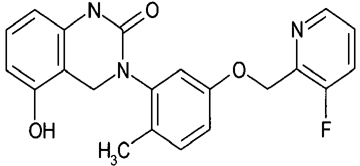 |
| 306 | 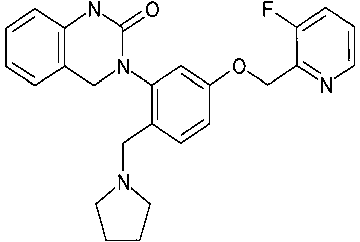 |
| 307 | 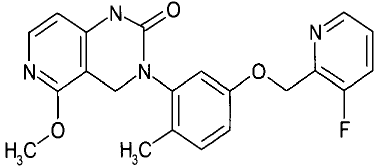 |
| 308 | 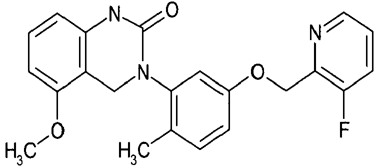 |
| 309 | 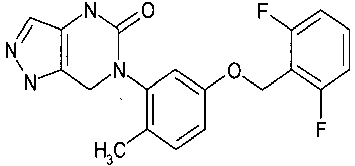 |
| 310 | 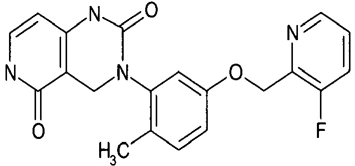 |
| 311 | 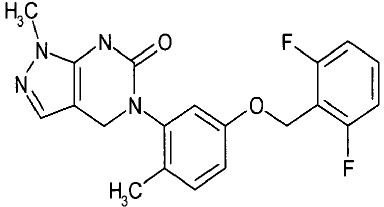 |
| 312 | 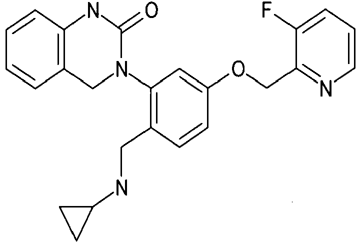 |
| 313 | 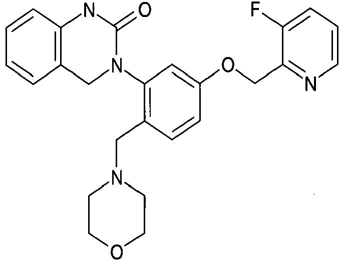 |
| 314 | 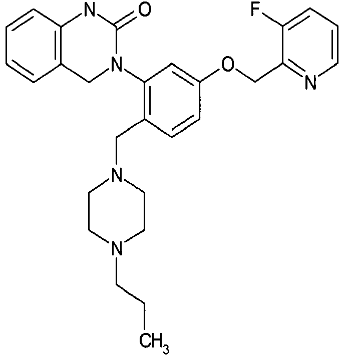 |
| 315 | 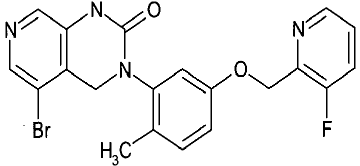 |
| 316 | 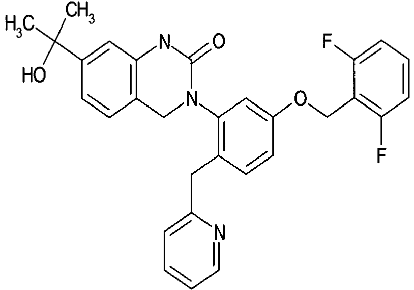 |
| 317 | 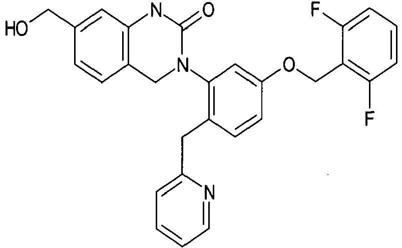 |
| 318 | 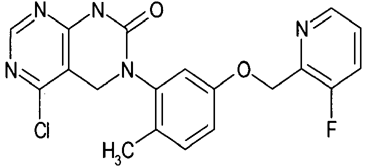 |
| 319 | 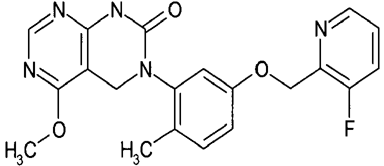 |
| 320 | 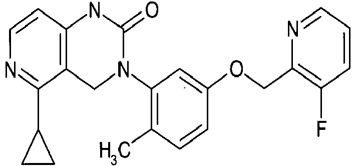 |

**[Table 1-21]**

| | |
|---|---|
| 321 | 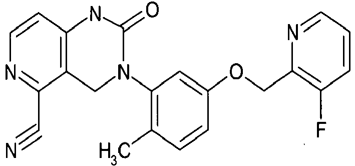 |
| 322 | 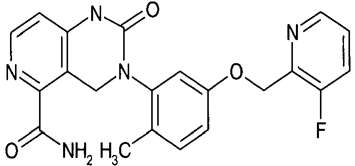 |
| 323 | 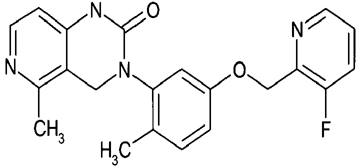 |
| 324 | 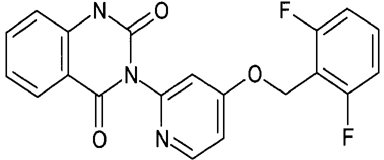 |
| 325 | 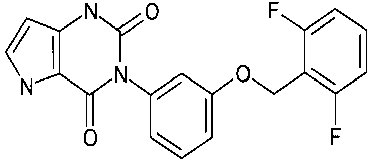 |
| 326 | 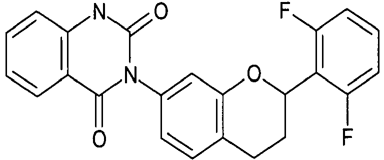 |
| 327 | 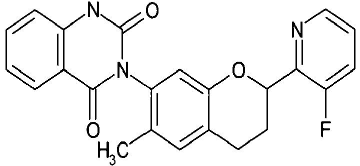 |
| 328 | 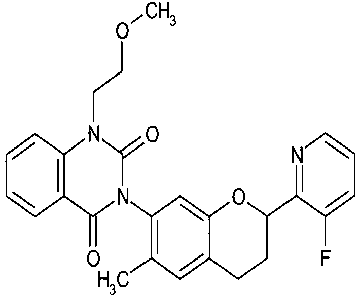 |
| 329 | 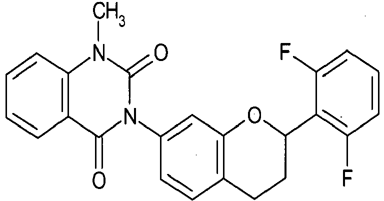 |
| 330 | 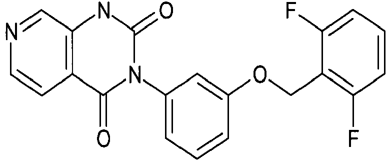 |
| 331 | 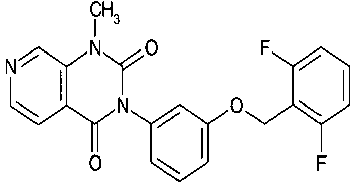 |
| 334 | 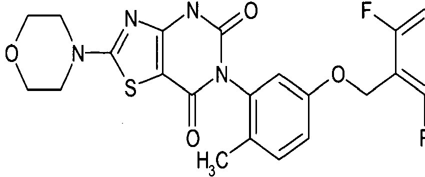 |
| 335 | 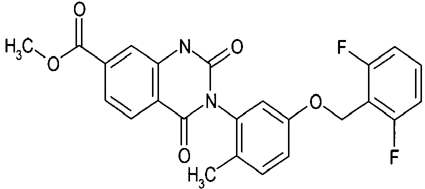 |
| 336 | 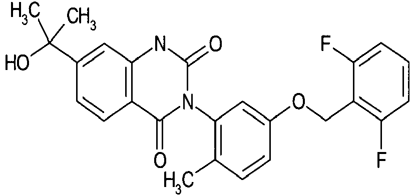 |
| 337 | 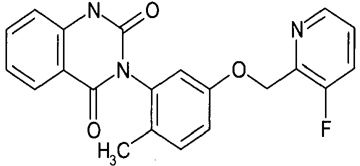 |
| 338 | 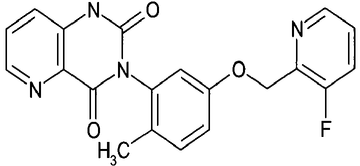 |

**[Table 1-22]**

| | |
|---|---|
| 339 | 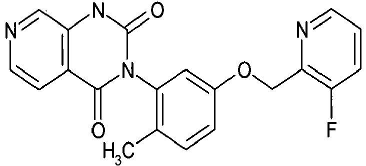 |
| 340 | 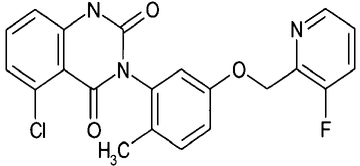 |
| 341 | 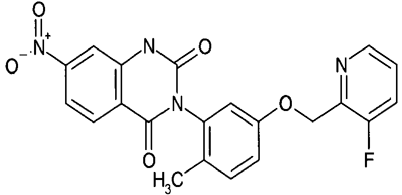 |
| 342 | 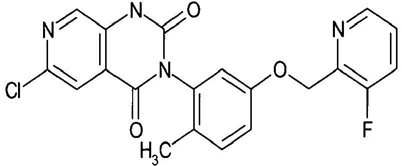 |
| 343 | 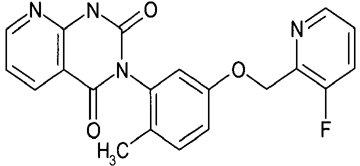 |
| 344 | 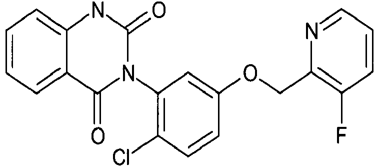 |
| 345 | 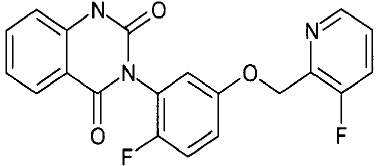 |
| 346 | 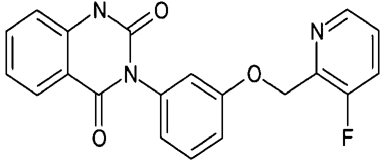 |
| 347 | 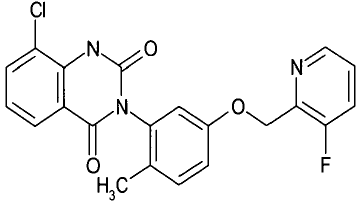 |
| 348 | 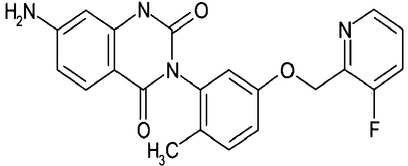 |
| 349 | 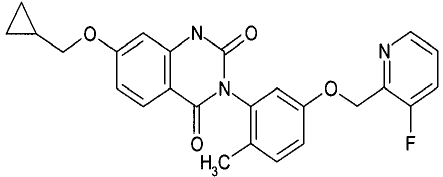 |
| 350 | 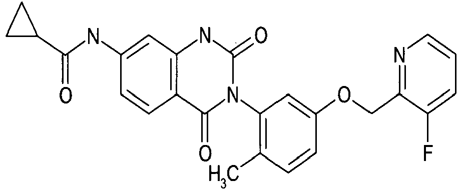 |
| 351 | 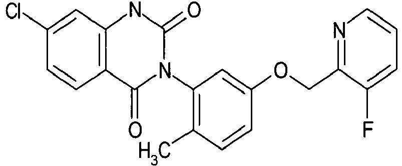 |
| 352 | 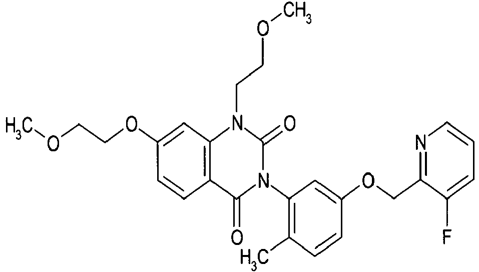 |
| 353 | 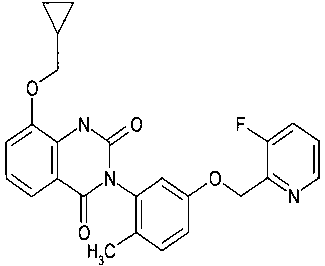 |
| 354 | 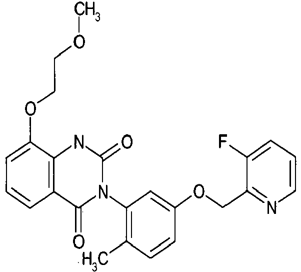 |

**[Table 1-23]**

| | |
|---|---|
| 355 | 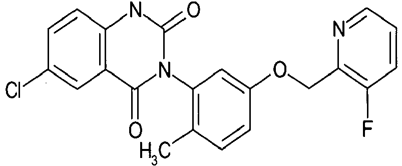 |
| 356 | 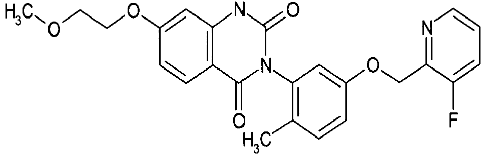 |
| 357 | 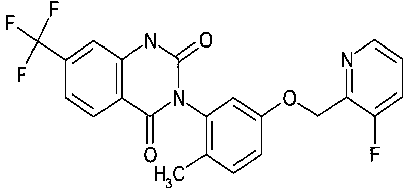 |
| 358 | 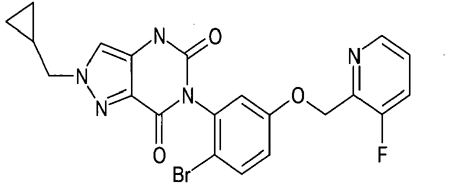 |
| 359 | 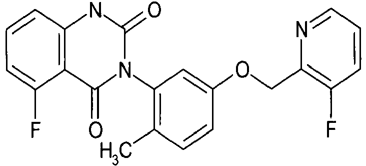 |
| 360 | 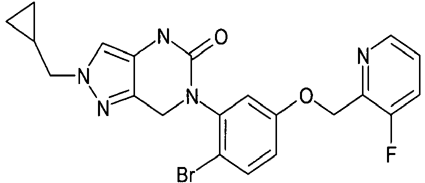 |
| 361 | 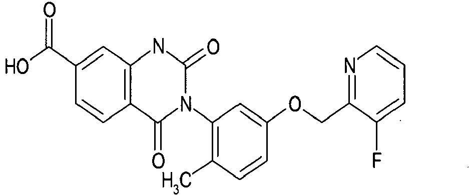 |
| 362 | 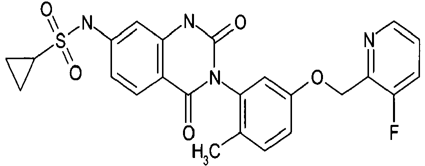 |
| 363 | 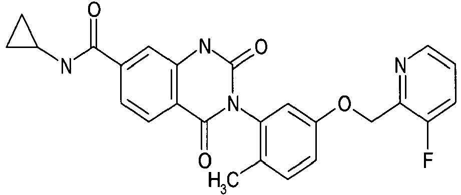 |
| 364 | 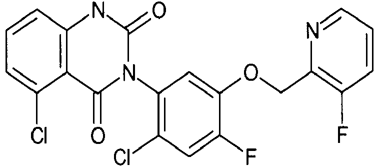 |
| 365 | 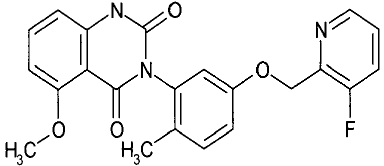 |
| 366 | 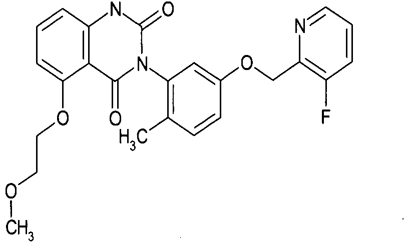 |
| 367 | 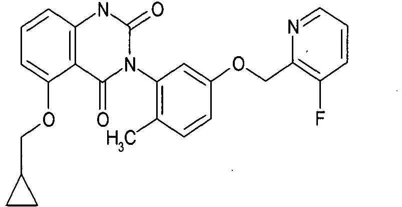 |
| 368 | 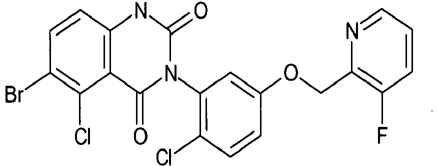 |
| 369 | 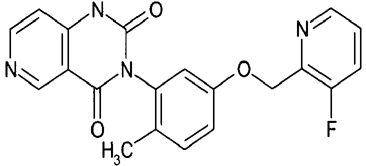 |
| 370 | 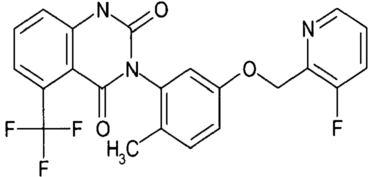 |

**[Table 1-24]**

| | |
|---|---|
| 371 | 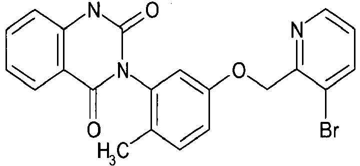 |
| 372 | 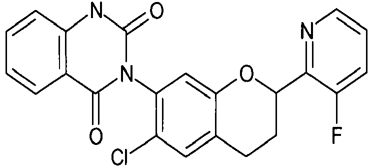 |
| 373 | 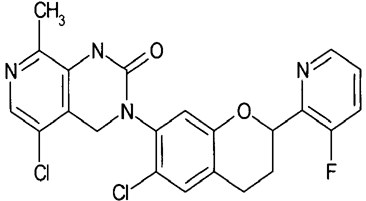 |

### Formulation Example 1 (production of capsule)

| | |
|---|---|
| 1) compound of Example 1 | 30 mg |
| 2) finely divided powder cellulose | 10 mg |
| 3) lactose | 19 mg |
| 4) magnesium stearate | 1 mg |
| total | 60 mg |

| | |
|---|---|
| 1), 2), 3) and 4) are mixed and filled in a gelatin capsule. | |

### Formulation Example 2 (production of tablets)

| | |
|---|---|
| 1) compound of Example 1 | 30 g |
| 2) lactose | 50 g |
| 3) cornstarch | 15 g |
| 4) calcium carboxymethylcellulose | 44 g |
| 5) magnesium stearate | 1 g |
| 1000 tablets | total 140 g |

The entire amount of 1), 2) and 3) and 4) (30 g) is kneaded with water, vacuum dried, and sieved. The sieved powder is mixed with 4) (14 g) and 5) (1 g), and the mixture is punched by a tableting machine, whereby 1000 tablets containing 30 mg of the compound of Ex. 1 per tablet are obtained.

### Experimental Example 1

### In vitro binding test

As an assay buffer, 100 mM Tris-HCl, pH 7.5, 140 mM NaCl, 2 mM EDTA, 0.5 mM dithiothreitol, 5% glycerol, 0.05% Tween20 was used. Sf9 microsome fractions expressing human and mouse FLAPs were dispensed by 50 µL to a 96-well plate at 2 µg/mL. Furthermore, a test compound (25 µL) diluted 4-fold of the final concentration with the assay buffer was added and mixed therewith, and the mixture was incubated at room temperature for 10 min. Furthermore, 120 nM [³H]-7-chloro-3-(5-((3-fluoropyridin-2-yl)methoxy)-2-methylphenyl)-3,4-dihydroquinazolin-2(1H)-one was added by 25 µL, and mixed therewith, and the mixture was incubated at room temperature for 60 min. Thereafter, the cells were transferred to a 96-well GF/B Unifilter plate with a FilterMate cell harvester, and washed 3 times with a wash buffer (100 mM Tris-HCl pH 7.5, 0.05% Tween20, 200 µL) cooled to 4°C. The GF/B Unifilter plate was dried at 37°C, Microscint O was added and measured by TopCount NXT (PerkinElmer).

As for the inhibitory activity, the inhibitory rate was calculated wherein the [³H]-7-chloro-3-(5-((3-fluoropyridin-2-yl)methoxy)-2-methylphenyl)-3,4-dihydroquinazolin-2(1H)-one binding amount in the presence of 10 µM sodium 3-(3-(tert-butylsulfanyl)-1-(4-chlorobenzyl)-5-isopropyl-1H-indol-2-yl)-2,2-dimethylpropanoate was 100%, and the [³H]-7-chloro-3-(5-((3-fluoropyridin-2-yl)methoxy)-2-methylphenyl)-3,4-dihydroquinazolin-2(1H)-one binding amount in the presence of DMSO was 0%. The results are shown in Table 2.

**[Table 2-1]**

| **Example No.** | **inhibitory rate (%)** |
|---|---|
| 1 | 94 |
| 2 | 102 |
| 3 | 97 |
| 4 | 103 |
| 5 | 100 |
| 6 | 98 |
| 7 | 98 |
| 8 | 103 |
| 9 | 104 |
| 10 | 94 |
| 11 | 109 |
| 12 | 99 |
| 13 | 101 |
| 14 | 105 |
| 15 | 102 |
| 16 | 102 |
| 17 | 110 |
| 18 | 88 |
| 19 | 102 |
| 20 | 96 |
| 21 | 103 |
| 22 | 109 |
| 23 | 92 |
| 24 | 96 |
| 25 | 99 |
| 26 | 101 |
| 27 | 82 |
| 28 | 98 |
| 29 | 99 |
| 30 | 105 |
| 31 | 98 |
| 32 | 101 |
| 33 | 106 |
| 34 | 111 |
| 35 | 93 |
| 36 | 115 |
| 37 | 107 |
| 38 | 104 |
| 39 | 94 |
| 40 | 108 |
| 41 | 108 |
| 42 | 116 |
| 43 | 107 |
| 44 | 113 |
| 45 | 99 |
| 46 | 104 |
| 47 | 114 |
| 48 | 105 |
| 49 | 120 |
| 50 | 67 |
| 51 | 105 |
| 52 | 15 |
| 53 | 03 |
| 54 | 115 |
| 55 | 106 |
| 56 | 110 |
| 57 | 105 |
| 58 | 117 |
| 59 | 107 |
| 60 | 113 |
| 61 | 91 |
| 62 | 100 |
| 63 | 106 |
| 64 | 94 |
| 65 | 105 |
| 66 | 98 |

**[Table 2-2]**

| | |
|---|---|
| 67 | 104 |
| 68 | 113 |
| 70 | 101 |
| 71 | 107 |
| 72 | 104 |
| 73 | 109 |
| 74 | 104 |
| 75 | 103 |
| 76 | 105 |
| 77 | 84 |
| 78 | 105 |
| 79 | 98 |
| 80 | 107 |
| 81 | 102 |
| 82 | 101 |
| 83 | 96 |
| 84 | 106 |
| 85 | 102 |
| 86 | 108 |
| 87 | 118 |
| 88 | 96 |
| 89 | 98 |
| 90 | 118 |
| 91 | 109 |
| 92 | 106 |
| 93 | 112 |
| 94 | 104 |
| 95 | 89 |
| 96 | 84 |
| 97 | 90 |
| 98 | 94 |
| 99 | 94 |
| 100 | 100 |
| 101 | 96 |
| 102 | 101 |
| 103 | 94 |
| 104 | 91 |
| 105 | 96 |
| 106 | 94 |
| 107 | 93 |
| 108 | 104 |
| 109 | 106 |
| 110 | 99 |
| 111 | 103 |
| 112 | 103 |
| 113 | 114 |
| 114 | 106 |
| 115 | 106 |
| 116 | 102 |
| 117 | 106 |
| 118 | 104 |
| 119 | 107 |
| 120 | 100 |
| 121 | 111 |
| 122 | 106 |
| 123 | 98 |
| 124 | 104 |
| 125 | 95 |
| 126 | 113 |

**[Table 2-3]**

| | |
|---|---|
| 127 | 98 |
| 128 | 111 |
| 129 | 114 |
| 130 | 101 |
| 131 | 101 |
| 132 | 104 |
| 133 | 100 |
| 134 | 91 |
| 135 | 104 |
| 136 | 111 |
| 137 | 104 |
| 138 | 109 |
| 139 | 98 |
| 140 | 99 |
| 141 | 102 |
| 142 | 102 |
| 143 | 89 |
| 144 | 92 |
| 145 | 104 |
| 146 | 97 |
| 147 | 104 |
| 148 | 97 |
| 149 | 107 |
| 150 | 98 |
| 151 | 90 |
| 152 | 103 |
| 153 | 104 |
| 154 | 100 |
| 155 | 101 |
| 156 | 95 |
| 157 | 104 |
| 158 | 99 |
| 159 | 97 |
| 160 | 93 |
| 161 | 90 |
| 162 | 95 |
| 163 | 98 |
| 164 | 102 |
| 165 | 104 |
| 166 | 106 |
| 67 | 95 |
| 168 | 91 |
| 169 | 92 |
| 170 | 91 |
| 171 | 99 |
| 172 | 80 |
| 173 | 70 |
| 174 | 54 |
| 175 | 51 |
| 176 | 95 |
| 177 | 84 |
| 178 | 109 |
| 179 | 91 |
| 180 | 81 |
| 181 | 91 |
| 182 | 108 |
| 183 | 96 |
| 184 | 97 |
| 185 | 98 |

**[Table 2-4]**

| | |
|---|---|
| 186 | 62 |
| 187 | 103 |
| 188 | 83 |
| 189 | 86 |
| 190 | 55 |
| 192 | 51 |
| 193 | 97 |
| 194 | 71 |
| 195 | 97 |
| 196 | 97 |
| 197 | 101 |
| 198 | 94 |
| 199 | 60 |
| 200 | 100 |
| 201 | 104 |
| 202 | 72 |
| 203 | 81 |
| 204 | 103 |
| 205 | 77 |
| 206 | 81 |
| 207 | 87 |
| 208 | 96 |
| 209 | 102 |
| 210 | 96 |
| 211 | 86 |
| 212 | 113 |
| 213 | 114 |
| 214 | 110 |
| 215 | 79 |
| 216 | 104 |
| 217 | 87 |
| 218 | 95 |
| 219 | 106 |
| 220 | 106 |
| 221 | 72 |
| 222 | 103 |
| 223 | 95 |
| 224 | 87 |
| 225 | 100 |
| 226 | 52 |
| 227 | 90 |
| 228 | 95 |
| 229 | 76 |
| 230 | 87 |
| 231 | 103 |
| 232 | 104 |
| 233 | 87 |
| 234 | 85 |
| 235 | 79 |
| 236 | 65 |
| 237 | 100 |
| 238 | 84 |
| 239 | 86 |
| 240 | 76 |
| 241 | 94 |
| 242 | 102 |
| 243 | 100 |
| 244 | 113 |
| 245 | 99 |

**[Table 2-5]**

| | |
|---|---|
| 246 | 109 |
| 247 | 99 |
| 248 | 98 |
| 249 | 90 |
| 250 | 70 |
| 251 | 68 |
| 252 | 105 |
| 253 | 93 |
| 254 | 98 |
| 255 | 81 |
| 256 | 85 |
| 257 | 97 |
| 258 | 116 |
| 259 | 92 |
| 260 | 106 |
| 261 | 116 |
| 262 | 94 |
| 263 | 97 |
| 264 | 101 |
| 265 | 82 |
| 266 | 105 |
| 267 | 100 |
| 268 | 83 |
| 269 | 111 |
| 270 | 104 |
| 271 | 100 |
| 272 | 95 |
| 273 | 100 |
| 274 | 106 |
| 275 | 101 |
| 276 | 101 |
| 277 | 99 |
| 278 | 96 |
| 279 | 103 |
| 280 | 62 |
| 281 | 107 |
| 282 | 98 |
| 283 | 100 |
| 284 | 105 |
| 285 | 102 |
| 286 | 112 |
| 287 | 95 |
| 288 | 104 |
| 289 | 80 |
| 290 | 97 |
| 291 | 94 |
| 292 | 107 |
| 293 | 84 |
| 294 | 65 |
| 295 | 102 |
| 296 | 95 |
| 297 | 106 |
| 298 | 104 |
| 299 | 113 |
| 300 | 105 |
| 301 | 104 |
| 302 | 104 |
| 303 | 108 |
| 304 | 64 |

**[Table 2-6]**

| | |
|---|---|
| 305 | 103 |
| 306 | 74 |
| 307 | 100 |
| 308 | 85 |
| 309 | 51 |
| 310 | 95 |
| 311 | 69 |
| 312 | 86 |
| 313 | 64 |
| 314 | 58 |
| 315 | 103 |
| 316 | 50 |
| 317 | 54 |
| 318 | 62 |
| 319 | 52 |
| 320 | 101 |
| 321 | 90 |
| 322 | 97 |
| 323 | 94 |
| 324 | 50 |
| 325 | 65 |
| 326 | 95 |
| 327 | 107 |
| 328 | 11 |
| 329 | 103 |
| 330 | 72 |
| 331 | 51 |
| 334 | 93 |
| 335 | 103 |
| 336 | 108 |
| 337 | 104 |
| 338 | 90 |
| 339 | 106 |
| 340 | 107 |
| 341 | 102 |
| 342 | 101 |
| 343 | 01 |
| 344 | 114 |
| 345 | 114 |
| 346 | 96 |
| 347 | 110 |
| 348 | 101 |
| 349 | 105 |
| 350 | 101 |
| 351 | 107 |
| 352 | 107 |
| 353 | 106 |
| 354 | 104 |
| 355 | 94 |
| 356 | 102 |
| 357 | 104 |
| 358 | 101 |
| 359 | 97 |
| 360 | 105 |
| 361 | 98 |
| 362 | 110 |
| 363 | 97 |
| 364 | 108 |
| 365 | 94 |

**[Table 2-7]**

| | |
|---|---|
| 366 | 95 |
| 367 | 100 |
| 368 | 108 |
| 369 | 106 |
| 370 | 97 |
| 371 | 96 |
| 372 | 95 |

### Industrial Applicability

The compound of the present invention has a superior FLAP inhibitory action, and is useful as a prophylactic or therapeutic agent for arteriosclerosis and the like.

This application is based on a patent application No. 2012-185725 filed in Japan (filing date: August 24, 2012), the contents of which are incorporated in full herein.

## Claims

1. A compound represented by the formula (I): 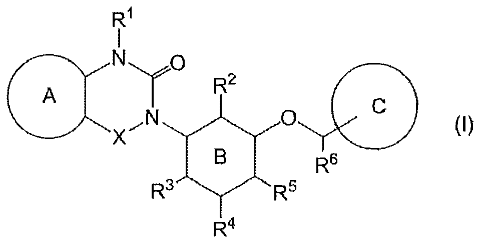 wherein
X is C(=O) or CH₂;
ring A is an optionally substituted 5-membered nitrogen-containing heterocycle, an optionally substituted 6-membered heterocycle, or an optionally substituted benzene;
ring B is a 6-membered aromatic heterocycle or benzene;
ring C is an optionally further substituted 5- or 6-membered heterocycle or a benzene further having substituent(s);
R¹ is a hydrogen atom or an optionally substituted C₁₋₆ alkyl group;
R² is a hydrogen atom or an optionally substituted C₁₋₆ alkyl group or absent;
R³ is a hydrogen atom or a substituent or absent;
R⁴ is a hydrogen atom or an optionally substituted C₁₋₆ alkyl group or absent;
R⁵ is a hydrogen atom, a halogen atom or a C₁₋₆ alkyl group or absent;
R⁶ is a hydrogen atom or a C₁₋₆ alkyl group,
or R⁵ and R⁶ optionally form a dihydropyran structure together with a carbon atom bonded thereto,
or a salt thereof.

2. The compound according to claim 1, wherein the formula (I) is the following formula (IA): 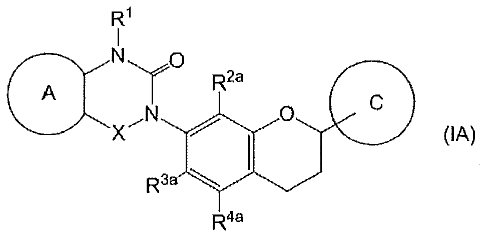 wherein
X is C(=O) or CH₂;
ring A is an optionally substituted 5-membered nitrogen-containing heterocycle, an optionally substituted 6-membered heterocycle, or an optionally substituted benzene;
ring C is an optionally further substituted 5- or 6-membered heterocycle, or benzene further having substituent(s);
R¹ is a hydrogen atom or an optionally substituted C₁₋₆ alkyl group;
R^{2a} is a hydrogen atom or an optionally substituted C₁₋₆ alkyl group;
R^{3a} is a hydrogen atom or a substituent; and
R^{4a} is a hydrogen atom or an optionally substituted C₁₋₆ alkyl group,
or a salt thereof.

3. 5-Chloro-3-((2S)-6-chloro-2-(3-fluoropyridin-2-yl)-3,4-dihydro-2H-chromen-7-yl)-8-methyl-3,4-dihydropyrido[3,4-d]pyrimidine-2(1H)-one or a salt thereof.

4. (+)-5-Chloro-8-(3,5-dimethyl-1,2-oxazol-4-yl)-3-(2-(3-fluoropyridin-2-yl)-6-methyl-3,4-dihydro-2H-chromen-7-yl)-3,4-dihydropyrido[3,4-d]pyrimidine-2(1H)-one or a salt thereof.

5. 3-((2R)-2-(3-Fluoropyridin-2-yl)-6-methyl-3,4-dihydro-2H-chromen-7-yl)-5-(trifluoromethyl)-3,4-dihydropyrido[4,3-d]pyrimidine-2(1H)-one or a salt thereof.

6. A medicament comprising the compound according to claim 1 or a salt thereof.

7. The medicament according to claim 6, which is a 5-lipoxygenase activating protein inhibitor.

8. The medicament according to claim 6, which is a prophylactic or therapeutic agent for arteriosclerosis.

9. The compound according to claim 1 or a salt thereof for use for the prophylaxis or treatment of arteriosclerosis.

10. A method of inhibiting a 5-lipoxygenase activating protein in a mammal, comprising administering an effective amount of the compound according to claim 1 or a salt thereof to the mammal.

11. A method for the prophylaxis or treatment of arteriosclerosis in a mammal, comprising administering an effective amount of the compound according to claim 1 or a salt thereof to the mammal.

12. Use of the compound according to claim 1 or a salt thereof in the production of a prophylactic or therapeutic agent for arteriosclerosis.
